(19) Europäisches Patentamt / European Patent Office / Office européen des brevets

(11) **EP 4 597 451 A2**

(12) **EUROPEAN PATENT APPLICATION**

(43) Date of publication:
06.08.2025 Bulletin 2025/32

(21) Application number: 25185029.3

(22) Date of filing: 15.10.2018

(51) International Patent Classification (IPC):
*G06V 10/82* *(2022.01)*

(52) Cooperative Patent Classification (CPC):
**G06F 18/24133; G06N 3/045; G06N 3/084; G06V 10/454; G06V 10/82; G16B 20/20; G16B 40/20;** G06N 3/044; G06N 3/048; G06N 3/082; G06N 20/20; Y02A 90/10

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 16.10.2017 US 201762573144 P
16.10.2017 US 201762573149 P
16.10.2017 US 201762573153 P
07.11.2017 US 201762582898 P

(62) Document number(s) of the earlier application(s) in accordance with Art. 76 EPC:
18796330.1 / 3 622 520

(71) Applicant: Illumina Inc.
San Diego, CA 92122 (US)

(72) Inventors:
• GAO, Hong
San Diego, CA 92122 (US)
• FARH, Kai-How
San Diego, CA 92122 (US)
• SUNDARAM, Laksshman
San Diego, CA 92122 (US)
• MCRAE, Jeremy Francis
San Diego, CA 92122 (US)

(74) Representative: Robinson, David Edward Ashdown
Marks & Clerk LLP
1 New York Street
Manchester M1 4HD (GB)

Remarks:
This application was filed on 24.06.2025 as a divisional application to the application mentioned under INID code 62.

(54) **DEEP LEARNING-BASED TECHNIQUES FOR TRAINING DEEP CONVOLUTIONAL NEURAL NETWORKS**

(57) The technology disclosed relates to a neural network-based variant pathogenicity classifier for indicating a likelihood that a variant amino acid is benign or pathogenic. The disclosure particularly relates to a computer-implemented method comprising inputting, into a neural network based variant pathogenicity classifier trained to classify a variant amino acid sequence as benign or pathogenic a reference amino acid sequence for a protein; a variant amino acid sequence for the protein comprising a variant amino acid; and a conservation profile representing conservation of amino acids in a reference protein sequence across aligned protein sequences of other species; and generating, as an output of the neural network-based variant pathogenicity classifier, a pathogenicity score indicating a likelihood that the variant amino acid is benign or pathogenic.

EP 4 597 451 A2

Classify Chimpanzee Missense SNPs with Matching Reference Codons with Humans as Benign

FIG. 46

**Description**

**APPENDIX**

The Appendix includes a bibliography of potentially relevant references listed in a paper authored by the inventors. The subject matter of the paper is covered in the US Provisionals to which this Application claims priority to/benefit of. These references can be made available by the Counsel upon request or may be accessible via Global Dossier. The paper is the first listed reference.

**PRIORITY APPLICATIONS**

This application claims priority to or the benefit of US Provisional Patent Application No. 62/573,144, titled, "Training a Deep Pathogenicity Classifier Using Large-Scale Benign Training Data," by Hong Gao, Kai-How Farh, Laksshman Sundaram and Jeremy Francis McRae, filed October 16, 2017 (Attorney Docket No. ILLM 1000-1/IP-1611-PRV); US Provisional Patent Application No. 62/573,149, titled, "Pathogenicity Classifier Based On Deep Convolutional Neural Networks (CNNS)," by Kai-How Farh, Laksshman Sundaram, Samskruthi Reddy Padigepati and Jeremy Francis McRae, filed October 16, 2017 (Attorney Docket No. ILLM 1000-2/IP-1612-PRV); US Provisional Patent Application No. 62/573,153, titled, "Deep Semi-Supervised Learning that Generates Large-Scale Pathogenic Training Data," by Hong Gao, Kai-How Farh, Laksshman Sundaram and Jeremy Francis McRae, filed October 16, 2017 (Attorney Docket No. ILLM 1000-3 /IP-1613-PRV); and US Provisional Patent Application No. 62/582,898, titled, "Pathogenicity Classification of Genomic Data Using Deep Convolutional Neural Networks (CNNs)," by Hong Gao, Kai-How Farh and Laksshman Sundaram, filed November 7, 2017 (Attorney Docket No. ILLM 1000-4/IP-1618-PRV). These provisional applications are hereby incorporated by reference for all purposes.

**INCORPORATIONS**

The following are incorporated by reference for all purposes as if fully set forth herein:
PCT Patent Application No. PCT/US20181 , titled "DEEP CONVOLUTIONAL NEURAL NETWORKS FOR VARIANT CLASSIFICATION," by Laksshman Sundaram, Kai-How Farh, Hong Gao, Samskruthi Reddy Padigepati and Jeremy Francis McRae, filed contemporaneously on October 15, 2018 (Attorney Docket No. ILLM 1000-9/IP-1612-PCT), subsequently published as PCT Publication No. WO _____.
PCT Patent Application No. PCT/US2018/_____, titled "SEMI-SUPERVISED LEARNING FOR TRAINING AN ENSEMBLE OF DEEP CONVOLUTIONAL NEURAL NETWORKS," by Laksshman Sundaram, Kai-How Farh, Hong Gao and Jeremy Francis McRae, filed contemporaneously on October 15, 2018 (Attorney Docket No. ILLM 1000-10/IP-1613-PCT) , subsequently published as PCT Publication No. WO _____.
US Nonprovisional Patent Application titled "DEEP LEARNING-BASED TECHNIQUES FOR TRAINING DEEP CONVOLUTIONAL NEURAL NETWORKS," by Hong Gao, Kai-How Farh, Laksshman Sundaram and Jeremy Francis McRae, (Attorney Docket No. ILLM 1000-5/IP-1611-US) filed contemporaneously.
US Nonprovisional Patent Application titled "DEEP CONVOLUTIONAL NEURAL NETWORKS FOR VARIANT CLASSIFICATION," by Laksshman Sundaram, Kai-How Farh, Hong Gao and Jeremy Francis McRae, (Attorney Docket No. ILLM 1000-6/IP-1612-US) filed contemporaneously.
US Nonprovisional Patent Application titled "SEMI-SUPERVISED LEARNING FOR TRAINING AN ENSEMBLE OF DEEP CONVOLUTIONAL NEURAL NETWORKS," by Laksshman Sundaram, Kai-How Farh, Hong Gao and Jeremy Francis McRae, (Attorney Docket No. ILLM 1000-7/IP-1613-US) filed contemporaneously.
Document 1 - A. V. D. Oord, S. Dieleman, H. Zen, K. Simonyan, 0. Vinyals, A. Graves, N. Kalchbrenner, A. Senior, and K. Kavukcuoglu, "WAVENET: A GENERATIVE MODEL FOR RAW AUDIO," arXiv:1609.03499, 2016;
Document 2 - S. 0. Arik, M. Chrzanowski, A. Coates, G. Diamos, A. Gibiansky, Y. Kang. X. Li, J. Miller, A. Ng, J. Raiman, S. Sengupta and M. Shocybi, "DEEP VOICE: REAL-TIME NEURAL TEXT-TO-SPEECH," arXiv:1702.07825, 2017;
Document 3 - F. Yu and V. Koltun, "MULTI-SCALE CONTEXT AGGREGATION BY DILATED CONVOLUTIONS," arXiv:1511.07122, 2016;
Document 4 - K. He, X. Zhang, S. Ren, and J. Sun, "DEEP RESIDUAL LEARNING FOR IMAGE RECOGNITION," arXiv:1512.03385, 2015;
Document 5 - R.K. Srivastava, K. Greff, and J. Schmidhuber, "HIGHWAY NETWORKS," arXiv: 1505.00387, 2015;
Document 6 - G. Huang, Z. Liu, L. van der Maaten and K. Q. Weinberger, "DENSELY CONNECTED CONVOLUTIONAL NETWORKS," arXiv:1608.06993, 2017:
Document 7 - C. Szegedy. W. Liu,Y. Jin. P. Sermanet, S. Reed, D. Anguelov, D. Erhan, V. Vanhoucke, and A.

Rabinovich, "GOING DEEPER WITH CONVOLUTIONS," arXiv: 1409.4842, 2014:

Document 8 - S. Ioffe and C. Szegedy, "BATCH NORMALIZATION: ACCELERATING DEEP NETWORK TRAINING BY REDUCING INTERNAL COVARIATE SHIFT," arXiv: 1502.03167, 2015;

Document 9 - J. M. Wolterink, T. Leiner, M. A. Viergever, and I. Isgum, "DILATED CONVOLUTIONAL NEURAL NETWORKS FOR CARDIOVASCULAR MR SEGMENTATION IN CONGENITAL HEART DISEASE," arXiv:1704.03669, 2017;

Document 10 - L. C. Piqueras, "AUTOREGRESSIVE MODEL BASED ON A DEEP CONVOLUTIONAL NEURAL NETWORK FOR AUDIO GENERATION," Tampere University of Technology, 2016;

Document 11 - J. Wu, "Introduction to Convolutional Neural Networks," Nanjing University, 2017;

Document 12 - I.J. Goodfellow, D. Warde-Farley, M. Mirza, A. Courville, and Y. Bengio, "CONVOLUTIONAL NETWORKS", Deep Learning, MIT Press, 2016; and

Document 13 - J. Gu, Z. Wang, J. Kuen, L. Ma, A. Shahroudy, B. Shuai, T. Liu, X. Wang, and G. Wang, "RECENT ADVANCES IN CONVOLUTIONAL NEURAL NETWORKS," arXiv:1512,07108, 2017.

Document 1 describes deep convolutional neural network architectures that use groups of residual blocks with convolution filters having same convolution window size, batch normalization layers, rectified linear unit (abbreviated ReLU) layers, dimensionality altering layers, atrous convolution layers with exponentially growing atrous convolution rates, skip connections, and a softmax classification layer to accept an input sequence and produce an output sequence that scores entries in the input sequence. The technology disclosed uses neural network components and parameters described in Document 1. In one implementation, the technology disclosed modifies the parameters of the neural network components described in Document 1. For instance, unlike in Document 1, the atrous convolution rate in the technology disclosed progresses non-exponentially from a lower residual block group to a higher residual block group. In another example, unlike in Document 1, the convolution window size in the technology disclosed varies between groups of residual blocks.

Document 2 describes details of the deep convolutional neural network architectures described in Document 1.

Document 3 describes atrous convolutions used by the technology disclosed. As used herein, atrous convolutions are also referred to as "dilated convolutions". Atrous/dilated convolutions allow for large receptive fields with few trainable parameters. An atrous/dilated convolution is a convolution where the kernel is applied over an area larger than its length by skipping input values with a certain step, also called atrous convolution rate or dilation factor. Atrous/dilated convolutions add spacing between the elements of a convolution filter/kernel so that neighboring input entries (e.g., nucleotides, amino acids) at larger intervals are considered when a convolution operation is performed. This enables incorporation of long-range contextual dependencies in the input. The atrous convolutions conserve partial convolution calculations for reuse as adjacent nucleotides are processed.

Document 4 describes residual blocks and residual connections used by the technology disclosed.

Document 5 describes skip connections used by the technology disclosed. As used herein, skip connections ate also referred to as "highway networks".

Document 6 describes densely connected convolutional network architectures used by the technology disclosed.

Document 7 describes dimensionality altering convolution layers and modules-based processing pipelines used by the technology disclosed. One example of a dimensionality altering convolution is a 1 x 1 convolution.

Document 8 describes batch normalization layers used by the technology disclosed.

Document 9 also describes atrous/dilated convolutions used by the technology disclosed.

Document 10 describes various architectures of deep neural networks that can be used by the technology disclosed, including convolutional neural networks, deep convolutional neural networks, and deep convolutional neural networks with atrons/dilated convolutions.

Document 11 describes details of a convolutional neural network that can be used by the technology disclosed, including algorithms for training a convolutional neural network with subsampling layers (e.g., pooling) and fully-connected layers.

Document 12 describes details of various convolution operations that can be used by the technology disclosed.

Document 13 describes various architectures of convolutional neural networks that can be used by the technology disclosed.

## INCORPORATION" BY REFERENCE OF TABLES

## SUBMIITED ELECTRONICALLY WITH APPLICATION

Following table files in ASCII text format are submitted with this application and incorporated by reference. The names, creation dates and sizes of the files are:

| | | |
|---|---|---|
| SupplementaryTable1.txt | October 2, 2018 | 13 KB |
| SupplementaryTable2.txt | October 2, 2018 | 13 KB |
| SupplementaryTable3.txt | October 2, 2018 | 11 KB |
| SupplementaryTable4.txt | October 2, 2018 | 13 KB |
| SupplementaryTable6.txt | October 2, 2018 | 12 KB |
| SupplementaryTable7.txt | October 2, 2018 | 44 KB |
| SupplementaryTable13.txt | October 2, 2018 | 119 KB |
| SupplementaryTable18.txt | October 2, 2018 | 35 KB |
| SupplementaryTable20.txt | October 2, 2018 | 1027 KB |
| SupplementaryTable20Summary.txt | October 2, 2018 | 9 KB |
| SupplementaryTable21.txt | October 2, 2018 | 24 KB |
| SupplementaryTable21.txt | October 2, 2018 | 24 KB |
| SupplementaryTable18.txt | October 4, 2018 | 35 KB |
| DataFileS1.txt | October 4, 2018 | 138 MB |
| DataFileS2.txt | October 4, 2018 | 980 MB |
| DataFileS3.txt | October 4, 2018 | 1.01 MB |
| DataFileS4.txt | October 4, 2018 | 834 KB |
| Pathogenicity_prediction_model.txt | October 4, 2018 | 8.24 KB |

Supplementary Table 1: Details of the variants from each species used in the analysis. The table includes the intermediate results in the pipeline for each of these data sources. Note this table is provided in SupplementaryTable1.txt.

Supplementary Table 2: Depletion of missense variants present in other species at common human allele frequencies. The depletion was calculated based on the missense:synonymous ratio in common variams (> 0.1%) compared to rare variants (< 0.1%), using variants that were identical-by-state between human and the other species. Note this table is provided in SupplementaryTable2.txt.

Supplementary Table 3: Depletion of missense variants present in other species at common human allele frequencies, restricted only to genes with > 50% mean nucleotide conservation between human and other mammals. The depletion was calculated based on the missense:synonymous ratio in common variants (> 0.1%) compared to rare vanants (< 0.1%), using variants that were identical-by-state between human and the other species. Note this table is provided in SupplementaryTable3.txt.

Supplementary Table 4: Depletion of missense variants present as fixed substitutions in related species pairs at common human allele frequencies. The depletion was calculated based on the missense:synonymous ratio in common variants (> 0.1%) compared to rare variants (< 0.1%), using variants that were identical-by-state between human and the related species pair. Note this table is provided in SupplementaryTable4.txt.

Supplementary Table 6: Domain specific annotation of SCN2A gene. Wilcoxon rank sum p-values indicate the divergence of PrimateAI scores in the specific domain in comparison with the entire protein. The domains, highlighted in bold, cover approximately 7% of the protein, but have the majority of ClinVar pathogenic annotations. This correlates well with the average PrimateAI scores for the domains and are the top 3 pathogenic domains according to the PrimateAI model. Note this table is provided in SupplementaryTable6.txt.

Supplementary Table 7: Raw counts used in calculating the effect of allele frequency on expected missense:synonymous ratio. Expected counts of synonymous and missense variants were calculated based on variants in intronic regions, using trinucleotide context to control for mutational rate and gene conversion. Note this table is provided in SupplementaryTables.xlsx.

Supplementary Table 13: List of protein names from the Protein DataBank (PDB) used for training the deep learning models for 3-state secondary structure and 3-state solvent accessibility prediction. The label column indicates if the proteins are used in the training/validation/testing phases of model training. Note this table is provided in SupplementaryTable13.txt.

Supplementary Table 18: List of 605 genes that were nominally significant for disease association in the DDD study, calculated from protein-truncating variation only (p<0.05). Note this table is provided in SupplementaryTable18.txt.

Supplementary Table 20: Results of testing for enrichment of de novo mutations (DNMs) per gene, for all genes with at least one observed DNM. P-values are provided, when including all DNMs, and after removing missense DNMs with PrimateAI scores < 0.803. FDR corrected P-values are similarly provided. Counts of observed protein truncating (PTV) and missense DNMs are included, from just the DDD cohort and from the full meta-analysis cohort. Similar counts of observed and expected missense DNMs are also included, firstly when including all missense DNMs, and secondly after removing all missense DNMs with a PrimatcAI score < 0.803. Note this table is provided in SupplementaryTable20.txt and SupplementaryTable20Summary.txt.

Supplementary Table 21: Results from testing enrichment of de novo mutations in genes with FDR < 0.1. Counts of observed protein truncating (PTV) de novo mutations, and counts of other protein-altering de novo mutations, once with all missense de novo mutations and once with only damaging missense mutations, are included. P-values when including all missense sites, versus P-values after excluding low scoring missense sites are provided. Note this table is provided in SupplementaryTable21.txt.

DataFileS1: List of all variants present in other species. The ClinVar Significance column contains the available non-conflicting ClinVar annotations. Note this table is provided in DataFileSt.txt.

DataFileS2: List of all fixed substitutions from related species pairs. Note this table is provided in DataFileS2.txt.

DataFilcS3; List of withheld benign test variants IBS with primates. The benign test variants are non-common human variants that are IBS with >=1 primate species. Note this table is provided in DataFileS3.txt.

DataFileS4: List of unlabeled variants IBS with primates matched to withheld benign test variants. The unlabeled variants are matched with benign test variants, for mutational rate, coverage biases and alignability with primate species. Note this table is provided in DataFileS4.txt.

Pathogenicity_prediction truxlel: Code in Python programming language that enables the technology disclosed according to one implementation. Note this code file is provided in Pathogenicity_prediction_model.txt.

## FIELD OF THE TECHNOLOGY DISCLOSED

The technology disclosed relates to artificial intelligence type computers and digital data processing systems and corresponding data processing methods and products for emulation of intelligence (i.e., knowledge based systems, reasoning systems, and knowledge acquisition systems); and including systems for reasoning with uncertainty (e.g., fuzzy logic systems), adaptive systems, machine learning systems, and artificial neural networks. In particular, the technology disclosed relates to using deep learning-based techniques for training deep convolutional neural networks.

## BACKGROUND

The subject matter discussed in this section should not be assumed to be prior art merely as a result of its mention in this section. Similarly, a problem mentioned in this section or associated with the subject matter provided as background should not be assumed to have been previously recognized in the prior art. The subject matter in this section merely represents different approaches, which in and of themselves can also correspond to implementations of the claimed technology.

### Machine Learning

In machine learning input variables are used to predict an output variable. The input variables are often called features and are denoted by $X = (X_1, X_2, ..., X_k)$, where each $X_i, i \in 1, ..., k$ is a feature. The output variable is often called the response or dependent variable and is denoted by the variable $Y,$. The relationship between $Y$ and the corresponding $X$ can be written in a general form:

$$Y = f(X) + \in$$

In the equation above, $f$ is a function of the features $(X_1, X_2, ..., X_k)$ and $\in$ is the random error term. The error term is independent of X and has a mean value of zero.

In practice, the features X are available without having Y or knowing the exact relation between X and Y. Since the error term has a mean value of zero, the goal is to estimate $f$.

$$\hat{Y} = \hat{f}(X)$$

In the equation above, $\hat{f}$ is the estimate of $\in$, which is often considered a black box, meaning that only the relation between the input and output of $\hat{f}$ is known, but the question why it works remains unanswered.

The function $\hat{f}$ is found using learning. Supervised learning and unsupervised learning are two ways used in machine learning for this task. In supervised learning, labeled data is used for training. By showing the inputs and the corresponding outputs (-labels), the function $\hat{f}$ is opiumized such that it approximates the output. In unsupervised learning, the goal is to find a hidden structure from unlabeled data. The algorithm has no measure of accuracy on the input data, which distinguishes it from supervised learning.

## Neural Networks

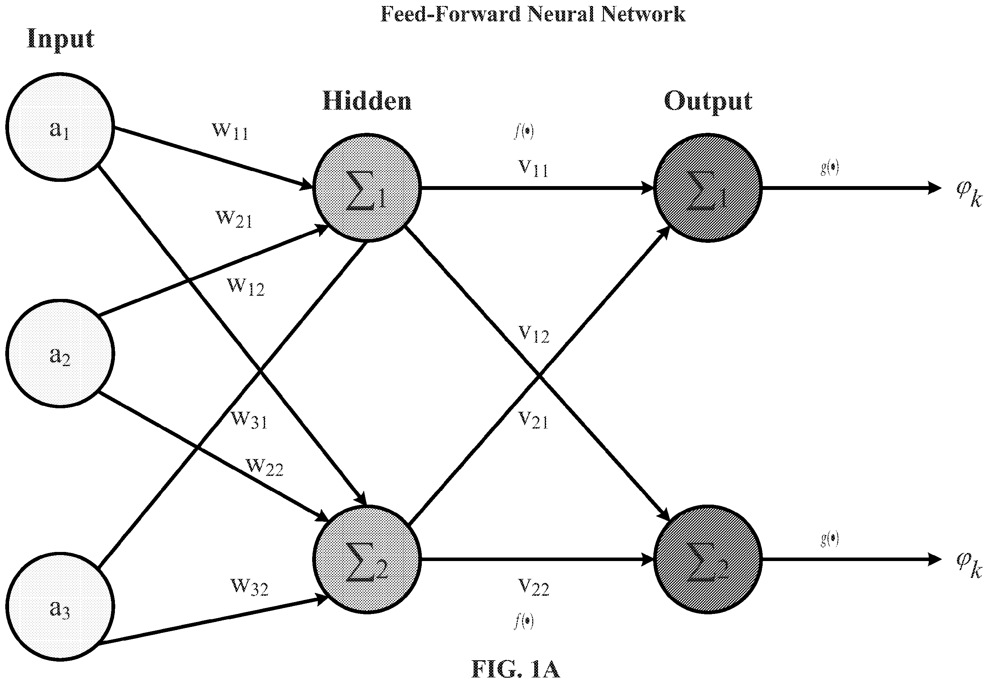

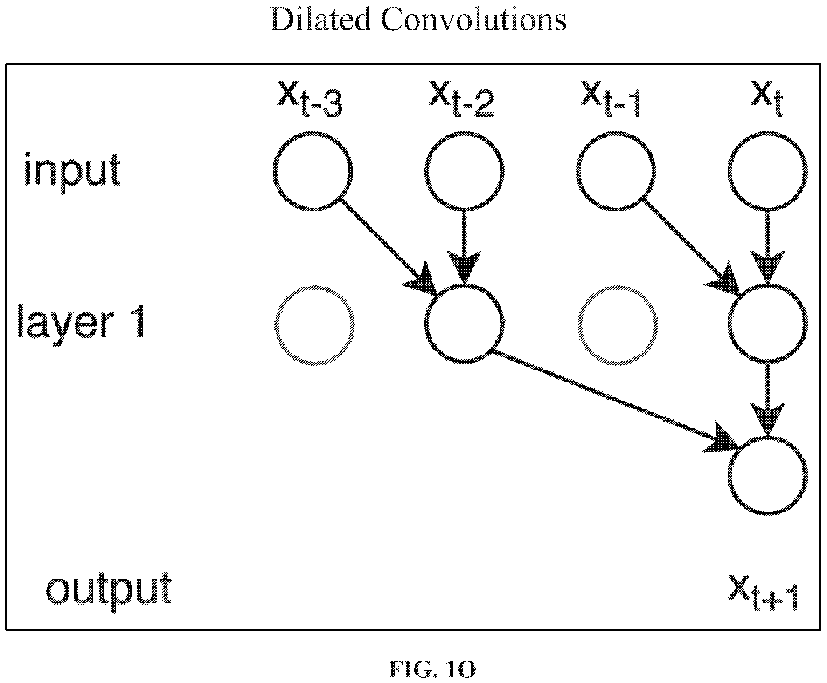

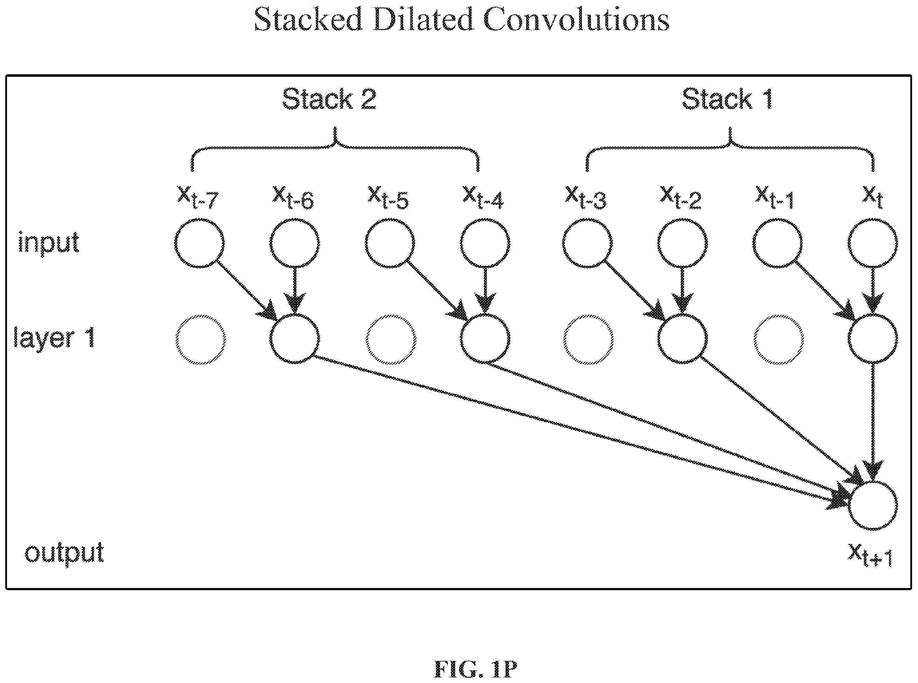

**FIG. 1A** shows one implementation of a fully connected neural network with multiple layers. A neural network is a system of interconnected arti ficial neurons (*e.g.*, $a_1$, $a_2$, $a_3$) that exchange messages between each other. The illustrated neural network has three inputs. two neurons in the hidden layer and two neurons in the output layer. The hidden layer has an activation function $f(\cdot)$ and the output layer has an activation function $g(\cdot)$. The connections have numeric weights (*e.g.*, $w_{11}$, $w_{21}$, $w_{12}$, $w_{31}$, $w_{22}$, $w_{32}$, $v_{11}$, $v_{22}$) that are tuned during the training process, so that a properly trained network responds correctly when fed an image to recognize. The input layer processes the raw input. the hidden layer processes the output from the input layer based on the weights of the connections between the input layer and the hidden layer. The output layer takes the output from the hidden layer and processes it based on the weights of the connections between the hidden layer and the output layer. The network includes multiple layers of feature-detecting neurons. Each layer has many neurons that respond to different combinations of inputs from the previous layers. These layers are constructed so that the first layer detects a set of primitive patterns in the input image data, the second layer detects patterns of patterns and the third layer detects patterns of those patterns.

A survey of application of deep learning in genomics can be found in the following publications:

- T. Ching et al., Opportunities And Obstacles for Deep Learning In Biology And Medicine, www.biorxiv.org:142760, 2017;
- Angermueller C, Pärnamaa T, Parts L, Stegle O. Deep Learning For Computational Biology. Mol Syst Biol. 2016;12:878;
- Park Y, Kellis M. 2015 Deep Learning For Regulatory Genomics. Nat. Biotechnol. 33, 825--826. (doi:10.1038/nbt.3313);
- Min, S., Lee, B. & Yoon, S. Deep Learning In Bioinformatics. Brief. Bioinfonn. bbw068 (2016);
- Leung MK, Delong A. Alipanahi B et al. Machine Learning In Genomic Medicine: A Review of Computational Problems and Data Sets 2016; and
- Libbrecht MW, Noble WS. Machine Learning Applications In Genetics and Genomics. Nature Reviews Genetics 2015;16(6):321-32.

## BRIEF DESCRIPTION OF THE DRAWINGS

In the drawings, like reference characters generally refer to like parts throughout the different views. Also, the drawings are not necessarily to scale, with an emphasis instead generally being placed upon illustrating the principles of the technology disclosed. In the following description, various implementations of the technology disclosed are described with reference to the following drawings, in which:

FIG. **1A** shows one implementation of a feed-forward neural network with multiple layers.

FIG. **1B** depicts one implementation of workings of a convolutional neural network.

FIG. **1C** depicts a block diagram of training a convolutional neural network in accordance with one implementation of the technology disclosed.

FIG. **1D** is one implementation or sub-sampling layers (average/max pooling) in accordance with one implementation of the technology disclosed.

FIG. **1E** shows one implementation of a ReLU non-linear layer in accordance with one implementation of the technology disclosed.

FIG. **1F** depicts one implementation of a two-layer convolution of the convolution layers.

FIG. **1G** depicts a residual connection that reinjects prior information downstream via feature-map addition.

FIG. **1H** depicts one implementation of residual blocks and skip-connections.

FIG. **1I** shows the batch normalization forward pass.

FIG. **1J** illustrates the batch normalization transform at test time.

FIG. **1K** shows the batch normalization backward pass.

FIG. **1L** depicts use of a batch normalization layer after and before a convolutional or densely connected layer.

FIG. **1M** shows one implementation of 1D convolution.

FIG. **1N** illustrates how global average pooling (GAP) works.

FIG. **1O** illustrates dilated convolutions.

FIG. **1P** shows one implementation of stacked dilated convolutions.

FIG. **1Q** shows an example computing environment in which the technology disclosed can be operated.

**FIG. 2** shows an example architecture of a deep residual network for pathogenicity prediction, referred to herein as "PrirnateA1".

**FIG. 3** depicts a schematic illustration of PrimateAI, the deep learning network architecture for pathogenicity classification.

**FIGs. 4A. 4B,** and **4C** are Supplementary Table 16 that show example model architecture details of the pathogenicity prediction deep learning model PrimateAI.

**FIGs. 5** and **6** illustrate the deep learning network architecture used for predicting secondary structure and solvent accessibility of proteins.

**FIGs. 7A** and **7B** are Supplementary Table 11 that show example model architecture details for the 3-state secondary structure prediction deep learning (DL) model.

**FIGs. 8A** and **8B** are Supplementary Table 12 that show example model architecture details for the 3-state solvent accessibility prediction deep learning model.

**FIG. 9** depicts one implementation of generating reference and alternative protein sequences from benign and pathogenic variants.

**FIG. 10** shows one implementation of aligning reference and alternative protein sequences.

**FIG. 11** is one implementation of generating position frequency matrices (abbreviated PFMs), also called position weight matrices (abbreviated PWMs) or position-specific scoring matrix (abbreviated PSSM).

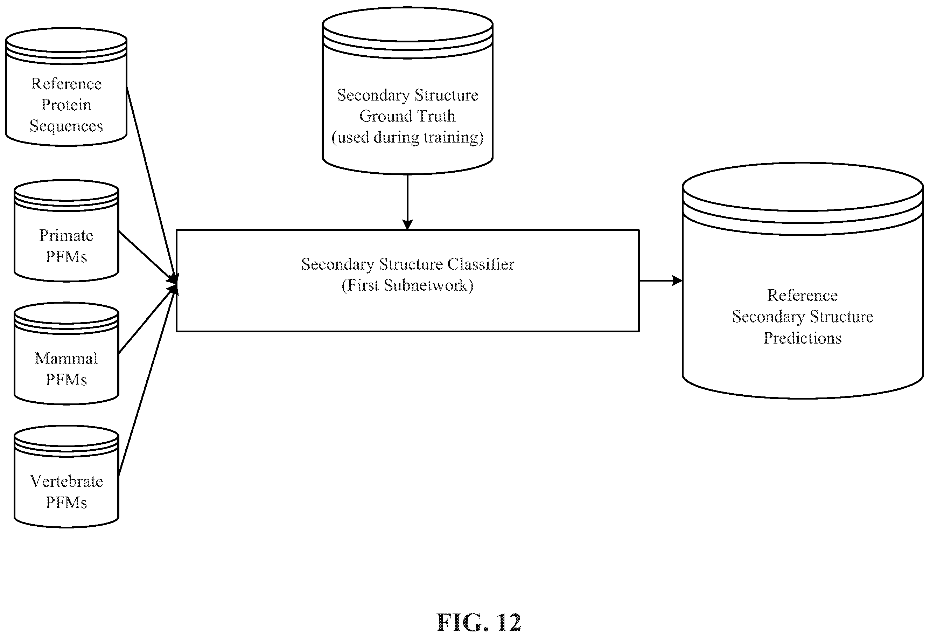

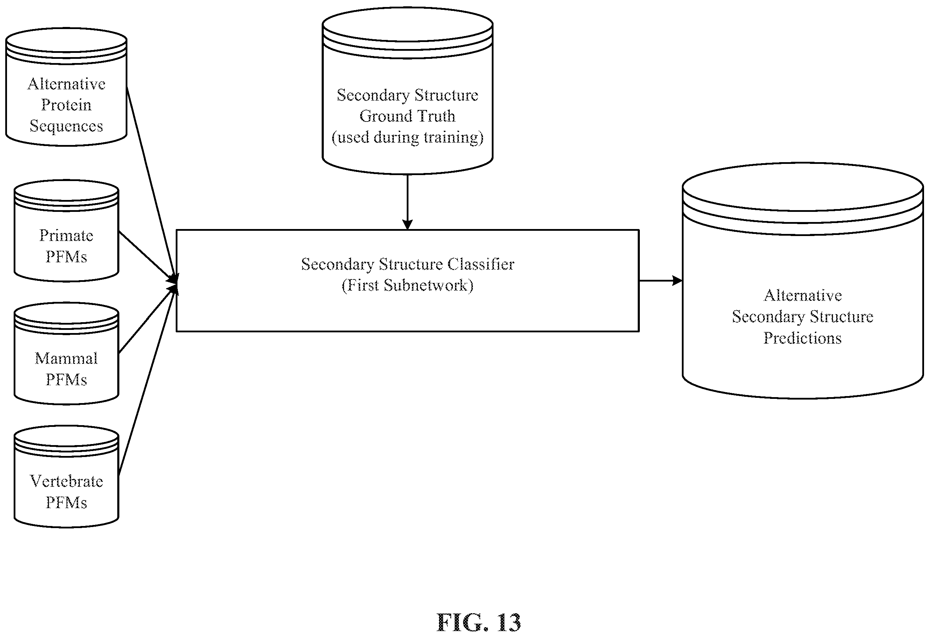

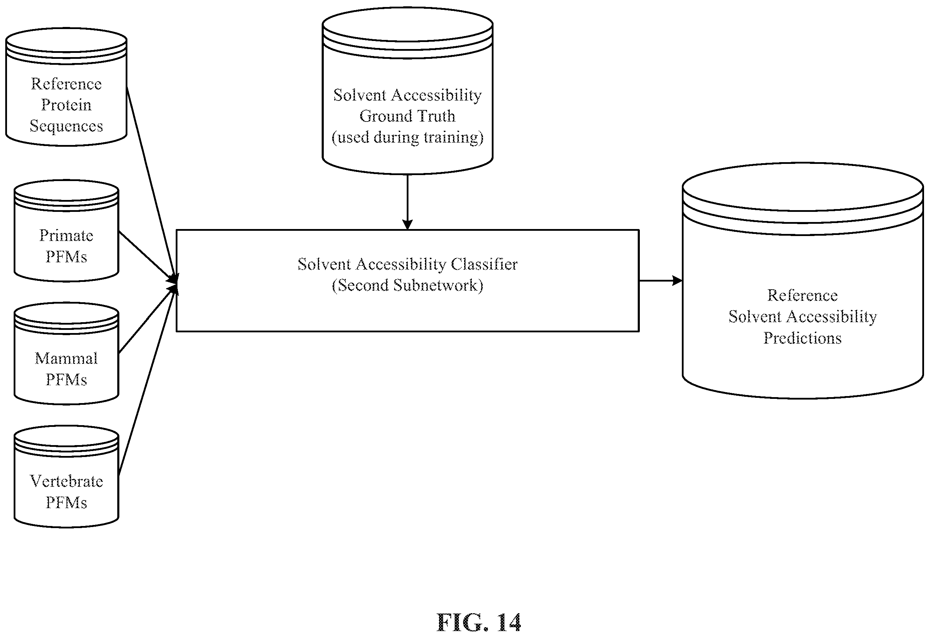

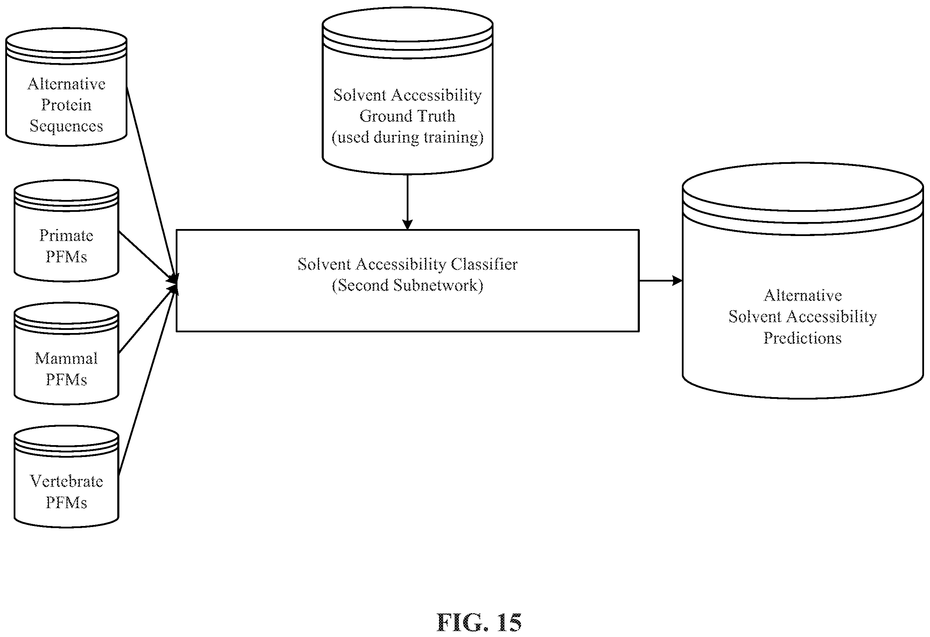

**FIGs. 12, 13, 14,** and **15** show processing of the secondary structure and solvent accessibility subnetwork.

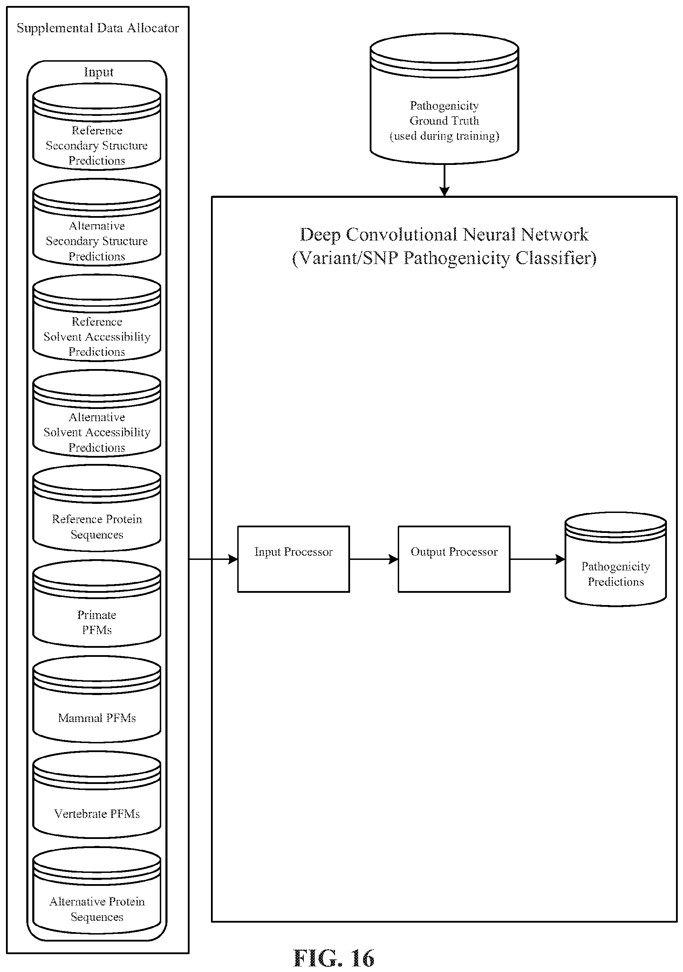

**FIG. 16** operation of a variant pathogenicity classifier. As used herein, the term variant also refers to single nucleotide polymorphisms (abbreviated SNPs) and generally to single nucleotide variants (abbreviated SNVs).

**FIG. 17** illustrates a residual block.

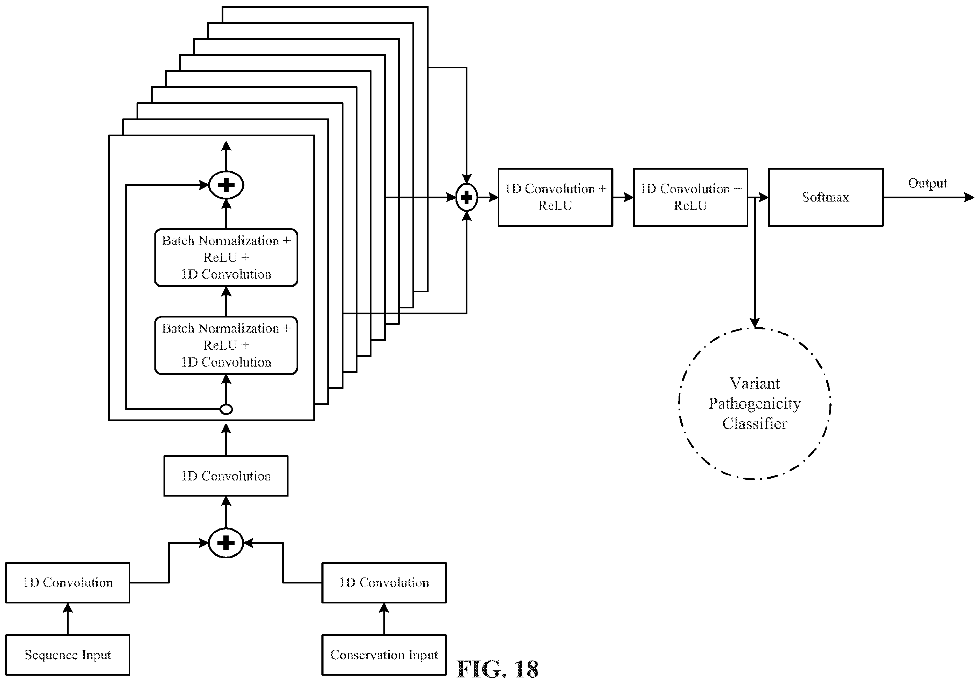

**FIG. 18** depicts a neural network architecture of the secondary structure and solvent accessibility subnetworks.

**FIG. 19** shows a neural network architecture of the variant pathogenicity classifier.

**FIG. 20** shows predicted pathogenicity score at each amino acid position in the SCN2A gene, annotated for key functional domains.

**FIG. 21D** shows a comparison of classifiers at predicting benign consequence for a test set of 10,000 common primate variants that were withheld from training.

**FIG. 21E** illustrates distributions of PrimateAI prediction scores for *de novo* missense variants occurring in Deciphering Developmental Disorders (DDD) patients compared to unaffected siblings, with corresponding Wilcoxon rank-sum P value.

**FIG. 21F** depicts comparison of classifiers at separating *de novo* missense variants in DDD cases versus controls. Wilcoxon rank-sum test P values are shown for each classifier.

**FIG. 22A** shows enrichment of *de novo* missense mutations over expectation in affected individuals from the DDD cohort within 605 associated genes that were significant for *de novo* protein truncating variation ($P<0.05$).

**FIG. 22B** depicts distributions of PrimateAI prediction scores for *de novo* missense variants occurring in DDD patients versus unaffected siblings within the 605 associated genes. with corresponding Wilcoxon rank-sum P value.

**FIG. 22C** shows comparison of various classifiers at separating *de novo* missense variants in cases versus controls within the 605 genes.

**FIG. 22D** depicts comparison of various classifiers, shown on a receiver operator characteristic curve, with area under curve (AUC) indicated for each classifier.

**FIG. 22E** illustrates classification accuracy and area under curve (AUC) for each classifier.

**FIGs. 23A, 23B, 23C,** and **23D** show impact of data used for training on classification accuracy.

**FIG. 24** illustrates correcting for the effect of sequencing coverage on the ascertainment of common primate variants.

**FIGs. 25A, 25B, 25C,** and **26** depict recognition of protein motifs by the disclosed neural networks. **FIG. 26** includes a line plot showing the effect of perturbing each position in and around the variant on the predicted deep learning score for the variant.

**FIG. 27** illustrates correlation patterns of weights mimic BLOSUM62 and Grantham score matrices.

**FIGs. 28A, 288,** and **28C** show performance evaluation of the deep learning network PrimateAI and other classifiers.

**FIGs. 29A** and **29B** illustrate distribution of the prediction scores of four classifiers.

**FIGs. 30A, 30B,** and **30C** compare the accuracy of the PrimateAI network and other classifiers at separating pathogenic and benign variants in 605 disease-associated genes.

**FIGs. 31A** and **31B** illustrate correlation between classifier performance on human expert-curated ClinVar variants and performance on empirical datasets.

**FIG. 32** is Supplementary Table 14 that shows performance of the 3-state secondary structure and 3-state solvent accessibility prediction models on annotated samples from the Protein DataBank.

**FIG. 33** is Supplementary Table 15 that shows performance comparison of deep learning network using annotated secondary structure labels of human proteins from DSSP database.

**FIG. 34** is Supplementary Table 17 that shows the accuracy values on the 10,000 withheld primate variants and the p-values for *de novo* variants in DDD cases vs controls for each of the 20 classifiers we evaluated.

**FIG. 35** is Supplementary Table 19 that shows comparison of the performance of different classifiers on *de novo* variants in the DDD case vs control dataset, restricted to 605 disease-associated genes.

**FIG. 36** shows a computing environment of the disclosed semi-supervised learner.

**FIGs. 37, 38, 39, 40,** and **41** show various cycles of the disclosed semi-supervised learning.

**FIG. 42** is an illustration of the iterative balanced sampling process.

**FIG. 43** illustrates one implementation of a computing environment used to generate the benign dataset.

**FIG. 44** depicts one implementation of generating benign human missense SNPs.

**FIG. 45** shows one implementation of human orthologous missense SNPs. A missense SNP in a non-human species that has matching reference and alternative codons with humans.

**FIG. 46** depicts one implementation of classifying, as benign, SNPs of a non-human primate species (e.g., Chimpanzee) with matching reference codons with humans.

**FIG. 47** depicts one implementation of calculating enrichment scores and comparing them.

**FIG. 48** depicts one implementation of the benign SNP dataset.

**FIGs. 49A, 49B, 49C, 49D.** and **49E** depict missense/synonymous ratios across the human allele frequency spectrum.

**FIGs. 50A, 50B, 50C,** and **50D** show purifying selection on missense variants identical-by-state with other species.

**FIG. 51** shows expected missense:synonymous ratios across the human allele frequency spectrum in the absence of purifying selection.

**FIGs. 52A, 52B, 52C,** and **52D** depict missense:synonymous ratios for CpG and non-CpG variants.

**FIGs. 53, 54,** and **55** illustrate missense:synonymous ratios of human variants identical by state with six primates.

**FIG. 56** is a simulation showing saturation of new common missense variants discovered by increasing the size of the human cohorts surveyed.

**FIG. 57** shows accuracy of PrimateAI across different conservation profiles in the genome.

**FIG. 58** is Supplementary Table 5 that shows contributions to the labeled benign training dataset from common human variants and variants present in non-human primates.

**FIG. 59** is Supplementary Table 8 that shows effect of allele frequency on expected missense:synonymous ratio.

**FIG. 60** is Supplementary Table 9 that shows ClinVar analysis.

**FIG. 61** is Supplementary Table 10 that shows the number of missense variants from other species found in ClinVar, according to one implementation.

**FIG. 62** is Table I that shows one implementation of discovery of 14 additional candidate genes in intellectual disability.

**FIG. 63** is Table 2 that shows one implementation of the mean difference in Grantham score between pathogenic and benign variants in ClinVar.

**FIG. 64** shows one implementation of per-gene enrichment analysis.

**FIG. 65** shows one implementation of genome-wide enrichment analysis.

**FIG. 66** is a simplified block diagram of a computer system that can be used to implement the technology disclosed.

## DETAILED DESCRIPTION

The following discussion is presented to enable any person skilled in the art to make and use the technology disclosed, and is provided in the context of a particular application and its requirements. Various modifications to the disclosed implementations will be readily apparent to those skilled in the art, and the general principles defined herein may be applied to other implementations and applications without departing from the spirit and scope of the technology disclosed. Thus, the technology disclosed is not intended to be limited to the implementations shown, but is to be accorded the widest scope consistent with the principles and features disclosed herein.

### Introduction

### Convolutional Neural Networks

A convolutional neural network is a special type of neural network. The fundamental difference between a densely connected layer and a convolution layer is this. Dense layers learn global patterns in their input feature space, whereas convolution layers learn local patters: in the case of images, patterns found in small 2D windows of the inputs. This key characteristic gives convolutional neural networks two interesting properties: (1) the patterns they learn are translation invariant and (2) they can learn spatial hierarchies of patterns.

Regarding the first, after learning a certain pattern in the lower-right corner of a picture, a convolution layer can recognize it anywhere: for example, in the upper-left corner. A densely connected network would have to learn the pattern anew if it appeared at a new location. This makes convolutional neural networks data efficient because they need fewer training samples to learn representations they have generalization power.

Regarding the second, a first convolution layer can learn small local patterns such as edges, a second convolution layer will learn larger patterns made of the features of the first layers, and so on. This allows convolutional neural networks to efficiently learn increasingly complex and abstract visual concepts.

A convolutional neural network learns highly non-linear mappings by interconnecting layers of artificial neurons arranged in many different layers with activation functions that make the layers dependent. It includes one or more convolutional layers, interspersed with one or more sub-sampling layers and non-linear layers, which are typically followed by one or more fully connected layers. Each element of the convolutional neural network receives inputs from a set of features in the previous layer. The convolutional neural network learns concurrently because the neurons in the same feature map have identical weights. These local shared weights reduce the complexity of the network such that when multi-dimensional input data enters the network, the convolutional neural network avoids the complexity of data reconstruction in feature extraction and regression or classification process.

Convolutions operate over 3D tensors, called feature maps, with two spatial axes (height and width) as well as a depth axis (also called the channels axis). For an RGB image, the dimension of the depth axis is 3, because the image has three color channels; red, green, and blue. For a black-and-white picture, the depth is 1 (levels of gray). The convolution operation extracts patches from its input feature map and applies the same transformation on all of these patches, producing an output feature map. This output feature map is still a 3D tensor: it has a width and a height. Its depth can be arbitrary. because the output depth is a parameter of the layer, and the different channels in that depth axis no longer stand for specific colors as in RGB input; rather, they stand for filters. Filters encode specific aspects of the input data: at a height level, a single filter could encode the concept "presence of a face in the input," for instance.

For example, the first convolution layer takes a feature map of size (28, 28, 1) and outputs a feature map of size (26, 26, 32): it computes 32 filters over its input. Each of these 32 output channels contains a 26 x 26 grid of values, which is a response map of the filter over the input, indicating the response of that filter pattern at different locations in the input. That is what the term feature map means: every dimension in the depth axis is a feature (or filter), and the 2D tensor output [:, :, n] is the 2D spatial map of the response of this filter over the input.

Convolutions are defined by two key parameters: (1) size of the patches extracted from the inputs-these are typically 1 x 1, 3 x 3 or 5 x 5 and (2) depth of the output feature map - the number of filters computed by the convolution. Often these start with a depth of 32, continue to a depth of 64, and terminate with a depth of 128 or 256.

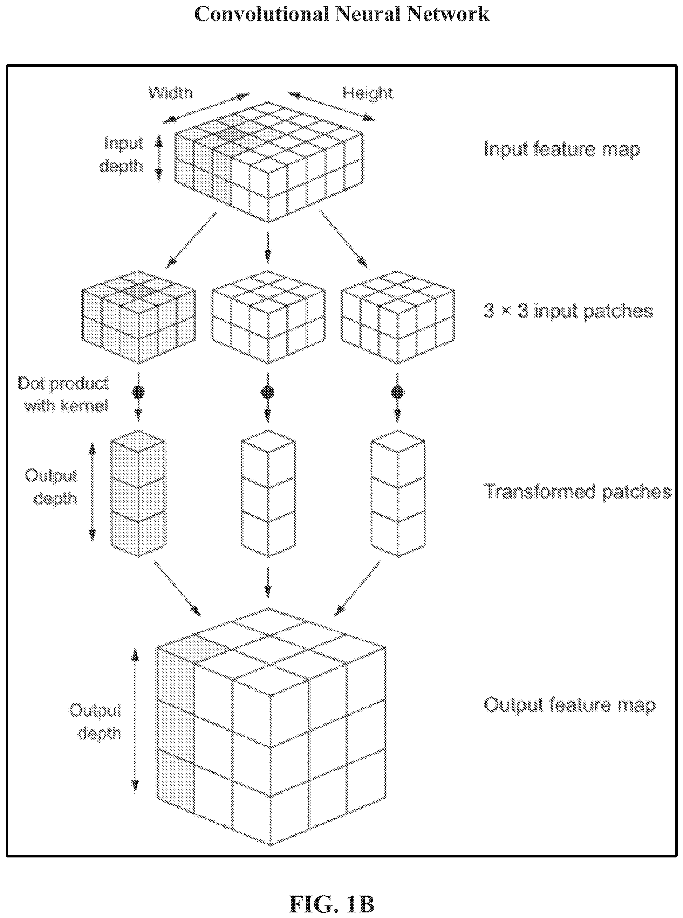

A convolution works by sliding these windows of size 3 x 3 or 5 x 5 over the 3D input feature map, stopping at every location, and extracting the 3D patch of surrounding features (shape (window_height, window_width, input_depth)). Each such 3D patch is ten transformed (via a tensor product with the same learned weight matrix, called the convolution kernel) into a 1D vector of shape (output_depth.). All of these vectors are then spatially reassembled into a 3D output map of shape (height, width, output_depth). Every spatial location in the output feature map corresponds to the same location in the input feature map (for example, the lower-right corner of the output contains information about the lower-right corner of the input). For instance, with 3 x 3 windows, the vector output [i, j, :] comes from the 3D patch input [i-I: i+1, j-1:J+1, :]. The full process is detailed in **FIG. 1B**.

**The** convolutional neural network comprises convolution layers which perform the convolution operation between the input values and convolution filters (matrix of weights) that are learned over many gradient update iterations during the traning, Let ($m$, $n$) be the filter size and $W$ be the matrix of weights, then a convolution layer performs a convolution of the $W$ with the input $X$ by calculating the dot product $W \cdot x + b$, where $x$ is an instance of $X$ and $b$ is the bias. **The** step size by which the convolution filters slide across the input is called the stride, and the filter area ($m \times n$) is called the receptive field. A same convolution filter is applied across different positions of the input, which reduces the number of weights learned. It also allows location invariant learning, i.e., if an important pattern exists in the input, the convolution filters learn it no matter where it is in the sequence.

### Training a Convolutional Neural Network

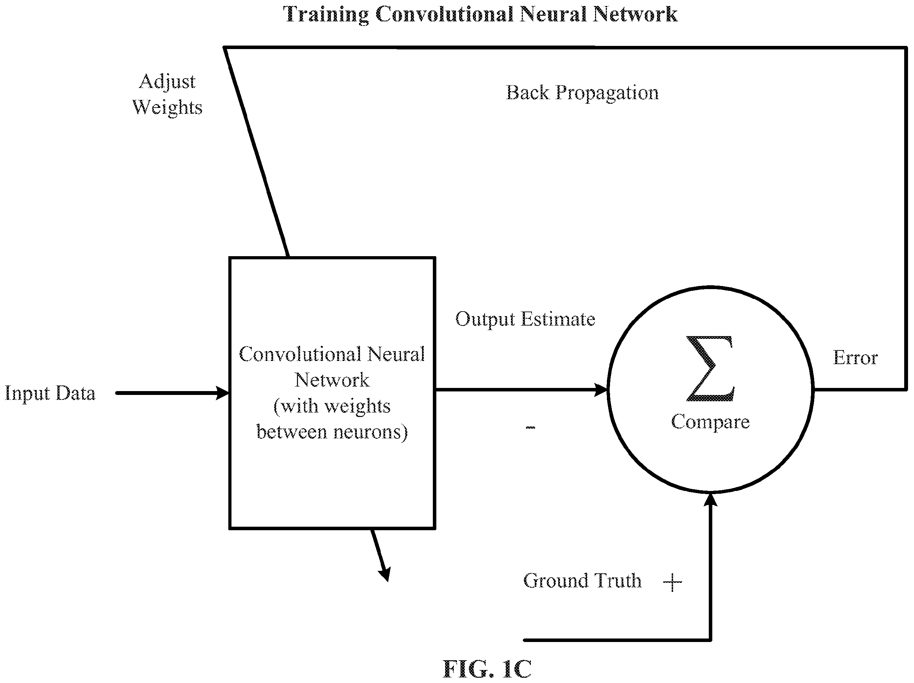

**FIG. 1C** depicts a block diagram of training a convolutional neural network in accordance with one implementation of the technology disclosed. The convolutional neural network is adjusted or trained so that the input data leads to a specific output estimate. The convolutional neural network is adjusted using back propagation based on a comparison of the output estimate and the ground truth until the output estimate progressively matches or approaches the ground truth.

The convolutional neural network is trained by adjusting the weights between the neurons based on the difference between the ground truth and the actual output. This is mathematically described as:

$$\Delta w_i = x_i \delta$$

$$where \: \delta = (ground \: truth) - (actual \: output)$$

In one implementation, the training rule is defined as:

$$W_{nm} \leftarrow W_{nm} + \alpha(t_m - \varphi_m)a_n$$

In the equation above: the arrow indicates an update of the value; $t_m$ is the target value of neuron $m$ ; $\varphi_m$ is the computed current output of neuron $m$ ; $a_n$ is input $n$; and $\alpha$ is the learning rate.

The intermediary step in the training includes generating a feature vector from the input data using the convolution layers, The gradient with respect to the weights in each layer, starting at the output, is calculated. This is referred to as the backward pass, or going backwards. The weights in the network are updated using a combination of the negative gradient and previous weights.

In one implementation, the convolutional neural network uses a stochastic gradient update algorithm (such as ADAM) that performs backward propagation of errors by means of gradient descent. One example of a sigmoid function based back propagation algorithm is described below:

$$\varphi = f(h) = \frac{1}{1 + e^{-h}}$$

In the sigmoid function above, $h$ is the weighted sum computed by a neuron. The sigmoid function has the following derivative:

$$\frac{\partial \varphi}{\partial h} = \varphi(1 - \varphi)$$

The algorithm includes computing the activation of all neurons in the network, yielding an output for the forward pass. The activation of neuron $m$ in the hidden layers is described as:

$$\varphi_m = \frac{1}{1 + e^{-h_m}}$$

$$h_m = \sum_{n=1}^{N} a_n w_{nm}$$

This is done for all the hidden layers to get the activation described as:

$$\varphi_k = \frac{1}{1 + e^{h_k}}$$

$$h_k = \sum_{m=1}^{M} \varphi_m v_{mk}$$

**Then,** the error and the correct weights are calculated per layer. The error at the output is computed as:

$$\delta_{ok} = (t_k - \varphi_k)\varphi_k(1 - \varphi_k)$$

The error in the hidden layers is calculated as:

$$\delta_{hm} = \varphi_m(1 - \varphi_m)\sum_{k=1}^{K} v_{mk}\delta_{ok}$$

The weights of the output layer are updated as:

$$V_{mk} \leftarrow V_{mk} + \alpha\delta_{ok}\varphi_m$$

The weights of the hidden layers are **updated** using the learning rate $\alpha$ as:

$$V_{nm} \leftarrow W_{nm} + \alpha\delta_{hm}a_n$$

In one implementation, the convolutional neural network uses a gradient descent optimization to compute the error across all the layers. In such an optimization, for an input feature vector $x$ and the predicted output $\hat{y}$. the loss function is defined as $l$ for the cost of predicting $\hat{y}$ when the target is $y$, i.e. $l(\hat{y}, y)$. The predicted output $\hat{y}$ is transformed from the input feature vector $x$ using function $f$. Function $f$ is parameterized by the weights of convolutional neural network, i.e. $\hat{y} = f_w(x)$. **The** loss function is described as $l(\hat{y}, y) = l(f_w(x), y)$. or $Q(z, w) = l(f_y(x), y)$ where $z$ is an input and output data pair $(x, y)$. **The** gradient descent optimization is performed by updating the weights according to:

$$V_{t+1} = \mu V_t - \alpha \frac{1}{n}\sum_{i=1}^{N} \nabla_{w_t} Q(z_t, w_t)$$

$$W_{t+1} = W_t + V_{t+1}$$

In the equations above, $\alpha$ is the learning rate. Also, the loss is computed as the average over a set of $n$ data pairs. **The** computation is terminated when the learning rate $\alpha$ is small enough upon linear convergence. In other implementations, the gradient is calculated using only selected data pairs fed to a Nesterov's accelerated gradient and an adaptive gradient to inject computation efficiency.

In one implementation, the convolutional neural network uses a stochastic gradient descent (SGD) to calculate the cost function, A SGD approximates the gradient with respect to the weights in the loss function by computing it from only one, randomized, data pair. $Z_1$, described as:

$$V_{t+1} = \mu V - \alpha \nabla_w Q(z_t, w_t)$$

$$W_{t+1} = W_t + V_{t+1}$$

In the equations above: $\alpha$ is the learning rate: $\mu$ is the momentum; and $t$ is the current weight state before updating. The convergence speed of SGD is approximately $O(1/t)$ when the learning rate $\alpha$ are reduced both fast and slow enough. In other implementations, the convolutional neural network uses different loss functions such as Euclidean loss and softmax loss. In a further implementation, an Adam stochastic optimizer is used by the convolutional neural network.

## Convolution Layers

The convolution layers of the convolutional neural actwork serve as feature extractors. Convolution layers act as adaptive feature extractors capable of learning and decomposing the input dura into hierarchical features. In one implementation, the convolution layers take two images as input and produce a third image as output. In such an implementation, convolution operates on two images in two-dirnension (2D), with one image being the input image and the other image, called the "kernel", applied as a filter on the input image, producing an output image. Thus, for an input vector $f$ of length $n$ and a kernel g of length $m$, the convolution $f * g$ of $f$ and g is defined as:

$$(f * g)(i) = \sum_{j=1}^{m} g(j) \cdot f(i - j + m/2)$$

The convolution operation includes sliding the kernel over the input image. For each position of the kernel, the overlapping values of the kernel and the input image are multiplied and the results are added. The sum of products is the value of the output image at the point in the input image where the kernel is centered. The resulting different outputs from many kernels are called feature maps.

Once the convolutional layers are trained, they are applied to perform recognition tasks on new inference data.

Since the convolutional layers learn from the training data, they avoid explicit feature extraction and implicitly learn from the training data. Convolution layers use convolution filter kernel weights, which are determined and updated as part of the training process. The convolution layers extract different features of the input, which are combined at higher layers. The convolutional neural network uses a various number of convolution layers, each with different convolving parameters such as kernel size, strides, padding, number of feature maps and weights.

### Sub-Sampling Layers

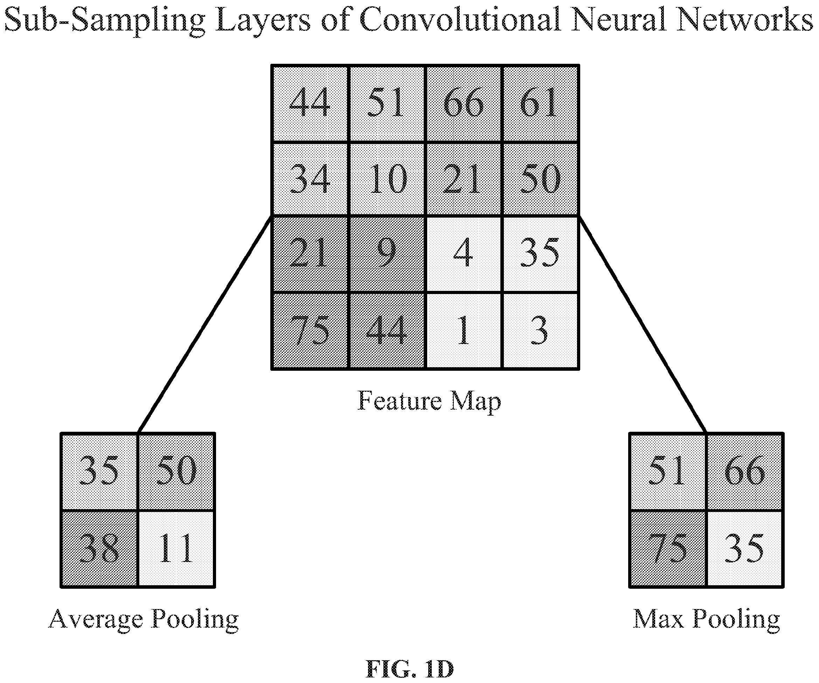

**FIG. 1D** is one implementation of sub-sampling layers in accordance with one implementation of the technology disclosed. Sub-sampling layers reduce the resolution of the features extracted by the convolution layers to make the extracted features or feature maps- robust against noise and distortion. In one implementation, sub-sampling layers employ two types of pooling operations, average pooling and max pooling. The pooling operations divide the input into non-overlapping two-dimensional spaces. For average pooling, the average of the four values in the region is calculated. For max pooling, the maximum value of the four values is selected.

In one implementation, the sub-sampling layers include pooling operations on a set of neurons in the previous layer by mapping its output to only one of the inputs in max pooling and by mapping its output to the average of the input in average pooling. In max pooling, the output of the pooling neuron is the maximum value that resides within the input, as described by:

$$\varphi_o = \max(\varphi_1, \varphi_2, \dots, \varphi_N)$$

In the equation above. $N$ is the total number of elements within a neuron act.

In average pooling, the output of the pooling neuron is the average value of the input values that reside with the input neuron set, as described by:

$$\varphi_o = \frac{1}{N} \sum_{n=1}^{N} \varphi_n$$

In the equation above, $N$ is the total number of elements within input neuron set.

In **FIG. 1D**. the input is of size $4 \times 4$. For $2 \times 2$ sub-sampling a $4 \times 4$ image is divided into four non-overlapping matrices of size $2 \times 2$. For average pooling, the average of the four values is the whole-integer output. for max pooling, the maximum value of the four values in the $2 \times 2$ matrix is the whole-integer output.

### Non-Linear Layers

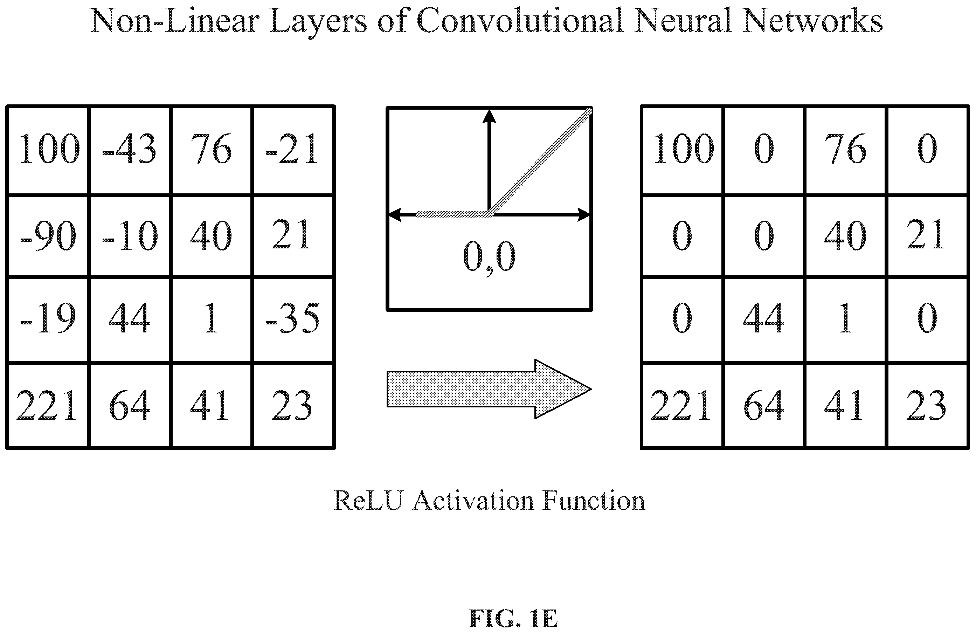

**FIG. 1E** shows one implementation of non-linear layers in accordance with one implementation of the technology disclosed. Non-linear layers use different non-linear trigger functions to signal distinct identification of likely features on each hidden layer. Non-linear layers use a variety of specific functions to implement the non-linear triggering, including the rectified linear units (ReLUs), hyperbolic tangent, absolute of hyperbolic tangent, sigmoid and continuous trigger (non-linear) functions. In one implementation, a ReLU activation implements the function y = $max(x, 0)$ and keeps the input and output sizes of a layer the same. The advantage of using ReLU is that the convolutional neural network is trained many times faster. ReLU is a non-continuous, non-saturating activation function that is linear with respect to the input if the input values are larger than zero and zero otherwise. Mathematically, a ReLU activation function is described as:

$$\varphi(h) = \max(h, 0)$$

$$\varphi(h) = \begin{cases} h & if \ h > 0 \\ 0 & if \ h \leq 0 \end{cases}$$

In other implementations, the convolutional neural network uses a power unit activation function, which is a continuous, non-saturatmg function described by:

$$\varphi(h) = (a + bh)^C$$

In the equation above, *a, b* and *c* are parameters controlling the shift, scale and power respectively. The power activation function is able to yield *x* and *y*-antisymmetric activation if *c* is odd and y-symmetric activation if *c* is even. In some implementations, the unit yields a non-rectified linear activation.

In yet other implementations, the convolutional neural network uses a sigmoid unit activation function. which is a continuous, saturating function described by the following logistic function:

$$\varphi(h) = \frac{1}{1 + e^{-\beta h}}$$

In the equation above, $\beta = 1$. The sigmoid unit activation function does not yield negative activation and is only antisymmetric with respect to the y-axis.

## Convolution Examples

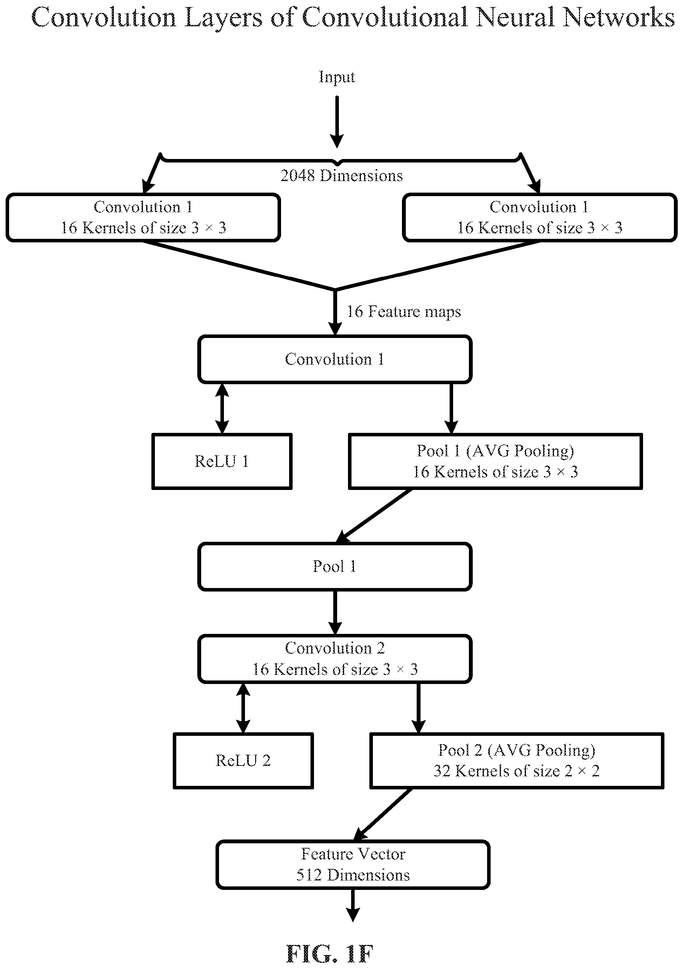

**FIG. 1F** depicts one implementation of a two-layer convolution of the convolution layers. In **FIG. 1F**, an input of size 2048 dimensions is convolved. At convolution I. the input is convolved by a convolutional layer comprising of two channels of sixteen kernels of size $3 \times 3$. The resulting sixteen feature maps are then rectified by means of the ReLU activation function at ReLU1 and then pooled in Pool 1 by means of average pooling using a sixteen channel pooling layer with kernels of size $3 \times 3$. At convolution 2, the output of Pool 1 is then convolved by another convolutional layer comprising of sixteen channels of thirty kernels with a size of $3 \times 3$. This is followed by yet another ReLU2 and average pooling in Pool 2 with u kernel size of $2 \times 2$. **The** convolution layers use varying number of strides and padding, for example, zero, one, two and three. The resulting feature vector is five hundred and twelve (512) dimensions, according to one implementation.

In other implementations, the convolutional neural network uses different numbers of convolution layers, sub-sampling layers, non-linear layers and fully connected layers. In one implementation, the convolutional neural network is a shallow network with fewer layers and more neurons per layer, for example, one, two or three fully connected layers with hundred (100) to two hundred (200) neurons per layer. In another implementation, the convolutional neural network is a deep network with more layers and fewer neurons per layer, for example, five (5), six (6) or eight (8) fully connected layers with thirty (30) to fifty (50) neurons per layer.

## Forward Pass

The output of a neuron of row *x*, column y in the $l^{th}$ convolution layer and $k^{th}$ feature map for/number of convolution cores in a feature map is determined by the following equation:

$$O_{x,y}^{(l,k)} = \tanh\left(\sum_{t=0}^{f-1} \sum_{r=0}^{k_h} \sum_{c=0}^{k_w} W_{(r,c)}^{(k,t)} O_{(x+r,x+c)}^{(l-1,t)} + Bias^{(l,k)}\right)$$

**The** output of a neuron of row *x*, column *y* in the $l^{th}$ sub-sample layer and $k^{th}$ feature map is determined by the following equation:

$$O_{x,y}^{(l,k)} = \tanh\left(W^{(k)} \sum_{r=0}^{S_h} \sum_{c=0}^{S_w} O_{(x \times S_h + r, y \times S_w + c)}^{(l-1,k)} + Bias^{(l,k)}\right)$$

The output of an $i^{th}$ neuron of the $l^{th}$ output layer is determined by the following equation:

$$O_{(l,i)} = \tanh\left(\sum_{j=0}^{H} O_{(l-1,j)} W_{(i,j)}^{l} + Bias^{(l,i)}\right)$$

## Backpropagation

The output deviation of a $k^{th}$ neuron in the output layer **is** determined **by the** following equation:

$$d(O_k^o) = y_k - t_k$$

The input deviation of a $k^{th}$ neuron in the output layer is determined by the following equation:

$$d(I_k^o) = (y_k - t_k)\varphi'(v_k) = \varphi'(v_k)d(O_k^o)$$

The weight and bias variation of a $k^{th}$ neuron in the output layer is determined by the following equation:

$$\Delta W_{k,x}^o = d(I_k^o)y_{k,x}$$

$$\Delta Bias_k^o = d(I_k^o)$$

The output bias of a $k^{th}$ neuron in the hidden layer is determined by the following equation:

$$d(O_k^H) = \sum_{i=0}^{i<84} d(I_i^o)W_{i,k}$$

The input bias of a $k^{th}$ neuron in the hidden layer is determined by the following equation:

$$d(I_k^H) = \varphi'(v_k)d(O_k^H)$$

The weight and bias variation in row $x$, column $y$ in a $m^{th}$ feature map of a prior layer receiving input from $k$ neurons in the hidden layer is determined by the following equation:

$$\Delta W_{m,x,y}^{H,k} = d(I_k^H)y_{x,y}^m$$

$$\Delta Bias_k^H = d(I_k^H)$$

The output bias of row x, column y in a $m^{th}$ feature map of sub-sample layer $S$ is determined by the following equation:

$$d(O_{x,y}^{S,m}) = \sum_{k}^{170} d(I_{m,x,y}^H)W_{m,x,y}^{H,k}$$

The input bias of raw x, column $y$ in a $m^{th}$ feature map of sub-sample layer $S$ is determined by the following equation:

$$d(I_{x,y}^{S,m}) = \varphi'(v_k)d(O_{x,y}^{S,m})$$

The weight and bias variation in row $x$, column $y$ in a $m^{th}$ feature map of sub-sample layer $S$ and convolution layer C is determined by the following equation:

$$\Delta W^{S,m} = \sum_{x=0}^{fh} \sum_{y=0}^{fw} d(I_{[x/2],[y/2]}^{S,m})O_{x,y}^{C,m}$$

$$\Delta Bias^{S,m} = \sum_{x=0}^{fh} \sum_{y=0}^{fw} d(O_{x,y}^{S,m})$$

The output bias of row x, column $y$ in a $k^{th}$ feature map of convolution layer C is determined by the following equation:

$$d(O_{x,y}^{C,k}) = d(I_{[x/2],[y/2]}^{S,k})W^{r^k}$$

The input bias of row $x$, column $y$ in a $k^{th}$ feature map of convolution layer C is determined by the following equation:

$$d(I_{x,y}^{C,k}) = \varphi'(v_k)d(O_{x,y}^{C,k})$$

The weight and bias variation in row $r$. column $c$ in an $m^{th}$ convolution core of a $k^{th}$ feature map of $l^{th}$ convolution layer $C$:

$$\Delta W_{r,c}^{k,m} = \sum_{x=0}^{fh} \sum_{y=0}^{fw} d(I_{x,y}^{C,k})O_{x+r,y+c}^{l-1,m}$$

$$\Delta Bias^{C,k}) = \sum_{x=0}^{fh} \sum_{y=0}^{fw} d(I_{x,y}^{C,k})$$

## Residual Connections

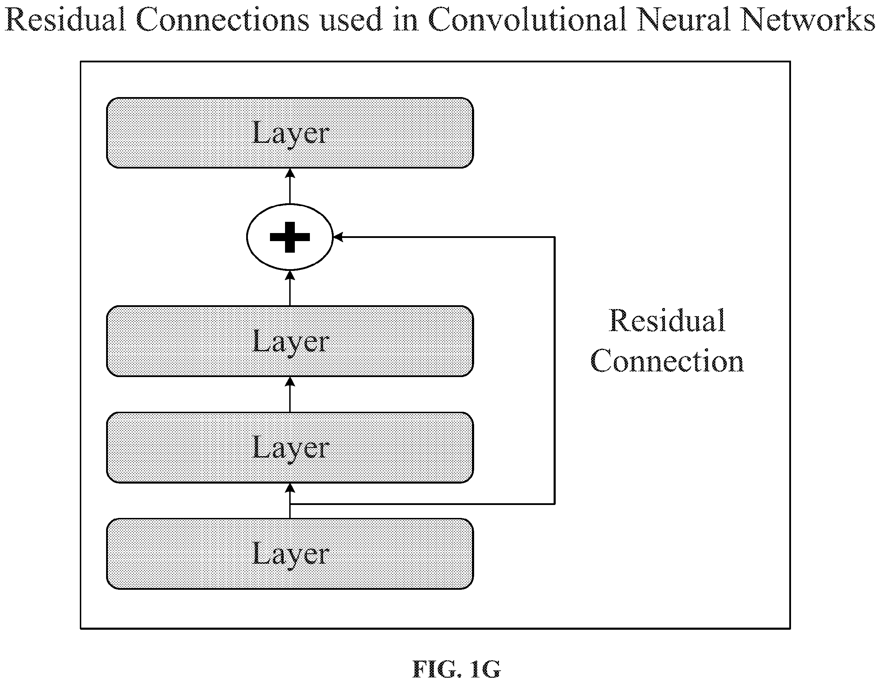

**FIG. 1G** depicts a residual connection that reinjects prior mformation downstream via feature-map addition. A residual connection comprises reinjecting previous representations into the downstream flow of data by adding a past output tensor to a later output tensor, which helps prevent information loss along the data-processing flow. Residual connections tackle two common problems that plague any large-scale deep-learning model: vanishing gradients and representational bottlenecks. In general, adding residual connections to any model that has more than 10 layers is likely to be beneficial. As discussed above, a residual connection comprises making the output of an earlier layer available as input to a later layer, effectively creating a shortcut in a sequential network. Rather than being concatenated to the later activation, the earlier output is summed with the later activation, which assumes that both activations are the same size. If they are of different sizes, a linear transformation to reshape the earlier activation into the target shape can be used. Additional information about residual connections can be found in K. He, X. Zhang. S. Ren, and J. Sun, "DEEP RESIDUAL LEARNING FOR IMAGE RECOGNITION," arXiv:1512.03385, 2015, which is hereby incorporated by reference for all purposes as if fully set forth herein.

## Residual Learning and Skip-Connections

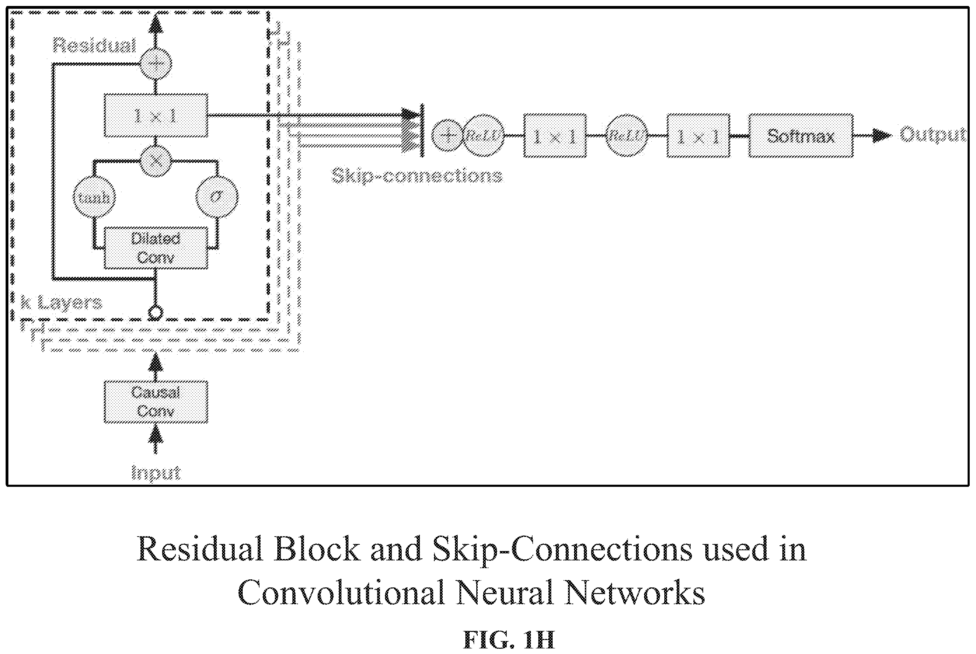

**FIG. 1H** depicts one implementation of residual blocks and skip-connections. The main idea of residual learning is that the residual mapping is much easier to be learned than the original mapping. Residual network stacks a number of residual units to alleviate the degradation of training accuracy. Residual blocks make use of special additive skip connections to combat vanishing gradients in deep neural networks. At the beginning of a residual block, the data flow is separated into two streams: the first carries the unchanged input of the block, while the second applies weights and non-linearitics. At the end of the block, the two streams are merged using an element-wise sum. The main advantage of such constructs is to allow the gradient to flow through the network more easily. Additional information about residual blocks and skip-connections can be found in A. V. D. Oord, S. Dieleman, H. Zen, K. Simonyan, O. Vinyals, A. Graves, N. Kalchbrenner, A. Senior, and K. Kavukeuoglu, "WAVENET: A GENERATIVE MODEL FOR RAW AUDIO," arXiv:1609.03499, 2016.

Benefited from residual network, deep convolutional neural networks (CNNs) can be easily trained and improved accuracy has been achieved for image classification and object detection. Convolutional feed-forward networks connect the output of the $l^{th}$ layer as input to the $(l + 1)^{th}$ layer, which gives rise to the following layer transition: $x_i = H_j(x_{l-1})$. Residual blocks add a skip-connection that bypasses the non-linear transformations with an identify function: $x_l = H_l(x_{l-1}) + x_{l-1}$. An advantage of residual blocks is that the gradient can flow directly through the identity function from later layers to the earlier layers. However, the identity function and the output of $H_l$ are combined by summation, which may impede the information flow in the network.

## Dilated Convolutions

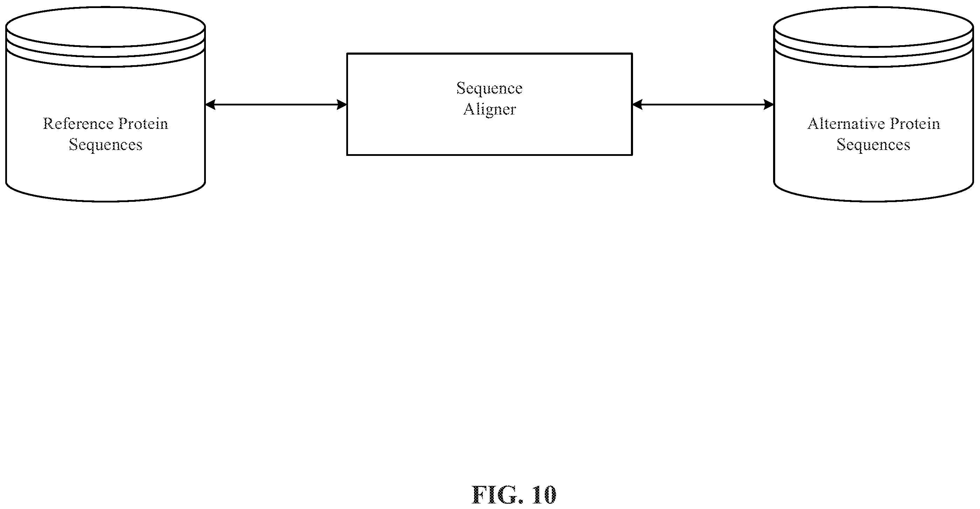

**FIG. 10** illustrates dilated convolutions. Dilated convolutions, sometimes called atrous convolutions, which literally means with holes. The French name has its origins in the algorithme a trous, which computes the fast dyadic wavelet transform. In these type of convolutional layers, the inputs corresponding to the receptive field of the filters are not neighboring points. This is illustrated in **FIG. 10**. The distance between the inputs is dependent on the dilation factor.

**WaveNet**

The WaveNet is a deep neural network for generating raw audio waveforms. The WaveNet distinguishes itself from other convolutional networks since it is able to take relatively large 'visual fields' at low cost. Moreover, it is able to add conditioning of the signals locally and globally, which allows the WaveNet to be used as a text to speech (TTS) engine with multiple voices, is the TTS gives local conditioning and the particular voice the global conditioning.

The main building blocks of the WaveNet are the causal dilated convolutions. As an extension on the causal dilated convolutions, theWaveNet also allows stacks of these convolutions, as shown in FIG. IP. To obtain the same receptive field with dilated convolutions in this figure, another dilation layer is required. The stacks are a repetition of the dilated convolutions, connecting the outputs of dilated convolution layer to a single output. This enables the WaveNet to get a large 'visual' field of one output node at a relatively low computational cost. For comparison, to get a visual field of 512 inputs, a fully convolutional network (FCN) would require 511 layers. In the case of a diluted convolutional network, we would need eight layers, The stacked dilated convolutions only need seven layers with two stacks or six layers with four stacks. To get an idea of the differences in computational power required for covering the same visual field, the following table shows the number of weights required in the network with the assumption of one filter per layer and a filter width of two. Furthermore, it is assumed that the network is using binary encoding of the 8 bits.

| Network type | No. stacks | No. weights per channel | Total No. of weights |
|---|---|---|---|
| FCN | 1 | $2.6 \cdot 10^5$ | $2.6 \cdot 10^6$ |
| WN | 1 | 1022 | 8176 |
| WN | 2 | 1022 | 8176 |
| WN | 4 | 508 | 4064 |

The WaveNet adds a skip connection before the residual connecnon is made, which bypasses all the following residual blocks. Bach of these skip cormections is summed before passing them through a series of activation functions and convolutions. Intuitively, this is the sum of the information extracted in each layer.

**Batch Normalization**

Batch normalization is a method for accelerating deep network training by making data standardization an integral part of the network architecture. Batch normalization can adaptively normalize data even as the mean and variance change over time during training. It works by internally maintaining an exponential moving average of the batch-wise mean and variance of the data seen during training. The main effect of batch normalization is that it helps with gradient propagation much like residual connections and thus allows for deep networks. Some very deep networks can only be trained if they include multiple Batch Normalization layers, Additional information about batch normalization can be found in S. Ioffe and C. Szegedy, "BATCH NORMALIZATION: ACCELERATING DEEP NETWORK TRAINING BY REDUCING INTERNAL COVARIATE SHIFT," arXiv: 1502.03167, 2015, which is hereby incorporated by reference for all purposes as if fully set forth herein.

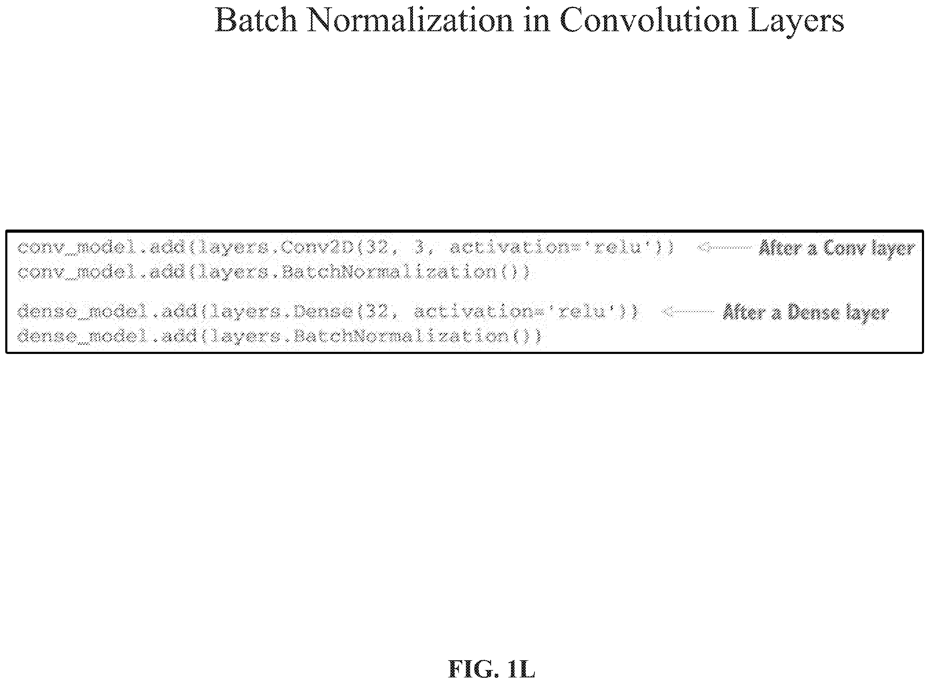

Batch normalization can be seen as yet another layer that can be inserted into the model architecture, just like the fully connected or convolutional layer, The BatchNonnalization layer is typically used after a convolutional or densely connected layer. It can also be used before a convolutional or densely connected layer. Both implementations can be used by the technology disclosed and are shown in **FIG. 1L**. The BatchNormalization layer takes an axis argument, which specifies the feature axis that should be normalized. This argument defaults to - 1, the last axis in the input tensor. This is the correct value when using Dense layers, Conv 1D layers, RNN layers, and Conv2D layers with data_format set to "channels_last". But in the niche use case of Conv2D layers with date_format set to "channels_first", the features axis is axis 1: the axis argument in BatchNormalization can be set to I.

Batch normalization provides a definition for feed-forwarding the input and computing the gradients with respect to the parameters and its own input via a backward pass. In practice, batch normalization layers are inserted after a convolutional or fully connected layer, but before the outputs are fed into an activation function. For convolutional layers, the different elements of the same feature map - i.e. the activations - at different locations are normalized in the same way in order to obey the convolutional property. Thus, all activations in a mini-batch are normalized over all locations, rather than per activation.

The internal covariate shift is the major reason why deep architectures have been notoriously slow to train. This stems from the fact that deep networks do not only have to learn a new representation at each layer, but also have to account for the change in their distribution.

The covariate shift in general is a known problem in the deep learning domain and frequently occurs in real-world problems. A common covariate shift problem is the difference in the distribution of the training and test set which can lead to suboptimal generalization performance. This problem is usually handled with a standardization or whitening preprocessing step. However, especially the whitening operation is computationally expensive and thus impractical in an online setting, especially if the covariate shift occurs throughout different layers.

The internal covariate shift is the phenomenon where the distribution of network activations change across layers due to the change in network parameters during training. Ideally, each layer should be transformed into a space where they have the same distribution but the functional relationship stays the same. In order to avoid costly calculations of covariance matrices to decorrelate and whiten the data at every layer and step, we nomialize the distribution of each input feature in each layer across each mini-batch to have zero mean and a standard deviation of one.

**Forward Pass**

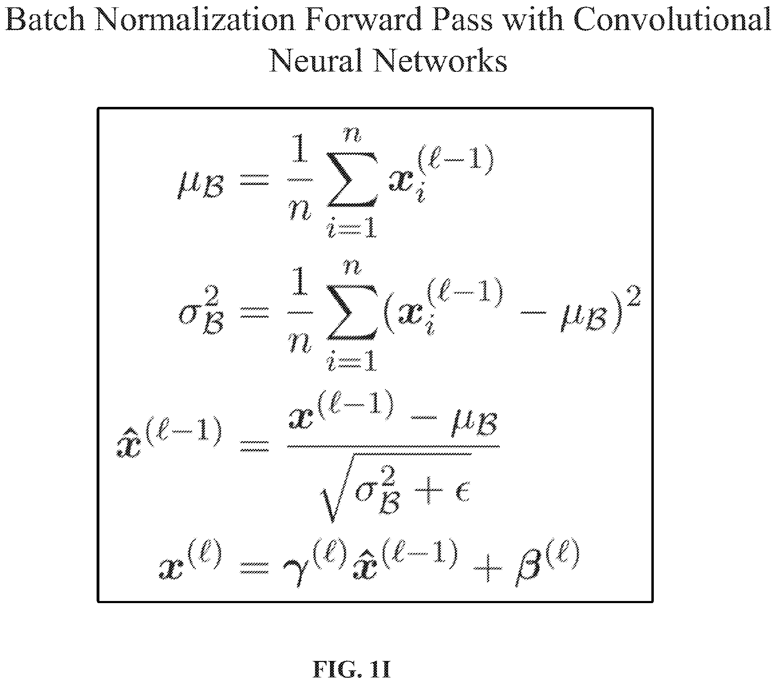

During the forward pass, the mini-barch mean and variance are calculated. With these mini-batch statistics, the data is normalized by subtracting the mean and dividing by the standard deviation. Finally. the data is scaled and shi fred with the learned scale and shift parameters. The batch nonnalization forward **pass** $f_{SN}$ is depicted in **FIG. 1I.**

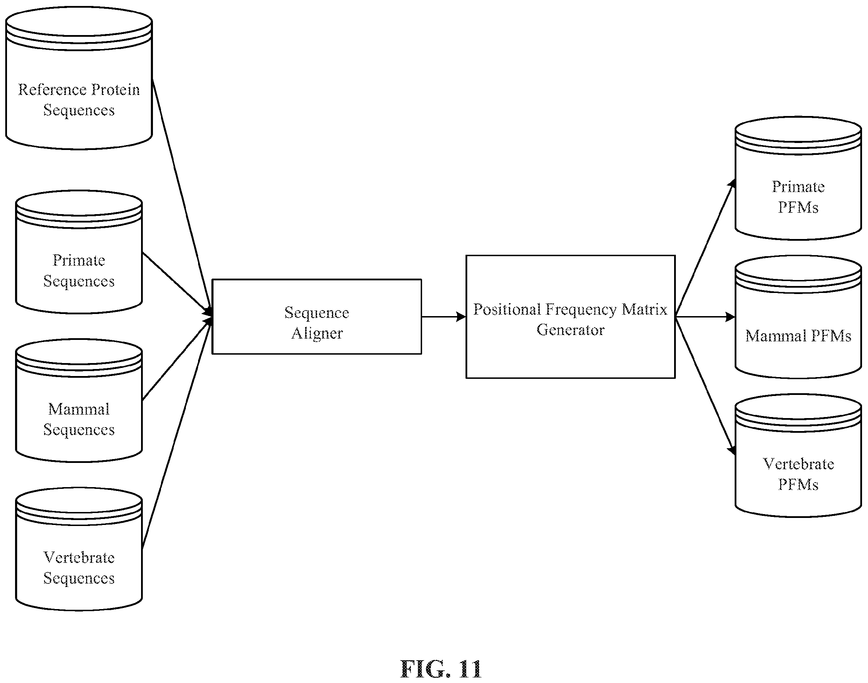

In **FIG. 11.** $\mu_\beta$ is the batch mean and $\sigma_\beta^2$ is the batch variance, respectively. The learned scale and shift parameters are denoted by $\gamma$ and $\beta$, respectively. For clarity, the batch normalization procedure is described herein per activation and omit the corresponding indices.

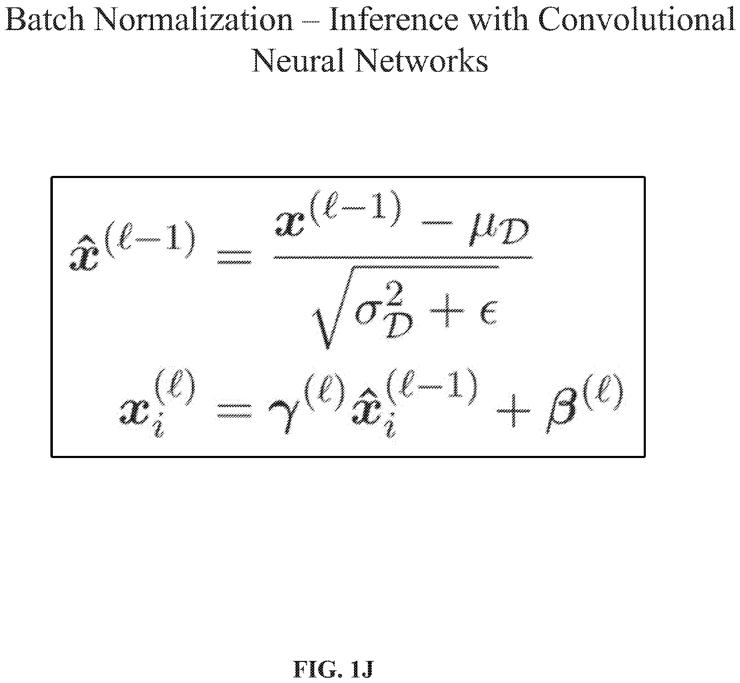

Since normalization is a differentiable transform, the errors are propagated into these learned parameters and are thus able to restore the representational power of the network by learning the identity transform. Conversely, by leaming scale and shift parameters that are identical to the corresponding batch statistics, the batch normalization transform would have no effect on the network, if that was the optimal operation to perform. At test time, the batch mean and variance are replaced by the respective population statistics since the input does not depend on other samples from a mini-barch. Another method is to keep running averages of the batch statistics during training and to use these to compute the network output at test time. At test time, the batch normalization transform can be expressed as illustrated in **FIG. 1J.** In

**FIG. 1J**, $\mu_D$ and $\sigma_D^2$ denote the population mean and variance, rather than the batch statistics, respectively.

**Backward Pass**

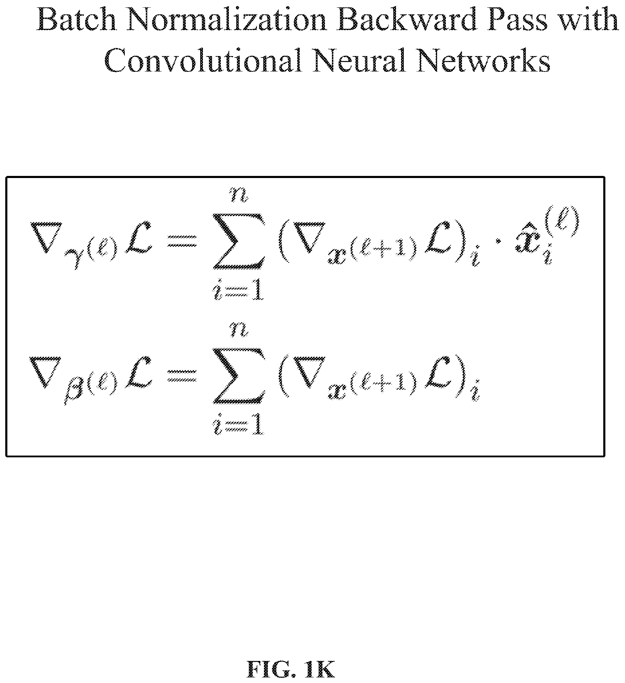

Since normalization is a differentiable operation, the backward pass can be computed as depicted in **FIG. 1K.**

**1D Convolution**

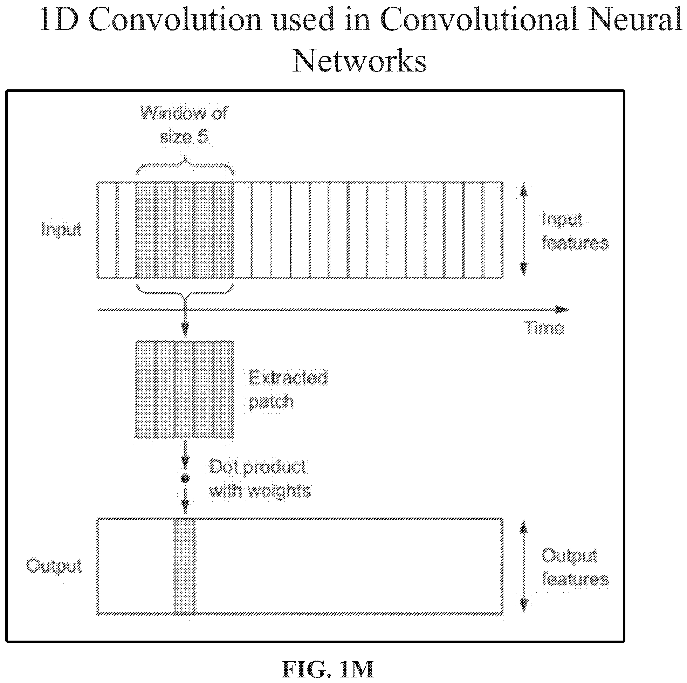

1D convolutions extract local 1D patches or subsequences from sequences, as shown in **FIG. 1M.** 1D convolution obtains each output timestep from a temporal patch in the input sequence. 1D convolution layers recognize local patters in a sequence. Because the same input transformation is performed on every patch, a pattern learned at a certain position in the input sequences can be later recognized at a different position, making 1D convolution layers translation invariant for temporal transtanons. For instance, a 1D convolution layer processing sequences of bases using convolution windows of size 5 should be able to learn bases or base sequences of length 5 or less, and it should be able to recognize the base motifs in any context in an input sequence. A base-level 1D convolution is thus able to learn about base morphology.

**Global Average Pooling**

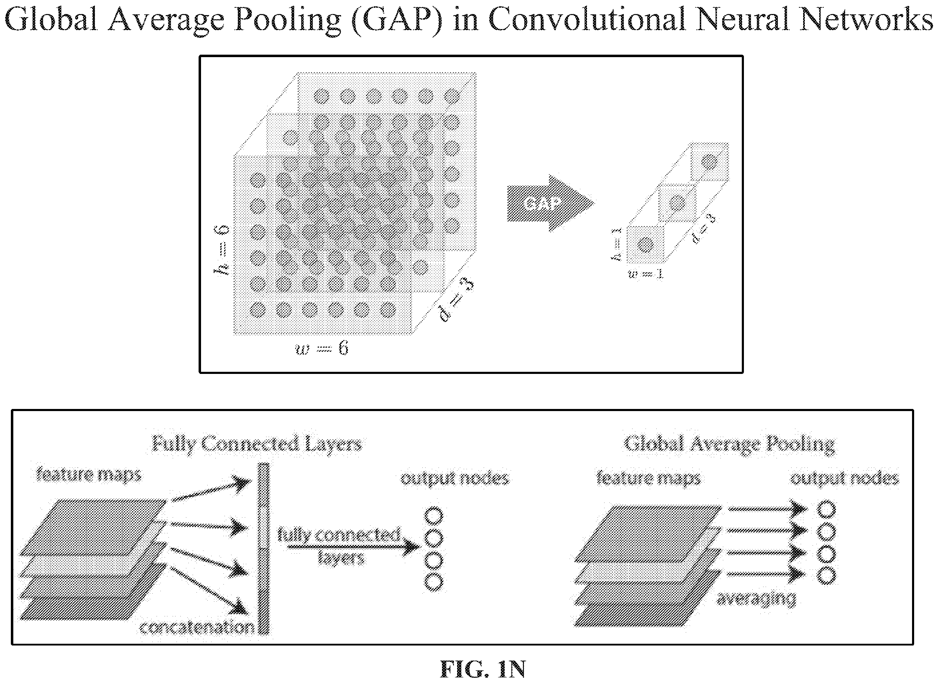

**FIG. 1N** illustrates how global average pooling (GAP) works. Global average pooling can be use used to replace fully connected (FC) layers for classification, by taking the spatial average of features in the last layer for scoring. The reduces the training load and bypasses overfitting issues. Global average pooling applies a structural prior to the model and it is equivalent to hnear transformation with predefined weights. Global average pooling reduces the number of parameters and eliminates the fully connected layer. Fully connected layers are typically the most parameter and connection intensive layers, and global average pooling provides much lower-cost approach to achieve similar results. The main idea of global average pooling is to generate the average value from each last layer feature map as the confidence factor for scoring. feeding directly into the softmax layer.

Global average pooling have three benefits; (1) there are no extra parameters in global average pooling layers thus overfitting is avoided at global average pooling layers; (2) since the output of global average pooling is the average of the whole feature map, global average pooling will he more robust to spatial translations; and (3) because of the huge number of parameters in fully connected layers which usually take over 50% in all the parameters of the whole network, replacing them by global average pooling layers can significantly reduce the size of the model, and this makes global average pooling very useful in model cotnpression.

Global average pooling makes sense, since stronger features in the last layer are expected to have a higher average value. In some implementations, global average pooling can be used as a proxy for the classification score. The feature maps under global average pooling can be interpreted as confidence maps, and force correspondence between the feature maps and the categories. Global average pooling can be particularly effective if the last layer features are at a sufficient abstraction for direct classification; however, global average pooling alone is not enough if multilevel features should be combined into groups like parts models, which is best performed by adding a simple fully connected layer or other classifier after the global average pooling.

## Deep Learning in Genomies

Genetic variations can help explain many diseases. Every human being has a unique genetic code and there are lots of genetic variants within a group of individuals. Most of the deleterious genetic variants have been depleted from genomes by natural selection. It is important to identify which genetics variations are likely to be pathogenic or deleterious. This will help researchers focus on the likely pathogenic genetic variants and accelerate the pace of diagnosis and cure of many diseases.

Modeling the properties and functional effects (e.g., pathogenicity) of variants is an important but challenging task in the field of genomics. Despite the rapid advancement of functional genomic sequencing technologies, interpretation of the functional consequences of variants remains a great challenge due to the complexity of cell type-specific transcription regulation systems.

Advances in biochemical technologies over the past decades have given rise to next generation sequencing (NGS) platforms that quickly produce genomic data at much lower costs than ever before. Such overwhelmingly large volumes of sequenced DNA remain difficult to annotate. Supervised machine learning algorithms typically perform well when large amounts of labeled data are available. In bioinformatics and many other data-rich disciplines, the process of labeling instances is costly: however, unlabeled instances are inexpensive and readily available. For a scenario in which the amount of labeled data is relatively small and the amount of unlabeled data is substantially larger, semi-supervised learning represents a cost-effective alternative to manual labeling.

An opportunity arises to use semi-supervised algorithms to construct deep learning-based pathogenicity classifiers that accurately predict pathogenicity of variants. Databases of pathogenic variants that are free from human ascertainment bias may result.

Regarding pathogenicity classifiers, deep neural networks are a type of artificial neural networks that use multiple nonlinear and complex transforming layers to successively model high-level features. Deep neural networks provide feedback via backpropagation which carries the difference between observed and predicted output to adjust parameters. Deep neural networks have evolved with the availability of large training datasets, the power of parallel and distributed computing, and sophisticated training algorithms. Deep neural networks have facilitated major advances in numerous domains such as computer vision, speech recognition, and natural language processing.

Convolutional neural networks (CNNs) and recurrent neural networks (RNNs) are components of deep neural networks. Convolutional neural networks have succeeded particularly in image recognition with an architecture that comprises convolution layers, nonlinear layers, and pooling layers. Recurrent neural networks are designed to utilize sequential information of input data with cyclic connections among building blocks like perceptrons, long short-term memory units, and gated recurrent units. In addition, many other emergent deep neural networks have been proposed for limited contexts, such as deep spatio-temporal neural networks, multi-dimensional recurrent neural networks, and convolutional auto-encoders.

The goal of maining deep neural networks is optimization of the weight parameters in each layer, which gradually combines simpler features into complex features so that the most suitable hierarchical representations can be learned from data. A single cycle of the optimization process is organized as follows, First, given a training dataset, the forward pass sequentially computes the output in each layer and propagates the function signals forward through the network. In the final output layer, an objective loss function measures error between the inferenced outputs and the given labels. To minimize the training error, the backward pass uses the chain rule to backpmpagate error signals and compute gradients with respect to all weights throughout the neural network. Finally, the weight parameters are updated using optimization algorithms based on stochastic gradient descent. Whereas batch gradient descent performs parameter updates for each complete dataset, stochastic gradient descent provides stochastic approximations by performing the updates for each small set of data examples. Several optimization algorithms stem from stochastic gradient descent. For example, the Adagrad and Adam training algorithms perform stochastic gradient descent while adaptively modifying learning rates based on update frequency and moments of the gradients for each parameter, respectively.

Another core element in the training of deep neural networks is regularization, which refers to strategies intended to avoid overfitting and thus achieve good generalization performance. For example, weight decay adds a penalty term to the objective loss function so that weight parameters converge to smaller absolute values. Dropout randomly removes hidden units from neural networks during training and can be considered an ensemble of possible subnetworks. To enhance the capabilities of dropout, a new activation function, maxout, and a variant of dropout for recurrent neural networks called mnDrop have been proposed. Furthermore, batch normalization provides a new regularization method through normalization of scalar features for each activation within a mini-batch and learning each mean and variance as parameters.

Given that sequenced data are multi- and high-dimensional, deep neural networks have great promise for bioinformatics research because of their broad applicability and enhanced prediction power. Convolutional neural networks have been adapted to solve sequence-based problems in genomics such as motif discovery, pathogenic variant identification, and gene expression inference. Convolutional neural networks use a weight-sharing strategy that is especially useful for studying DNA because it can capture sequence motifs, which are short, recurring local patterns in DNA that are presumed to have significant biological functions. A hallmark of convolutional neural networks is the use of convolution filters. Unlike traditional classification approaches that are based on elaborately-designed and manually-crafted features, convolution filters perform adaptive learning of features, analogous to a process of mapping raw input data to the informative representation of knowledge. In this sense, the convolution filters serve as a series of motif scanners, since a set of such filters is capable of recognizing relevant patterns in the input and updating themselves during the training procedure. Recurrent neural networks can capture long-range dependencies in sequential data of varying lengths, such as protein or DNA sequences.

Therefore, a powerful computational model for predicting the pathogenicity of variants can have enormous benefits for both hasic science and translational research.

Common polymorphisms represent natural experiments whose fitness has been tested by generations of natural selection. Comparing the allele frequency distributions for human missense and synonymous substitutions, we find that the presence of a missense variant at high allele frequencies in a non-human primate species reliably predicts that the variant is also under neutral selection in the human population. In contrast, common variants in more distant species experience negative selection as evolutionary distance increases.

We employ common variation from six non-human primate species to train a semi-supervised deep learning network that accurately classifies clinical *de novo* missense mutations using sequence alone. With over 500 known species, the primate lineage contains sufficient common variation to systematically model the effects of most human variants of unknown significance.

The human reference genome harbors more than 70 million potential protein-altering missense substitutions. the vast majority of which are rare mutations whose effects on human health have not been characterized. These variants of unknown significance present a challenge for genome interpretation in clinical applications, and are a roadblock to the long term adoption of sequencing for population-wide screening and individualized medicine.

Cataloguing common variation across diverse human populations is an effective strategy for identifying clinically benign variation, but the common variation available in modem day humans is limited by bottleneck events in our species' distant past. Humans and chimpanzees share 99% sequence identity, suggesting that natural selection operating on chimpanzee variants has the potential to model the effects of variants that are identical-by-state in human. The mean coalescence time for neutral polymorphisms in the human population is a fraction of the species" divergence time, hence, naturally occurring chimpanzee variation largely explores mutational space that is non-overlapping with human variation, aside from rare instances of haplotypes maintained by balancing selection.

The recent availabil ity of aggregated exome data from 60,706 humans enables us to test this hypothesis by comparing the allele frequency spectra for missense and synonymous mutations. Singleton variants in ExAC closely match the expected 2.2:1 missense:synonymous ratio predicted by *de novo* mutation after adjusting for mutational rate using trinucleotide context, but at higher allele frequencies the number of observed missense variants decreases due to the filtering out of deleterious variants by natural selection. The pattern of missense:synonymous ratios across the allele frequency spectrum indicates that a large fraction of missense variants with population frequency < 0.1% are mildly deleterious, that is, neither pathogenic enough to warrant immediate removal from the population, nor neutral enough to be allowed to exist at high allele frequencies, consistent with prior observations on more limited population data. These findings support the widespread empirical practice by diagnostic labs of filtering out variants with greater than 0.1%~1% allele frequency as likely benign for penetrant genetic disease, aside from a handful of well-documented exceptions caused by balancing selection and founder effects.

Repeating this analysis with the subset of human variants that are identical-by-state with common chimpanzee variants (observed more than once in chimpanzee population sequencing), we find that the missense:synonymous ratio is largely constant across the allele frequency spectrum. The high allele frequency of these variants in the chimpanzee population indicates that they have already been through the sieve of natural selection in chimpanzee, and their neutral impact on fitness in human populations provides compelling evidence that the selective pressures on missense variants are highly concordant in the two species. The lower missense:synonymous ratio observed in chimpanzee is consistent with the larger effective population size in ancestral chimpanzee populations enabling more efficient filtering of mildly deleterious variants.

In contrast, rare chimpanzee variants (observed only once in chimpanzee population sequencing) show a modest decrease in missense:synonymous ratio at higher allele frequencies. Simulating an identically sized cohort from human variation data, we estimate that only 64% of the variants observed once in a cohort of this size would have an allele frequency greater than 0.1% in the general population, compared to 99.8% for the variants seen multiple times in the cohort, indicating that not all of the rare chimpanzee variants have been through the sieve of selection. Overall, we estimate that 16% of the ascertained chimpanzee missense variants have an allele frequency less than 0.1% in the general population, and would be subject to negative selection at higher allele frequencies.

We next characterize human variants that are identical-by-state with variation observed in other non-human primate species (Bonobo, Gorilla, Orangutan, Rhesus, and Marmoset). Similar to chimpanzee, we observe that the missense:synonymous ratios are roughly equivalent across the allele frequency spectrum, other than a slight depletion of missense variation at high allele frequencies, which would be anticipated due to the inclusion of a small number of rare variants (~5~15%). These results imply that the selective forces on missense variants are largely concordant within the primate lineage at least out to new world monkeys, which are estimated to have diverged from the human ancestral lineage ~35 million years ago.

Human missense variants that are identical-by-state with variants in other primates are strongly enriched for benign consequence in ClinVar. After excluding variants with unknown or conflicting annotations, we observe that human variants with primate orthologs are approximately 95% likely to be annotated as Benign or Likely Benign in ClinVar, compared to 45% for missense variation in general. The small fraction of ClinVar variants that are classified as pathogenic from non-human primates is comparable to the fraction of pathogenic ClinVar variants that would be observed by ascertaining rare variants from a similar sized cohort of healthy humans, A substantial fraction of these variants annotated as Pathogenic or Likely Pathogenic indicate that received their classifications prior to the advent of large allele frequency databases, and might be curated differently today.

The field of human genetics has long relied upon model organisms to infer the clinical impact of human mutations, but the long evolutionary distance to most genetically tractable animal models raises concerns about the extent to which these findings are generalizable back to human. To examine the concordance of natural selection on missense variants in human and more distant species, we extend our analysis beyond the primate lineage to include largely common variation from four additional mammalian species (mouse, pig, goat, cow) and two species of more distant vertebrates (chicken, zebrafish). In contrast to the prior primate analyses, we observe that missense variation is markedly depleted at common allele frequencies compared to rare allele frequencies, especially at greater evolutionary distances, indicating that a substantial fraction of common missense variation in more distant species would experience negative selection in human populations. Nonetheless, the observation of a missense variant in more distant vertebrates still increases the likelihood of benign consequence, as the fraction of common missense variants depleted by natural selection is far less than the -50% depletion for human missense variants at baseline. Consistent with these results, we find that human missense variants that have been observed in mouse, dog, pig, and cow are approximately 85% likely to be annotated as Benign or Likely Benign in ClinVar, compared to 95% for primate variation and 45% for the ClinVar database as a whole.

The presence of closely related pairs of species at varying evolutionary distances also provides an opportunity to evaluate the functional consequences of fixed missense substitutions in human populations. Within closely related pairs of species (branch length < 0.1) on the mammalian family tree, we observe that fixed missense variation is depleted at common allele frequencies compared to rare allele frequencies, indicating that a substantial fraction of mter-species fixed substitutions would be non-neutral in human, even within the primate lineage. A comparison of the magnitude of missense depletion indicates that inter-species fixed substitutions are significantly less neutral than within-species polymorphisms. Intriguingly, inter-species variation between closely related mammals are not substantially more pathogenic in ClinVar (83% likely to be annotated as Benign or Likely Benign) compared to within-species common polymorphisms, suggesting that these changes do not abrogate protein function, but rather reflect tuning of protein function that confer species-specific adaptive advantages.

The large number of possible variants of unknown significance and the crucial importance of accurate variant classification for clinical applications has inspired multiple attempts to tackle the problem with machine learning, but these efforts have largely been limited by the insufficient quantity of common human variants and the dubious quality of annotations in curated databases, variation from the six non-human primates contributes over 300,000 unique missense variants that are non-overlapping with common human variation and largely of benign consequence, greatly enlarging the size of the training dataset that can be used for machine learning approaches.

Unlike earlier models which employ a large number of human-engineered features and meta-classifiers, we apply a simple deep learning residual network which takes as input only the amino acid sequence flanking the variant of interest and the orthologous sequence alignments in other species. To provide the network with information about protein structure, we train two separate networks to learn secondary structure and solvent accessibility from sequence alone, and incorporate these as sub-networks in the larger deep learning network to predict effects on protein structure. Using sequence as a starting point avoids potential biases in protein structure and functional domain annotation, which may be incompletely ascertained or inconsistently applied.

We use semi-supervised learning to overcome the problem of the training set containing only variants with benign labels, by initially training an ensemble of networks to separate likely benign primate variants versus random unknown variants that are matched for mutation rate and sequencing coverage. This ensemble of networks is used to score the complete set of unknown variants and influence the selection of unknown variants to seed the next iteration of the classifier by biasing towards unknown variants with more pathogenic predicted consequence, taking gradual steps at each iteration to prevent the model from prematurely converging to a suboptimal result.

Common primate variation also provides a clean validation dataset for evaluating existing methods that is completely independent of previously used training data, which has been hard to evaluate objectively because of the proliferation of meta-classifiers. We evaluated the performance of our model, along with four other popular classification algorithms (Sift, Polyphen2, CADD, M-CAP), using 10,000 held-out primate common variants. Because roughly 50% of all human missense variants would be removed by natural selection at common allele frequencies, we calculated the 50tb-percentile score for each classifier on a set of randomly picked missense variants that were matched to the 10,000 held-out primate common variants by mutational rate, and used that threshold to evaluate the held-out primate common variants. The accuracy of our deep learning model was significantly better than the other classifiers on this independent validation dataset, using either deep learning networks that were trained only on human common variants, or using both human common variants and primate variants.

Recent trio sequencing studies have catalogued thousands of *de novo* mutations in patients with neurodevelopmental disorders and their healthy siblings, enabling assessment of the strength of various classification algorithms in separating *de novo* missense mutations in cases versus controls. For each of the four classification algorithms, we scored each *de novo* missense variant in cases versus controls, and report the p-value from the Wilcoxon rank-sum test of the difference between the two distributions, showing that the deep learning method trained on primate variants ($p\sim10^{-33}$) performed far better than the other classifiers ($p\sim10^{-13}$) to $10^{-19}$) on this clinical scenario. From the ~1.3-fold enrichment of *de novo* missense variants over expectation previously reported for this cohort, and prior estimates that ~20% of missense variants produce loss-of-function effects, we would expect a perfect classifier to separate the two classes with a p-value of $p\sim10^{-40}$, indicating that our classifier still has room for improvement.

The accuracy of the deep learning classifier scales with the size of the training dataset, and variation data from each of the six primate species independently contributes to boosting the accuracy of the classifier. The large number and diversity of extant non-human primate species, along with evidence showing that the selective pressures on protein-altering variants are largely concordant within the primate lineage, suggests systematic primate population sequencing as an effective strategy to classify the millions of human variants of unknown significance that currently limit clinical genome interpretation. Of the 504 known non-human primate species, roughly 60% face extinction due to hunting and habitat logs, motivating urgency for a worldwide conservation effort that would benefit both these unique and irreplaceable species and our own.

Although not as much aggregate whole genome data is available as exome data, limiting the power to detect the impact of natural selection in deep intronic regions, we were also able to calculate the observed vs expected counts of cryptic splice mutations far from exonic regions. Overall, we observe a 60% depletion in cryptic splice mutations at a distance > 50nt from an exon-intron boundary. The attenuated signal is likely a combination of the smaller sample size with whole genome data compared to exame, and the greater difficulty of predicting the impact of deep intronic variants.


## Terminology

All literature and similar material cited in this application, including, but not limited to, patents, patent applications, articles, books, treatises, and web pages, regardless of the format of such literature and similar materials, are expressly incorporated by reference in their entirety. In the event that one or more of the incorporated lirerature and similar materials differs from or contradicts this application, including but not limited to defined terms, term usage, described techniques, or the like, this application controls.

As used herein, the following terms have the meanings indicated.

A base refers to a nucleotide base or nucleotide. A (adenine), C (cytosine), T (thymine), or G (guanine).

This application uses the terms "protein" and "translated sequence" interchangeably.

This application uses the terms "codon" and "base triplet" interchangeably.

This application uses the terms "amino acid" and "translated unit" interchangeably.

This application uses the phrases "variant pathogenicity classifier", "convolutional neural network-based classifier for variant classification", and "deep convolutional neural network-based classifier for variant classification" interchangeably.

The term "chromosome" refers to the heredity-bearing gene carrier of a living cell, which is derived from chromatin strands comprising DNA and protein components (especially histones). The conventional internationally recognized individual human genome chromosome numbering system is employed herein.

The term "site" refers to a unique position (e.g., chromosome ID, chromosome position and orientation) on a reference genome. In some implementations, a site may be a residue, a sequence tag, or a segment's position on a sequence. The term "locus" may be used to refer to the specific location of a nucleic acid sequence or polymorphism on a reference chromosome.

The term "sample" herein refers to a sample, typically derived from a biological fluid, cell, tissue, organ, or organism containing a nucleic acid or a mixture of nucleic acids containing at least one nucleic acid sequence that is to be sequenced and/or phased. Such samples include, but are not limited to sputum/oral fluid, amniotic fluid, blood, a blood fraction, fine needle biopsy samples (e.g., surgical biopsy, fine needle biopsy, etc.). urine, peritoneal fluid, pleural fluid, tissue explant, organ culture and any other tissue or cell preparation, or fraction or derivative thereof or isolated therefrom. Although the sample is often taken from a human subject (e.g., patient), samples can be taken from any organism having chromosomes, including, but not limited to dogs, cats, horses, goats, sheep, cattle, pigs. etc. The sample may be used directly as obtained from the biological source or following a pretreatment to modify the character of the sample. For example, such pretreatment may include preparing plasma from blood, diluting viscous fluids and so forth. Methods of pretreatment may also involve, but are not limited to, filtration, precipitation, dilution, distillation, mixing, centrifugation, freezing, lyophilization, concentration, amplification, nucleic acid fragmentation, inactivation of interfering components, the addition of reagents, lysing, etc.

The term "sequence" includes or represents a strand of nucleotides coupled to each other. The nucleotides may be based on DNA or RNA. It should be understood that one sequence may include multiple sub-sequences. For example, a single sequence (e.g., of a PCR amplicon) may have 350 nucleotides. The sample read may include multiple sub-sequences within these 350 nucleotides. For instance, the sample read may include first and second flanking sub-sequences having, for example, 20-50 nucleotides. The first and second flanking sub-sequences may be located on either side of a repetitive segment having a corresponding sub-sequence (e.g., 40-100 nucleotides). Each of the flanking sub-sequences may include (or include portions of) a primer sub-sequence (e.g., 10-30 nucleotides). For ease of reading, the term "sub-sequence" will be referred to as "sequence," but it is understood that two sequences are not necessarily separate from each other on a common strand. To differentiate the various sequences described herein, the sequences may be given different labels (e.g., target sequence, primer sequence, flanking sequence, reference sequence, and the like). Other terms, such as "allele," may be given di fferent labels to differentiate between like objects.

The term "paired-end sequencing" refers to sequencing methods that sequence both ends of a target fragment. Paired-end sequencing may facilitate detection of genomic rearrangements and repetitive segments, as well as gene fusions and novel transcripts. Methodology for paired-end sequencing are described in PCT publication WO07010252, PCT application Serial No. PCTGB20071003798 and US patent application publication US 2009/0088327, each of which is incorporated by reference herein. In one example, a series of operations may be performed as follows; (a) generate clusters of nucleic acids; (b) linearize the nucleic acids; (c) hybridize a first sequencing primer and carry out repeated cycles of extension, scanning and deblocking, as set forth above; (d) "invert" the target nucleic acids on the flow cell surface by synthesizing a complimentary copy; (e) linearize the resynthesized strand: and (f) hybridize a second sequencing primer and carry out repeated cycles of extension, scanning and deblocking, as set forth above. The inversion operation can be carried out be delivering reagents as set forth above for a single cycle of bridge amplification.

The term "reference genome" or "reference sequence" refers to any particular known genome sequence, whether partial or complete, of any organism which may be used to reference identified sequences from a subject. For example, a reference genome used for human subjects as well as many other organisms is found at the National Center for Biotechnology Information at ncbi.nlm.nih.gov. A "genome" refers to the complete genetic information of an organism or virus, expressed in nucleic acid sequences. A genome includes both the genes and the noncoding sequences of the DNA. The reference sequence may be larger than the reads that are aligned to it. For example, it may be at least about 100 times larger, or at least about 1000 times larger, or at least about 10,000 times larger, or at least about 105 times larger, or at least about 106 times larger, or at least about 107 times larger. In one example, the reference genome sequence is that of a full length human genome. In another example, the reference genome sequence is limited to a specific human chromosome such as chromosome 13. In some implementations, a reference chromosome is a chromosome sequence from human genome version hg19. Such sequences may be referred to as chromosome reference sequences, although the term reference genome is intended to cover such sequences. Other examples of reference sequences include genomes of other species, as well as chromosomes, sub-chromosomal regions (such as strands). etc., of any species. In various implementations, the reference genome is a consensus sequence or other combination derived from multiple individuals. However, in certain applications, the reference sequence may be taken from a particular individual.

The term "read" refers to a collection of sequence data that describes a fragment of a nucleotide sample or reference. The term "read" may refer to a sample read and/or a reference read. Typically, though not necessarily, a read represents a short sequence of contiguous base pairs in the sample or reference. The read may be represented symbolically by the base pair sequence (in ATCG) of the sample or reference fragment. It may be stored in a memory device and processed as appropriate to determine whether the read matches a reference sequence or meets other criteria. A read may be obtained directly from a sequencing apparatus or indirectly from stored sequence information concerning the sample. In some cases, a read is a DNA sequence of sufficient length (e.g., at least about 25 bp) that can be used to identify a larger sequence or region, e.g., that can be aligned and specifically assigned to a chromosome or genomic region or gene.

Next-generation sequencing methods include, for example, sequencing by synthesis technology (Illumina), pyrosequencing (454), ion semiconductor technology (Ion Torrent sequencing), single-molecule real-time sequencing (Pacific Biosciences) and sequencing by ligation (SOLiD sequencing). Depending on the sequencing methods, the length of each read may vary from about 30 by to more than 10,000 bp. For example, Illumina sequencing method using SOLID sequencer generates nucleic acid reads of about 50 bp. For another example, Ion Torrent Sequencing generates nucleic acid reads of up to 400 bp and 454 pyrosequencing generates nucleic acid reads of about 700 bp. For yet another example, single-molecule real-time sequencing methods may generate reads of 10,000 bp to 15,000 bp. Therefore, in certain implementations, the nucleic acid sequence reads have a length of 30-100 bp, 50-200 bp, or 50-400 bp.

The terms "sample read", "sample sequence" or "sample fragment" refer to sequence data for a genomic sequence of interest from a sample. For example, the sample read comprises sequence data from a PCR amplicon having a forward and reverse primer sequence. The sequence data can be obtained from any select sequence methodology. The sample read can be, for example, from a sequencing-hy-synthesis (SBS) reaction, a sequencing-by-ligation reaction, or any other suitable sequencing methodology for which it is desired to determine the length and/or identity of a repetitive element. The sample read can be a consensus (e.g., averaged or weighted) sequence derived from multiple sample reads. In certain implementations, providing a reference sequence comprises identifying a locus-of-interest based upon the primer sequence of the PCR amplicon.

The term "raw fragment" refers to sequence data for a portion of a genomic sequence of interest that at least partially overlaps a designated position or secondary position of interest within a sample read or sample fragment. Non-limiting examples of raw fragments include a duplex stitched fragment, a simplex stitched fragment, a duplex un-stitched fragment and a simplex un-stitched fragment. The term "raw" is used to indicate that the raw fragment includes sequence data having some relation to the sequence data in a sample read, regardless of whether the raw fragment exhibits a supporting variant that corresponds to and authenticates or confirms a potential variant in a sample read. The term "raw fragment" does not indicate that the fragmeng necessarily includes a supporting variant that validates a variant call in a sample read. For example, when a sample read is determined by a variant call application to exhibit a first variant, the variant call application may determine that one or more raw frogments lack a corresponding type of "supporting" variant that may otherwise be expected to occur given the variant in the sample read.

The terms "mapping", "aligned," "alignment," or "aligning" refer to the process of comparing a read or tag to a reference sequence and thereby determining whether the reference sequence contains the read sequence. If the reference sequence contains the read, the read may be mapped to the reference sequence or, in certain implementations, to a particular location in the reference sequence. In some cases, alignment simply tells whether or not a read is a member of a particular reference sequence (i.e., whether the read is present or absent in the reference sequence). For example, the alignment of a read to the reference sequence for human chromosome 13 will tell whether the read is present in the reference sequence for chromosome 13. A tool that provides this information may be called a set membership tester. In same cases, an alignment additionally indicates a location in the reference sequence where the read or tag maps to. For example, if the reference sequence is the whole human genome sequence, an alignment may indicate that a read is present on chromosome 13, and may further indicate that the read is on a particular strand and/or site of chromosome 13.

The term "indel" refers to the insertion and/or the deletion of bases in the DNA of an organism. A micro-indel represents an indel that results in a net change of 1 to 50 nucleotides. In coding regions of the genome, unless the length of an indel is a multiple of 3, it will produce a frameshift mutation. Indels can be contrasted with point mutations. An indel inserts and deletes nucleotides from a sequence, while a point mutation is a form of substitution that replaces one of the nucleotides without changing the overall number in the DNA. Indels can also be contrasted with a Tandem **Base** Mutation (TBM), which may be defined as substitution at adjacent nucleotides (primarily substitutions at two adjacent nucleotides, but substitutions at three adjacent nucleotides have been observed).

The term "variant" refers to a nucleic acid sequence that is different from a nucleic acid reference. Typical nucleic acid sequence variant includes without limitation single nucleotide polymorphism (SNP), short deletion and insertion polymorphisms (Indel), copy number variation (CNV), microsatellite markers or short tandem repeats and structural variation. Somatic variant calling is the effort to identify variants present at low frequency in the DNA sample. Somatic variant calling is of interest in the context of cancer treatment. Cancer is caused by an accumulation of mutations in DNA. A DNA sample from a tumor is generally heterogeneous, including some normal cells, some cells at an early stage of cancer progression (with fewer mutations), and some late-stage cells (with more mutations). Because of this heterogeneity, when sequencing a tumor (e.g., from an FFPE sample), somatic mutations will often appear at a low frequency. For example, a SNV might be seen in only 10% of the reads covering a given base. A variant that is to be classified as somatic or germline by the variant classifier is also referred to herein as the "variant under test".

The term "noise" refers to a mistaken variant call resulting from one or more errors in the sequencing process and/or in the variant call application.

The term "variant frequency" represents the relative frequency of an allele (variant of a gene) at a particular locus in a population. expressed as a fraction or percentage. For example, the fraction or percentage may be the fraction of all chromosomes in the population that carry that allele. By way of example, sample variant frequency represents the relative frequency of an allele/variant at a particular locus/position along a genomic sequence of interest over a "population" corresponding to the number of reads and/or samples obtained for the genomic sequence of interest from an individual. As another example, a baseline vanant frequency represents the relative frequency of an allele/variant as a particular locus/position along one or more baseline genomic sequences where the "population" corresponding to the number of reads and/or samples obtained for the one or more baseline genomic sequences from a population of normal individuals.

The term "variant allele frequency (VAF)" refers to the percentage of sequenced reads observed matching the variant divided by the overall coverage at the target position. VAF is a measure of the proportion of sequenced reads carrying the variant.

The terms "position", "designated position", and "locus" refer to a location or coordinate of one or more nucleotides within a sequence of nucleotides. The terms "position". "designated position", and "locus" also refer to a location or coordinate of one or more base pairs in a sequence of nucleotides.

The term "haplotype" refers to a combination of alleles at adjacent sites on a chromosome that are inherited together. A haplotype may be one locus, several loci, or an entire chromosome depending on the number of recombination events that have occurred between a given set of loci, if any occurred.

The term "threshold" herein refers to a numeric or non-numeric value that is used as a cutoff to characterize a sample, a nucleic acid, or portion thereof (e.g., a read). A threshold may be varied based upon empirical analysis. The threshold may be compared to a measured or calculated value to determine whether the source giving rise to such value suggests should be classified in a particular manner. Threshold values can be identified empirically or analytically. The choice of a threshold is dependent on the level of confidence that the user wishes to have to make the classification. The threshold may be chosen for a particular purpose (e.g., to balance sensitivity and selectivity). As used herein, the term "threshold" indicates a point at which a course of analysis may be changed and/or a point at which an action may be triggered. A threshold is not required to be a predetermined number. Instead, the threshold may be, for instance, a function that is based on a plurality of factors. The threshold may be adaptive to the circumstances. Moreover, a threshold may indicate an upper limit, a lower limit, or a range between limits.

In some implementations, a metric or score that is based on sequencing data may be compared to the threshold. As used herein, the terms "metric" or "score" may include values or results that were determined from the sequencing data or may include functions that are based on the values or results that were determined from the sequencing data. Like a threshold, the metric or score may be adaptive to the circumstances. For instance, the metric or score may be a normalized value. As an example of a score or metric, one or more implementations may use count scores when analyzing the data. A count score may be hased on the number of sample reads. The sample reads may have undergone one or more filtering stages such that the sample reads have at least one common characteristic or quality. For example, each of the sample reads that are used to determine a count score may have been aligned with a reference sequence or may be assigned as a potential allele. The number of sample reads having a common characteristic may be counted to determine a read coimt. Count scores may be based on the read count. In some implementations, the count score may be a value that is equal to the read count. In other implementations, the count score may be based on the read count and other information. For example, a count score may be based on the read count for a particular allele of a genetic locus and a total number of reads for the genetic locus. In some implementations, the count score may be based on the read count and previously-obtained data for the genetic locus. In some implementations, the count scores may be normalized scores between predetermined values. The count score may also be a function of read counts from other loci of a sample or a function of read counts from other samples that were concurrently run with the sample-of-interest. For instance, the count score may be a function of the read count of a particular allele and the read counts of other loci in the sample and/or the read counts from other samples. As one example, the read counts from other loci and/or the read counts from other samples may be used to normalize the count score for the particular allele.

The terms "coverage" or "fragment coverage" refer to a count or other measure of a number of sample reads for the same fragment of a sequence. A read count may represent a count of the number of reads that cover a corresponding fragment. Alternatively, the coverage may be determined by multiplying the read count by a designated factor that is based on historical knowledge, knowledge of the sample, knowledge of the locus, etc.

The term "read depth" (conventionally a number followed by "$\times$") refers to the number of sequenced reads with overlapping alignment at the target position. This is often expressed as an average or percentage exceeding a cutoff over a set of intervals (such as exons, genes, or panels). For example, a clinical report might say that a panel average coverage is 1,105$\times$ with 98% of targeted bases covered >100$\times$.

The terms "base call quality score" or "Q score" refer to a PHRED-scaled probability ranging from 0-20 inversely proportional to the probability that a single sequenced base is correct. For example, a T base call with Q of 20 is considered likely correct with a confidence P-value of 0.01. Any base call with Q<20 should be considered low quality, and any variant identified where a substantial proportion of sequenced reads supporting the variant are of low quality should be considered potentially false positive.

The terms "variant reads" or "variant read number" refer to the number of sequenced reads supporting the presence of the variant.

**Sequencing Process**

Implementations set forth herein may be applicable to analyzing nucleic acid sequences to identify sequence variations. Implementations may be used to analyze potential variants/alleles of a genetic position/locus and determine a genotype of the genetic **locus** or, in other words, provide a genotype call for the locus. By way of example, nucleic acid sequences may be analyzed in accordance with the methods and systems described in US Patent Application Publication No. 2016/0085910 and US Patent Application Publication No. 2013/0296175. the complete subject matter of which are expressly incorporated by reference herein in their entirety.

In one implementation, a sequencing process includes receiving a sample that includes or is suspected of including nucleic acids, such as DNA. The sample may be from a known or unknown source, such as an animal (e.g., human), plant, bacteria, or fungus. The sample may be taken directly from the source. For instance, blood or saliva may be taken directly from an individual. Alternatively, the sample may not be obtained directly from the source. Then, one or more processors direct the system to prepare the sample for sequencing. The preparation may include removing extraneous material and/or isolating certain material (e.g., DNA). The biological sample may be prepared to include features for a particular assay. For example, the biological sample may be prepared for sequencing-by-synthesis (SBS). In certain implementations, the preparing may include amplification of certain regions of a genome. For instance, the preparing may include amplifying predetermined genetic loci that are known to include STRs and/or SNPs. The genetic loci may be amplified using predetermined primer sequences.

Next, the one or more processors direct the system to sequence the sample. The sequencing may be performed through a variety of known sequencing protocols. In particular implementations, the sequencing includes SBS. In SBS, a plurality of fluorescently-labeled nucleotides are used to sequence a plurality of clusters of amplified DNA (possibly millions of clusters) present on the surface of an optical substrate (e.g., a surface that at least partially defines a channel in a flow cell). The flow cells may contain nucleic acid samples for sequencing where the flow cells are placed within the appropriate flow cell holders.

The nucleic acids can be prepared such that they comprise a known primer sequence that is adjacent to an unknown target sequence. To initiate the first SBS sequencing cycle, one or more differently labeled nucleotides, and DNA polymerase, etc., can be flowed into/through the flow cell by a fluid flow subsystem. Either a single type of nucleotide can be added at a time, or the nucleotides used in the sequencing procedure can be specially designed to possess a reversible termination property, thus allowing each cycle of the sequencing reaction to occur simultaneously in the presence of several types of labeled nucleotides (e.g., A. C. T, G). The nucleotides can include detectable label moieties such as fluorophores. Where the four nucleotides are mixed together, the polymerase is able to select the correct base to incorporate and each sequence is extended by a single base. Non-incorporated nucleotides can be washed away by flowing a wash solution through the flow cell. One or more lasers may excite the nucleic acids and induce fluorescence. The fluorescence emitted from the nucleic acids is based upon the fluorophores of the incorporated base, and different fluorophores may emit different wavelengths of emission light. A deblocking reagent can be added to the flow cell to remove reversible terminator groups from the DNA strands that were extended and detected. The deblocking reagent can then be washed away by flowing a wash solution through the flow cell. The flow cell is then ready for a further cycle of sequencing starting with introduction of a labeled nucleotide as set forth above. The fluidic and detection operations can be repeated several times to complete a sequencing run. Example sequencing methods are described, for example, in Bentley et al., Nature 456:53-39 (2008), International Publication No. WO 04/018497: U.S. Pat. No. 7,057,026: International Publication No. WO 91/06678; International Publication No. WO 07/123744; U.S. Pat. No. 7,329,492, U.S. Patent No. 7,211,414; U.S. Patent No. 7,315,019: U.S. Patent No. 7,405,281, and U.S. Patent Application Publication No. 2008/0108082, each of which is incorporated herein by reference.

In some implementations, nucleic acids can be attached to a surface and amplified prior to or during sequencing. For example, amplification can be carried out using bridge amplification to form nucleic acid clusters on a surface. Useful bridge amplification methods are described, for example, in U.S. Patent No. 5,641,658; U.S. Patent Application Publication No. 2002/0055100; U.S. Patent No. 7,115,400; U.S. Patent Application Publication No. 2004/0096853; U.S. Patent Application Publication No. 2004/0002090; U.S. Patent Application Publication No. 2007/0128624; and U.S. Patent Application Publication No. 2008/0009420, cach of which is incorporated herein by reference in its entirety. Another useful method for amplifying nucleic acids on a surface is rolling circle amplification (RCA), for example, as described in Lizardi et al., Nat. Genet. 19:225-232 (1998) and U.S. Patent Application Publication No. 2007/0099208 A1, each of which is incorporated herein by reference.

One example SBS protocol exploits modified nucleotides having removable 3' blocks, for example, as described in International Publication No. WO 04/018497, U.S. Patent Application Publication No. 2007/0166705A1, und U.S. Patent No. 7,057,026, each of which is incorporated herein by reference. For example, repeated cycles of SBS reagents can be delivered ro a flow cell having target nucleic acids attached thereto, for example, as a result of the bridge amplification protocol. The nucleic acid clusters can be converted to single stranded form using a linearization solution. The linearization solution can contain, for example, a restriction endonuclease capable of cleaving one strand of each cluster. Other methods of cleavage can be used as an altemarive to restriction enzymes or nicking enzymes, including inter alia chemical cleavage (e.g., cleavage of a diol linkage with periodate), cleavage of abasic sites by cleavage with endonuclease (for example 'USER', as supplied by NEB, Ipswich, Mass., USA, part number M5505S), by exposure to heat or alkali, cleavage of ribonucleotides incorporated into amplification products otherwise comprised of deoxyribonucleotides, photochemical cleavage or cleavage of a peptide linker. After the linearization operation a sequencing primer can be delivered to the flow cell under conditions for hybridization of the sequencing primer to the target nucleic acids that are to be sequenced.

A flow cell can then be contacted with an SBS extension reagent having modified nucleotides with removable 3' blocks and fluorescent labels under conditions to extend a primer hybridized to each target nucleic acid by a single nucleotide addition. Only a single nucleotide is added to each primer because once the modified nucleotide has been incorporated into the growing polynucleotide chain complementary to the region of the template being sequenced there is no free 3'-OH group available to direct further sequence extension and therefore the polymerase cannot add further nucleotides. The SBS extension reagent can be removed and replaced with scan reagent containing components that protect the sample under excitation with radiation. Example components for scan reagent are described in U.S. Patent Application Publication No. 2008/0280773 A1 and U.S. Patent Application No. 13/018,255, each of which is incorporated herein by reference. The extended nucleic acids can then be fluorescently detected in the presence of scan reagent. Once the fluorescence has been detected, the 3' block may be removed using a deblock reagent that is appropriate to the blocking group used. Example deblock reagents that are useful for respective blocking groups are described in WO004018497, US 2007/4016675A1 and U.S. Patent No. 7,057,026, each of which is incorporated herein by reference. The deblock reagent can be washed away leaving target nucleic acids hybridized to extended printers having 3'-OH groups that are now competent for addition of a further nucleotide. Accordingly the cycles of adding extension reagent, scan reagent, and deblock reagent, with optional washes between one or more of the operations, can be repeated until a desired sequence is obtained. The above cycles can be carried out using a single extension reagent delivery operation per cycle when each of the modified nucleotides has a different label attached thereto, known to correspond to the particular base. The different labels facilitate discrimination between the nucleotides added during each incorporation operation. Alternatively, each cycle can include separate operations of extension reagent delivery followed by separate operations of scan reagent delivery and detection, in which case two or more of the nucleotides can have the same label and can be distinguished based on the known order of delivery.

Although the sequencing operation has been discussed above with respect to a particular SBS protocol, it will be understood that other protocols for sequencing any of a variety of other molecular analyses can be carried out as desired.

Then, the one or more processors of the system receive the sequencing data for subsequent analysis. The sequencing data may be formatted in various manners, such as in a .BAM file. The sequencing data may include, for example, a number of sample reads. The sequencing data may include a plurality of sample reads that have corresponding sample sequences of the nucleotides. Although only one sample read is discussed, it should be understood that the sequencing data may include, for example, hundreds, thousands, hundreds of thousands, or millions of sample reads. Different sample reads may have different numbers of nucleotides. For example, a sample read may range between 10 nucleotides to about 500 nucleotides or more. The sample reads may span the entire genome of the source(s). As one example, the sample reads are directed toward predetermined genetic loci, such as those genetic loci having suspected STRs or suspected SNPs.

Each sample read may include a sequence of nucleotides, which may be referred to as a sample sequence, sample fragment or a target sequence. The sample sequence may include, for example, primer sequences, flanking sequences, and a target sequence. The number of nucleotides within the sample sequence may include 30, 40, 50, 60, 70, 80, 90, 100 or more. In some implementations, one or more of the sample reads (or sample sequences) includes at least 150 nucleotides, 200 nucleotides, 300 nucleotides, 400 nucleotides. 500 miclcotides, or more. In some implementations, the sample reads may include more than 1000 nucleotides, 2000 nucleotides, or more. The sample reads (or the sample sequences) may include primer sequences at one or both ends.

Next, the one or more processors analyze the sequencing data to obtain potential variant call(s) and a sample variant frequency of the sample variant call(s). The operation may also be referred to as a variant call application or variant caller. Thus, the variant caller identifies or detects variants and the variant classifier classifies the detected variants as somatic or germline. Alternative variant callers may be utilized in accordance with implementations herein, wherein different variant callers may be used based on the type of sequencing operation being performed, based on features of the sample that are of interest and the like. One non-limiting example of a variant call application, such as the Pisecs™

application by Illumina Inc. (San Diego. CA) hosted at https://github.com/Illumina/Pisces and described in the article Dunn, Tamsen &. Berry, Gwenn & Emig-Agius. Dorothea & Jiang, Yu & Iyer, Anita & Udar, Nitin & Strömberg, Michael. (2017). Pisces: An Accurate and Versatile Single Sample Somatic and Germline Variant Caller. 595-595. 10.114513107411.3108203, the complete subject matter of which is expressly incorporated herein by reference in its entirety.

Such a variant call application can comprise four sequentially executed modules:

**(1) Pisces Read Stitcher.** Reduces noise by stitching paired reads in a BAM (read one and read two of the same molecule) into consensus reads. The output is a stitched BAM.

(2) Pisces Variant Caller. Calls small SNVs, insertions and deletions. Pisces includes a variant-collapsing algorithm to coalesce variants broken up by read boundaries, basic filtering algorithms, and a simple Poisson-based variant confidence-scoring algorithm. The output is a VCF.

(3) Pisces Variant Quality Recalibrator (VQR): In the event that the variant calls overwhelmingly follow a pattern associated with thermal damage or FFPE deamination, the VQR step will downgrade the variant Q score of the suspect variant calls. The output is an adjusted VCF.

(4) Pisces Variant Phaser (Scylla): Uses a read-backed greedy clustering method to assemble small variants into complex alleles from clonal subpopulations. This allows for the more accurate determination of functional consequence by downstream tools. The output is an adjusted VCF.

Additionally or alternatively, the operation may utilize the variant call application Strelka™ application by Illumina Inc. hosted at https://github.com/Illumina/strelka and described in the article T Saunders. Christopher & Wong, Wendy & Swamy, Sajani & Becq, Jennifer & J Murray, Lisa & Cheetham, Keira. (2012), Strelka: Accurate somatic small-variant calling from sequenced tumor-normal sample pairs. Bioinformatics (Oxford, England). 28. 1811-7. 10.1093/bioinformatics/bts271, the complete subject matter of which is expressly incorporated herein by reference in its entirety. Furthermore, additionally or alternatively, the operation may utilize the variant call application Strelka2™ application by Illumina Inc. hosted at https://github.com/Illumine/strelka and described in the article Kim, S., Scheffler. K.. Halpern, A.L., Bekritsky, M.A., Noh, E., Källberg, M., Chen, X., Beyter, D., Krusche, P., and Saunders, C.T. (2017). Strelka2: Fast and accurate variant calling for clinical sequencing applications, the complete subject matter of which is expressly incorporated herein by reference in its entirety. Moreover, additionally or alternatively, the operation may utilize a variant annotation/call tool, such as the Nirvana™ application by Illumina Inc. hosted at https://github.com/Illumina/Nirvana/wiki and described in the article Stromberg, Michael & Roy, Rajat & Lajugie, Julien & Jiang, Yu & Li, Haochen & Margulies, Elliott. (2017). Nirvana: Clinical Grade Variant Annotator. 596-596.10.1145/3107411.3108204, the complete subject matter of which is expressly incorporated herein by reference in its entirety.

Such a variant annotation/call tool can apply different algorithmic techniques such as those disclosed in Nirvana:

a. Identifying all overlapping transcripts with Interval Array: For functional annotation. we can identify all transcripts overlapping a variant and an interval tree can be used. However, since a set of intervals can be static, we were able to further optimize it to an Interval Array. An interval tree returns all overlapping transcripts in $O(\min(n, k \lg n))$ time, where n is the number of intervals in the tree and k is the number of overlapping intervals. In practice, since k is really small compared to n for most variants, the effective runtime on interval tree would be $O(k \lg n)$. We improved to $O(\lg n + k)$ by creating an interval array where all intervals are stored in a sorted array so that we only need to find the first overlapping interval and then enumerate through the remaining (k-1).

b. CNVs/SVs (Yu): annotations for Copy Number Variation and Structural Variants can be provided. Similar to the annotation of small variants, transcripts overlapping with the SV and also previously reported structural variants can be annotated in online databases. Unlike the small variants, not all overlapping transcripts need be annotated, since too many transcripts will be overlapped with a large SVs. Instead, all overlapping transcripts can be annotated that belong to a partial overlapping gene. Specifically, for these transcripts, the impacted introns, exons and the consequences caused by the structural variants can be reported. An option to allow output all overlapping transcripts is avai table, but the basic information for these transcripts can be reported, such as gene symbol, flag whether it is canonical overlap or partial overlapped with the transcripts. For each SV/CNV, it is also of interest to know if these variants have been studied and their frequencies in different populations. Hence, we reported overlapping SVs in external databases, such as 1000 genomes, DGV and ClinGen. To avoid using an arbitrary cutoff to determine which SV is overlapped, instead all overlapping transcripts can be used and the reciprocal overlap can be calculated, i.e. the overlapping length divided by the minimum of the length of these two SVs.

c. Reporting supplementary annotations : Supplementary annotations are of two types: small and structural variants (SVs). SVs can be modeled as intervals and use the interval array discussed above to identify overlapping SVs. Small variants are modeled as points and matched by position and (optionally) allele. As such, they are searched using a binary-search-like algorithm. Since the supplementary annotation database can be quite large, a much smaller index is created to map chromosome positions to file locations where the supplementary annotation resides. The index is a sorted array of objects (made up of chromosome position and **file** location) that can be binary searched using position. To keep the index size small, multiple positions (up to a certain max count) are compressed to one object that stores the values for the first position and only deltas for subsequent positions. Since we use Binary search, the runtime is O(lg n), where n is the number of items in the database.

d. VEP cache files

e. Transcript Database : The Transcript Cache (cache) and Supplementary database (SAdb) files are serialized dump of data objects such as transcripts and supplementary annotations. We use Ensembl VEP cache as our data source for cache. To create the cache, all transcripts are inserted in an interval array and the final state of the array is stored in the cache files. Thus, during annotation, we only need to load a pre-computed interval array and perform searches on it. Since the cache is loaded up in memory and searching is very fast (described above), finding overlapping transcripts is extremely quick in Nirvana (profiled to less than 1% of total runtime?).

f. Supplementary Database : The data sources for SAdb are listed under supplementary material. The SAdb for small variants is produced by a k -way merge of all data sources such that each object in the database (identified by reference name and position) holds all relevant supplementary annotations. Issues encountered during parsing data source files have been documented in detail in Nirvana's home page. To limit memory usage, only the SA index is loaded up in memory. This index allows a quick lookup of the file location for a supplementary annotation. However, since the data has to be fetched from disk, adding supplementary annotation has been identified as Nirvana's largest bottleneck (profiled at -30% of total runtime.)

g. Consequence and Sequence Ontology : Nirvana's functional annotation (when provided) follows the Sequence Ontology (SO) (http://www.sequenceontology.org/) guidelines. On occasions, we had the opportunity to identify issues in the current SO und collaborate with the SO team to improve the state of annotation.

Such a variant annotation tool can include pre-processing. For example, Nirvana included a large number of annotations from External data sources, like ExAC, EVS, 1000 Genomes project, dbSNP, ClinVar, Cosmic, DGV and ClinGen. To make full use of these databases, we have to sanitize the information from them. We implemented different strategy to deal with different conflicts that exist from different data sources. For example, in case of multiple dbSNP entries for the same position and alternate allele, we join all ids into a comma separated list of ids: if there are multiple entries with different CAF values for the same allele, we use the first CAF value. For contlicting ExAC and EVS entries, we consider the number of sample counts and the entry with higher sample count is used. In 1000 Genome Projects, we removed the allele frequency of the conflicting allele. Another issue is inaccurate information. We mainly extracted the allele frequencies information from 1000 Genome Projects, however, we noticed that for GRCh38, the allele frequency reported in the info field did not exclude samples with genotype not available, leading to deflated frequencies for variants which are not available for all samples. To guarantee the accuracy of our annotation, we use all of the individual level genotype to compute the true allele frequencies. As we know, the same variants can have different representations based on different alignments. To make sure we can accurately report the information for already identified variants, we have to preprocess the variants from different resources to make them have consistent representation. For all external data sources, we trimmed alleles to remove duplicated nucleotides in both reference allele and alternative allele. For ClinVar, we directly parsed the xml file we performed a five-prime alignment for all variants, which is often used in vcf file. Different databases can contain the same set of information. To avoid unaccessary duplicates, we removed some duplicated information. For example, we removed variants in DGV which has data source as 1000 genome projects, since we already reported these variants in 1000 genomes with more detailed information.

In accordance with at least some implementations, the variant call application provides calls for low frequency variants, germline calling and the like. As non-limiting example, the variant call application may run on tumor-only samples and/or tumor-normal paired samples. The variant call application may search for single nucleotide variations (SNV), multiple nucleotide variations (MNV), indels and the like. The variant call application identifies variants, while filtering for mismatches due to sequencing or sample preparation errors. For each variant, the variant caller identifies the reference sequence, a position of the variant, and the potential variant sequence(s) (e.g., A to C SNV, or AG to A deletion). The variant call application identifies the sample sequence (or sample fragment). a reference sequence/fragment, and a variant call as an indication that a variant is present. The variant call application may identify raw fragments, and output a designation of the raw fragments, a count of the number of raw fragments that verify the potential variant call, the position within the raw fragment at which a supporting variant occurred and other relevant information. Non-limiting examples of raw fragments include a duplex stitched fragment, a simplex stitched fragment, a duplex un-stitched fragment and a simplex un- stitched fragment.

The variant call application may output the calls in various formats, such as in a .VCF or .GVCF file. By way of example only, the variant call application may be included in a MiSeqReporter pipeline (e.g., when implemented on the MiSeq® sequencer instrument). Optionally, the application may be implemented with various workflows, The analysis may include a single protocol or a combination of protocols that analyze the sample reads in a designated manner to obtain desired information,

Then, the one or more processors perform a validation operation in connection with the potential variant call. The validation operation may be based on a quality score, and/or a hierarchy of tiered tests, as explained hereafter. When the validation operation authenticates or verifies that the potential variant call, the validation operation passes the variant call information (from the variant call application) to the sample report generator. Alternatively, when the validation operation invalidates or disqualifies the potential variant call, the validation operation passes a corresponding indication (e.g., a negative mdicator, a no call indicator, an in-valid call indicator) to the sample report generator. The validation operation also may pass a confidence score related to a degree of confidence that the variant call is correct or the in-valid call designation is correct.

Next, the one or more processors generate and store a sample report. The sample report may include, for example. information regarding a plurality of genetic loci with respect to the sample. For example, for each genetic locus of a predetermined set of genetic loci, the sample report may at least one of provide a genotype call; indicate that a genotype call cannot be made: provide a confidence score on a certainty of the genotype call; or indicate potential problems with an assay regarding one or more genetic loci. The sample report may also indicate a gender of an individual that provided a sample and/or indicate that the sample include multiple sources. As used herein, a "sample report" may include digital data (e.g., a data file) of a genetic locus or predetermined set of genetic locus and/or a printed report of the genetic locus or the set of genetic loci. Thus, generating or providing may include creating a data file and/or printing the sample report, or displaying the sample report.

The sample report may indicate that a variant call was determined, but was not validated. When a variant call is determined invalid, the sample report may indicate additional information regarding the basis for the determination to not validate the variant call. For example, the additional information in the report may include a description of the raw fragments and an extent (e.g., a count) to which the raw fragments support or contradict the variant call. Additionally or alternatively, the additional information in the report may include the quality score obtained in accordance with implementations described herein.

## **Variant Call Application**

Implementations disclosed herein include analyzing sequencing data to identify potential variant calls. Variant calling may be performed upon stored data for a previously performed sequencing operation. Additionally or alternatively, it may be performed in real time while a sequencing operation is being performed. Each of the sample reads is assigned to corresponding genetic loci. The sample reads may be assigned to corresponding genetic loci based on the sequence of the nucleotides of the sample read or, in other words, the order of nucleotides within the sample read (e.g., A, C, G, T). Bused on this analysis, the sample read may be designated as including a possible variant/allele of a particular genetic locus. The sample read may be collected (or aggregated or binned) with other sample reads that have been designated as including possible variants/alleles of the genetic locus. The assigning operation may also be referred to as a calling operation in which the sample read is identified as being possibly associated with a particular genetic position/locus. The sample reads may be analyzed to locate one or more identifying sequences (e.g., primer sequences) of nucleotides that differentiate the sample read from other sample reads. More specifically, the identifying sequence(s) may identify the sample read from other sample reads as being associated with a particular genetic locus.

The assigning operation may include analyzing the series of n nucleotides of the identifying sequence to determine if the series of n nucleotides of the identifying sequence effectively matches with one or more of the select sequences. In particular implementations, the assigning operation may include analyzing the first n nucleotides of the sample sequence to determine if the first n nucleotides of the sample sequence effectively matches with one or more of the select sequences. The number n may have a variety of values, which may be programmed into the protocol or entered by a user. For example, the number n may be defined as the number of nucleotides of the shortest select sequence within the database. The number n may be a predetermined number. The predetermined number may be, for example, 10, 11, 12, 13, 14, 15, 16, 17, 18, 19, 20, 21, 22, 23, 24, 25, 26, 27, 28, 29, or 30 nucleotides. However, fewer or more nucleotides may be used in other implementations. The number n may also be selected by an individual, such as a user of the system. The number n may be based on one or more conditions. For instance, the number n may be defined as the number of nucleotides of the shortest primer sequence within the database or a designated number, whichever is the smaller number. In some implementations, a minimum value for n may be used, such as 15, such that any primer sequence that is less than 15 nucleotides may be designated as an exception.

In some cases, the series of n nucleotides of an identifying sequence may not precisely match the nucleotides of the select sequence. Nonetheless, the identifying sequence may effectively match the select sequence if the identifying sequence is nearly identical to the select sequence. For example, the sample read may be called for a genetic locus if the series of a nucleotides (e.g., the first n nucleotides) of the identifying sequence match a select sequence with no more thin a designated number of mismatches (e.g., 3) and/or a designated number of shifts (e.g., 2). Rules may be established such that each mismatch or shift may count as a difference between the sample read and the primer sequence. If the number of differences is less than a designated number, then the sample read may be called for the corresponding genetic locus (i.e.. assigned to the corresponding genetic locus). In some implementations, a matching score may be determined that is based on the number of differences between the identifying sequence of the sample read and the select sequence associated with a genetic locus. If the matching score passes a designated matching threshold, then the genetic locus that corresponds to the select sequence may be designated as a potential locus for the sample read. In some implementations, subsequent analysis may be performed to determine whether the sample read is called the the genetic locus.

If the sample read effectively matches one of the select sequences in the database (i.e., exactly matches or nearly matches as described above), then the sample read is assigned or designated to the genetic locus that correlates to the select sequence. This may be referred to as locus calling or provisional-locus calling, wherein the sample read is called for the genetic locus that correlates to the select sequence. However, as discussed above, a sample read may be called for more than one genetic locus. In such implementations, further analysis may be performed to call or assign the sample read for only one of the potential genetic loci. In some implementations, the sample read that is compared to the database of reference sequences is the first read from paired-end sequencing. When performing paired-end sequencing, a second read (representing a raw fragment) is obtained that correlates to the sample read. After assigning, the subsequent analysis that is performed with the assigned reads may be based on the type of genetic locus that has been called for the assigned read.

Next, the sample reads are analyzed to identify potential variant calls. Among other things, the results of the analysis identify the potential variant call, a sample variant frequency, a reference sequence and a position within the genomic sequence of interest at which the variant occurred. For example, if a genetic locus is known for including SNPs, then the assigned reads that have been called for the genetic locus may undergo analysis to identify the SNPs of the assigned reads. If the genetic locus is known for including polymorphic repetitive DNA elements, then the assigned reads may be analyzed to identify or characterize the polymorphic repetitive DNA elements within the sample reads. In some implementations, if an assigned read effectively matches with a STR locus and a SNP locus, a warning or flag may be assigned to the sample read. The sample read may be designated as both a STR locus and an SNP locus. The analyzing may include aligning the assigned reads in accordance with an alignment protocol to detennine sequences and/or lengths of the assigned reads. The alignment protocol may include the method described in International Patent Application No. PCT/US2013/030867 (Publication No. WO 2014/142831), filed) on March 15, 2013, which is herein mcorporated by reference in its entirety.

Then, the one or more processors analyze raw fragments to determine whether supporting variants **exist** at corresponding positions within the raw fragments. Various types of raw fragments may be identified. For example, the variant caller may identify a type of raw fragment that exhibits a variant that validates the original variant call. For example, the type of raw fragment may represent a duplex stitched fragment, a simplex stitched fragment, a duplex un-stitched fragment or a simplex un-stitched fragment. Optionally other raw fragments may be identified instead of or in addition to the foregoing examples. In connection with identifying each type of raw fragment, the variant caller also identifies the position, within the raw fragment, at which the supporting variant occurred, as well as a count of the number of raw fragments that exhibited the supporting variant. For example, the variant caller may output an indication that 10 reads of raw fragments were identified to represent duplex stitched fragments having a supporting variant at a particular position X. The variant caller may also output indication that five reads of raw fragments were identified to represent simplex un-stitched tragments having a supporting variant at a particular position Y. The variant caller may also output a number of raw fragments that corresponded to reference sequences and thus did not include a supporting variant that would otherwise provide evidence validating the potential variant call at the genomic sequence of interest.

Next, a count is maintained of the raw fragments that include supporting variants, as well as the position at which the supporting variant occurred. Additionally or alternatively, a count may be maintained of the raw fragments that did not include supporting variants at the position of interest (relative to the position of the potential variant call in the sample read or sample fragment). Additionally or alternnatively, a count may be maintained of raw fragments that correspond to a reference sequence and do not authenticate or confirm the potential variant call. The information determined is output to the variant call validation application, including a count and type of the raw fragments that support the potential variant call, positions of the supporting variance in the raw fragments, a count of the raw fragments that do not support the potential variant call and the like.

When a potential variant call is identified, the process outputs an indicating of the potential variant call, the variant sequence, the variant position and a reference sequence associated therewith. The variant call is designated to represent a "potential" variant as errors may cause the call process to identify a false variant. In accordance with implementations herein, the potential variant call is analyzed to reduce and eliminate false variants or false positives. Additionally or alternatively, the process analyzes one or more raw fragments associated with a sample read and outputs a corresponding variant call associated with the raw fragments.

## Benign Training Set Generation

Millions of human genomes and exomes have been sequenced, but their clinical applications remain limited due to the difficulty of distinguishing disease-causing mutations from benign genetic variation. Here we demonstrate that common missense variants in other primate species are largely clinically benign in human, enabling pathogenic mutations to be systematically identified by the process of elimination. Using hundreds of thousands of common variants from population sequencing of six non-human primate species, we train a deep neural network that identifies pathogenic mutations in rare disease patients with 88% accuracy and enables the discovery of 14 new candidate genes in intellectual disability at genome-wide significance. Cataloging common variation from additional primate species would improve interpretation for millions of variants of uncertain significance, further advancing the clinical utility of human genome sequencing.

The clinical actionability of diagnostic sequencing is limited by the difficulty of interpreting rare genetic variants in human populations and inferring their impact on disease risk. Because of their deleterious effects on fitness, clinically significant genetic variants tend to be extremely rare in the population and, for the vast majority, their effects on human health have not been determined. The large number und rarity of these variants of uncertain clinical significance present a formdable obstacle to the adoption of sequencing for individualized medicine and population-wide health screening.

Most penetrant Mendelian diseases have very low prevalence in the population, hence the observation of a variant at high frequencies in the population is strong evidence in favor of benign consequence. Assaying common variation across diverse human populations is an effective strategy for cataloguing benign variants, but the total amount of common variation in present-day humans is limited due to bottleneck events in our species' recent history, during which a large fraction of ancestral diversity was lost. Population studies of present-day humans show a remarkable inflation from an effective population size ($N_e$) of less than 10,000 individuals within the last 15,000-65.000 years, and the small pool of common polymorphisms traces back to the limited capacitance for variation in a population of this size. Out of more than 70 million potential protein-altering missense substitutions in the reference genome, only roughly 1 in 1,000 are present at greater than 0.1% overall population allele frequency.

Outside of modern human populations, chimpanzees comprise the next closest extant species, and share 99.4% amino acid sequence identity. The near-identity of the protein-coding sequence in humans and chimpanzees suggests that purifying selection operating on chimpanzee protein-coding variants might also model the consequences on fitness of human mutations that are identical-by-state.

Because the mean time for neutral polymorphisms to persist in the ancestral human lineage (-4N, generations) is a fraction of the species' divergence time (~6 million years ago), naturally occurring chimpanzee variation explores mutational space that is largely non-overlapping except by chance, aside from rare instances of haplotypes maintained by balancing selection. If polymorphisms that are identical-by-state similarly affect fitness in the two species, the presence of a variant at high allele frequencies in chimpanzee populations should indicate benign consequence in human, expanding the catalog of known variants whose benign consequence has been established by purifying selection.

## Results - Common Varlants in Other Primates are Largely Benign in Human

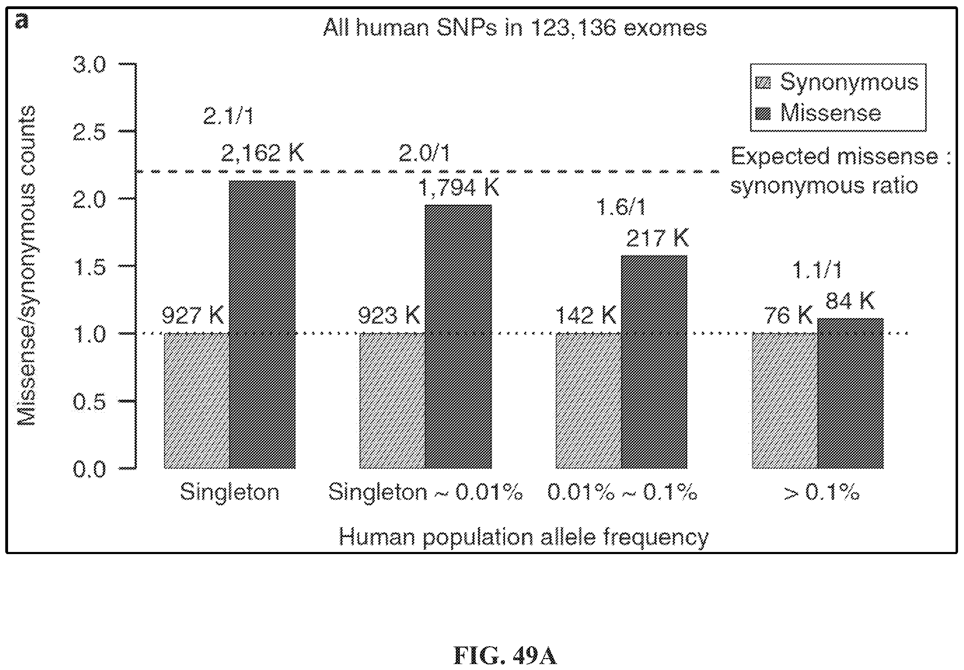

The recent availability of aggregated exome data. comprising 123,136 humans collected in the Exome Aggregation Consortium (ExAC) and Genome Aggregation Database (gnomAD), allows us to measure the impact of natural selection on missense and synonymous mutations across the allele frequency spectrum. Rare singleton variants that are observed only once in the cohort closely match the expected 2.2/1 missense/synonymous ratio predicted by *de novo* mutation after adjusting for the effects of trinucleotide context on mutational rate **(FIG. 49A, FIG. 51,** and **FIGs. 524A, 528, 52C,** and **52D),** but at higher allele frequencies the number of observed missense variants decreases due to the purging of deleterious mutations by natural selection. The gradual decrease of missense/synonynmus ratios with increasing allele frequency is consistent with a substantial fraction of missense variants of population frequency < 0.1% having a mildly deleterious consequence despite being observed in healthy individuals. These findings support the widespread empirical practice by diagnostic laboratories of filtering out variants with greater than 0.1% to ~1% allele frequency as probably benign for penetrant genetic disease, aside from a handful of well-documented exceptions due to balancing selection and founder effects.

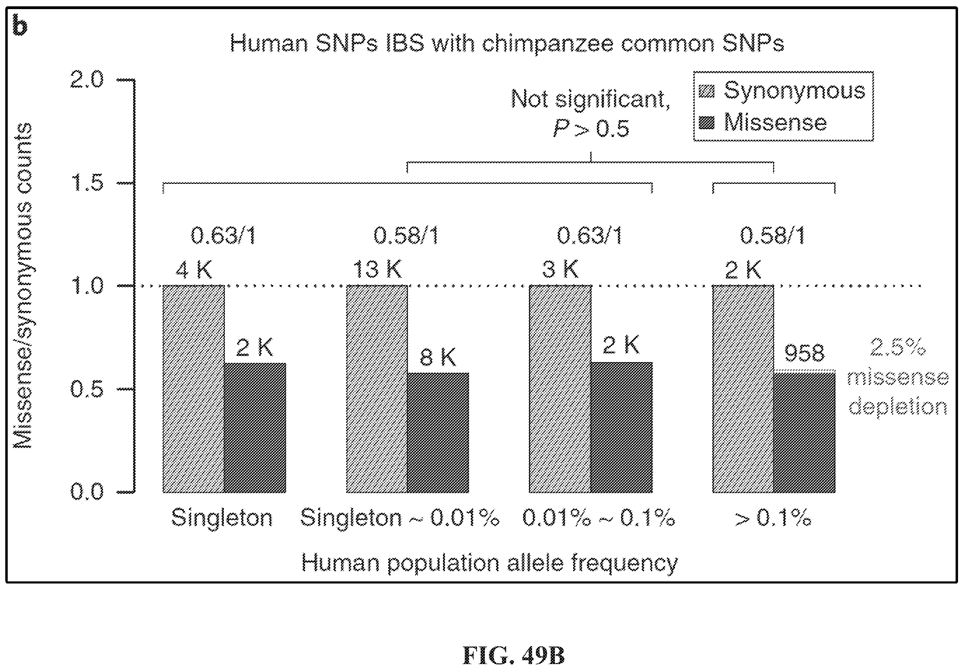

We identified common chimpanzee variants that were sampled two or more times in a cohort of 24 unrelated individuals; we estimate that 99.8% of these variants are common in the general chimpanzee population (allele frequency (AF) > 0.1%), indicating that these variants have already passed through the sieve of purifying selection. We examined the human allele frequency spectrum for the corresponding identical-by-state human variants **(FIG. 49B,)** excluding the extended major histocompatibility complex region as a known region of balancing selection, along with variants lacking a one-to-one mapping in the multiple sequence alignment. For human varients that are identical-by-state with common chimpanzee variants, the missense/synonymymus ratio is largely constant across the human allele frequency spectrum (P > 0.5 by Chi-squared ($\chi$2) test), which is consistent with the absence of negative seleciion against common chimpanzee variants in the human population and concordant selection coefficients on missense variants in the two species. The low missense/synonymous ratio observed in human variants that are identical-by-state with common chimpanzee variants is consistent with the larger effective population size in chimpanzee ($N_e$ ~ 73,000), which enables more efficient filtering of mildly deleterious variation.

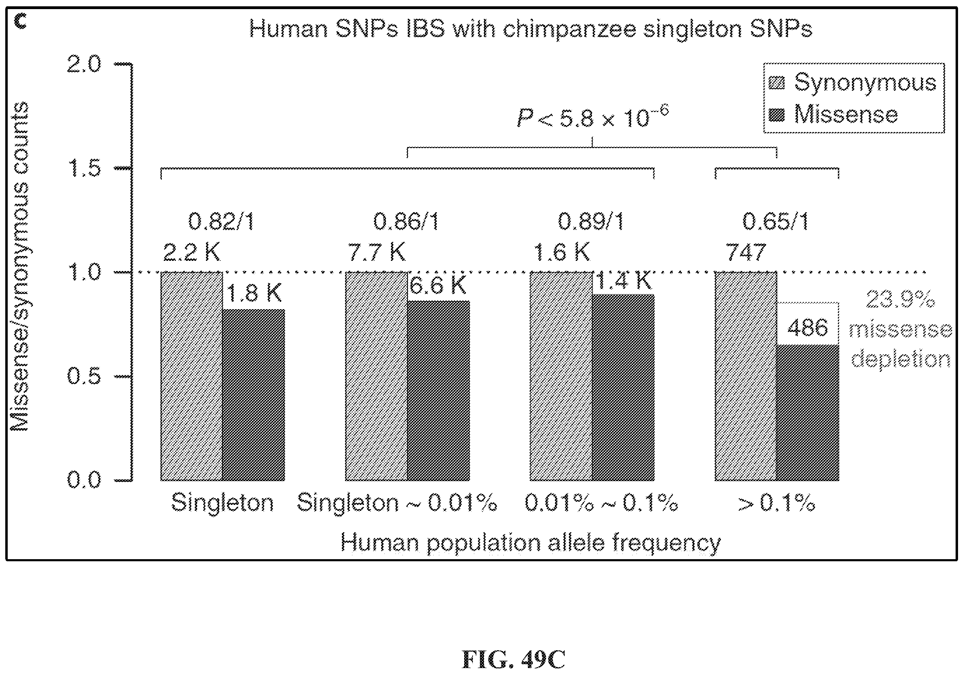

In contrast, for singleton chimpanzee variants (sampled only once in the cohort), we observe a significant decrease in the missense/synonymous ratio at common allele frequencies (P < 5.8 × 10⁻⁶; **FIG. 49C),** indicating that 24% of singleton chimpanzee missense variants would be filtered by purifying selection in human populations at allele frequencies greater than 0.1%. This depletion indicates that a significant fraction of the chimpanzee singleton variants are rare deleterious mutations whose damaging effects on fitness have prevented them from reaching common allele frequencies in either species. We estimate that only 69% of singleton variants are common (AF > 0.1%) in the general chimpanzee population.

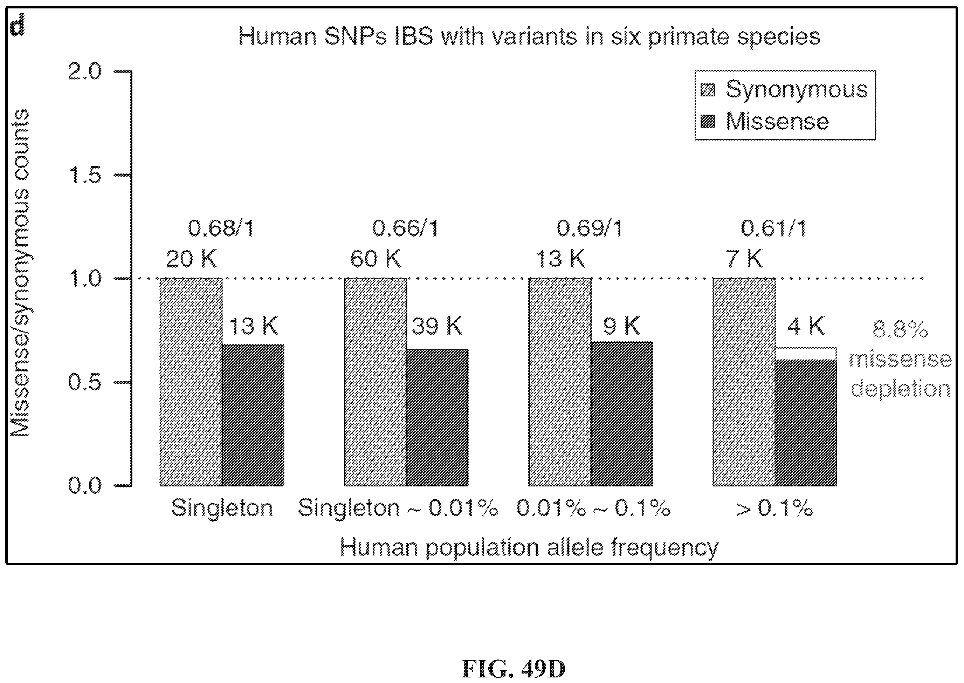

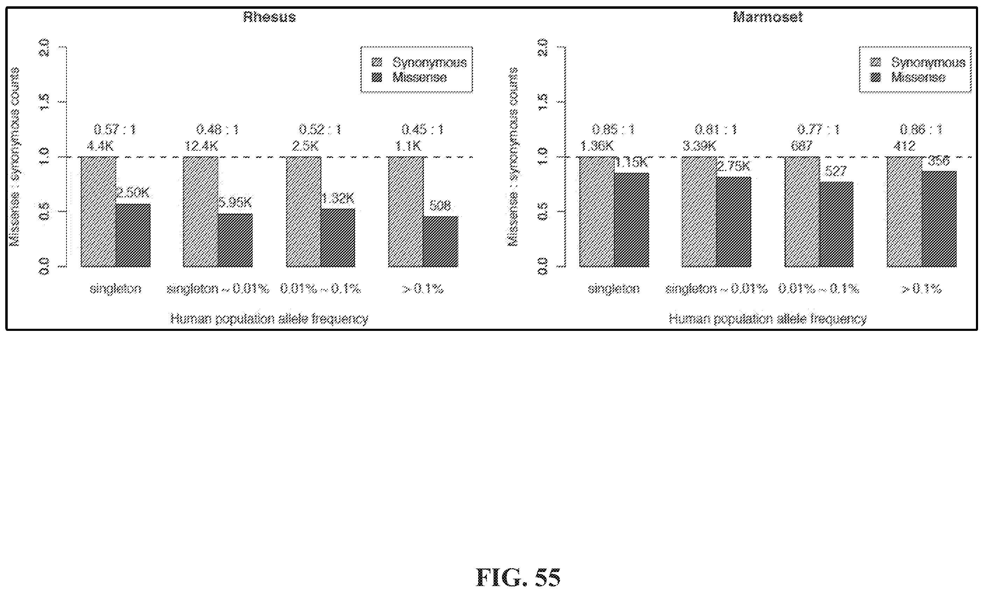

We next identified human variants that are identical-by-state with variation observed in at least one of six non-human primate species. Variation in each of the six species was ascertained from either the great ape genome project (chimp, bonobo, gorilla, and orangutan) or were submitted to the Single Nucleotide Polymorphism Database (dbSNP) from the primate genome projects (thesus, marmoset), and largely represent common variants based on the limited number of individuals sequenced and the low missense:synonymous ratios observed for each species (Supplementary Table 1). Similar to chimpanzee, we found that the missense/synonymous ratios for variants from the six non-human primate species are roughly equal across the human allele frequency spectrum, other than a mild depletion of missense variation at common allele frequencies **(FIG. 49D, FIGs. 53, 34,** and **55,** Supplementary Data File 1), which is expected due to the inclusion of a minority of rare variants (~16% with under 0.1% allele frequency in chimpanzee, and less in other species due to fewer individuals sequenced). These results suggest that the selection coefficients on identical-by-state missense variants are concordant within the primate lineage at least out to New World monkeys, which are estimated to have diverged from the human ancestral lineage -35 million years ago.

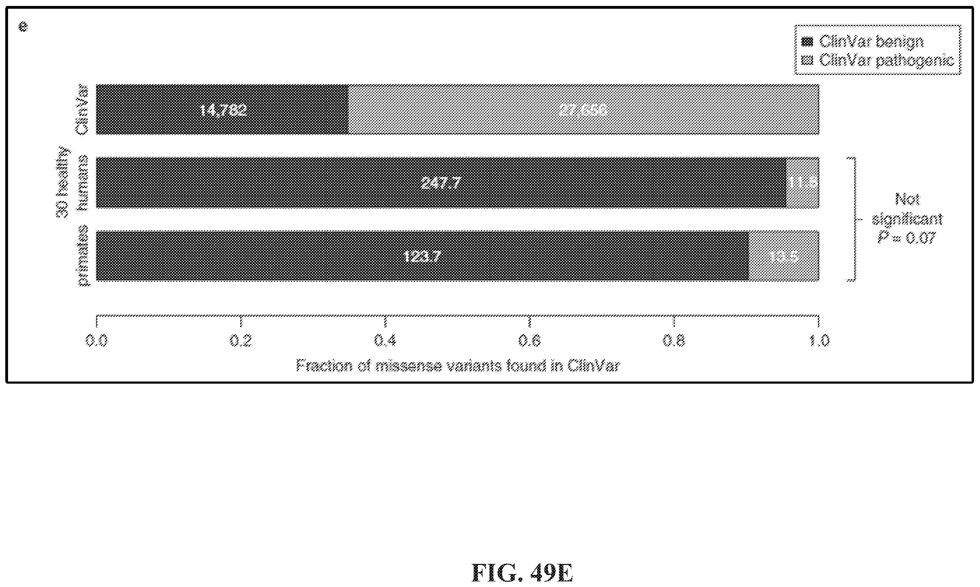

We found that human missense variants that are identical-by-state with observed primate variants are strongly enriched for benign consequence in the ClinVar database. After excluding variants of uncertain significance and those with conflicting annotations, ClinVar variants that are present in at least one non-human primate species are annotated as benign or likely benign 90% of the time on average, compared to 35% for ClinVar missense variants in general (P < 10⁻⁴⁰; **FIG. 49E).** The pathogenicity of ClinVar annotations for primate variants is slightly greater than that observed from sampling a similarly sized cohort of healthy humans (~95% benign or likely benign consequence, P = 0.07) excluding human variants with greater than 1% allele frequency to reduce curation bias.

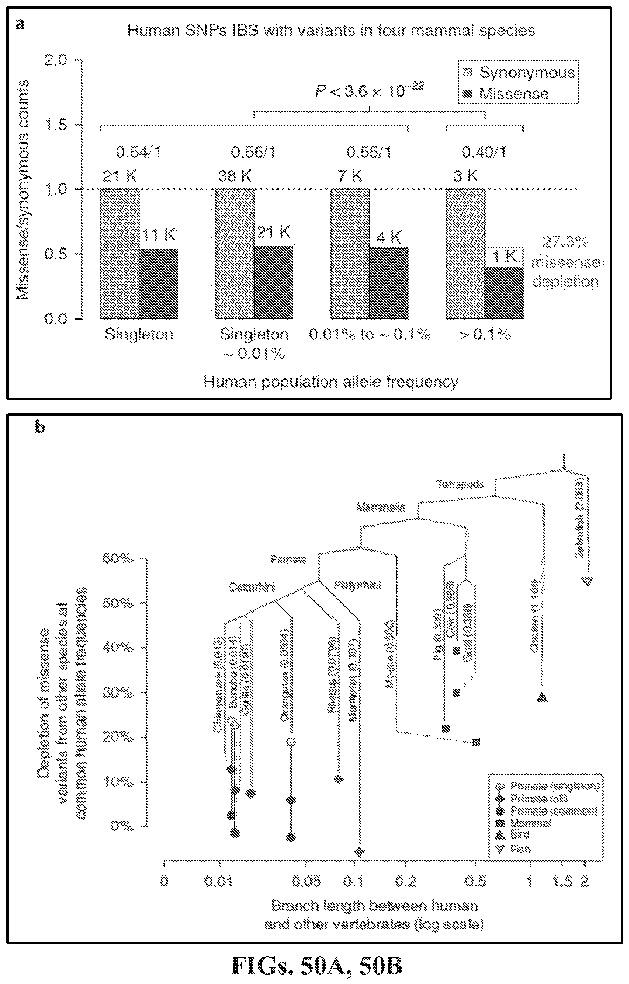

The field of human genetics has long relied on model organisms to infer the clinical impact of human mutations, but the long evolutionary distance to most genetically tractable animal models raises concerns about the extent to which findings on model organisms are generalizable back to human, We extended our analysis beyond the primate lineage to include largely common variation from four additional mammalian species (mouse, pig, goat, and cow) and two species of more distant vertebrates (chicken and zebrafish). We selected species with sufficient genome-wide ascertainment of variation in the dbSNP, and confirmed that these are largely common variants, based on missense/synonymous ratios being much lower than 2.2/1. In contrast to our primite analyses, human missense mutations that are identical-by-state with variation in more distant species are markedly depleted at common allele frequencies **(FIG. 50A),** and the magnitude of this depletion increases at longer evolutionary distances **(FIG. 50B** and Supplementary Tables 2 and 3).

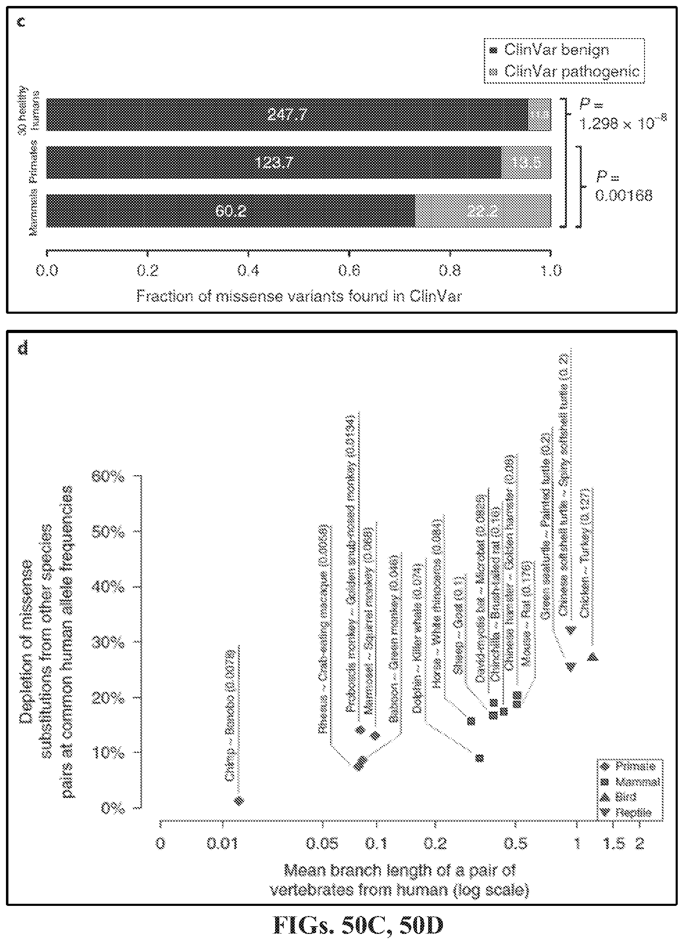

The missense mutations that are deleterious in human, yet tolerated at high allele frequencies in more distant species, indicate that the coefficients of selection for identical-by-state missense mutations have diverged substantially between humans and more distant species. Nonetheless, the presence of a missense variant in more distant mammals still increases the likelihood of benign consequence, as the fraction of missense variants depleted by natural selection at common allele frequencies is less than the ~50% depletion observed for human missense variants in general **(FIG. 49A).** Consistent with these results, we found that ClinVar missense variants that have been observed in mouse, pig, goat, and cow are 73% likely to be annotated with benign or likely benign consequence, compared to 90% the primate variation (P < 2 × 10⁻⁸; **FIG. 50C),** and 35% for the ClinVar database overall.

To confirm that evolutionary distance, and not domestication artifact, is the primary driving force for the divergence of the selection coefficients, we repeated the analysis using fixed substitutions between pairs of closely related species in lieu of intra-species polymorphisms across a broad range of evolutionary distances (FIG. 50D, Supplementary Table 4 and Supplementary Data File 2). We found that the depletion of human missense variants thar are identical-by-state with inter-species fixed substitutions increases with evolutionary branch length, with no discernable difference for wild species compared to those exposed to domestication. This concurs with earlier work in fly and yeast, which found that the number of identical-by-state fixed missense substitutions was lower than expected by chance in divergent lineages.

**Deep Learning Network for Variant Pathogenicity Classification**

The technology disclosed provides a deep learning network for variant pathogenicity classification. The importance of variant classification for clinical applications has inspired numerous attempts to use supervised machine learning to address the problem, but these efforts have been hindered by the lack of an adequately sized truth dataset containing confidently labeled benign and pathogenic variants for training.

Existing databases of human expert curated variants do not represent the entire genome, with ~50% of the variants in the ClinVar database coming from only 200 genes (~1% of human protein-coding genes). Moreover, systematic studies identify that many human expert annotations have questionable supporting evidence, underscoring the difficulty of interpreting rare variants that may be observed in only a single patient. Although human expert interpretation has become increasingly ngorous, classification guidelines are largely formulated around consensus practices and are at risk of reinforcing existing tendencies. To reduce human interpretation biases. recent classifiers have been trained on common human polymorphisms or fixed human-chimpanzee substitutions, but these classifiers also use as their input the prediction scores of earlier classifiers that were trained on human curated databases. Objective benchmarking of the performance of theft, various methods haft been elusive in the absence of an independent, bias-free truth dataset.

Variation from the six non-human primates (chimpanzee, bonobo, gorilla, orangutan, rhesus, and marmoset) contributes over 300,000 unique missense **variants that are** non-overlapping with common human variation, and largely **represent** common variants of benign consequence that have been through the sieve of purifying selection, greatly enlarging the training dataset available for machine learning approaches. On average, each primate species contributes more variants than the whole of the ClinVar database (~42,000 missense variants as of November 2017, after excluding variants of uncertain significance and those with conflicting annotations). Additionally, this content is free from biases in human interpretation.

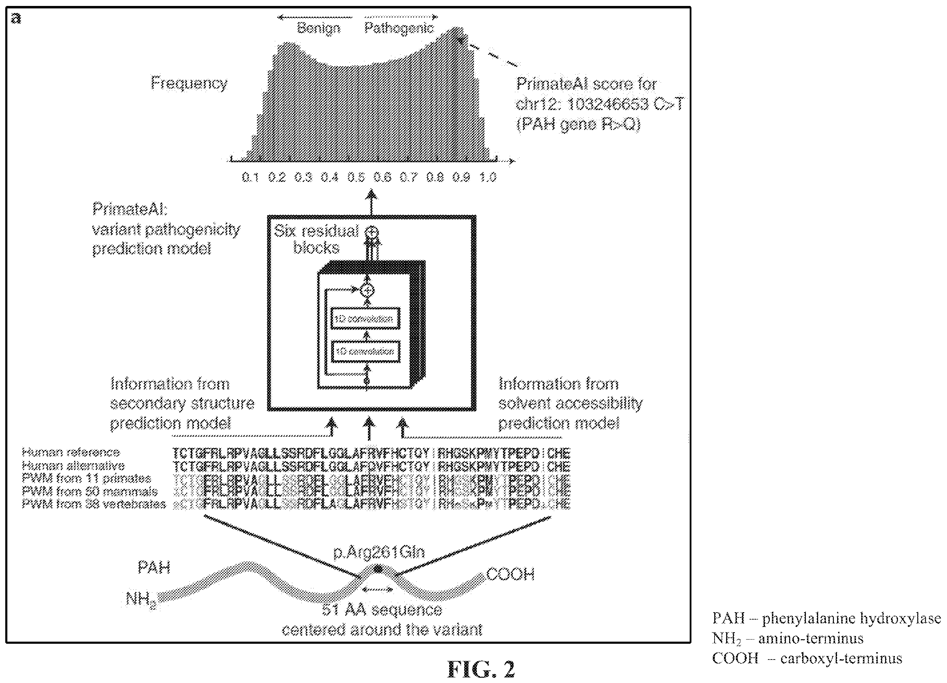

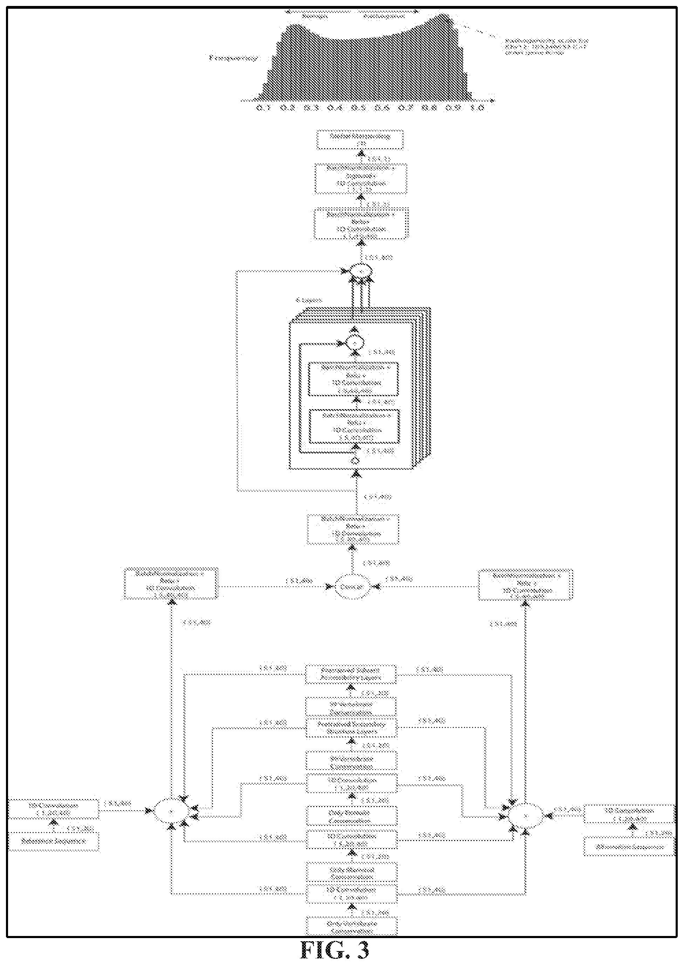

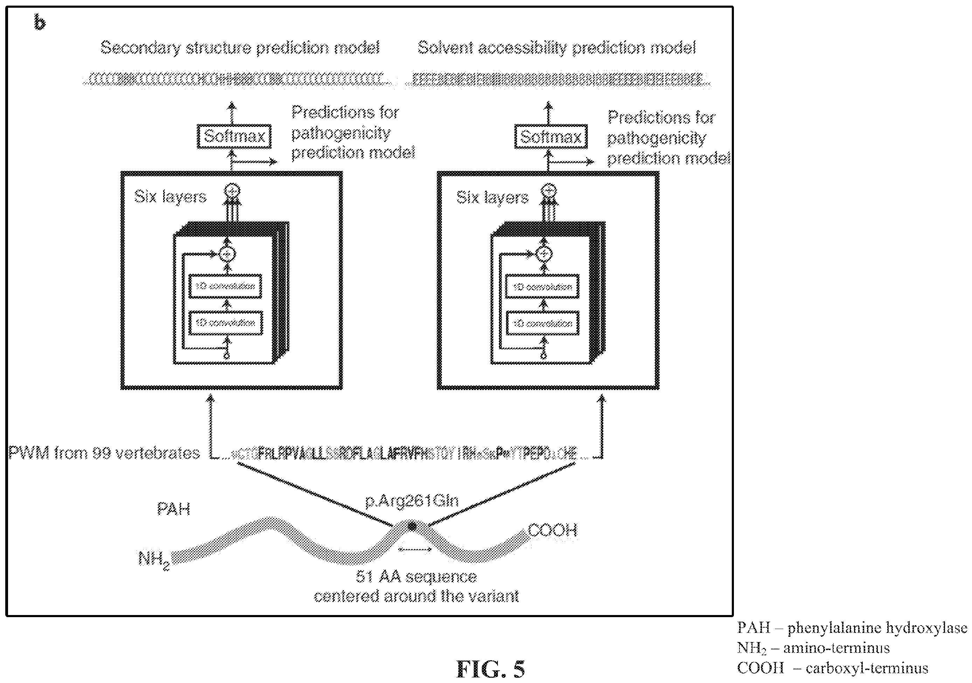

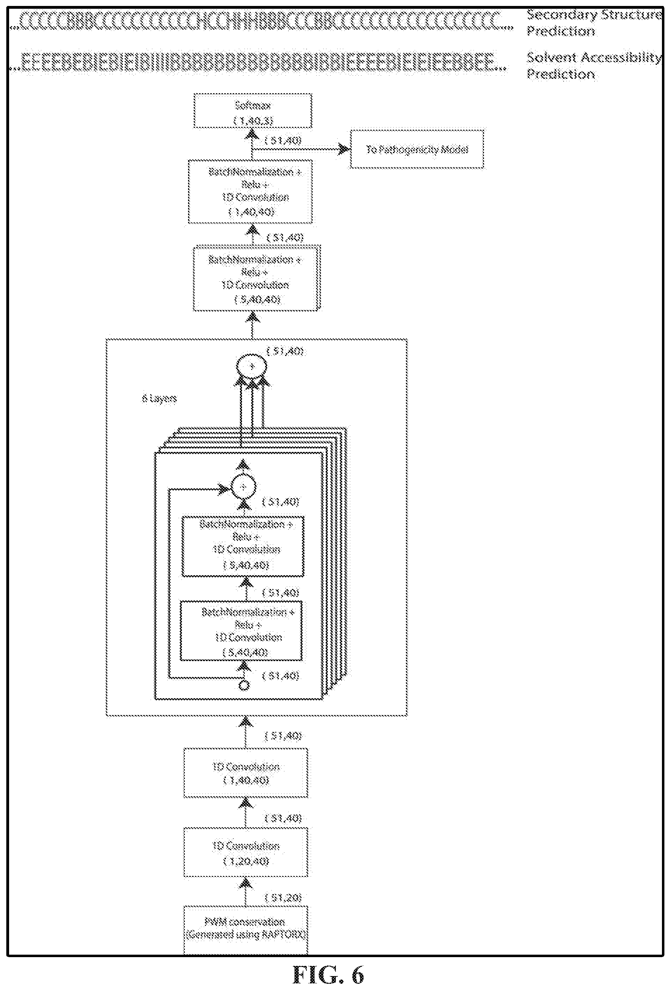

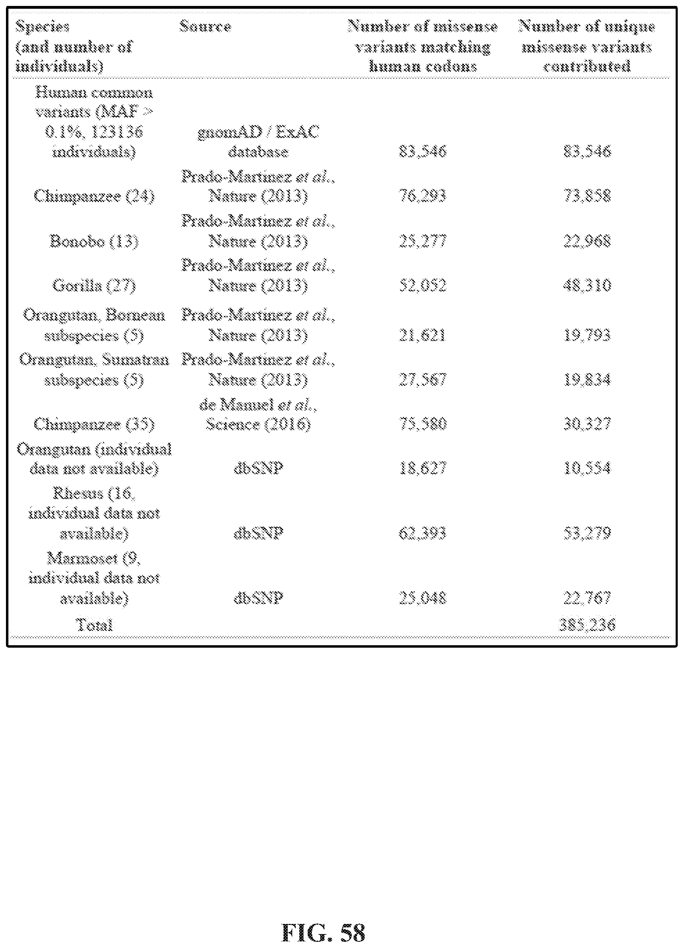

Using a dataset comprising common human variants (AF>0.1%) and primate variation (Supplementary Table 5 **(FIG. 58),** we trained a novel deep residual network. PrimateAI which takes as input the amino acid sequence flanking the variant of interest and the orthologous sequence alignments in other species **(FIG. 2** and **FIG.** 3). Unlike existing classifiers that employ human-engineered features, our deep learning network learns to extract features directly from the primary sequence. To incorporate information about protein structure, we trained separate networks to predict the secondary structure and solvent accessibility from the sequence alone, and then included these as subnetworks in the full model **(FIG. 5** and **FIG. 6).** Given the small number of human proteins that have been successfully crystallized, inferring structure from the primary sequence has the advantage of avoiding biases due to incomplete protein structure and functional domain annotation. The total depth of the network, with protein structure included, was 36 layers of convolutions, comprising roughly 400,000 trainable parameters.

To train a classifier using only variants with benign labels, we framed the prediction problem as whether a given mutation is likely to be observed as a common variant in the population. Several factors influence the probability of observing a variant at high allele frequencies, of which we are interested only in deleteriousness; other factors include mutation rate, technical arti facts such as sequencing coverage, and factors impacting neutral genetic drift such as gene conversion.

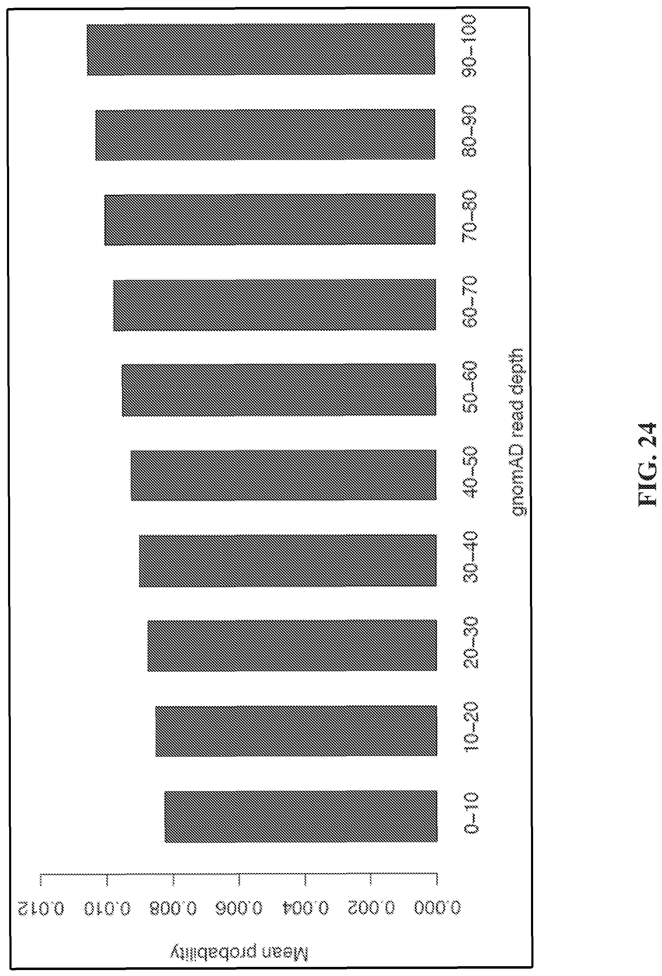

We matched each variant in the benign training set with a missense mutation that was absent in 123,136 exomes from the ExAC database, controlling for each of these confounding factors, and trained the deep learning network to distinguish between benign variants and matched controls **(FIG. 24).** As the number of unlabeled variants greatly exceeds the size of the labeled benign training dataset, we trained eight networks in parallel, each using a different set of unlabeled variants matched to the benign training dataset, to obtain a consensus prediction.

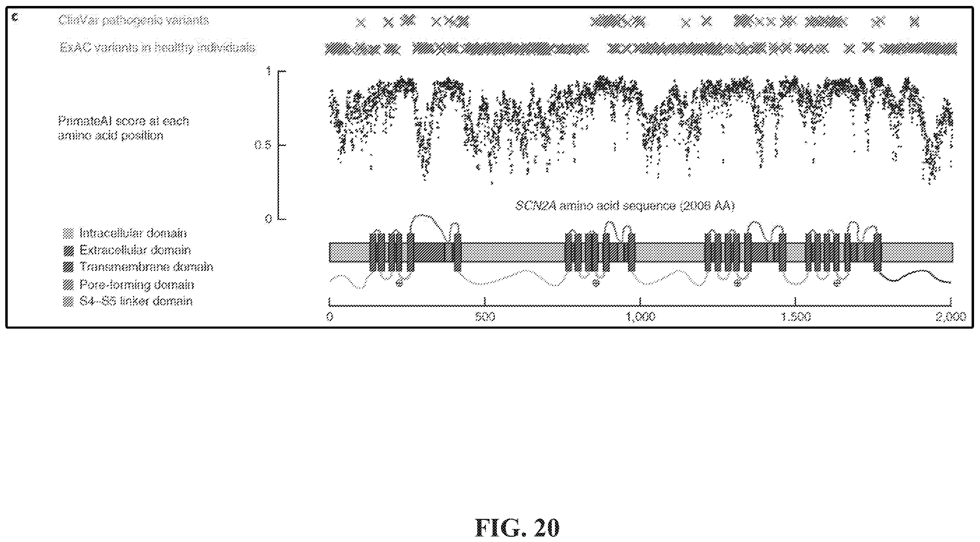

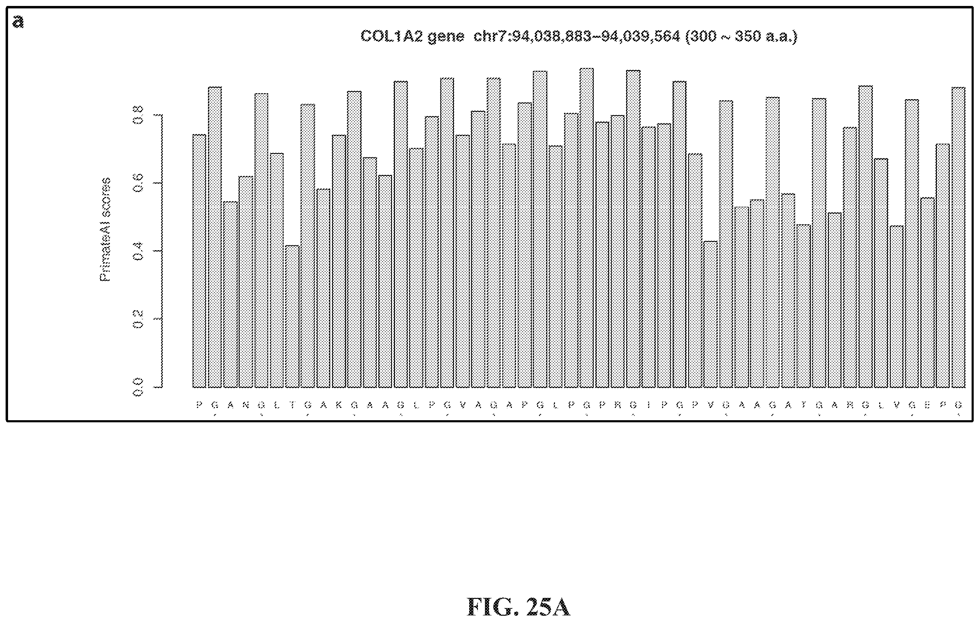

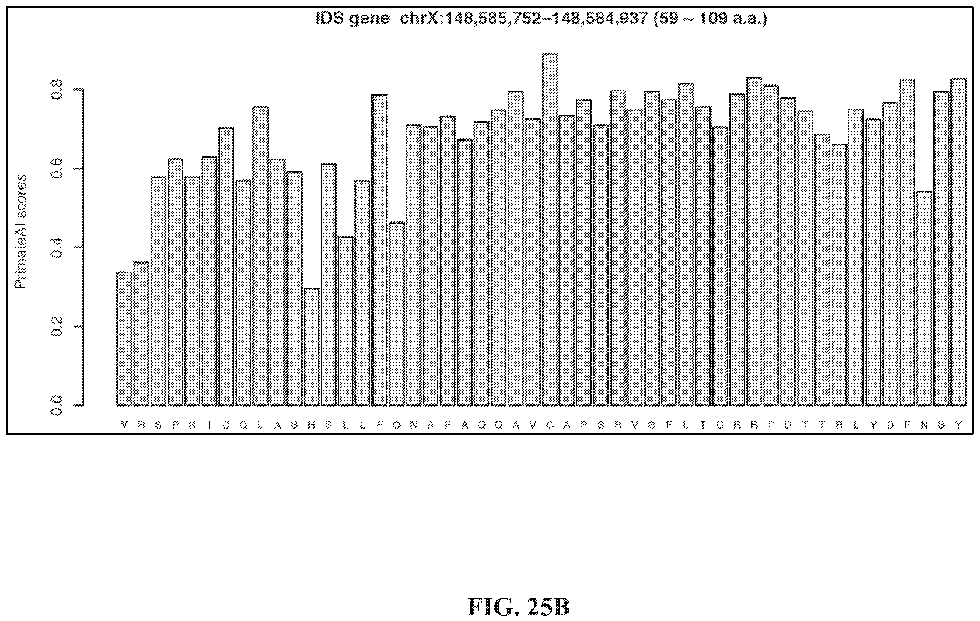

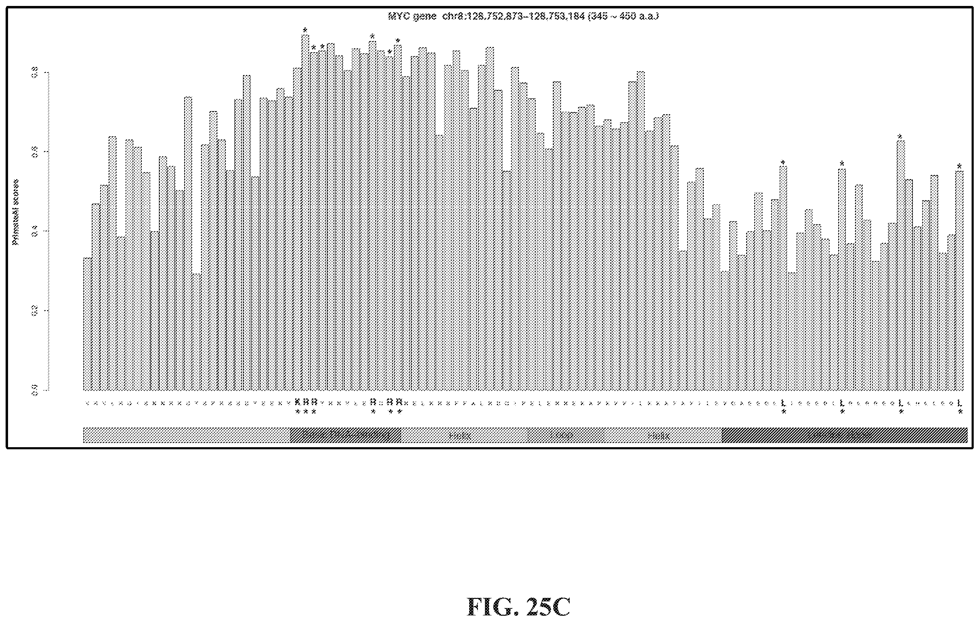

Using only the primary amino acid sequence as its input, the deep learning network accurately assigns high pathogenicity scores to residues at useful protein functional domains, as shown for the voltage-gated sodium channel SCN2A **(FIG. 20),** a major disease gene in epilepsy, autism, and intellectual disability. The structure of SCN2A comprises four homologous repeats. each containing six transmembrane helixes (S1-S6). On membrane depolarization, the positively charged S4 transmembrane helix moves towards the extracellular side of the membrane, causing the S5/S6 pore-forming domains to open via the S4- S5 linker. Mutations in the S4, S4-S5 linker, and S5 domains, which are clinically associated with early onset epileptic encephalopathy, are predicted by the network to have the highest pathogenicity scores in the gene, and are depleted for variants in the healthy population (Supplementary Table 6). We also found that the network recognizes important amino acid positions within domains, and assigns the highest pathogenicity scores to mutations at these positions, such as the DNA-contacting residues of transcription factors and the catalytic residues of enzymes **(FIGs. 25A, 25B, 25C.** and **26).**

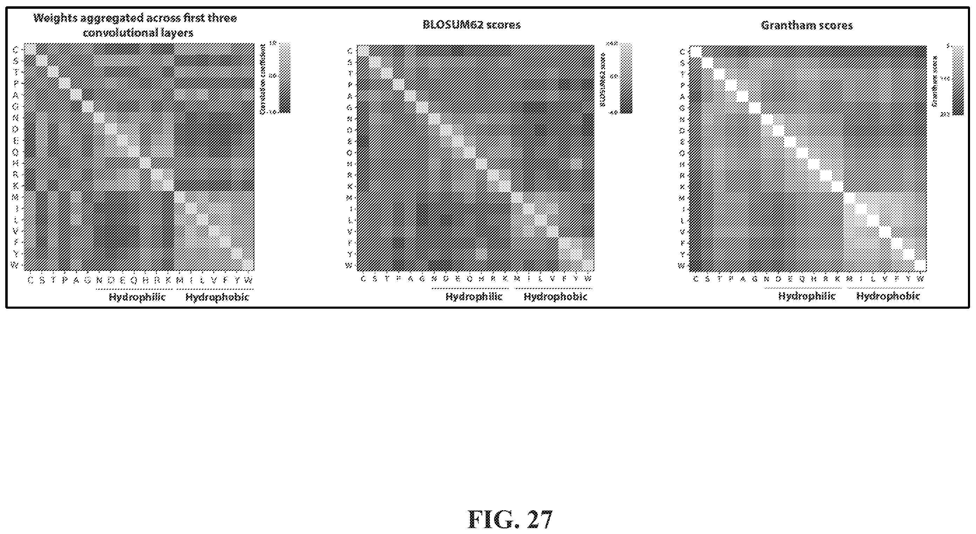

To better understand how the deep learning network derives insights into protein structure and function from the primary sequence, we visualized the trainable parameters from the first three layers of the network. Within these layers, we observed that the network learns correlations between the weights of different amino acids that approximate existing measurements of amino acid distance such as Grantham score **(FIG. 27).** The outputs of these initial layers become the inputs for later layers, enabling the deep learning network to construct progressively higher order representations of the data.

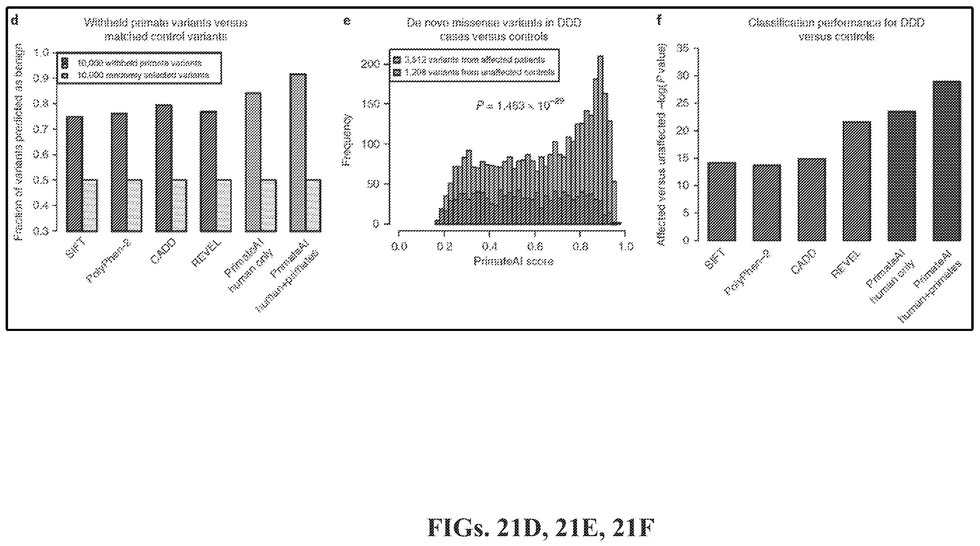

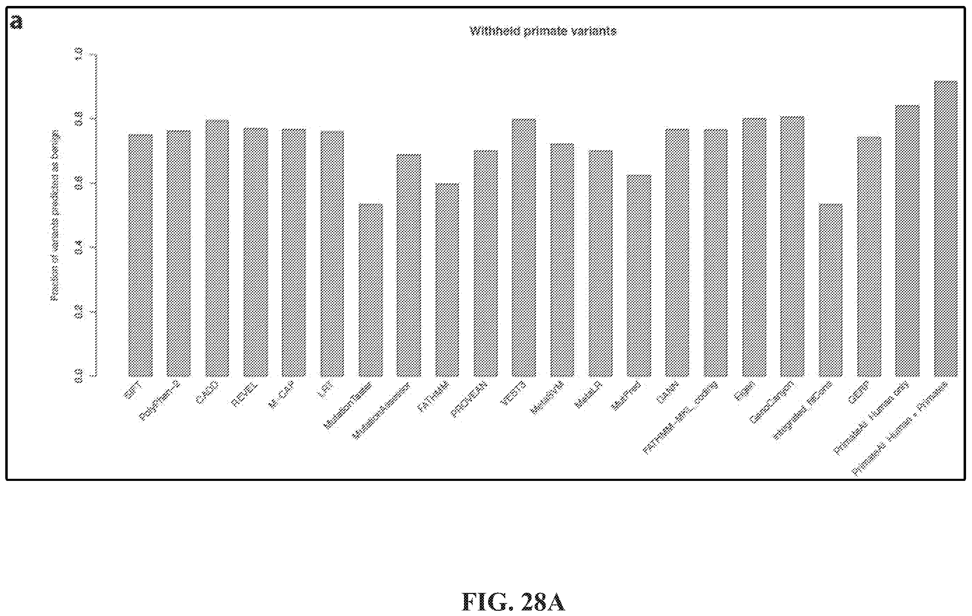

We compared the performance of our network with existing classification algorithms, using 10,000 common primate variants that were withheld from training. Because -50% of all newly arising human missense vanants are filtered by purifying selection at common allele frequencies **(FIG. 49A),** we determined the 50th-percentile score for each classifier on a set of 10,000 randomly selected variants that were matched to the 10,000 common primate variants by mutational rate and sequencing coverage, and evaluated the accuracy of each classifier at that threshold **(FIG. 21D, FIG. 28A.** and Supplementary Data File 4). Our deep learning network (91% accuracy) surpassed the performance of other classifiers (80% accuracy for the next best model) at assigning benign consequence to the 10,000 withheld common primate variants.

Roughly half the improvement over existing methods comes from using the deep learning network and half comes from augmenting the training dataset with primate variation, as compared to the accuracy of the network trained with human variation data only **(FIG. 21D).** To test the classification of variants of uncertain significance in a clinical scenario, we evaluated the ability of the deep learning network to distinguish between *de novo* mutations occurring in patients with neurodevelopmental disorders versus healthy controls. By prevalence, neurodevelopmental disorders constitute one of the largest categories of rare genetic diseases, and recent trio sequencing studies have implicated the central role of *de novo* missense and protein truncating mutations.

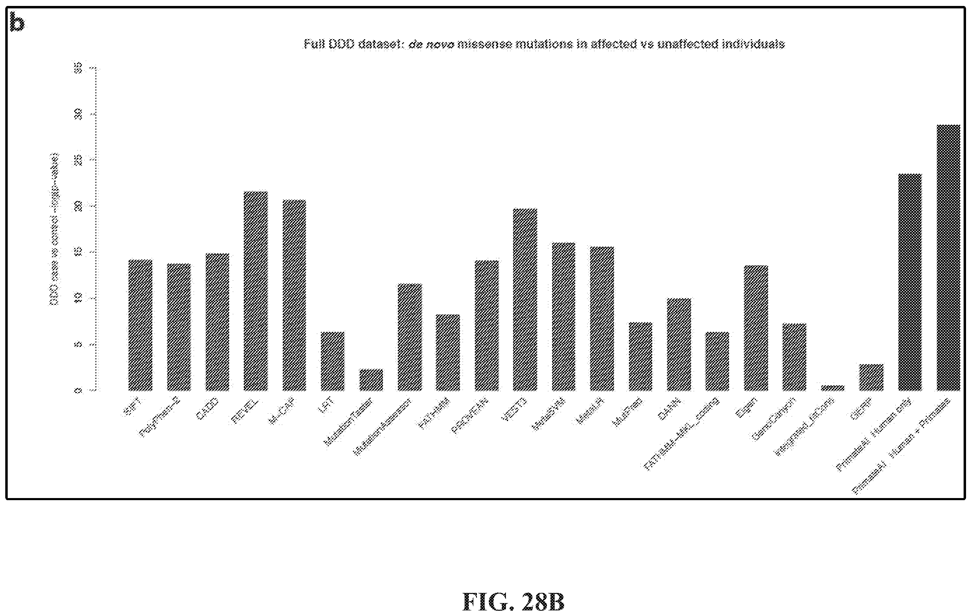

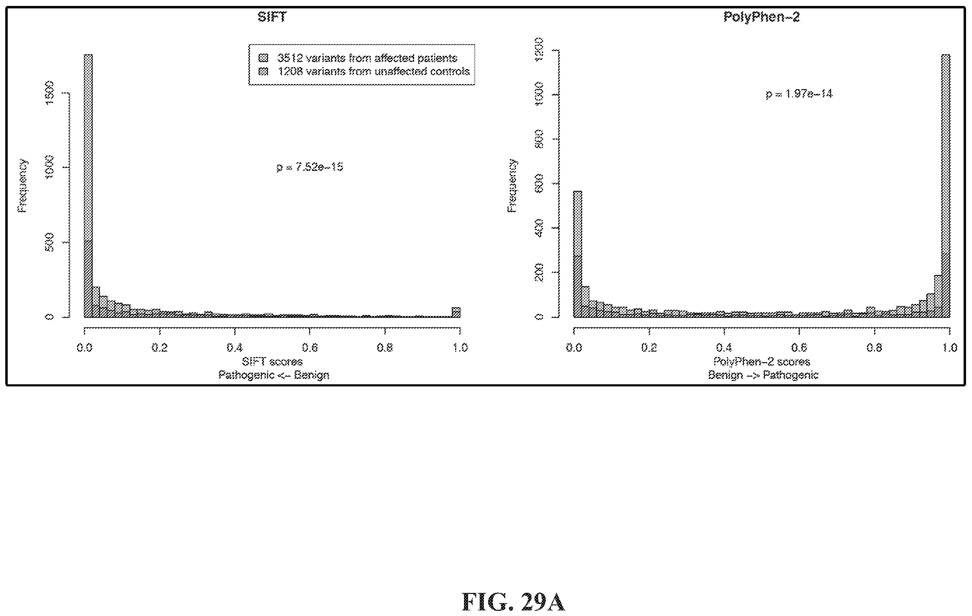

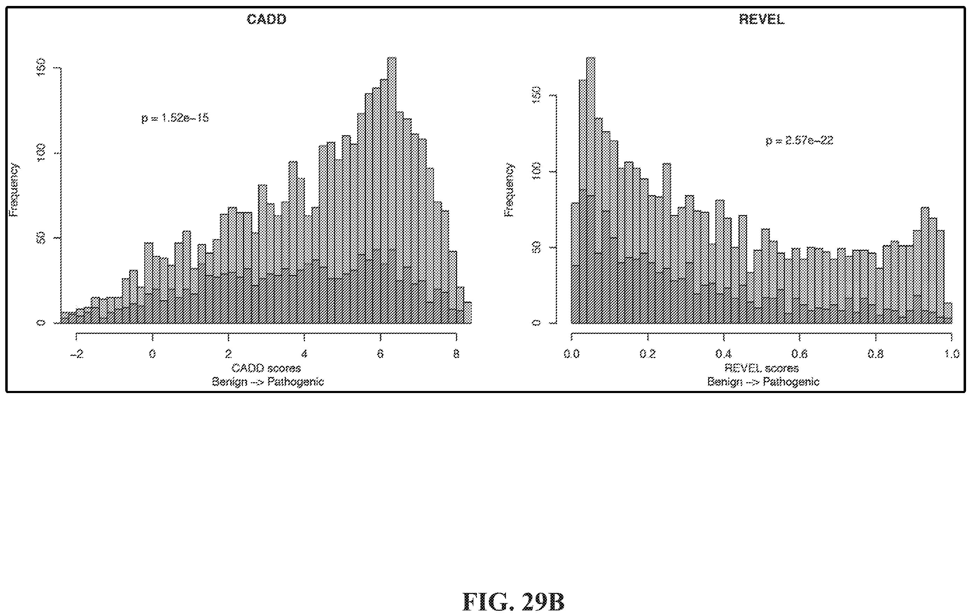

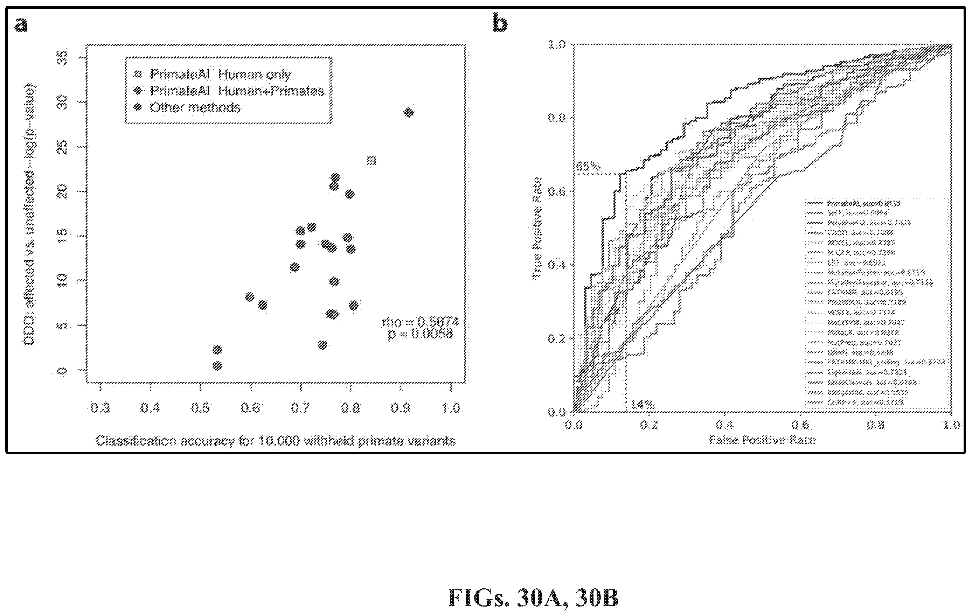

We classified each confidently called *de novo* missense variant in 4.293 affected individuals from the Deciphering Developmental Disorders (DDD) cohort versus *de novo* missense variants from 2,517 unaffected siblings in the Simon's Simplex Collection (SSC) cohort, and assessed the difference in prediction scores between the two distributions with the Wilcoxon rank-sum test **(FIG. 21E** and **FIGs. 29A** and **29B).** The deep learning network clearly outperforms other classifiers (In this task ($P<10^{-28}$; **FIG. 21F** and **FIG. 28B).** Moreover, the performance of the various classifiers on the withheld primate variant dataset and the DDD cases versus controls dataset was correlated (Spearman p = 0.57, P<0.01), indicating good agreement between the two datasets for evaluating pathogenicity, despite using entirely different sources and methodologies **(FIG. 30A).**

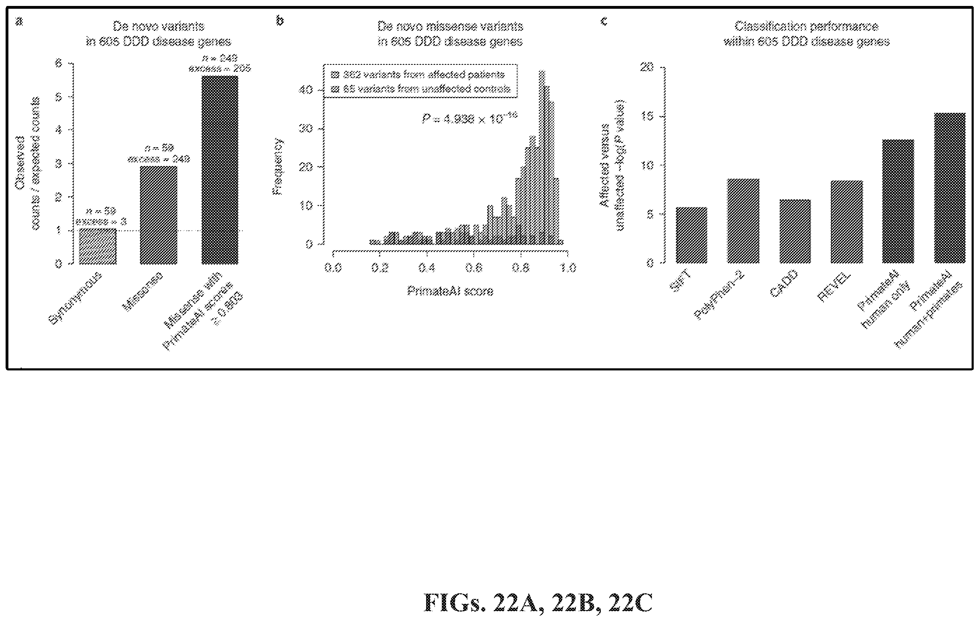

We next naught to estimate the accuracy of the deep learning network at classifying benign versus pathogenic mutations within the same gene. Given that the DDD population largely comprises index cases of affected children without affected first degree relatives, it is important to show that the classifier has not inflated its accuracy by favoring pathogenicity in genes with *de novo* dominanant modes of inheritance. We restricted the analysis to 605 genes that were nominally significant for disease association in the DDD study, calculated from protein-truncating variation only (P<0.05). Within these genes, *de novo* missense mutations are enriched 3/1 compared to expectation **(FIG. 22A),** indicating that -67% are pathogenic.

The deep learning network was able to discriminate pathogenic and benign *de novo* variants within the same set of genes ($P< 10^{-15}$; **FIG. 22B),** outperforming other methods by a large margin **(FIGs, 22C** and **28C).** At a binary cutoff of $\geq$ 0.803 **(FIGs, 22D** and **30B),** 65% of *de novo* missense mutations in cases are classified by the deep learning network as pathogenic, compared to 14% of *de novo* missense mutations in controls, corresponding to a classification accuracy of 88% (F**IGs. 22E** and **30C).** Given frequent incomplete penetrance and variable expressivity in neurodevelopmental disorders, this figure probably underestimates the accuracy of our classifier due to the inclusion of partially penetrant pathogenic variants in controls.

## Novel Candidate Gene Discovery

Applying a threshold of $\geq$ 0.803 to stratify pathogenic missense mutations increases the enrichment of *de* novo missense mutations in DDD patients from 1.5-fold to 2.2-fold, close to protein-truncating mutations (2.5-told), while relinquishing less than one-third of the total number of variants enriched above expectation. This substantially improves statistical power, enabling discovery of 14 additional candidate genes in **intellectual** disability, which had previously not reached the genome-wide significance threshold in the original DDD study (Table 1).

## Comparison with Human Expert Curation

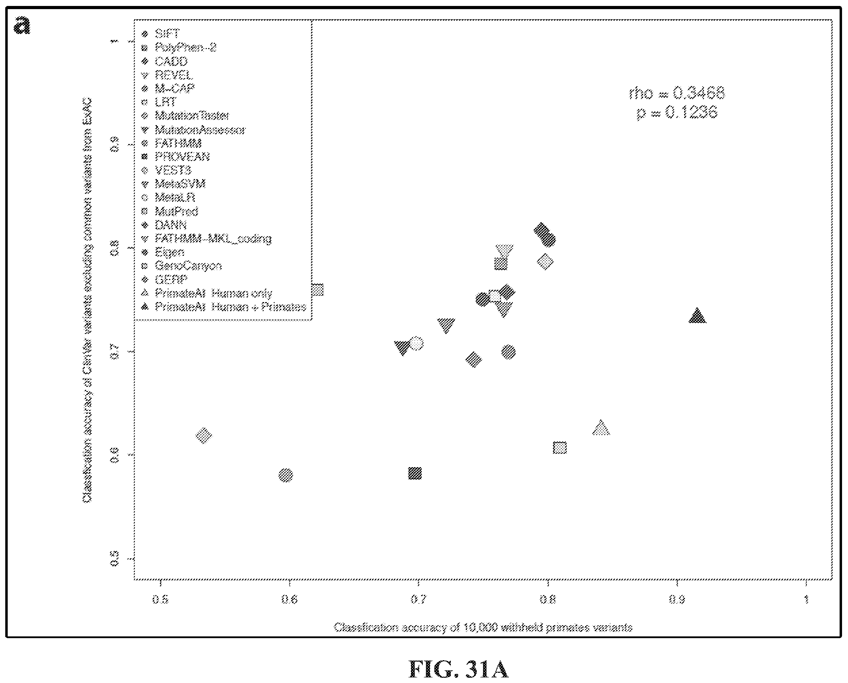

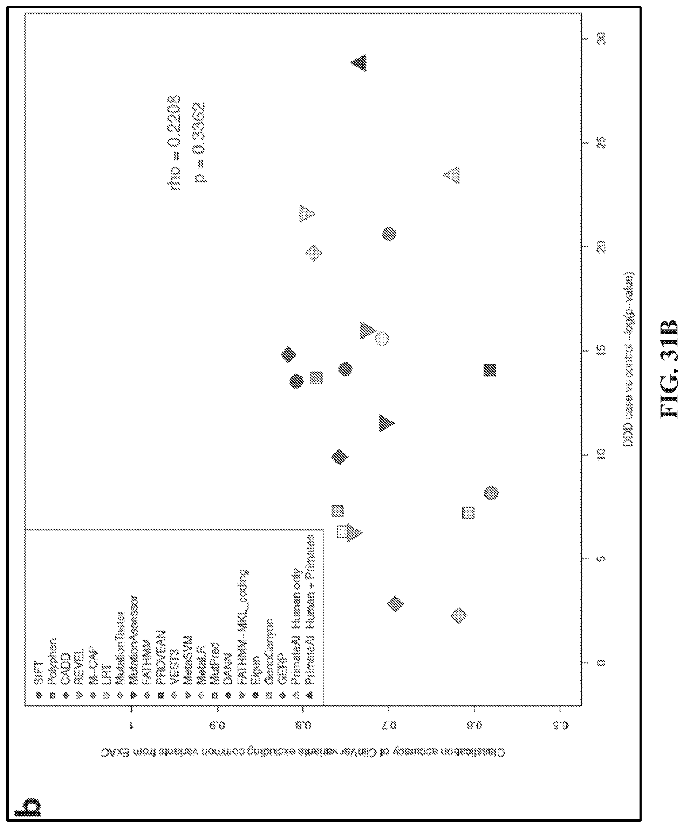

We examined the performance of various classifiers on recent human expert-curated variants from the ClinVar database, but found that the performance of classifiers on the ClinVar dataset was not significantly correlated with either the withheld primate variant dataset or the DDD case versus control dataset (P = 0.12 and P = 0.34, respectively) **(FIGs. 31A** and **31B).** We hypothesize that existing classifiers have biases from human expert civation, and while these human heuristics tend to be in the right direction, they may not be optimal. One example is the mean difference in Grantham score between pathogenic and benign variants in ClinVar, which is twice as large as the difference between *de novo* variants in DDD cases versus controls within the 605 disease-associated genes (Table 2). In comparison, human expert curation appears to underutilize protein structure, especially the importance of the residue being exposed at the surface where it can be available to interact with other molecules. We observe that both ClinVar pathogenic mutations and DDD *de* novo mutations are associated with predicted solvent-exposed residues, but that the difference in solvent accessibility between benign and pathogenic ClinVar variants is only half that seen for DDD cases versus controls. These findings are suggestive of ascertainment bias in favor of factors that are more straightforward for a human expert to interpret, such as Grantham score and conservation. Machine learning classifiers trained on human curated databases would be expected to reinforce these tendencies.

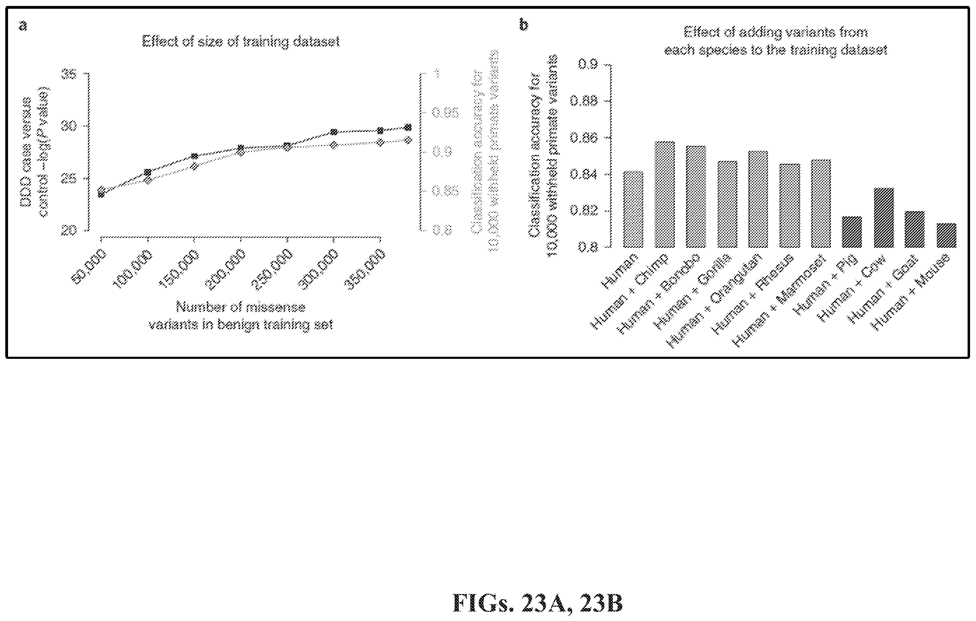

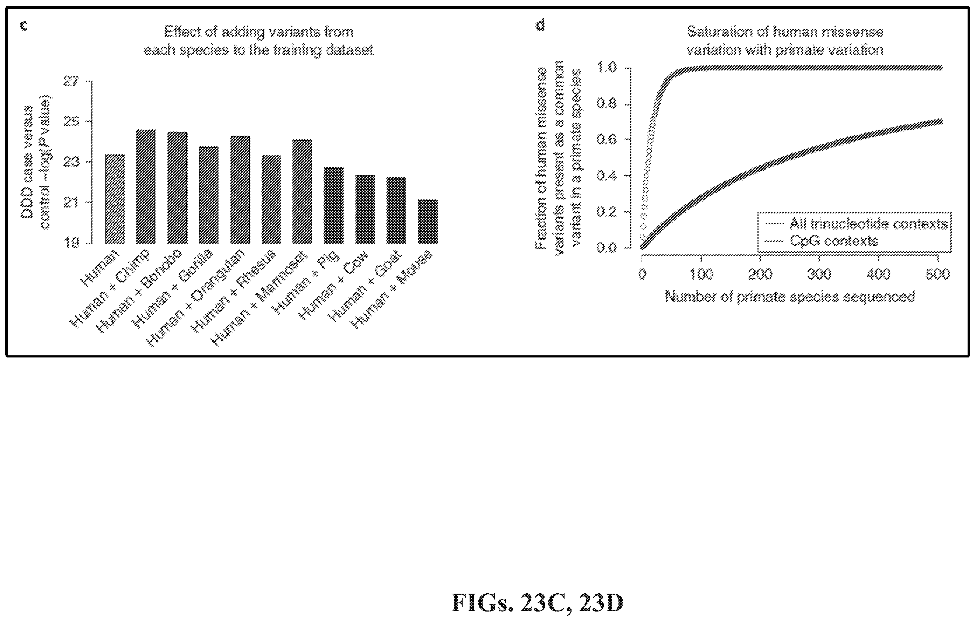

Out results suggest that systematic primate population sequencing is an effective strategy to classify the millions or human variants of uncertain significance that currently limit clinical genome interpretation, The accuracy of our deep learning network on both withheld common primate variants and clinical variants increases with the number of benign variants used to train the network **(FIG. 23A).** Moreover, training on variants from each of the six non-human primate species independently contributes to an increase in the performance of the network, whereas training on variants from more distant mammals negatively impacts the performance of the network **(FIGs. 23B** and **23C).** These results support the assertion that common primate variants are largely benign in human with respect to penetrant Mendelian disease, while the same cannot be said of variation in more distant species.

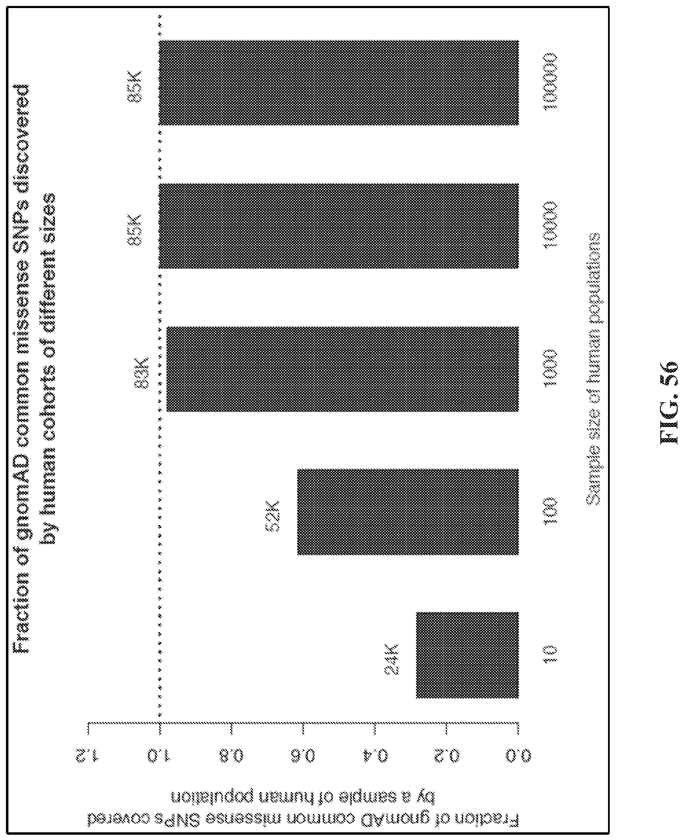

Although the number of non-human primate genomes examined in this study is small compared to the number of human genomes and exomes that have been sequenced, it is important to note that these additional primates contribute a disporhonate amount of information about common benign variation. Simulations with Ex AC show that discovery of common human variants (> 0.1% allele frequency) platesus quick ly after only a few hundred individuals **(FIG. 56),** and further healthy population sequencing into the millions mainly contributes additional rare variants. Unlike common variants, which are known to be largely clinically benign based on allele frequency, rare variants in healthy populations may cause recessive genetic diseases or dominant genetic diseases with incomplete penetrance. Because each primate species carries a different pool of common variants, sequencing several dozen members of each species is an effective strategy to systematically catalog benign missense variation in the primate lineage. Indeed, the 134 individuals from six non-human primate species examined in this study contribute nearly four times as many common missense variants as the 123,136 humans from the Ex AC study (Supplementary Table 5 **(FIG. 58)).** Primate population sequencing studies involving hundreds of individuals may be practical even with the relatively small numbers of unrelated individuals residing is wildlife sanctuaries and zoos, thus minimizing the disturbance to wild populations, which is important from the standpoint of conservation and ethical treatment of non-human primates.

Present-day human populations carry much lower genetic diversity than most non-human primate species, with roughly half the number of single nucleotide variants per individual as chimpanzee, gori Ila, and gibbon, and one-third as many variants per individual as orangutan. Although genetic diversity levels for the majority of non-hitman primate species are not known, the large number of extant non-hinnan primate species allows us to extrapolate that the majority of possible benign human missense positions are likely to be covered by a common variant in at least one primate species, enabling pathogenic variants to be systematically identified by the process of elimination **(FIG. 23D).** Even with only a subset of these species sequenced, increasing the training data size will enable more accurate prediction of missense consequence with machine learning. Finally, while our findings focus on missense variation, this strategy may also be applicable for inferring the consequences of noncoding variation, particularly in conserved regulatory regions where there is sufficient alignment between human and primate genomes to unambiguously determine whether a variant is identical-by-state.

Of the 504 known non-human primate species, roughly 60% face extinction due to poaching and widespread habitat loss. The reduction in population size and potential extinction of these species represents an irreplaceable loss in genetic diversity, motivating urgency for a worldwide conservation effort that would benefit both these unique and irreplaceable species and our own.

### Data Generation and Alignment

Coordinates in the application refer to human genome build UCSC hg19/GRCh37, including the coordinates for variants in other species mapped to hg19 using multiple sequence alignments. Canonical transcripts for protein-coding DNA sequence and multiple sequence alignments of 99 vertebrate genomes and branch length were downloaded from the UCSC genome browser.

We obtained human exome polymorphism data from the Exome Aggregation Consortium (ExAC)/Genome Aggregation Database (gnomAD exomes) v2.0. We obtained primate variation data from the great ape genome sequencing project, which comprises whole genome sequencing data and genotypes for 24 chimpanzees, 13 bonobos, 27 gorillas, and 10 orangutans. We also included variation from 35 chimpanzees from a separate study of chimpanzee and bonobos, but because of differences in variant calling methodology, we excluded these from the population analysis, and used them only for training the deep learning model. In addition, 16 rhesus individuals and 9 marmoset individuals were used to assay variation in the original genome projects for these species, but individual-level information was not available. We obtained variation data for rhesus, marmoset, pig, cow, goat, mouse, chicken, and zebrafish from dbSNP. dbSNP also included additional orangutan variants, which we only used for training the deep learning model, since individual genotype information was not available for the population analysis. To avoid effects due to balancing selection, we also excluded variants from within the extended major histocompatability complex region (chr6: 28,477,797-33,448,354) for the population analysis.

We used the multiple species alignment of 99 vertebrates to ensure orthologous one-to-one mapping to human protein-coding regions and prevent mapping to pseudogenes. We accepted variants as identical-by-state if they occurred in either reference/alternative orientation. To ensure that the variant had the same predicted protein-coding consequence in both human and the other species, we required that the other two nucleotides in the codon were identical between the species, for both missense and synonymous variants. Polymorphisms from each species included in the analysis are listed in Supplementary Data File 1 and detailed metrics are shown in Supplementary Table 1.

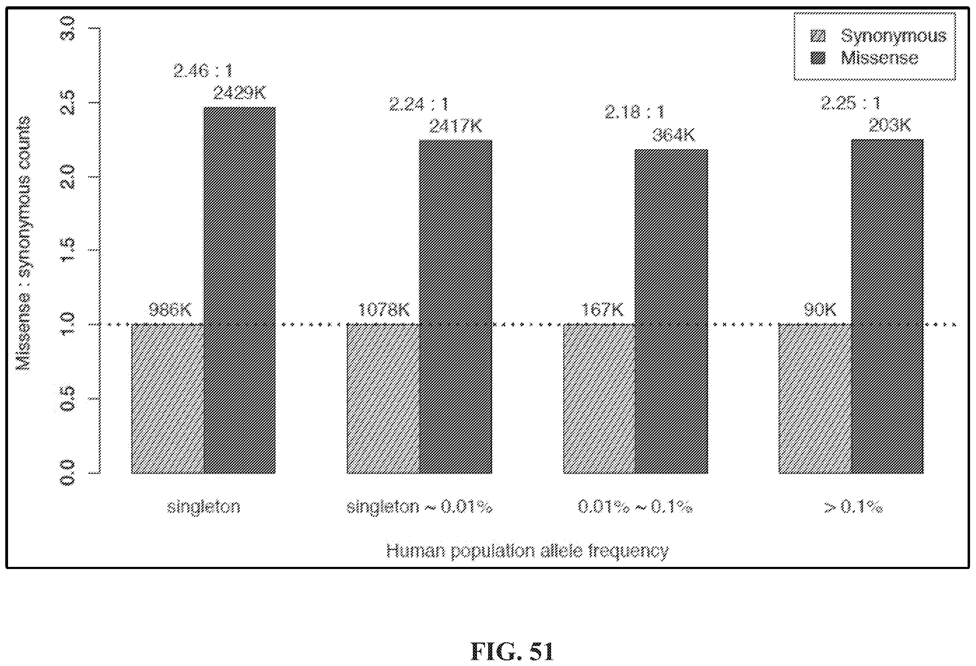

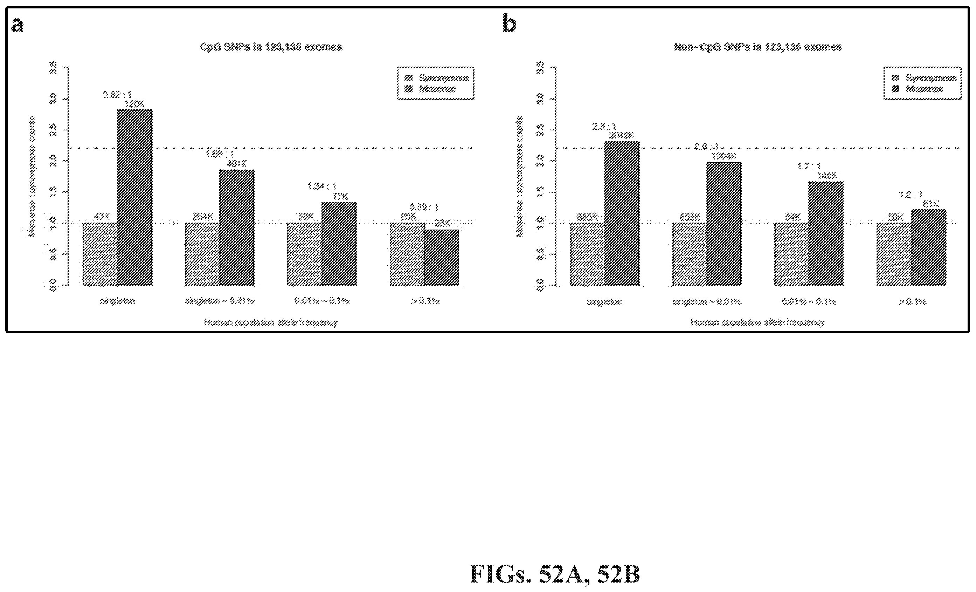

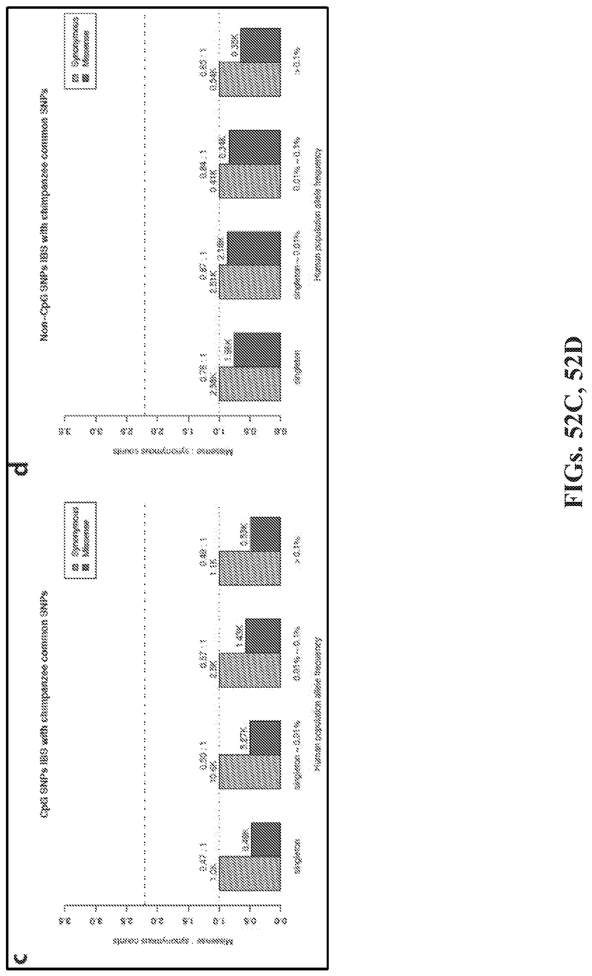

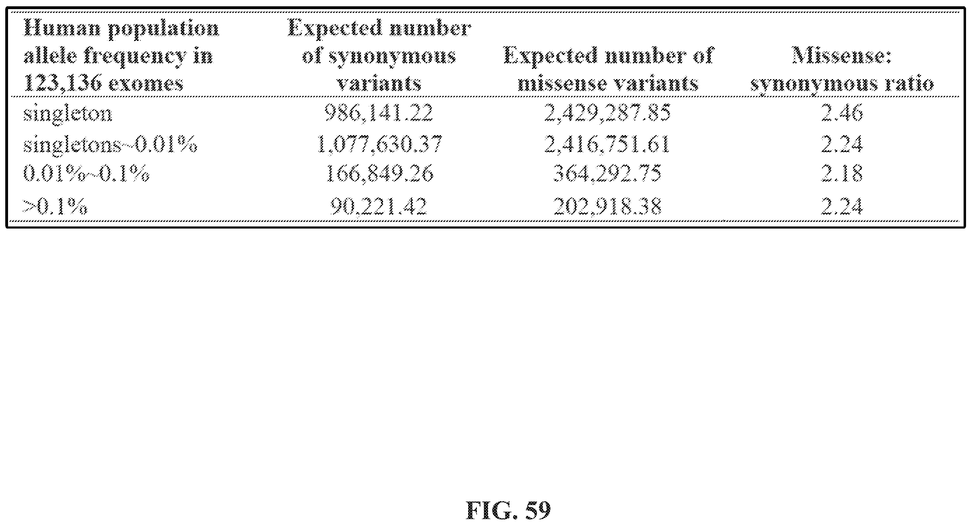

For each of the four allele frequency categories **(FIG. 49A),** we used variation in intronic regions to estimate the expected number of synonymous and missense variants in each of 96 possible trinucleotide contexts and to correct for mutational rate **(FIG. 51** and Supplementary Tables 7, 8 **(FIG. 59).** We also separately analyzed identical-by-state CpG dinucleotide and non-CpG dinucleotide variants, and verified that the missense/synonymous ratio was flat across the allele frequency spectrum for both classes, indicating that our analysis holds for both CpG and non-CpG variants, despite the large difference is their mutation rate **(FIGs. 52A, 52B, 52C,** and **52D).**

## Depletion of Human Missense Variants that are Identical-by-State with Polymorphisms in other Species

To evaluate whether variants present in other species would be tolerated at common allele frequencies (> 0.1%) in human, we identified human variants that were identical-by-state with variation in the other species. For each of the variants, we assigned them to one of the four categories based on their allele frequencies in human populations (singleton, more than singleton ~0.01%, 0.01% to -0.1%. > 0.1%), and estimated the decrease in missense/synonymous ratios (MSR) between the rare (< 0.1%) and common (> 0.1%) variants. The depletion of identical-by-state missense variants at common human allele frequencies (> 0.1%) indicates the fraction of variants from the other species that are sufficiently deleterious that they would be filtered our by natural selection at common allele frequencies in human:

$$\% \text{ depletion} = \frac{\text{MSR}_{rare} - \text{MSR}_{comm}}{\text{MSR}_{rare}}$$

The missense/synonymous ratios and the percentages of depletion are computed per species and are shown in **FIG. 50B** and Supplementary Table 2. In addition, for chimpanzee common variants **(FIG. 49B),** chimpanzee singleton variants **(FIG. 49C),** and mammal variants **(FIG. 50A),** we performed the Chi-squared test ($\chi^2$) test of homogeneity on the $2 \times 2$ contingency table to test if the differences in missense/synomymous ratios between rare and common variants were significant.

Because sequencing was only performed on limited numbers of individuals from the great ape genome project, we used the human allele frequency spectrum from ExAC to estimate the fraction of sampled variants that were rare (< 0.1%) or common (> 0.1%) in the general chimpanzee population. We sampled a cohort of 24 humans based on the ExAC allele frequencies and identified missense variants that were observed either once, or more than once, in this cohort. Variants thar were observed more than once had a 99.8% chance of being common (> 0.1%) in the general population, whereas variants that were observed only once in the cohort had a 69% chance of being common in the general population. To verify that the observed depletion for missense variants in more distant mammals was not due to a confounding effect of genes that are better conserved, and hence more accurately aligned, we repeated the above analysis, restricting only to genes with >50% average nucleotide identity in the multiple sequence alignment of 11 primates and 50 mammals compared with human (see Supplementary Table 3).

This removed -7% of human protein-coding genes from the analysis, without substantially affecting the results. Additionally, to ensure that our results were not affected by issues with variant calling, or domestication artifacts (since most of the species selected from dbSNP were domesticated), we repeated the analyses using fixed substitutions from pairs of closely related species in lieu of intraspecies polymorphisms (FIG. 50D, Supplementary Table 4, and Supplementary Data File 2).

## ClinVar Analysis of Polymorphism Data for Human, Primates, Mammals, and other Vertebrates

To examine the clinical impact of variants that are identical-by-state with other species, we downloaded the ClinVar database, excluding those variants that had contlicting annotations of pathogenicity or were only labeled as variants of uncertain significance. Following the filtering steps shown in Supplementary Table 9, there are a total of 24,853 missense variants in the pathogenic category and 17,775 missense variants in the benign category.

We counted the number of pathogenic and benign ClinVar variants that were identical-by-state with variation in humans, non-human primates, mammals, and other vertebrates. For human, we simulated a cohort of 30 humans, sampled from ExAC allele frequencies. The numbers of benign and pathogenic variants for each species are shown in Supplementary Table 10.

## Generation of Benign and Unlabeled Variants for Model Training

We constructed a benign training dataset of largely common benign missense variants from human and non-human primates for machine learning. The dataset comprises common human variants (> 0.1% allele frequency; 83,546 variants), and variants from chimpanzee, bonobo, gorilla, and orangutan, rhesus, and marmoset (301,690 unique primate variants). The number of benign training variants contributed by each source is shown in Supplementary Table 5.

We trained the deep learning network to discriminate between a set of labeled benign variants and an unlabeled set of variants that were matched to control for trinucleotide context, sequencing coverage, and alignability between the species and human. To obtain an unlabeled training dataset, we started with all possible missense variants in canonical coding regions. We excluded variants that were observed in the 123,136 exomes from ExAC, and variants in start or stop codons. In total, 68,258,623 unlabeled missense variants were generated. This was filtered to correct for regions of poor sequencing coverage, and regions where there was not a one-to-one alignment between human and primate genomes when selecting matched unlabeled variants for the primate variants.

We obtained a consensus prediction by training eight models that use the same set of labeled benign variants and eight randomly sampled sets of unlabeled variants, and taking the average of their predictions. We also set aside two randomly sampled sets of 10,000 primate variants for validation and testing, which we withheld from training (Supplementary Data File 3). For each of these sets, we sampled 10,000 unlabeled variants that were matched by trinucleotide context, which we used to normalize the threshold of each classifier when comparing between different classification algorithms (Supplementary Data File 4). In other implementations, fewer or additional models can be used in the ensemble, ranging from 2 to 500.

We assessed the classification accuracy of two versions of the deep learning network, one trained with common human variants only, and one trained with the full benign labeled dataset including both common human variants and primate variants.

## Architecture of the Deep Learning Network

For each variant, the pathogenicity prediction network takes as input the 51-length amino acid sequence centered at the variant of interest, and the outputs of the secondary structure and solvent accessibility networks **(FIG. 2 and FIG. 3)** with the missense variant substituted in at the central position. Three 51-length position frequency matrices are generated from multiple sequence alignments of 99 vertebrates, including one for 11 primates, one for 50 mammals excluding primates, and one for 38 vertebrates excluding primates and mammals.

The secondary structure deep learning network predicts a three-state secondary structure at each amino acid position: alpha helix (H), beta sheet (B), and coils (C) (Supplementary Table 11). The solvent accessibility network predicts a three-state solvent accessibility at each amino acid position: buried (B), intermediate (1), and exposed (E) (Supplementary Table 12). Both networks only take the flanking amino acid sequence as their inputs, and were trained using labels from known non-redundant crystal structures in the Protein DataBank (Supplementary Table 13). For the input to the pretrained three-state secondary structure and three-state solvent accessibility networks, we used a single length position frequency matrix generated from the multiple sequence alignments for all 99 vertebrates, also with length 51 and depth 20. After pre-training the networks on known crystal structures from the Protein DataBank, the final two layers for the secondary structure and solvent models were removed and the output of the network was directly connected to the input of the pathogenicity model. The best testing accuracy achieved for the three-state secondary structure prediction model was 79.86% (Supplementary Table 14). There was no substantial difference when comparing the predictions of the neural network when using DSSP-annotated (Define Secondary Structure of Proteins) structure labels for the approximately -4,000 human proteins that had crystal structures versus using predicted structure labels only (Supplementary Table 15).

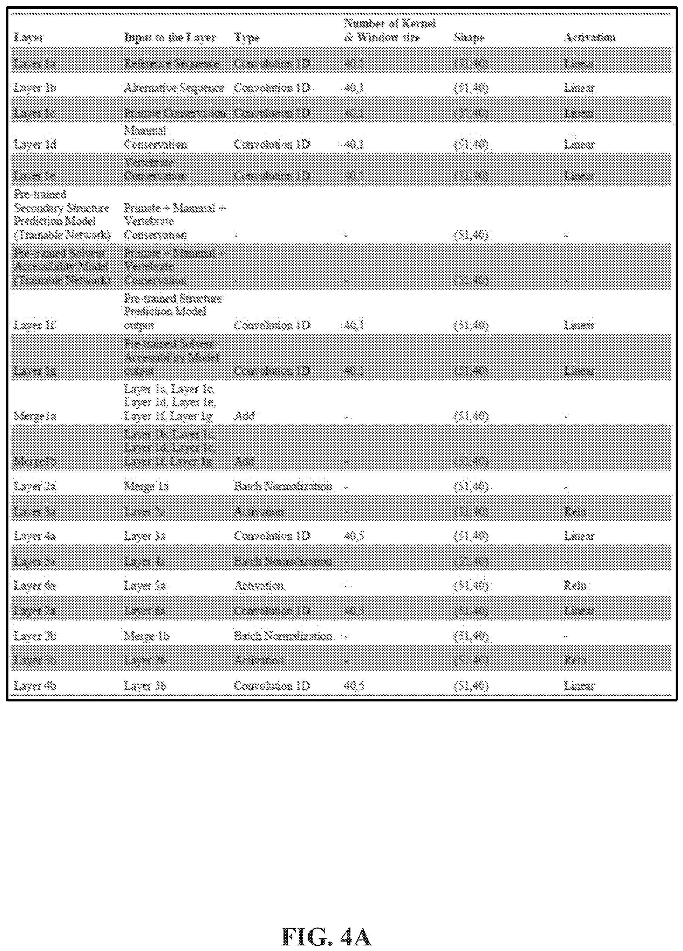

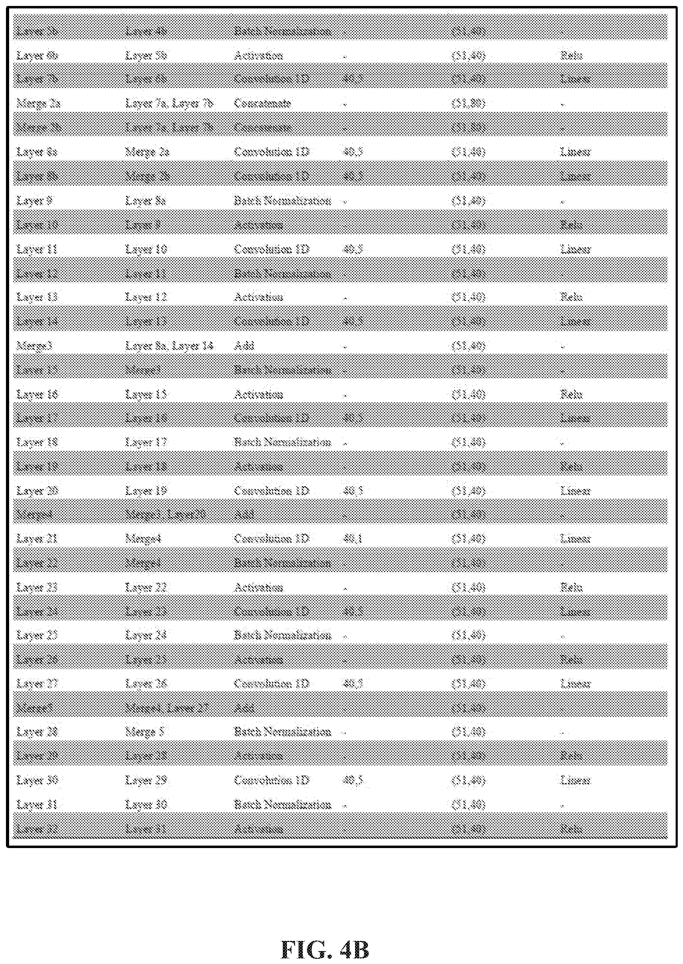

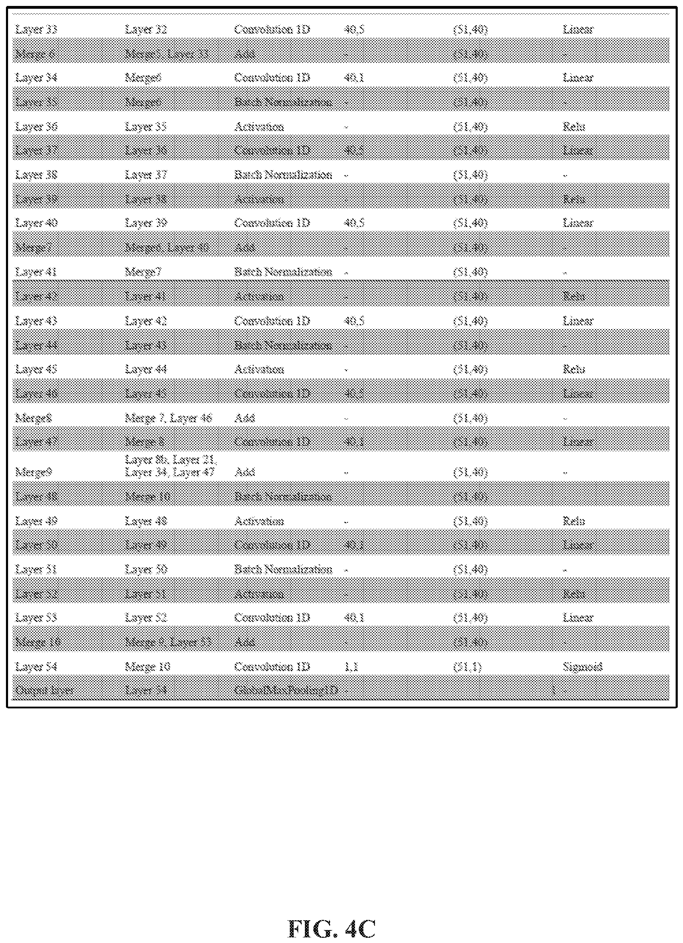

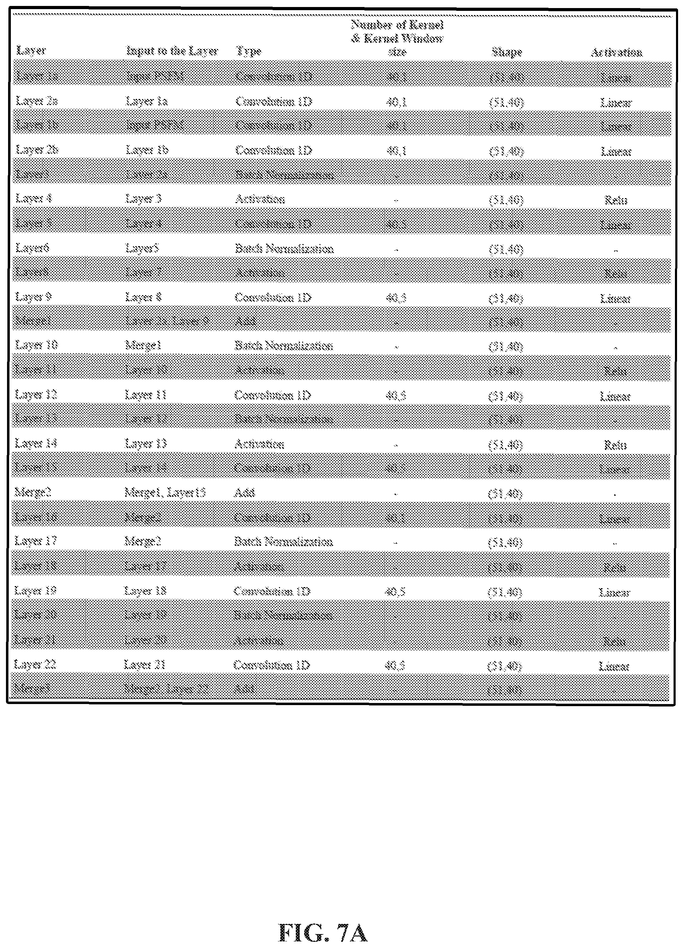

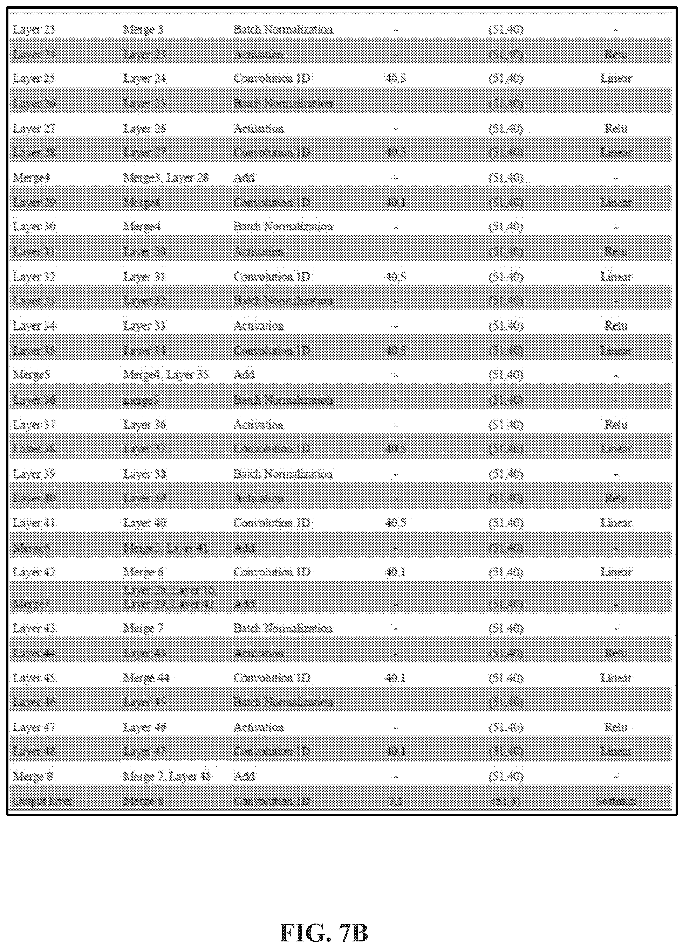

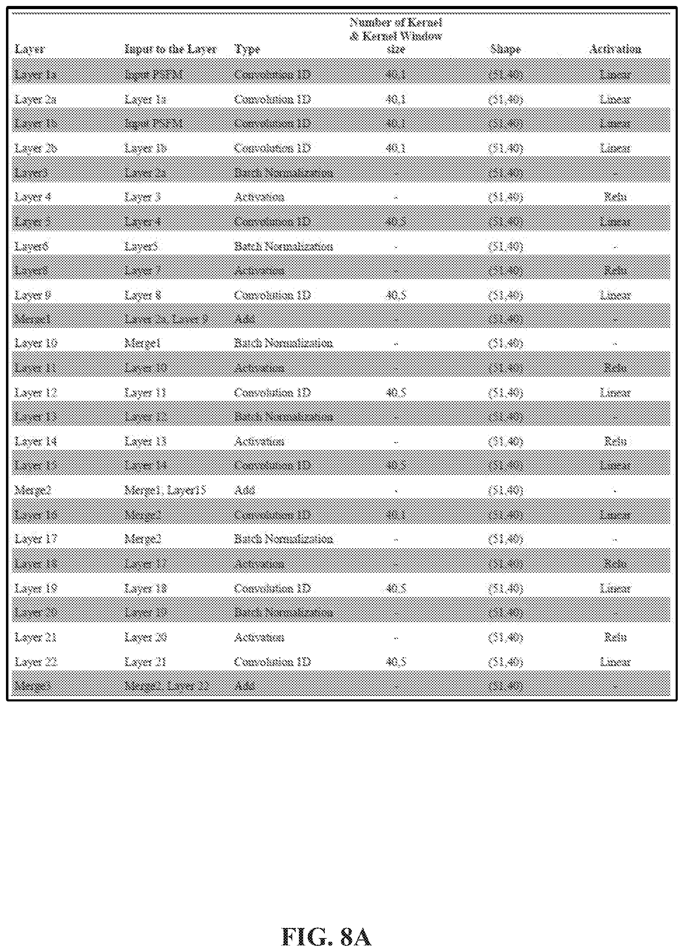

Both our deep learning network for pathogenicity prediction (PrimateAI) and deep learning networks for predicting secondary structure and solvent accessibility adopted the architecture of residual blocks. The detailed architecture for PrimateAI is described in **(FIG. 3)** and Supplementury Table 16 ( **FIGs. 4A, 4B,** and **4C).** The detailed architecture for the networks for predicting secondary structure and solvent accessibility is described in **FIG. 6 and** Supplementary Tables 11 **(FIGs. 7A** and **7B)** and 12 **(FIGs. 8A** and **88).**

## Benchmarking of Classifier Performance on a Withheld Test Set of 10,000 Primate Variants

We used the 10,000 withheld primate variants in the test dataset to benchmark the deep learning network as well as the other 20 previously published classifiers, for which we obtained prediction scores from the database dbNSFP. The performance for each of the classifiers on the 10,000 withheld primate variant test set is also provided in **FIG. 28A.**

Because the different classifiers had widely varying score distributions, we used 10,000 randomly selected unlabeled variants that were matched to the test set by trinucleotide context to identify the 50th-percentile threshold for each classifier. We benchmarked each classifier on the fraction of variants in the 10,000 withheld primate variant test set that were classified as benign at the 50th-percentile threshold for that classifier, to ensure fair comparison between the methods.

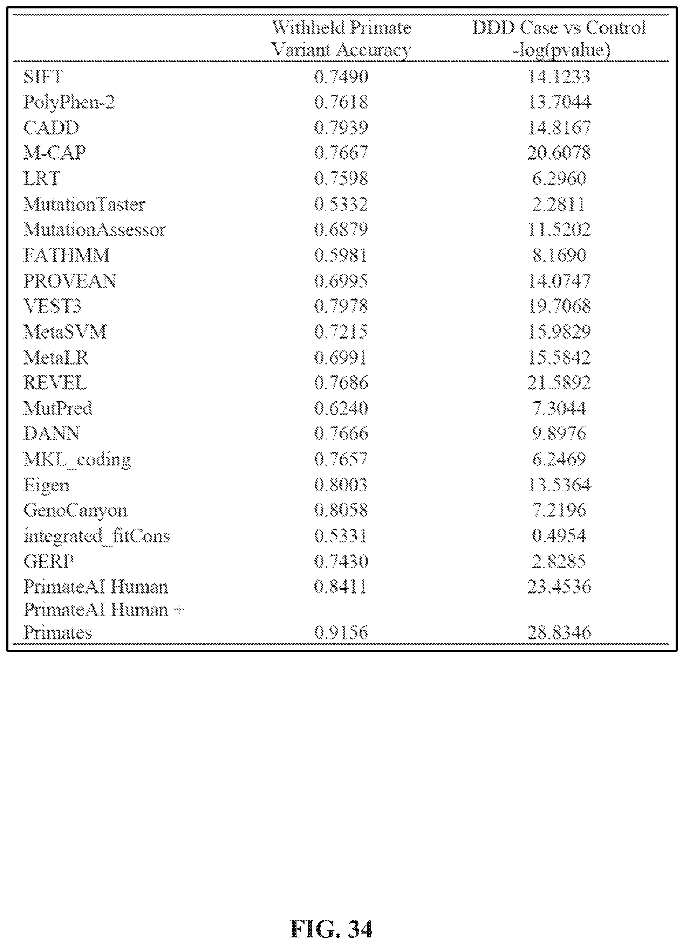

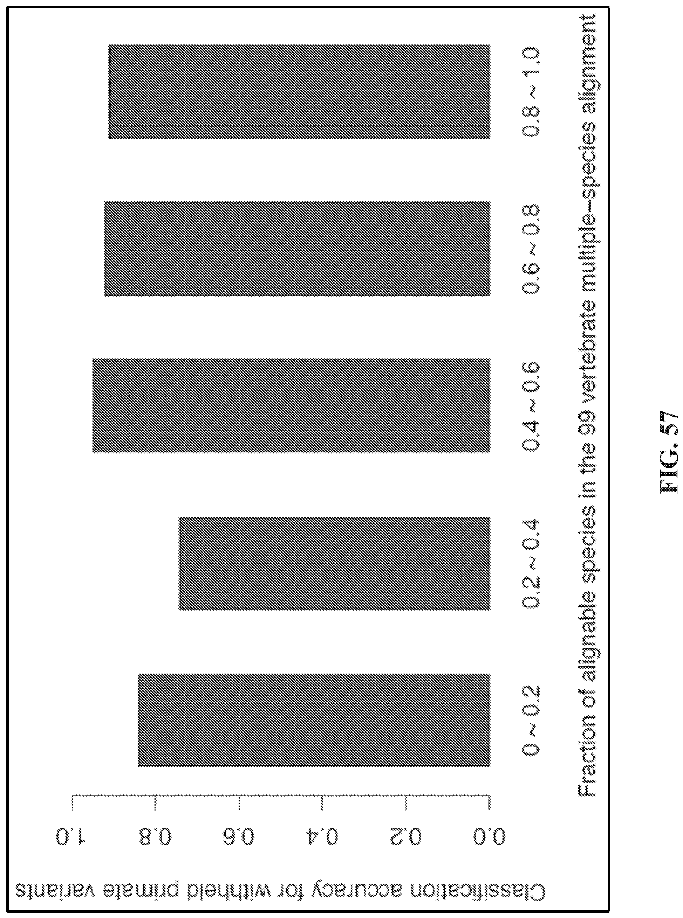

For each of the classifiers, the fraction of withheld primate test variants predicted as benign using the 50th-percentile threshold is also shown in in **FIG. 28A** and Supplementary Table 17 **(FIG. 34)**. We also show that the performance of PrimateAI is robust with respect to the number of al igned species at the variant position, and generally performs well as long as sufficient conservation information from mammals is available, which is true for most protein-coding sequence **(FIG. 57).**

## Analysis of *de novo* Variants from the DDD Study

We obtained published *de novo* variants from the DDD study, and *de novo* variants from the healthy sibling controls in the SSC autism study. The DDD study provides a confidence level for *de novo* variants, and we excluded variants from the DDD dataset with a threshold of <0.1 as potential false positives due to variant calling errors. In one implementation, in total, we had 3,512 missense *de novo* variants from DDD affected individuals and 1,208 missense *de novo* variants from healthy controls. The canonical transcript annotations used by UCSC for the 99-vertebrate mmultiple-sequence alignment differed slightly from the transcript annotations used by DDD, resulting in a small difference in the total counts of missense variants. We evaluated the classification methods on their ability to discriminate between *de novo* missense variants in the DDD affected individuals versus *de novo* missense variants in unaffected sibling controls from the autism studies. For each classifier, we reported the P value from the Wilcoxon rank-sum test of the difference between the prediction scores for the two distributions (Supplementary Table 17 **(FIG. 34)**.

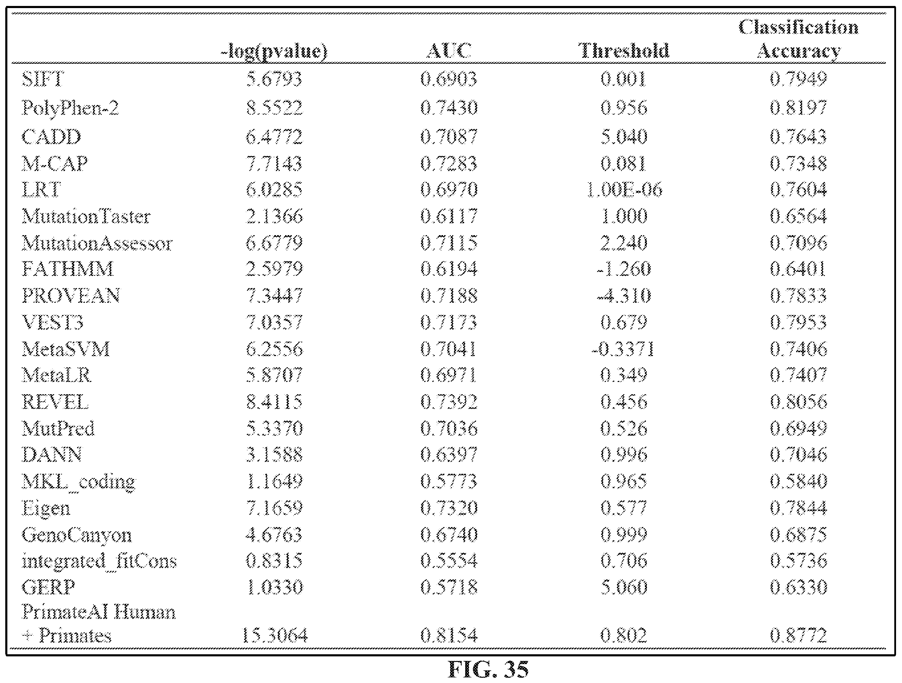

To measure the accuracy of various classifiers at distinguishing benign and pathogenic variation within the same disease gene, we repeated the analysis on a subset of 605 genes that were enriched for *de novo* protein-inmcating variation is the DDD cohort (P<0.05, Poisson exact text) (Supplementary Table 18). Within these 605 genes, we estimated that two-thirds of the *de novo* variants in the DDD dataset were pathogenic and one-third were benign, based on the 3/1 enrichment of *de novo*, missense mutations over expectation. We assumed minimal incomplete penetrance and that the *de novo* missense mutations in the healthy controls were benign. For each classifier, we identified the threshold that produced the same number of benign or pathogenic predictions as the empirical proportions observed in these datasets, and used this threshold as a binary cutoff to estimate the accuracy of each classifier at distinguishing *de novo* mutations in cases versus controls. To construct a receiver operator characteristics curve, we treated pathogenic classification of *de novo* DDD variants as true positive calls, and treated classification of *de novo* variants in healthy controls as pathogenic as being false positive calls. Because the DDD dataset contains one-third benign *de novo* variants, the area under the curve (AUC) for a theoretically perfect classifier is less than one. Hence, a classifier with perfect separation of benign and pathogenic variants would classify 67% of *de novo* variants in the DDD patients as true positives, 33% of *de novo* variants in the DDD patients as false negatives, and 100% of *de* novo variants in controls as true negatives, yielding a maximum possible AUC of 0.837 **(FIGs. 29A** and **29B** and Supplementary Table 19 **(FIG. 35))**.

## Novel Candidate Gene Discovery

We tested enrichment of *de novo* mutations in genes by comparing the observed number of *de novo* mutations to the number expected under a null mutation model. We repeated the enrichment analysis performed in the DDD **study,** and report genes that are newly genome-wide significant when only counting *de novo* missense mutations with a Primate AI score of >0.803. We adjusted the genome-wide expectation for *de novo* damaging missense variation by the fraction of missense variants that meet the PrimateAI threshold of >0.803 (roughly one-fifth of all possible missense mutations gentane-wide). As per the DDD study, each gene required four tests, one testing pitirein truncating enrichment and one testing enrichment of protein-altering *de novo* mutations, and both tested for just the DDD cohort and for a larger meta-analysis of neurodevelopmental trio sequencing cohorts. The enrichment of protein-altering *de novo* mutations was combined by Fisher's method with a test of the clustering of missense *de novo* mutations within the coding sequence (Supplementary Tables 20, 21). The P value for each gene was taken from the minimum of the four tests, and genome-wide significance was determined as $P < 6.757 \times 10^{-7}$ (a = 0.05, 18,500 genes with four tests).

## ClinVar Classification Accuracy

Since most of the existing classifiers are either trained directly or indirectly on ClinVar content, such as using prediction scores from classifiers that are trained on ClinVar, we limited analysis of the ClinVar dataset so that we only used ClinVar variants that had been added since 2017. There was substantial overlap among the recent ClinVar variants and other databases, and hence we further filtered to remove variants found at common allele frequencies (>0. 1%) in ExAC, or present in HGMI) (Human Gene Mutation Database), LOVD (Leiden Open Variation Database), or Uniprot (Universal Protein Resource). After excluding variants annotated ordy as uncertain significance and those with conflicting annotations, we were left with 177 missense variants with benign annotation and 969 missense variants with pathogenic annotation. We scored these ClinVar variants using both the deep learning network and the other classification methods. For each classifier, we identified the threshold that produced the same number of benign or pathogenic predictions as the empirical proportions observed in these datasets, and used this threshold as a binary cutoff to estimate the accuracy of each classifier **(FIGs. 31A** and **31B).**

## Impact of Increasing Training Data Size and Using Different Sources of Training Data

To evaluate the impact of training data size on the performance of the deep learning network, we randomly sampled a subset of variants from the labeled benign training set of 385,236 primate and common human variants, and kept the underlying deep learning network architecture the same. To show that variants from each individual primate species contributes to classification accuracy whereas variants from each individual mammal species lower classification accuracy, we trained deep learning networks using a training dataset comprising 83,546 human variants plus a constant number of randomly selected variants for each species, again keeping the underlying network architecture the same, according to one implementation. The constant number of vatiants we added to the training set (23,380) was the total number of variants available in the species with the lowest number of missense vanants, i.e. bonobo. We repeated the training procedures five times to get the median performance of each classifier.

## Saturation of All Possible Human Missense Mutations with Increasing Number of Primate Populations Sequenced

We investigated the expected saturation of all ~70 million possible human missense mutations by common variants present in the 504 extant primate species, by simulating variants based on the trinucleotide context of human common missense variants (>0.1% allele frequency) observed in ExAC. For each primate species, we simulated four times the number of common missense variants observed in human (~83,500 missense variants with allele frequency >0.1%), because humans have roughly half the number of variants per individual as other primate species, and about ~50% of human missense variants have been filtered out by purifying selection at >0.1% allele frequency **(FIG. 49A).**

To model the fraction of human common missense variants (>0.1% allele frequency) discovered with increasing size of human cohorts surveyed **(FIG. 56),** we sampled genotypes according to ExAC allele frequencies and report the fraction of common variants that were observed at least once in these simulated cohorts.

In one implementation, for practical application of PrimareAI scores, a threshold of >0.8 is preferred for likely pathogenic classification, <0.6 for likely benign, and 0.6-0.8 as intermediate in genes with dominant modes of inheritance, on the basis of the enrichment of *de* novo variants in cases as compared to controls **(FIG. 21D),** and a threshold of >0.7 for likely pathogenic and <0.5 for likely benign in genes with recessive modes of inheritance.

FIG. 2 shows an example architecture of a deep residual network for pathogenicity prediction, referred to herein as "PrimateAI". In **FIG. 2,** 1D refers to I-dimensional convolutional layer. Predicted pathogenicity is on a **scale** from 0 (benign) to I (pathogenic). The network takes as input the human amino acid (AA) reference and alternate sequence (51 AAs) centered at the variant, the position weight matrix (PWM) conservation profiles calculated from 99 vertebrate species, and the outputs of secondary structure and solvent accessibility prediction deep learning networks, which predict three-state protein secondary structure (helix-H, beta sheet-B, and coil-C) and three-state solvent accessibility (buried-B, intermediate-I, and exposed-E).

FIG. 3 depicts a schematic illustration of PrimateAI. the deep learning network architecture for pathogenicity classification. The inputs to the model include 51 amino acids (AA) of flanking sequence for both the reference sequence and the sequence with the variant substituted in, conservation represented by three 51-AA-length position-weighted matrices from primate, mammal, and vertebrate alignments, and the outputs of pre-trained secondary structure network and solvent accessibility network (also 51 AA in length).

**FIGs. 4A, 4B,** and **4C** are Supplementary Table 16 that show example model architecture details of the pathogenicity prediction deep learning model PrimateAI. The shape specifies the shape of the output tensor at each layer of the model and the activation is the activation given to the neurons of the layer. Inputs to the model are the position-specific frequency matrices (5I AA length, 20 depth) for the flanking amino acid sequence around the variant, the one-hot encoded human reference and alternate sequences (51 AA length, 20 depth), and the output from the secondary structure and solvent accessibility models (51 AA length, 40 depth).

The illustrated example uses 1D convolutions. In other implementations, the model can use different types of convolutions such as 2D convolutions, 3D convolutions, dilated or atrous convolutions, transposed convolutions, separable convolutions, and depthwise separable convolutions. Some layers also use ReLU activation function which greatly accelerates the convergence of stochastic gradient descent compared to saturating nonlinearities such as sigmoid or hyperbolic tangent. Other examples of activation functions that can be used by the technology disclosed include parametric ReLU, leaky ReLU, and exponennal linear unit (ELU).

Some layers also use batch normalization (Ioffe and Szegedy 2015). Regarding batch normalization, distribution of each layer in a convolution neural network (CNN) changes during training and it varies from one layer to another. This reduces the convergence speed of the optimization algorithm. Batch normalization is a technique to overcome this problem. Denoting the input of a batch normalization layer with x and its output using z, batch normalization applies the following transformation on x:

$$z = \frac{x - \mu}{\sqrt{\sigma^2 + \varepsilon}} \gamma + \beta$$

Batch normalization applies mean-variance normalization on the input x using $\mu$ and $\sigma$ and linearly scales and shifts it using $\gamma$ and $\beta$. The normalization parameters $\mu$ and $\sigma$ are computed for the current layer over the traming set using a method called exponential moving average. In other words, they are not trainable parameters. In contrast, $\gamma$ and $\beta$ are trainable parameters. The values for $\mu$ and $\sigma$ calculated during training are used in forward pass during inference.

**FIGs. 5** and **6** illustrate the deep learning network architecture used for predicting secondary structure and solvent accessibility of proteins. The input to the model is a position-weighted matrix using conservation generated by the RaptorX software (for training on Protein Data Bank sequences) or the 99-vertebrate alignments (for training and inference on human protein sequences). The output of the second to last layer, which is 51 AAs in length, becomes the input for the deep learning network for pathogenicity classification.

**FIGs. 7A** and **7B** are Supplementary Table 11 that show example model architecture details for the 3-state secondary structure prediction deep learning (DL) model. The shape specifies the shape of the output tensor at each layer of the model and the activation is the activation given to the neurons of the layer. Inputs to the model were the position-specific frequency matrices (51 AA length, 20 depth) for the flanking amino acid sequence around the variant.

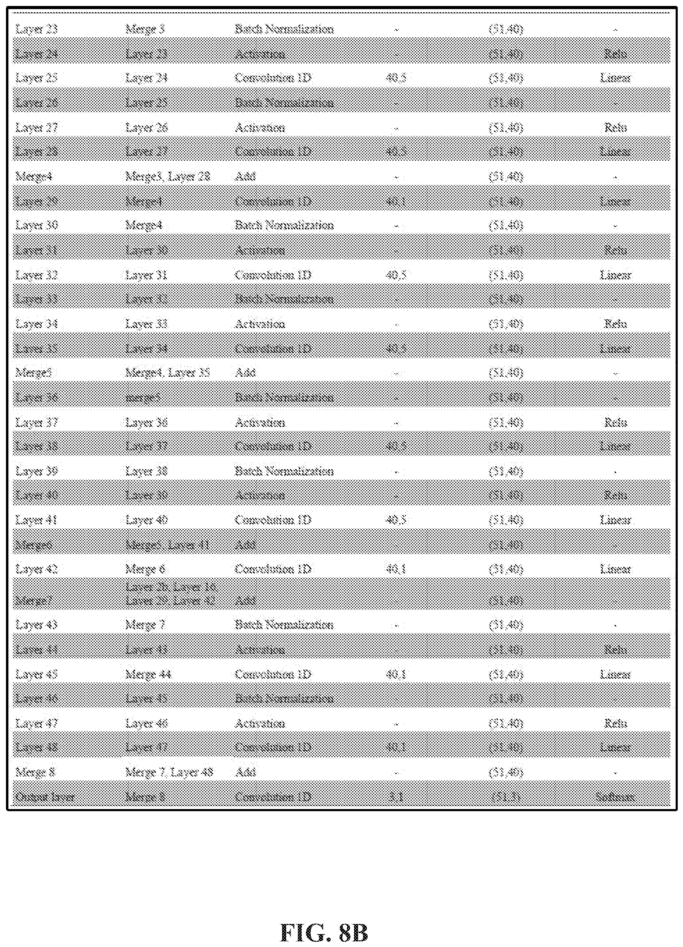

**FIGs. 8A** and **8B** are Supplementary Table 12 that show example model architecture details for the 3-state solvent accessibility prediction deep learning model. The shape specifies the shape of the output tensor at each layer of the model and the activation is the activation given to the neurons of the layer. Inputs to the model were the position-specific frequency matrices (51 AA length, 20 depth) for the flanking amino acid sequence around the variant.

**FIG. 20** shows predicted pathogenicity score at each amino acid position in the SCN2A gene, annotated for key functional domains. Plotted along the gene is the average PrimateAI score for missense substitutions at each amino acid position.

**FIG. 21D** shows a comparison of classifiers at predicting benign consequence for a test set of 10,000 common primate variants that were withheld from training. The y axis represents the percentage of primate variants correctly classified as benign, after normalizing the threshold of each classifier to its 50th-percentile score on a set of 10,000 random variants that were matched for mutational rate.

**FIG. 21E** illustrates distributions of PrimateAI prediction scores for *de novo* missense variants occurring in Deciphering Developmental Disorders (DDD) patients compared to unaffected siblings, with corresponding Wilcoxon rank-sum P value.

**FIG. 21F** depicts comparison of classifiers at separating *de novo* missense variants in DDD cases versus controls. Wilcoxon rank-sum test P values are shown for each classifier.

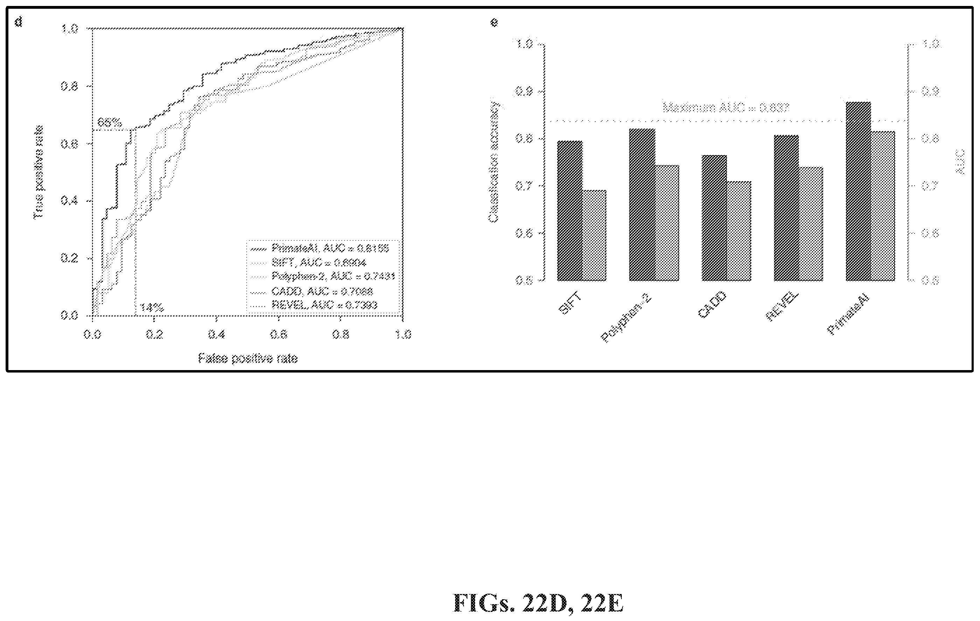

**FIGs. 22A, 228, 22C, 22D,** and **22E** illustrate classification accuracy within 605 DDD genes with P<0.05. **FIG. 22A** shows enrichment of *de nove* missense mutations over expectation in affected individuals from the DDD cohort within 605 associated genes that were significant for *de novo* protein truncating variation (P<0.05). **FIG. 22B** depicts distributions of PrimateAI prediction scores for *de novo* missense variants occurring in DDD patients versus unaffected siblings within the 605 associated genes, with corresponding Wilcoxon rank-sum P value.

**FIG. 22C** shows comparison of various classifiers at separating *de novo* missense variants in cases versus controls within the 605 genes. The y axis shows the P values of the Wilcoxon rank-sum test for each classifier.

**FIG. 22D** depicts comparison of various classifiers, shown on a receiver operator characteristic curve, with AUC indicated for each classifier.

**FIG. 22E** illustrates classification accuracy and AUC for each classifier. The classification accuracy shown is the average of the true positive and true negative error rates, using the threshold where the classifier would predict the same number of pathogenic and benign variants as expected based on the enrichment shown in FIG. **22A.** To take into account the fact that 33% of the DDD *de now* missense variants represent background, the maximum achievable AUC for a perfect classifier is indicated with a dotted line.

**FIGs. 23A, 23B, 23C,** and **23D** show impact of data used for training on classification accuracy. Deep learning networks trained with increasing numbers of primate and human common variants up to the full dataset (385,236 variants).

In **FIG. 23A,** classification performance for each of the networks is benchmarked on accuracy for the 10,000 withheld primate variants and *de novo* variants in DDD cases versus controls.

**FIGs. 23B** and **23C** show performance of networks trained using datasets comprising 83,546 human common variants plus 23,380 variants from a single primate or mammal species, according to one implementation. Results are shown for each network trained with different sources of common variation, benchmarked on 10,000 withheld primate variants **(FIG. 23B),** and on *de novo* missense variants **(FIG. 23C)** in DDD cases versus controls.

**FIG. 23D** depicts expected saturation of all possible human benign missense positions by identical-by-state common variants (>0.1%) in the 504 extant primate species. The y axis shows the fraction of human missense variants observed in at least one primate species, with CpG missense variants indicated in green, and all missense variants indicated in blue. To simulate the common variants in each primate species, we sampled from the set of all possible single nucleotide substitutions with replacemens, matching the trinucleotide context distribution observed for common human variants (>0.1% allele frequency) in ExAC.

**FIG. 24** illustrates correcting for the effect of sequencing coverage on the ascertainment of common primate variants. The probability of observing a given variant in a nonhuman primate species is inversely correlated with the sequencing depth at that position in the ExAC/gnomAD exome dataset. In contrast, the lower gnomAD read depth did not affect the probability of observing a common human variant at that position (>0.1% allele frequency) because the large number of human exomes sequenced makes ascertainment of common variation almost guaranteed. When picking matched variants for each of the primate variants for training the network, the probability of picking a variant was adjusted for the effects of sequencing depth, in addition to matching for trinucleotide context to control for mutational rate and gene conversion.

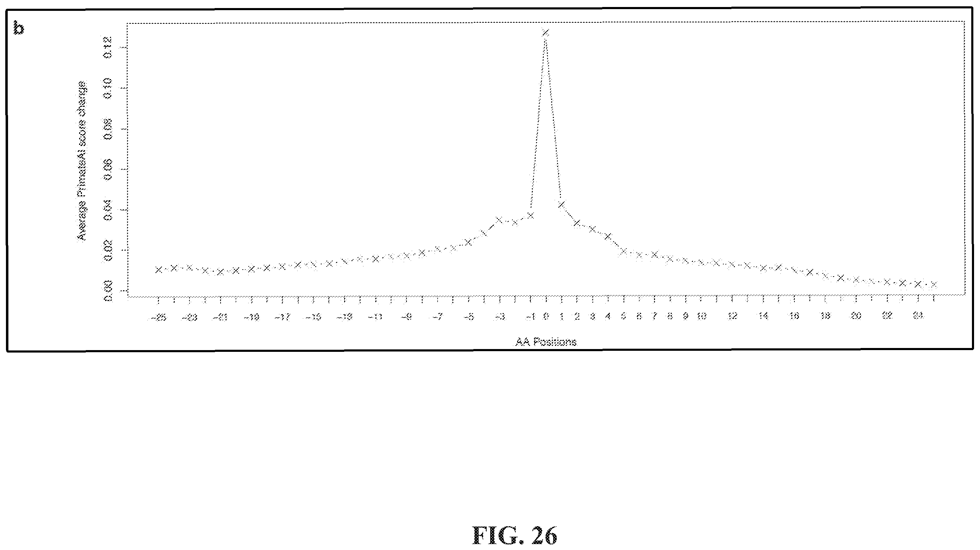

**FIGs. 25A, 25B, 25C,** and **26** depict recognition of protein motifs by the disclosed neural networks. Regarding **FIGs. 25A, 25B,** and **25C,** to illustrate the neural networks recognition of protein domains, we show the average PrimateAI scores for variants at each amino acid position in three different protein domains. In **FIG. 25A,** the collagen strand of COL1A2, with glycine in a repeating GXX motif is highlighted. Clinically identified mutations in collagen genes are largely due to missense mutations in glycine in GXX repeats, as these interfere with the nonnal assembly of collagen and exert strong dominant-negative effects. In **FIG. 25B,** the active site of the IDS sulfatase enzyme is highlighted, which contains a cysteine at the active site that is post-translationally modified to fonnylglycine. In **FIG. 25C,** the bHLHzip domain of the MYC transcription factor is shown. The basic domain contacts DNA via positively charged arginine and lysine residues (highlighted) that interact with the negatively charged sugar-phosphate backbone. The leucine-zipper domain comprises leucine residues spaced seven amino acids apart (highlighted), which are crucial for dimerization.

**FIG. 26** includes a line plot showing the effect of perturbing each position in and around the variant on the predicted deep learning score for the variant. We systematically zeroed out the inputs at nearby amino acids (positions -25 to +25) around the variant and measured the change in the neural network's predicted pathogenicity of the variant. The plot shows the average change in predicted pathogenicity score for perturbations at each nearby amino acid position for 5,000 randomly selected variants.

**FIG. 27** illustrates correlation patterns of weights mimic BLOSUM62 and Grantham score matrices. Correlation patterns of weights from the first three layers of the secondary-structure deep learning network show correlations between amino acids that are similar to BLOSUM62 and Grantham score matrices. The left heat map shows the correlation of parameter weights from the first convolutional layer following two initial upsampling layers of the secondary-structure deep learning network between amino acids encoded using a one-hot representation. The middle heat map shows BLOSUM62 scores between pairs of amino acids. The right heat map shows Grantham distance between amino acids. The Pearson correlation between deep learning weights and BLOSUM62 scores is 0.63 (P = $3.55 \times 10^{-9}$). The correlation between deep learning weights and Grantham scores is -0.59 (P = $4.36 \times 10^{-8}$). The correlation between BLOSUM62 and Grantham scores is -0.72 (P = $8.09 \times 10^{-13}$).

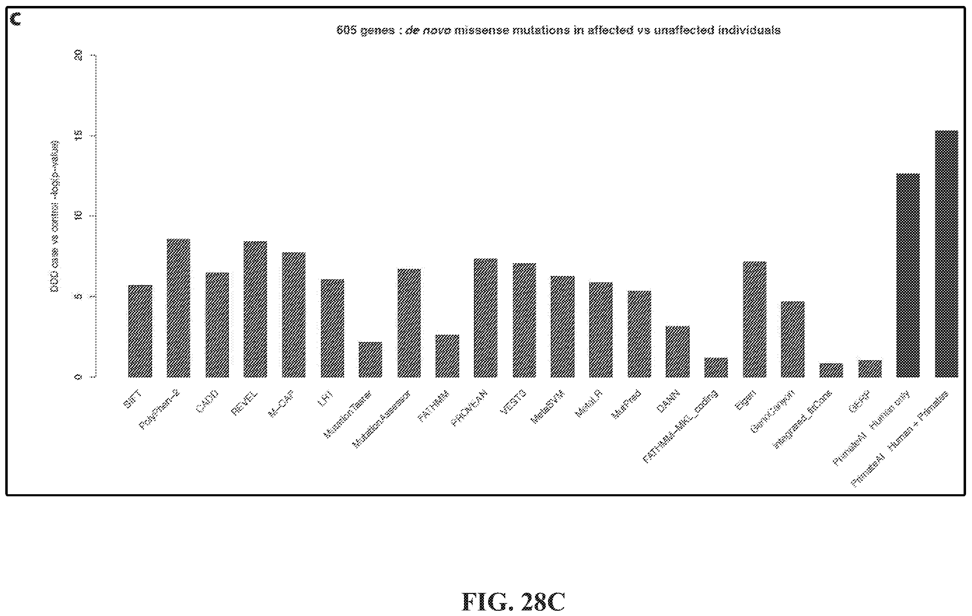

**FIGs. 28A, 288,** and **28C** show performance evaluation of the deep learning network PrimateAI and other classifiers. **FIG. 28A** depicts accuracy of the deep learning network PrimareAI at predicting a benign consequence for a test set of 10,000 primate variants that were withheld from training and comparison with other classifiers, including SIFT, PolyPhen-2. CADD, REVEL, M-CAP, LRT, MutationTaster, MutationAssessor, FATHMM, PROVEAN, VEST3, MetaSVM, MetaLR, MutPred, DANN, FATHMM-MKL_coding, Eigen, GenoCanyon, integrated_fitCons, and GERP. The y axis represents the percentage of primate variants classified as benign, based on normalizing the threshold for each classifier to its 50th-percentile score using a set of 10,000 randomly selected variants that were matched to the primate variants for trinucleotide context to control for mutational rate and gene conversion.

**FIG. 28B** depicts comparison of the performance of the PrimateAI network in separating *de novo* missense variants in DDD cases versus controls, along with the 20 existing methods listed above. The y axis shows the P values of the Wilcoxon rank-sum test for each classifier.

**FIG. 28C** depicts comparison of the performance of the PrimateAI network in separating de now missense variants in DDD cases versus unaffected controls within 605 disease-associated genes, with the 20 methods listed above. The y axis shows the P values of the Wilcoxon rank-sum test for each classifier.

**FIGs. 29A** and **29B** illustrate distribution of the prediction scores of four classifiers. Histograms of the prediction scores of four classifiers, including SIFT. PolyPhen-2, CADD, and REVEL. for *de novo* missense variants occurring in DDD cases versus unaffected controls, with corresponding Wilcoxon rank-sum P-values.

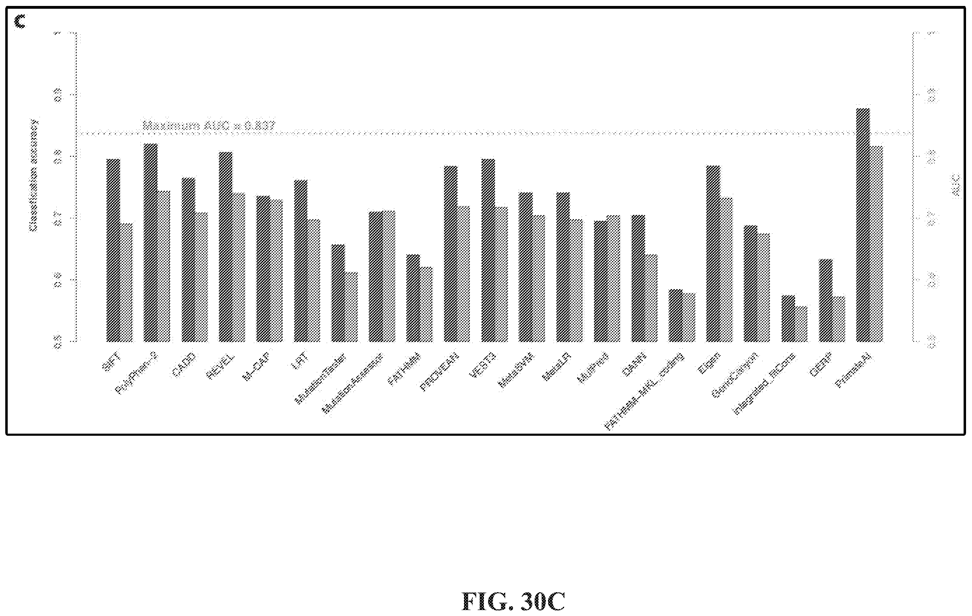

**FIGs. 30A, 30B,** and **30C** compare the accuracy of the PrimateAI network and other classifiers at separating pathogenic and benign variants in 605 disease-associated genes. The scatterplot in **FIG. 30A** shows the performance of each of the classifiers on DDD cases versus controls (y axis) and benign prediction accuracy on the withheld primate dataset (x axis). **FIG. 30B** compares different classifiers an separating de novo missense variants in cases versus controls within the 605 genes, shown on a receiver operator characteristic (ROC) curve, with area under the curve (AUC) indicated for each classifier. **FIG. 30C** shows classification accuracy and AUC for the PrimateAI network and the 20 classifiers listed in **FIGs. 28A. 28B,** and **28C.** The classification accuracy shown is the average of the true positive and true negative rates, using the threshold where the classifier would predict the same number of pathogenic and benign variants as expected based on the enrichment in **FIG. 22A.** The maximum achievable AUC for a perfect classifier is indicated with a dotted line, assuming that *de novo* missense variants in DDD cases are 67% pathogenic variants and 33% benign, and *de novo* missense variants in controls are 100% benign.

**FIGs. 31A** and **31B** illustrate correlation between classifier performance on human expert-curated ClinVar vanunts and performance on empirical datasets. The scatterplot in **FIG.31A** shows the classification accuracy (y axis) on ClinVar variants on the 10,000 withheld primate variants (x axis) for each of the 20 other classifiers and the PrimateAI network trained with human-only or human + primates data. Shown are the Spearman correlation coefficient rho and associated P value. To limit the evaluation to data that were not used for training the classifiers. we only used ClinVar variants that were added between January 2017 and November 2017, and excluded common human variants from ExAC/gnomAD (>0.1% allele frequency). The ClinVar classification accuracy shown is the average of the true positive and true negative rates, using the threshold where the classifier would predict the same number of pathogenic and benign variants as observed in the ClinVar dataset.

The scatterplot in **FIG. 31B** shows the classification accuracy (y axis) on ClinVar variants the DDD cases versus controls full dataset (x axis) for each of the 20 other classifiers and the PrimateAI network trained with human-only or human + primates data.

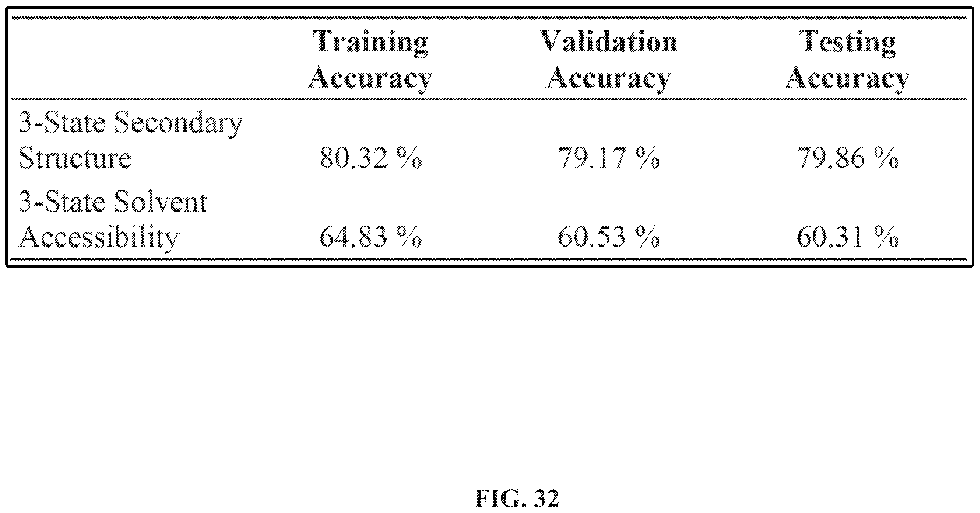

**FIG. 32** is Supplementary Table 14 that shows performance of the 3-state secondary structure and 3-state solvent accessibility prediction models on annotated samples from the Protein DataBank, using 6,367 unrelated protein sequences for training, 400 for validation, and 500 for testing. Only proteins with <25% sequence similarity were selected from the Protein DataBank. We report the accuracy of the deep learning networks as a performance metric, as the three classes are not grossly unbalanced for either secondary structure or solvent accessibility.

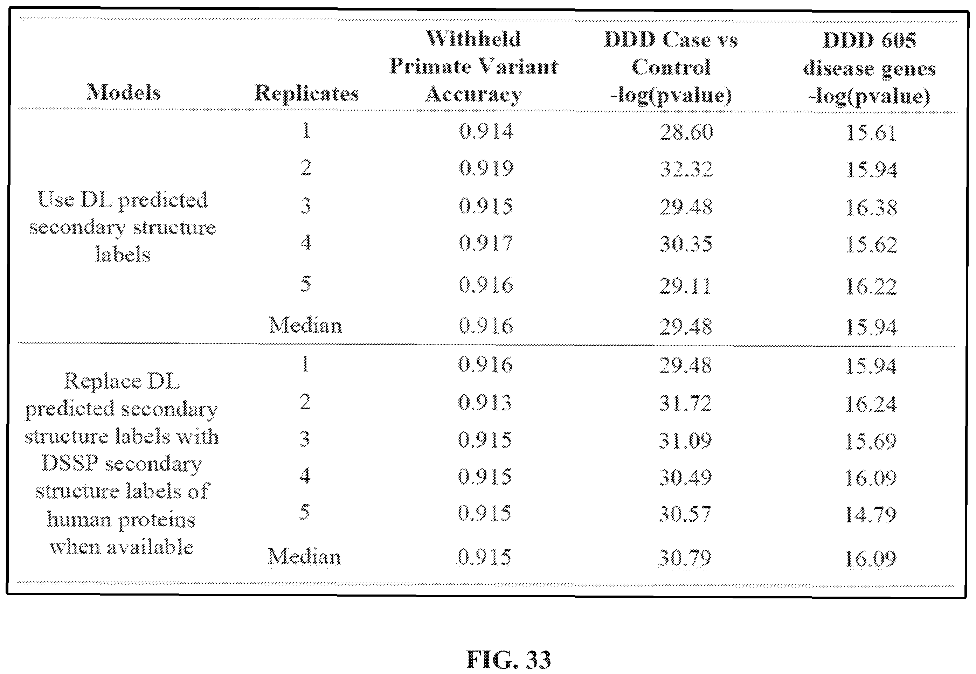

**FIG. 33** is Supplememary Table 15 that shows performance comparison of deep learning network using annotated secondary structure labels of human proteins from DSSP database when available with deep learning network using predicted secondary structure labels.

**FIG. 34** is Supplementary Table 17 that shows the accuracy values on the 10,000 withheld primate variants and the p-values for *de novo* variants in DDD cases vs controls for each of the 20 classifiers we evaluated. The PrimateAI model with Human data only is our deep learning network that was trained using a labeled benign training dataset comprising only of common human variants (83.5K variants with > 0.1% in the population), while PrimateAI model with Human + Primates data is our deep learning network trained on the full set of 385K labeled benign variants, comprising both common human variants and primate variants.

**FIG. 35** is Supplementary Table 19 that shows comparison of the performance of different classifiers on *de novo* variants in the DDD case vs control dataset, restricted to 605 disease-associated genes. To normalize between the different methods, for each classifier we identified the threshold where the classifier would predict the same number of pathogenic and benign variants as expected based on the enrichment in DDD and the control set. Classification accuracy shown is the average of the true positive and true negative error rates at this threshold.

**FIGs. 49A, 49B, 49C, 49D,** and **49E** depict missense/synonymous ratios across the human allele frequency spectrum. **FIG. 49A** shows missense and synonymous variants observed in 123,136 humans from the ExAC/gnomAD database were divided into four categories by allele frequency. Shaded grey bars represent counts of synonymous variants in each category; dark green bars represent missense variants. The height of each bar is scaled to the number of synonymous variants in each allele frequency category and the missense/synonymous counts and ratios are displayed after adjusting for mutation rate. **FIGs. 498** and **49C** illustrate allele frequency spectrum for human missense and synonymous variants that are identical-by-state (IBS) with chimpanzee common variants **(FIG. 49B)** and chimpanzee singleton variants **(FIG. 49C).** The depletion of chimpanzee missense variants at common human allele frequencies (>0.1%) compared to rare human allele frequencies (<0.1%) is indicated by the red box, along with accompanying Chi-squared ($\chi^2$) test *P* values.

**FIG. 49D** shows human variants that are observed in at least one of the non-human primate species. **FIG. 49E** illustrates counts of benign and pathogenic missense variants in the overall ClinVar database (top row), compared to ClinVar variants in a cohort of 30 humans sampled from ExAC/gnomAD allele frequencies (middle row), compared to variants observed in primates (bottom row). Conflicting benign and pathogenic assertions and variants annotated only with uncertain significance were excluded.

**FIGs. 50A, 50B, 50C,** and **50D** show purifying selection on missense variants identical-by-state with other species. **FIG. 50A** depicts allele frequency spectrum for human missense and synonymous variants that are identical-by-state with variants present in four non-primate mammalian species (mouse, pig, goat, and cow). The depletion of missense variants at common human allele frequencies (>0.1%) is indicated by the red box, along with the accompanying Chi-squared ($\chi^2$) test *P* value.

**FIG. 50B** is a scatter plot showing the depletion of missense variants observed in other species at common human allele frequencies (>0.1%) versus the species' evolutionary distance from human, expressed in units of branch length (mean number of substitutions per nucleotide position). The total branch length between each species and human is indicated next to the species' name. Depletion values for singleton and common variants are shown for species where variant frequencies were available, with the exception of gorilla, which contained related individuals.

**FIG. 50C** illustrates counts of benign and pathogenic missense variants in a cohort of 30 humans sampled from ExAC/gnomAD allele frequencies (top row), compared to variants observed in primates (middle row), and compared to variants observed in mouse, pig, goat, and cow (bottom row). Conflicting benign and pathogenic assertions and variants annotated only with uncertain significance were excluded.

**FIG. 50D** is a scatter plot showing the depletion of fixed missense substitutions observed in pairs of closely related species at common human allele frequencies (>0.1%) versus the species' evolutionary distance from human (expressed in units of mean branch length).

**FIG. 51** shows expected missense:synonymous ratios across the human allele frequency spectrum in the absence of purifying selection. Shaded gray bars represent the number of synonymous variants, and dark green bars represent the number of missense variants. The dotted line shows the baseline formed by synonymous vanants. Missense:synomymous ratios are indicated for each allele frequency category. According to one implementation, the expected missense and synonymous counts in each allele frequency category were calculated by taking intronic variants from the ExAC/gnomAD dataset comprising 123,136 exomes and using them to estimate the fraction of variants expected to fall into each of the four allele frequency categories, based on the trinucleotide context of the variant, which controls for mutational rate and GC bias in gene conversion.

**FIGs. 52A, 52B, 52C,** and **52D** depict missense:synonymous ratios for CpG and non-CpG variants. **FIGs. 52A** and **52B** show missense:synonymous ratios for CpG variants (**FIG. 52A**) and non-CpG variants (**FIG. 52A**) across the human allele frequency spectrum, using all variants from the ExAC/gnomAD exomes. **FIGs. 52C** and **52D** show missense:synonymous ratios for CpG variants **(FIG. 52C**) and non-CpG variants **(FIG. 52D**) across the human allele frequency spectrum, restricted to only human variants that are identical by state with chimpanzee common polymorphisms.

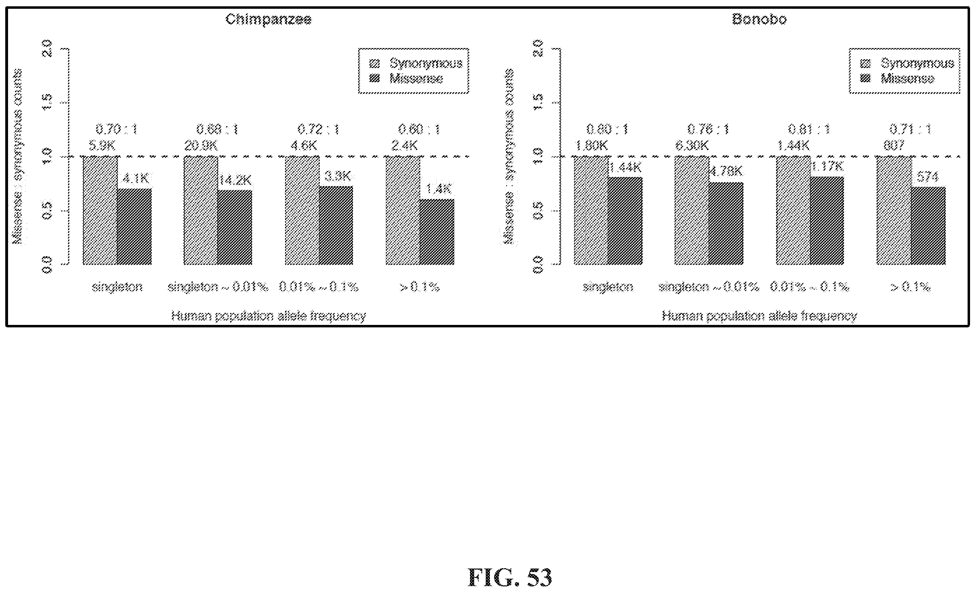

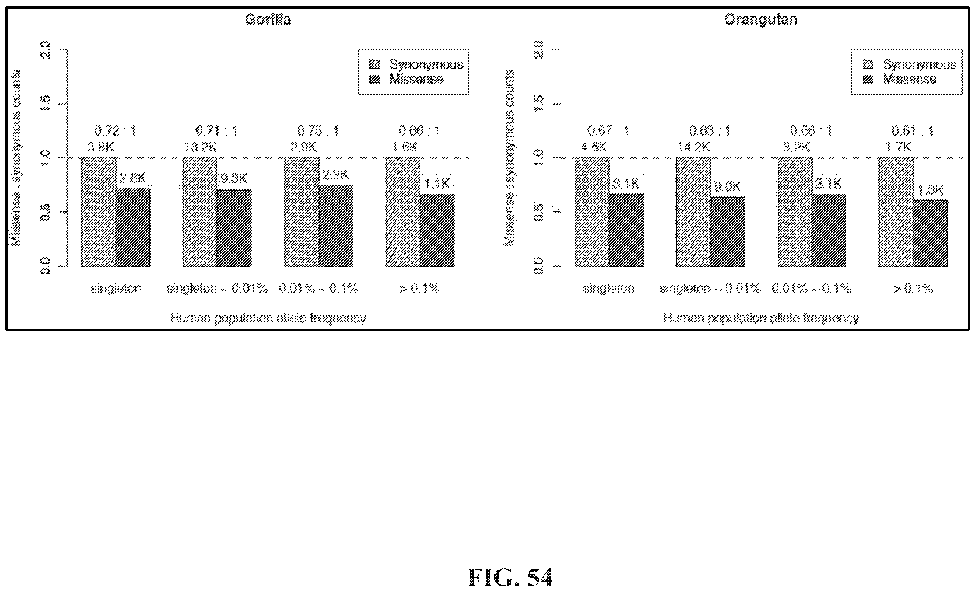

**FIGs. 53, 54,** and **55** illustrate missense:synonymous ratios of human variants identical by state with six primates. Patterns of missense:synonymous ratios across the human allele frequency spectrum for ExAC/gnomAD variants that are identical by state with variation present in chimpanzee, bonobo, gorilla, orangutan, rhesus, and marmoset.

**FIG. 56** is a simulation showing saturation of new common missense variants discovered by increasing the size of the human cohorts surveyed. In simulations. the genotypes of each sample were sampled according to gnomAD allele frequencies. The fraction of gnomAD common variants discovered is averaged across 100 simulations for each sample size from 10 to 100,000.

**FIG. 57** shows accuracy of PrimateAI across different conservation profiles in the genome. The x axis represents the percentage alignability of the 51 AA around a sequence with the 99-vertebrate alignments. The y axis represents the classification performance of PrimateAI accuracy for variants in each of the conservation bins, benchmarked on the test dataset of 10,000 withheld primate variants.

**FIG. 58** is Supplementary Table 5 that shows contributions to the labeled benign training dataset from common human variants and variants present in non-human primates.

**FIG. 59** is Supplementary Table 8 that shows effect of allele frequency on expected missense:synonymous ratio. Expected counts of synonymous and missense variants were calculated based on the allele frequency spectrum of variants in intronic regions at least 20-30nt away from exon boundaries, using trinucleotide context to control for mutational rate and gene conversion biases.

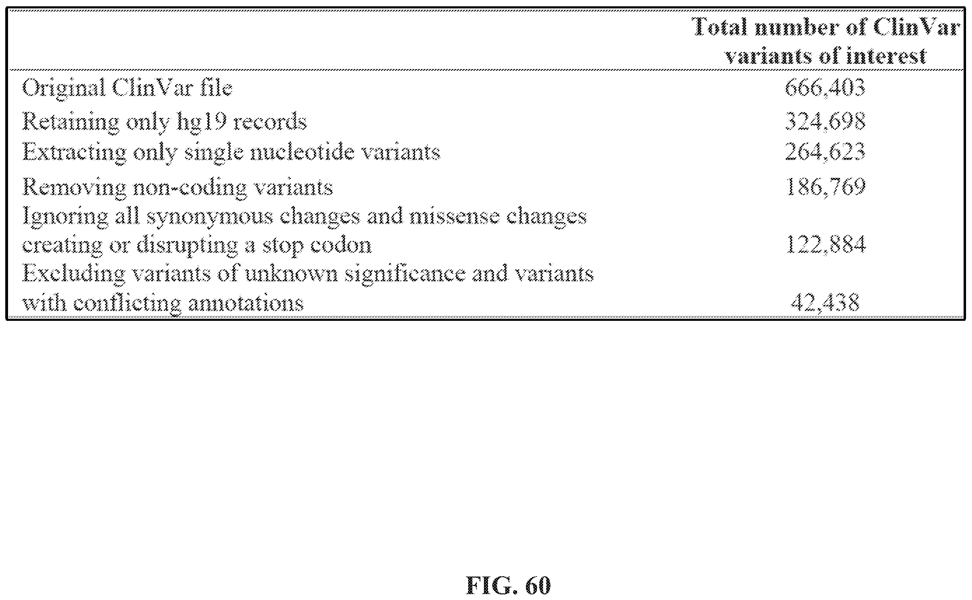

**FIG. 60** is Supplementary Table 9 that shows ClinVar analysis. According to one implementation, variants downloaded from the Nov. 2017 build of the ClinVar database were filtered to remove missense variants with conflicting annotations and exclude variants of uncertain significance, leaving 17.775 benign variants and 24,853 pathogenic variants.

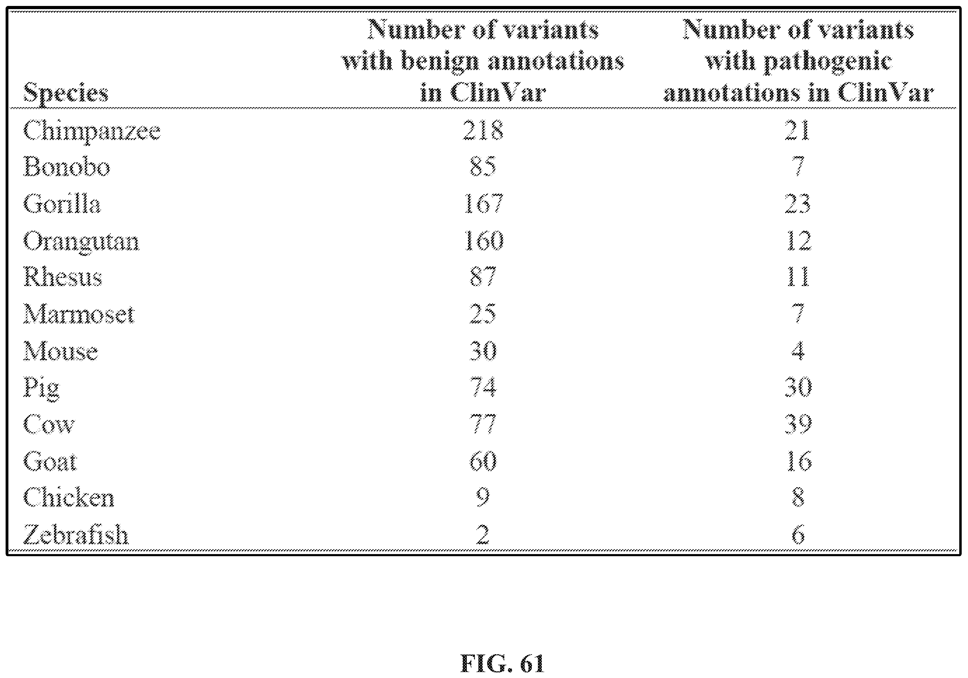

**FIG. 61** is Supplementary Table 10 that shows the number of missense variants from other species found in ClinVar, according to one implementation. Variants were required to be identical-by-state with the corresponding human variant, and to have identical nucleotides at the other two positions in the reading frame to ensure the same coding consequence.

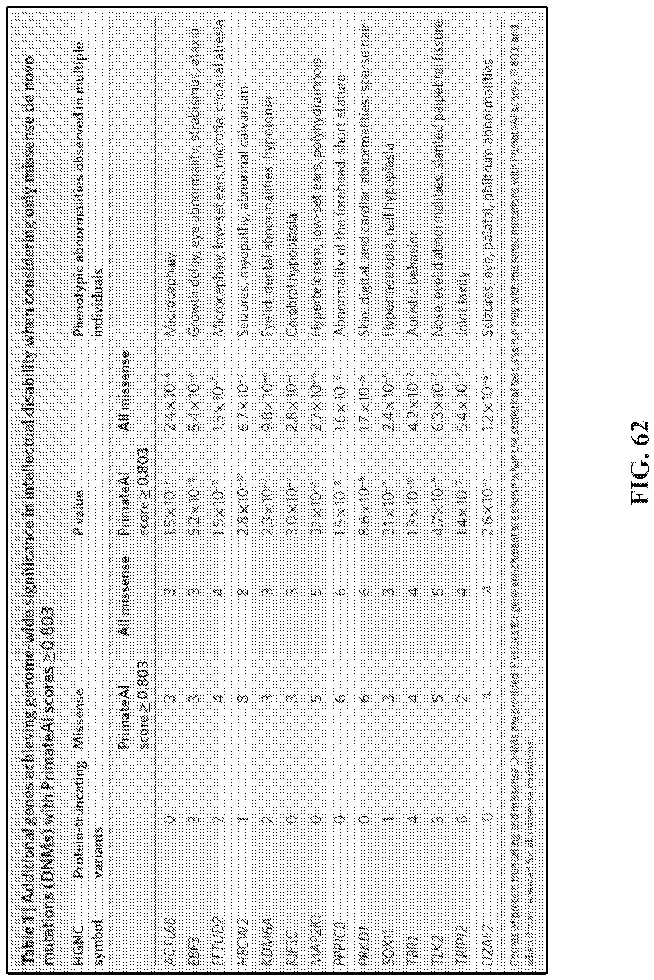

**FIG. 62** is Table 1 that shows one implementation of discovery of 14 additional candidate genes in intellectual disability, which had previously not reached the genome-wide significance threshold in the original DDD study.

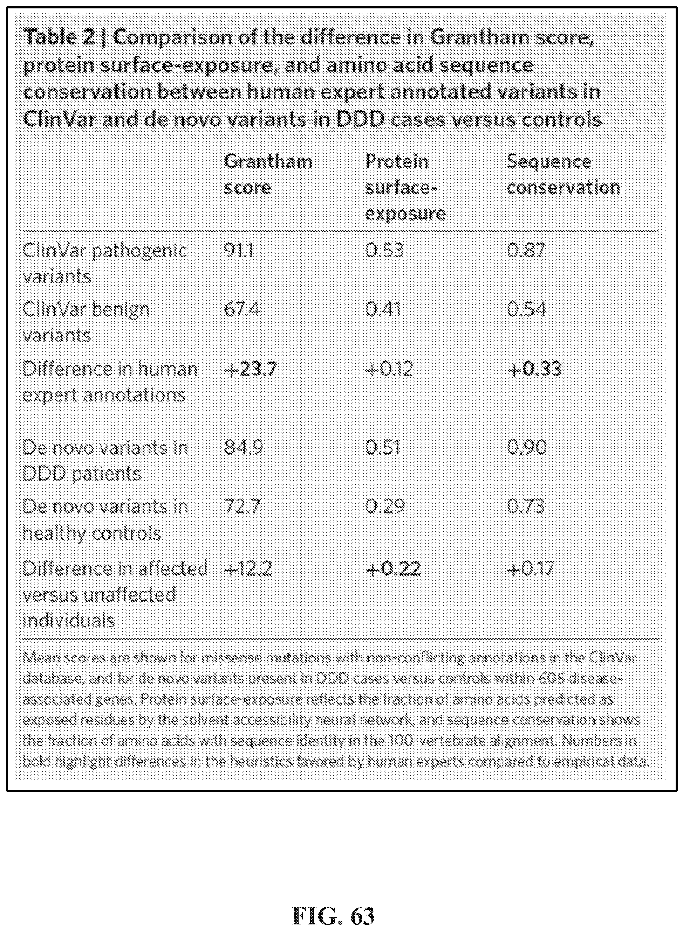

**FIG. 63** is Table 2 that shows one implementation of the mean difference in Grantham score between pathogenic and benign variants in ClinVar, which is twice as large as the difference between *de novo* variants in DDD cases versus controls within the 605 disease-associated genes.

## Data Generation

All coordinates used in the paper refer to human genome build UCSC hg19/GRCh37, including the coordinates for variants in other species, which were mapped to hg19 using multiple sequence alignments using the procedure described in this section. Protein-coding DNA sequence and multiple sequence alignments of 99 vertebrate genomes with human were downloaded from the UCSC genome browser for the hg19 build. (http://hgdownload.soe.uese.edu/gold enPath/hg19/multiz100way/alignments/knownCanonical.exonNuc.fa.gz). For genes with multiple canonical gene annotations, the longest coding transcript was selected.

We downloaded human exome polymorphism data from the Exome Aggregation Consortium (ExAC)/genome Aggregation Database (gnomAD) v2.0, which collected the whole-exome sequencing (WES) data of 123,136 individuals from eight subpopulations around the world (http://gnomad.broadinstitute.org/). We excluded variants that failed the default quality control filters as annotated in ExAC VCF file or fell outside canonical coding regions. To avoid effects due to balancing selection, we also excluded variants from within the extended MHC region (chr6: 28,477,797-33,448,354) for the primate analyses. The great ape genome sequencing project provides whole genome sequencing data and genotypes for 24 chimpanzees, 13 bonobos, 27 gorillas and 10 orangutans (including 5 from the Sumatran subspecies and 5 from the Bornean subspecies, which we collapsed for the downstream analyses). The study on chimpanzee and bonobos provides genome sequences of additional 35 chimpanzees. However, as variants for these additional chimpanzees were not called using the same methods as the great ape genome sequencing project, we excluded them from the allele frequency spectrum analysis, and used them only for training the deep learning model. Variation from these primate diversity studies were already mapped to the human reference (hg19). In addition, for Marmoset and Rhesus, 16 rhesus individuals and 9 marmoset individuals were used to assay variation in the original sequencing of these species' genomes. but individual-level information is not available.

The great ape genome sequencing project4 provides whole genome sequencing data and genotypes for 24 chimpanzees, 13 bonobos, 27 gorillas and 10 orangutans (including 5 from the Sumatran subspecies and 5 from the Bornean subspecies, which we collapsed for the downstream analyses). The study on chimpanzee and bonobos provides genome sequences of additional 35 chimpanzees. However, as variants for these additional chimpanzees were not called using the same methods as the great ape genome sequencing project, we excluded them from the allele frequency spectrum analysis. and used them only for training the deep learning model. Variation from these primate diversity studies were already mapped to the human reference (hg19). In addition, for Marmoset and Rhesus, 16 rhesus individuals and 9 marmoset individuals were used to assay variation in the original sequencing of these species' genomes, but individual-level information is not available.

To compare with other primates and mammals, we also downloaded SN Ps of other species from dbSNP, including rhesus, marmoset, pig, cow, goat, mouse, chicken, and zebrafish. dbSNP also included additional orangutan variants, which we only used for training the deep learning model, since individual genotype information was not available for the allele frequency spectrum analysis. We discarded other species, such as dog, cat, or sheep, as dbSNP provides limited numbers of variants for those species.

To map the variants to human. we used the multiple species alignment of 99 vertebrates to ensure orthologous 1:1 mapping to human protein-cnding regions. Mapping variants using the orthologous multiple species alignments was essential to remove artifacts caused by pseudogene or retrotransposed sequences, which occur when directly mapping SNPs between species using tools such as liftOver that allow many-to-1 mappings. In cases where the genome build of the species in dbSNP did not match the genome build of the species in the 99-vertebrate multiple sequence alignment, we used liftOver to update the variants to the genome build used in the multiple sequence alignment. We accepted variants as identical-by-state if they occurred in either reference/alternative orientation, for example, if the human reference was G and the alternative allele was A, this was considered to be identical-by-state with a variant in another species where the reference was A and the alternative allele was G. To ensure that the variant had the same predicted protein-coding consequence in both human and the other species, we required that the other two nucleotides in the codon are identical between the species, for both missense and synonymous variants. Polymorphisms from each species included in the analysis are listed in Supplementary Data File 1 and detailed metrics are shown in Supplementary Table 1.

To ensure that the variants from each dbSN P submitter batch were of high quality and aligned correctly to human, we calculated the missense:synonymous ratio for each batch. confirming that this was less than the expected ratio of 2.2:1; most species had ratios lower than 1:1, especially zebrafish and mouse, which would be expected to have very large effective population sizes. We excluded two batches of SNPs from cow which had unusually high missense:synonymous ratios from further analysis (snpBatch_1000_BULL_GENOMES_1059190.gz with ratio of 1.391 and snpBatch_COFACTOR_GENOMICS_1059634.gz with ratio of 2.568). The mean missense:synonymous ratio for the remaining cow batches was 0.8:1.

## Correction for the Effect of Allele Frequency on Missense:Synonymous Ratio, Mutational Rate, Genetic Drift, and GC-Biased Gene Conversion

In addition to the action of purifying selection, the observed depletion of human missense variants at high allele frequencies could also be affected by factors not related to natural selection. The probability of a neutral mutation appearing at a particular allele frequency in the population is a function of mutational rate, gene conversion, and genetic drift, and these factors could potentially introduce a bias in the missense:synonymous ratio across the allele frequency spectrum even in the absence of selective forces.

To compute the expected missense:synonymous ratios at each allele frequency category in the absence of protein-coding selection, we selected variants within intronic regions 31-50bp upstream and 21-50bp downstream of each exon. These regions were chosen to be distant enough to avoid effects of the extended splice motifs. Because these regions are near the edges of the exome capture sequence for ExAC/gnomAD exomes, to ensure fair ascertainment of variants we removed any chrX regions and excluded regions with mean read depth < 30. Each variant and its immediately upstream and downstream nucleotides falls within one of 64 trinucleotide contexts. If we mutate the middle nucleotide into three other bases, in total, $64 \times 3 = 192$ tri-nucleotide configurations are possible. As tri-nucleotide configurations and their reverse complements are equivalent, there are effectively 96 tri-nucleotide contexts. We observed that tri-nucleotide context has a very strong effect on mutational rate, and a smaller effect on GCbiased gene conversion, making tri-nucleotide context effective for modeling these variables.

Within these intronic regions, we took each variant from the 126,136 ExAC/gnomAD exomes and separated them into $4 \times 192$ categories, based on four categories of allele frequency (singleton, more than singleton~0.01%, 0,01% ~0.1%, > 0.1%) and 192 tri-nucleotide contexts. We normalized the number of variants observed in each of the $4 \times 192$ categories (allele frequency $\times$ tri-nucleotide context) by dividing by the total number of possible variants with that tri-nucleotide context (obtained by substituting each nucleotide in the intronic sequence three different ways). For each of the 192 tri-nucleotide contexts, we had thereby obtained the expected fraction of variants to fall in each of the 4 allele frequency categories in the absence of protein-coding selection. This implicitly models the effects of mutational rate, GC-biased gene conversion, and generic drift that are due to differences in tri-nucleotide context (Supplementary Table 7).

To get the expected missense:synonymous ratio in each allele frequency category, we counted the total number of possible synonymous and missense mutations in the human genome accessible by single nucleotide substitutions, and assigned each of them to one of the 192 trinucleotide contexts. For each context, we used the $4 \times 192$ table to calculate the number of variants expected to fall within each of the 4 allele frequency categories. Finally. we summed up the number of synonymous and missense variants across the 192 tri-nucleotide contexts, to obtain the total expected numbers of synonymous and missense variants in each of the four allele frequency categories (**FIG. 51** and Supplementary Table 8 **(FIG. 59))**.

The expected missense:synonymous ratios were nearly constant across the allele frequency spectrum and close to the ratio of 2.23:1 that would be expected for *de novo* variants in the absence of natural selection, with the exception of singleton variants, whose expected missense:synonymous ratio was 2.46:1. This indicates that due to the action of factors independent of protein-coding selective pressures (mutational rate, gene conversion. genetic drift), variants with the singleton allele frequency category in ExAC/gnomAD are expected to have a missense:synonymous ratio about 10% higher than that of *de novo* mutations by default. To correct for this, we adjusted down the missense:synonymous ratio for singletons by 10% in the allele frequency analyses **(FIGs. 49A, 49B, 49C, 49D,** and **49E** and **FIGs. 50A, 50B, 50C,** and **50D)**. This small adjustment reduced the estimated missense depletion for common human variants present in primates and other mammals (shown in **FIGs. 49A, 49B, 49C, 49D,** and **49E** and **FIGs. 50A, 50B, 50C,** and **50D**) by roughly -3.8%. The higher missense:synonymous ratio for singleton variants is due to transition mutations (which are more likely to create synonymous changes) having higher allele frequencies owing to higher mutation rate than transversion mutations (which are more likely to create missense changes).

Moreover, this explains the observed missense:synonymous ratio of 2.33:1 for singleton variants in ExAC/gnomAD, which exceeds the expected ratio for *de novo* mutations of 2.23:1. After factoring in the effects of allele frequency spectrum on missense:synonymous ratio, this actually reflects a 5.3% depletion of singleton variants compared to expectation, which would presumably be due to selection against pathogenic missense mutations with *de novo* dominant modes of inheritance. Indeed, when we consider only haploinsufficient genes with high probability of loss of function (pLI > 0.9), the missense:synonymous ratio for ExAC/gnomAD singleton variants is 2.04:1, indicating a depletion of around ~17% within haploinsufficient genes. This result is concordant with prior estimates that 20% of missense mutations are equivalent to loss of function mutations, assuming some degree of incomplete penetrance.

We also specifically examined missense:synonymous ratios for CpG and non-CpG variants across the human allele frequency spectrum, due to the large differences in their mutation rates **(FIGs. 52A, 52B, 52C,** and **52D).** We confirmed that for both CpG and non-CpG mutations, human variants that are identical-by-state with chimpanzee common polymorphisms have nearly constant missense:synonymous ratios across the allele frequency spectrum.

## Depletion of Human Missense Variants that are Identical-by-State with Polymorphisms in Other Species

To evaluate whether variants from other species would be tolerated at common allele frequencies (> 0.1%) in human, we identified human variants that were identical-by-state with variation in the other species. For each of the variants, we assigned them to one of the four categories based on their allele frequencies in human populations (singleton, more than singleton~0.01%, 0.01%~0.1%, > 0.1%), and estimated the decrease in missense:synonymous ratios (MSR) between the rare (< 0.1%) and common (> 0.1%) variants. The depletion of identical-by-state missense variants at common human **allele** frequencies (> 0.1%) indicates the fraction of variants from the other species that are sufficiently deleterious that they would be filtered out by natural selection at common allele frequencies in human.

$$\% \text{ depletion} = \frac{\text{MSR}_{\text{rare}} - \text{MSR}_{\text{comm}}}{\text{MSR}_{\text{rare}}}$$

The missense:synonymous ratios and the percentages of depletion were computed per species and are shown in **FIG. 50B** and Supplementary Table 2. In addition, for chimpanzee common variants **(FIG. 49A),** chimpanzee singleton variants (**FIG. 49C**), and mammal variants **(FIG. 50A),** we performed the Chi-squared ($\chi2$) test of homogeneity on 2×2 contingency table to test if the differences in missense:synonymous ratios between rare and common variants were significant.

Because sequencing was only performed on limited numbers of individuals from the great ape diversity project, we used the human allele frequency spectrum from ExAC/gnomAD to estimate the fraction of sampled variants which were rare (< 0.1%) or common (>0.1%) in the general chimpanzee population. We sampled a cohort of 24 individuals based on the ExAC/gnomAD allele frequencies, and identified missense variants that were observed either once, or more than once, in this cohort. Variants that were observed more than once had a 99.8% chance of being common (> 0.1%) in the general population, whereas variants that were observed only once in the cohort had a 69% chance of being common in the general population. In **FIGs. 49B** and **49C,** we show that as a consequence of some of the chimpanzee singleton variants being rare deleterious mutations, we observe depletion of singleton chimpanzee variants at high allele frequencies in human, but not for the common chimpanzee variants. Roughly half of chimpanzee variants observed in the cohort of 24 individuals were observed only once, and roughly half were observed more than once.

To confirm that the observed depletion for missense variants in more distant mammals was not due to a confounding effect of genes that are better conserved, and hence more accurately aligned, we repeated the above analysis, restricting only to genes with > 50% average nucleotide identity in the multiple sequence alignment of 11 primates and 50 mammals compared with human (see Supplementary Table 3). This removed ~7% of human protein-coding genes from the analysis, without substantially affecting the results.

## Fixed Substitutions Among Primates. Mammals and Distant Vertebrates

To ensure that our results using dbSNP variation were not affected by issues with the variant data, or domestication artifacts (since most of the species selected from dbSNP were domesticated), we also repeated the analyses using fixed substitutions from pairs of closely-related species in lieu of intra-species polymorphisms. We downloaded the phylogenetic tree of 100 vertebrate species (http://hgdownload.soe.ucse.edu/goldenPath/hg19/mul tiz100way/hg19.100way.commonNames.n h) from UCSC genome browser, with phylogenetic distance measured in branch length (mean number of nucleotide substitutions per position). We selected closely-related species pairs (branch length < 0.25) for further analysis. To identify fixed substitutions between closely related species pairs, we downloaded coding regions for the multiple sequence alignments of 99 vertebrate genomes with human, as well as the alignments of 19 mammalian (16 primates) genomes with human from the UCSC genome browser. The additional 19 mammalian multiple species alignment was necessary because some of the primate species, such as bonobo, were absent in the 99 vertebrate alignment (http://hgdownload.soe.ucsc.edu/goldenPath/hg38/multiz20way/alignments/knownCanonical.exo nNuc.fa.gz). In total, we obtained 15 pairs of closely related species, including live primate pairs, as listed in **FIG. 50D** and Supplementary Table 4.

We took the multiple sequence alignments of 19 mammalians or 99 vertebrate genomes with human within the canonical coding regions and obtained nucleotide substitutions between each selected pair of vertebrates, listed in Supplementary Data File 2. These substitutions were mapped to the human genome, requiring that the other two nucleotides in the codon were unchanged between human and the other species, and accepting the variant in either reference or alternative orientation. Using human variants that were identical-by-state with the fixed substitutions from related species pairs, we calculated missense:synonymous ratios for variants in the rare (< 0.1%) and common (> 0.1%) allele frequency categories to obtain the fraction of fixed substitutions under negative selection, as shown in Supplementary Table 4.

## ClinVar Analysis of Polymorphism Data for Human, Primates, Mammals, and Other Vertebrates

To examine the clinical impact of variants that are identical-by-state with other species, we downloaded the release variant summary for the ClinVar database (ftp://ftp.ncbi.nlm.nib.gov/pub/clinvar/clinvar_20171029.vcf.gz released on 02-Nov-2017)12. The database contained 324,698 variants on the hg19 genome build. of which 122,884 were missense single nucleotide variants mapping to our list of pmtein-coding genes (Supplementary Table 9). Most of the variants in the ClinVar database were not of missense consequence, and were excluded. Next, we filtered variants with conflicting interpretations of pathogenicity, and kept only those with benign, likely benign, pathogenic, and likely pathogenic annotations. We merged variants with Benign or Likely Bemgn annotations into a single category. as well merging variants with Pathogenic or Likely Pathogenic annotations. Following the filtering steps shown in Supplementary Table 9, there are a total of 24,853 variants in the pathogenic category and 17,775 variants in the benign category; the remainder were excluded because they arevariants of unknown significance or conflicting annotations.

To get a baseline for ClinVar missense variants in the human population, we examined ClinVar missense variants in a cohort of 30 individuals sampled from ExAC/gnomAD allele frequencies. This cohort size was chosen to reflect roughly the number of individuals sequenced in the primate diversity project study. We report the average number of pathogenic and benign variants in a cohort of 30 humans (**FIG. 49E)** from 100 such simulations. Because curators have systematically annotated common human variants with benign consequence in ClinVar, we excluded variants with greater than 1% allele frequency to avoid this curation bias.

We analyzed ClinVar variants that were identical-by-state with variation in primates, mammals and other vertebrates. The numbers of benign and pathogenic variants for each species are shown in Supplementary Table 10. A summary of the number of ClinVar variants that were present in humans, primates, and more distant mammals is shown in **FIGs. 49E** and **50B,** along with the results from Chi-squared ($\chi$2) test of homogeneity for differences in the ratio of benign to pathogenic variants.

## Generation of Benign Variants for Model Training

Variants that arc common in the human population are largely neutral aside from rare instances of founder effects or balancing selection, making them suitable as a benign training dataset for machine learning that is unaffected by biases in human interpretation. We used allele frequency data from 123,136 exomes from the ExAC/gnomAD database (release v2.0), excluding variants that did not pass filters, which left us with 83,546 missense variants with overall population allele frequency >= 0.1% within canonical protein-coding tenscripts.

Based on our earlier results showing that variants present in primates arc largely benign in human, we created a benign training dataset for machine learning comprising common human variants (> 0.1% allele frequency), variants from chimpanzee, bonobo, gorilla, and orangutan from the great ape diversity project and additional primate sequencing, and rhesus, orangutan, and marmoset variants from dbSNP. In total, 301.690 unique primate variants were added to the benign training set, according to one implementation. The number of benign training variants contributed by each source is shown in Supplementary Table 5.

A caveat is that while most primate variants are common in their respective populations, a minority of them are rare variants. Because the non-human primate species have had limited numbers of individuals sequenced, we would expect that the set of ascertained variants generally represent common variation. Indeed, we find that the missense:synonymous ratio for variants from each of the primate species is less than half of the expected 2.23:1 ratio for *de novo* mutation, indicating that these are mostly common variants that have already passed through the sieve of selection. Moreover, for the chimpanzee cohort, we have estimated that ~84% of the ascertained variants are present at common allele frequencies (> 0.1%) in their inspective populations. Since ~50% of newly arising missense mutations are filtered by purifying selection at common human allele frequencies (> 0.1%) (**FIG. 49A**), this figure is consistent with ~1 6% rare variants accounting for the observed depletion of 8.8% of human missense variants that are identical-by-state with observed primate variation (**FIG. 49D**).

Applying the estimate that ~20% of human missense mutations are loss of function equivalent, primate variants would be expected to comprise 3.2% fully pathogenic mutations, 91.2% benign mutations (tolerated at > 0.1% allele frequency), and 5.6% intennediate mutations that do not fully abrogate gene function, yet are deleterious enough to be filtered out at common allele frequencies (> 0.1%). Despite the known imperfections in this training dataset, the classification accuracy of the deep learning network was much better when trained on a benign training dataset comprising both common human variants and primate variants, compared to common human variants alone. Therefore, it appears that at the current classification accuracy, the amount of training data available is the stronger limitation. As larger numbers of individuals are sequenced in each primate species, it will be possible to prepare training datasets that contain a higher fraction of primate common variants, reducing contamination from pathogenic variants in the training dataset and further improving classification performance.

### Generation of Unlabeled Variants to Complement the Benign Training Dataset

All possible missense variants were generated from each base position of canonical coding regions by substituting the nucleotide at the position withthe other three nucleotides. We excluded variants that were observed in the 123,136 exomes from ExAC/gnomAD, and variants in start or stop codons. In total, 68,258,623 unlabeled variants were generated. We assigned each of the unlabeled variants to one of the 96 different tri-nucleotide context categories. We trained the deep learning network using a semi-supervised approach, by sampling variants from this unlabeled dataset that match the variants in the benign dataset by tri-nucleotide context, and training the classifier to discriminate between benign and unlabeled training examples.

### Additionat Filtering of Unlabeled Variants

By presenting examples of benign and unlabeled variants along with the flanking amino acid sequence, the deep learning network learns regions of proteins that are highly intolerant of mutations. However, the absence of common variants within a region of the protein sequence may be due to strong purifying selection, or it may be due to technical artifacts that prevent variants from being called in the region. To correct for the latter, we removed variants from both the benign and unlabeled datasets from regions where the ExAC/gnomAD dataset had mean coverage < 1. Similarly, when matching unlabeled variants to primate variants in the benign dataset during training, we excluded unlabeled variants from regions where that primate did not have orthologous alignable sequence with human in the multiple sequence alignment.

### Withheld Primate Variants for Validation and Testing, and *de novo* Variants from Affected and Unaffected Individuals

For validating and testing of the deep learning network, we randomly sampled two sets of 10,000 primate variants for validation and testing, which we withheld from training. The rest of the primate variants, along with the common human variants (> 0.1% allele frequency), were used as the benign dataset for training the deep learning network. In addition, we also sampled two sets of 10,000 unlabeled variants that were matched to the withheld primate variants for the validation set and the test set.

We used the 10,000 withheld primate variants in the validation set and the matched 10,000 unlabeled variants to monitor the performance of the deep learning network during the course of training, by measuring the ability of the network to discriminate between variants in the two sets. This enabled us to determine a stopping point for training and avoid overfitting, once the performance of the network had saturated.

We used the 10,000 withheld primate variants in the test dataset to benchmark the deep learning network as well as the other 20 classifiers. Because the different classifiers had widely varying score distributions, we used these unlabeled variants to identify the 50th-percentile threshold for each classifier. We benchmarked each classifier on the fraction of variants in the 10,000 withheld primate variant test set that were classified as benign at the 50th-percentile threshold for that classifier, to ensure fair comparison between the methods.

To evaluate the perfomance of the deep learning network in clinical settings using *de novo* variants in affected individuals with neurodevelopmental disorders and *de novo* variants in healthy controls, we downloaded the *de novo* variants from the Deciphering Developmental Disorders (DDD) study, and *de novo* variants from the healthy sibling controls in the Simons Simplex Collection (SSC) autism study. The DDD study provides a confidence level for *de novo* variams, and we excluded variants from the DDD dataset with a threshold of < 0.1 as potential false positives due to variant calling errors. In total, we had 3,512 missense *de novo* variants from DDD affected individuals and 1,208 missense *de novo* variants from healthy controls.

To better model the real-world clinical scenario of distinguishing between benign and pathogemc variants of uncertain significance within a panel of candidate disease genes, we limited the analysis to only *de novo* variants within 605 genes that were associated with disease in the DDD study (p<0.05), calculated only from protein-truncating variation (Supplementary Table 18). We evaluated the gene-specific enrichment of protein-truncating *de novo* mutations by computing the statistical significance under a null hypothesis of the expected number of *de novo* mutations given the gene-specific mutation rate and the number of considered chromosomes. We selected the 605 genes with a nominal P-value < 0.05. We calculated the excess of synonymous and missense *de novo* mutations within the 605 genes (**FIG. 22A**) as the ratio of counts of observed *de novo* mutations versus expected *de novo* mutations, as well as the difference of observed *de*

*novo* mutations minus expected *de novo* mutations. Within these 605 genes, we observed 380 de *novo* missense mutations from the DDD affected individuals (**FIG. 22A**). For each of the classifiers, including our own, a small fraction of variants did not have predictions, generally because they did not map to the same transcript models used by the classifier. Hence, for our deep learning network, we performed the downstream analyses in **FIGs. 22A, 22B, 22C, 22D.** and **22E** using 362 *de novo* missense mutations from DDD affected individuals and 65 *de novo* missense mutations from healthy controls.

**Saturation of All Possible Human Misseune Mutations with Increasing Number of Primate Populations Sequenced**

We investigated the expected saturation of all -70M possible human missense mutations by common variants present in the 504 extant primate species. For each primate species, we simulated 4 times the number of common missense variants observed in human (~83,500 missense variants with allele frequency > 0.1%), because humans appear to have roughly half the number of variants per individual as other primate species, and about ~50% of human missense variants have been filtered out by purifying selection at > 0.1% allele frequency (**FIG. 49A**). We assigned simulated variants based on the observed distribution of human common missense variants in the 96 tri-nucleotide contexts. For example, if 2% of human common missense variants were from the CCG>CTG tri-nucleotide context, we would require that 2% of the simulated variants were randomly sampled CCG>CTG mutations. This has the effect of controlling for the effects of mutational rate, genetic drift, and gene conversion bias, using tri-nucleotide context.

The curves in **FIG. 23D** show the cumulative saturation of the ~70M possible human missense mutations by common variants present in any of the 504 primate species, assuming that we ascertain all common variants in each primate species (> 0.1% allele frequency). From **FIG. 49A,** roughly ~50% of human missense mutations are sufficiently deleterious in both human and other primates to prevent them from rising to common allele frequencies (> 0.1%), and therefore the curves in **FIG. 23D** represent the fraction of non-deleterious human missense mutations saturated by common primate variation as the number of primate species grows. We show that with 504 primate species, the majority of non-deleterious human missense mutations will be saturated, with non-deleterious CpG mutations saturated with a much smaller number of species due to their higher mutation rate.

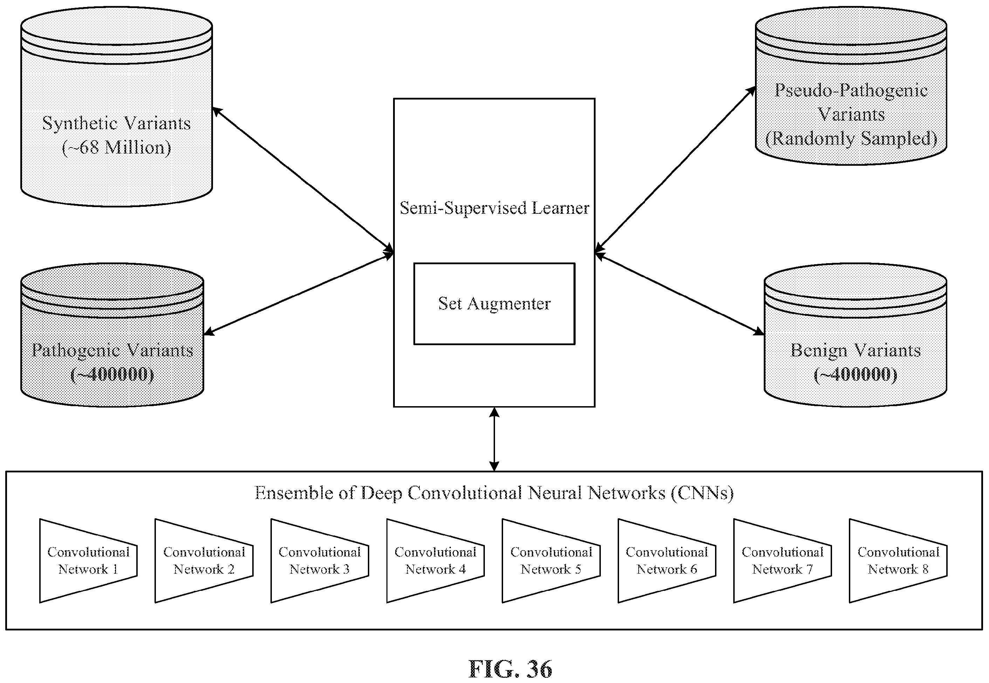

To model the fraction of human common missense variants (> 0.1% allele frequency) discovered with increasing size of human cohorts surveyed (**FIG. 36**), we sampled genotypes according to gnomAD allele frequencies. The fraction of gnomAD common missense variants discovered was averaged across 100 simulations for each sample size from 10 to 100K.

**Prediction of Secondary Structure and Solvent Accessibility**

The deep learning network for pathogenicity prediction contains 36 total convolutional layers, including 19 convolutional layers for the secondary structure and solvent accessibility prediction networks, and 17 for the main pathogenicity prediction network which takes as put the results of the secondary structure and solvent accessibility networks. Because the crystal structures of most human proteins are unknown, we trained two models to enable the network to learn protein structure from primary sequence. Both models used the same network architecture and inputs shown in **FIG. 6.** The inputs to the secondary structure and solvent accessibility networks is a 51 length $\times$ 20 amino acid position frequency matrix encoding conservation information from the multiple sequence alignment of human with 99 other vertebrates.

The secondary structure network is trained to predict 3-state secondary structure: alpha helix (H), beta sheet (B), and coils (C). The solvent accessibility network is trained to predict 3-state solvent accessibility: buried (B), intermediate (I), and exposed (E). Both networks only take primary sequence as their inputs, and were trained using labels from known crystal structures in the Protein DataBank. The models predict one state for each amino acid residue.

**Data Preparation for Prediction of Secondary Structure and Solvent Accessibility**

We used unrelated crystal structures from the Protein Databank for training the models. Amino acid sequences with more than 25% sequence similarity were removed. In total, 6,367 protein sequences were used for training, 400 for validation, and 500 for testing (Supplementary Table 13). The data used for training, including the amino acid sequence and the secondary structure and solvent accessibility labels is available from the RaptorX website: http://raptorx.uchicago.edu/download/.

Most solved crystal structures are of non-human proteins, hence for pre-training the secondary structure and solvent models, we used the RaptorX suite (based on PSI-BLAST) to obtain related sequences, since human-based multiple sequence alignments were generally not available. We generated multiple sequence alignments for the proteins using the CNFsearch1.66_release tool from RaptorX, and counted the amino acids at each position from the 99 closest alignments to fonn the position frequency matrix. For example, the specific commands using RaptorX to retrieve the multiple sequence alignment for the lu7lA.fasta protein were as follows: % ./buildFeature -i lu71A.fasta -c 10 -o ./TGT/lu71A.tgt % ./CNFsearch -a 30 -q lu71A

For each amino acid position in the dataset, we took a window from the position frequency matrix corresponding to the flanking 51 amino acids, and used this to predict the label for either secondary structure or solvent accessibility for the amino acid at the center of the 51- length amino acid sequence. The labels for secondary structure and relative solvent accessibility were directly obtained from the known 3D crystal structure of the protein using the DSSP software and did not require prediction from primary sequence. To incorporate the secondary structure and solvent accessibility networks as part of the pathogenicity prediction network, we computed position frequency matrices from the human-based 99 vertebrate multiple sequence alignments. Although the conservation matrices generated from these two methods are generally similar, we enabled backpropagation through the secondary structure and solvent accessibility models during training for pathogenicity prediction to allow fine-tuning of the parameter weights.

## Model Architecture and Training

We trained two separate deep convolutional neural network models to predict secondary structure and relative solvent accessibility of proteins. The two models have identical architecture and input data, but differ on the prediction states. We conducted detailed hyperparameter search to optimize the models for best performance. Both our deep learning network for pathogenicity prediction and deep learning networks for predicting secondary structure and solvent accessibility adopted the architecture of residual blocks, which has become widely adopted due to its success in image classification. The residual blocks comprise repeating units of convolution, interspersed with skip connections that allow information from earlier layers to skip over residual blocks. In each residual block, the input tayer is first batch normalized, followed by an activation layer using rectified linear units (ReLU). The activation is then passed through a 1D convolution layer. This intermediate output from the 1D convolution layer is again batch normalized and ReLU activated, followed by another 1D convolution layer. At the end of the second 1D convolution, we summed its output with the original input into the residual block, which acts as a skip connection by allowing the original input information to bypass the residual block. In such an architecture, termed a deep residual learning network by its authors, the input is preserved in its original state and the residual connections are kept free of nonlinear activations from the model, allowing effective training of deeper networks. The detailed architecture is provided in **FIG. 6** and Supplementary Tables 11 (**FIGs. 7A** and **7B**) and 12 (**FIGs. 8A** and **8B**).

Following the residual blocks, the softmax layer computes probabilities of the three states for each amino acid, among which the largest softmax probability determines the state of the amino acid. The model is trained with accumulated categorical cross entropy loss function for the whole protein sequence using the ADAM optimizer. Once the networks had been pretrained on secondary structure and solvent accessibility, instead of directly taking the output of the networks as inputs for the pathogenicity prediction network, we took the layer before the softmax layer, so that more information would pass through to the pathogenicity prediction network.

The best testing accuracy achieved for the 3-state secondary structure prediction model is 79.86 % (Supplementary Table 14), similar to the state-of-the-art accuracy predicted by DeepCNF model30. The best testing accuracy for the 3-state solvent accessibility prediction model is 60.31% (Supplementary Table 14), similar to the current best accuracy predicted by RaptorX on the similar training dataset. We also compared the predictions of the neural network when using DSSP-annotated structure labels for the approximately ~4000 human proteins that had crystal structures, versus using the standard PrimateAl model which uses predicted structure labels only. We did not obtain further improvement in pathogenicity prediction accuracy when using the DSSP-annorated labels (Supplemental Table 15).

## Input Features for Deep Learning Models for Pothotgenicity Prediction

The training dataset for the pathogenicity prediction network contains 385,236 labeled benign variants and 69,258,623 unlabeled variants after filtering, For each variant, we generated the following input features. The first input feature of each variant is its 51-length flanking amino acid sequence, i.e., 25 amino acids to each side of the variant obtained from the reference sequence of hg19, to provide the deep learning models the sequence context of the variant. In total, this flanking reference sequence is 51 amino acids in length. Through empirical observation we found that amino acid representation of the protein sequence was more effective than representing the protein coding sequence using nucleotides.

The second feature in the 51-length flanking human amino acid sequence with the alternative amino acid substituted at the central position by the variant. The alternative flanking sequence is the same as the reference flanking sequence in the first feature, except that the middle position of the sequence contains the alternative amino acid, instead of the reference amino acid. Both reference and alternative human amino acid sequences were converted to one- hot encoded vectors of length $51 \times 20$, where each amino acid is represented by a vector of 19 amino acids with value 0. and a single amino acid with value 1.

Three position frequency matrices (PFMs) are generated from multiple sequence alignments of 99 vertebrates for the variant, including one for 11 primates, one for 50 mammals excluding primates, and one for 38 vertebrates excluding primates and mammals. Each PFM has dimensions L x 20, where L is the length of flunking sequences around the variant (in our case, L represents 51 amino acids).

for the input to the pre-trained 3-state secondary structure and 3-state solvent accessibility networks, we used a single PFM matrix generated from the multiple sequence alignments for all 99 vertebrates, also with length 51 and depth 20. After pre-training the networks on known crystal structures from the Protein DataBank, the final two layers for the secondary structure and solvent models were removed (the global maxpool layer and the output layer), and the $51 \times 40$ shape of the previous layer's output was used as input for the pathogenicity prediction network. We allowed backpropagation through the structural layers of the network to fine-tune parameters.

## Semi-Supervised Learning

Because semi-supervised learning algorithms use both labeled and unlabeled instances in the training process, they can produce classifiers that achieve better performance than completely supervised learning algorithms that have only a small amount of labeled data available for training. The principle behind semi-supervised learning is that intrinsic knowledge within unlabeled data can be leveraged in order to strengthen the prediction capability of a supervised model that only uses labeled instances, thereby providing a potential advantage for semi-supervised learning. Model parameters learned by a supervised classifier from a small amount of labeled data may be steered towards a more realistic distribution (which more closely resembles the distribution of the test data) by the unlabeled data.

Another challenge that is prevalent in bioinformatics is the data imbalance problem. The data imbalance phenomenon arises when one of the classes to be predicted is underrepresented in the data because instances belonging to that class are rare (noteworthy cases) or hard to obtain. Ironically, minority classes are typically the most important to learn, because they may be associated with special cases.

An algorithmic approach to handle imbalanced data distributions is based on ensembles of classifiers. Limited amounts of labeled data naturally lead to weaker classifiers, but ensembles of weak classifiers tend to surpass the performance of any single constituent classifier. Moreover, ensembles typically improve the prediction accuracy obtained fmm a single classifier by a factor that validates the effort and cost associated with learning multiple models. Intuitively, aggregating several classifiers leads to better overfitting control, since averaging the high variability of individual classifiers also averages the classifiers" overfitting.

We pursued a semi-supervised learning strategy because of the lack of adequately sized datasets of confidently labeled pathogenic variants. Although the ClinVar database has over 300,000 entries, after removing variants of uncertain significance, there were only ~42,000 missense variants remaining with non-contlicting interpretations of pathogenicity.

Systematic reviews have also found that these entries often have insufficient climcal evidence to support their annotated pathogenicity. Moreover, most of the variants in human curated databases tend to be within a very small set of genes, making them mismatched for variants in benign training datasets, which are ascertained genome-wide using human common variants or chimpanzee-human fixed substitutions. Given how differently the datasets were ascertained, training a supervised learning model with human-curated variants as the pathogenic set and genome-wide common variants as the benign set is likely to introduce significant biases.

We trained the deep learning network to discriminate between a set of labeled benign variants and an unlabeled set of variants that were carefully matched to remove biases. According to one implementation, the set of 385,236 labeled benign variants comprised human common variants (> 0.1% allele frequency) from the ExAC/gnomAD database and vanants from six species of non-human primates.

We sampled a set of unlabeled variants, requiring marching with the benign variants on trinucleotide context (to control for mutational rate, genetic drift, and gene conversion), and adjusting for the impact of alignability and sequence coverage on variant ascertainment. Because the number of unlabeled variants greatly exceeds the labeled benign variants, we obtained a consensus prediction by training eight models that use the same set of labeled benign variants and eight randomly sampled sets of unlabeled variants and taking the average of their predictions.

The motivation of choosing semi-supervised learning is that human-curated variant databases are unreliable and noisy. and in particular, the lack of reliable pathogenic variants. We obtained a set of reliable benign variants from human common variants from gnomAD and primate variants. For pathogenic variants, we adopted an iterative balanced sampling approach to initially sampling pathogenic variants from a set of unknown variants (VUS variants without annotated clinical significance).

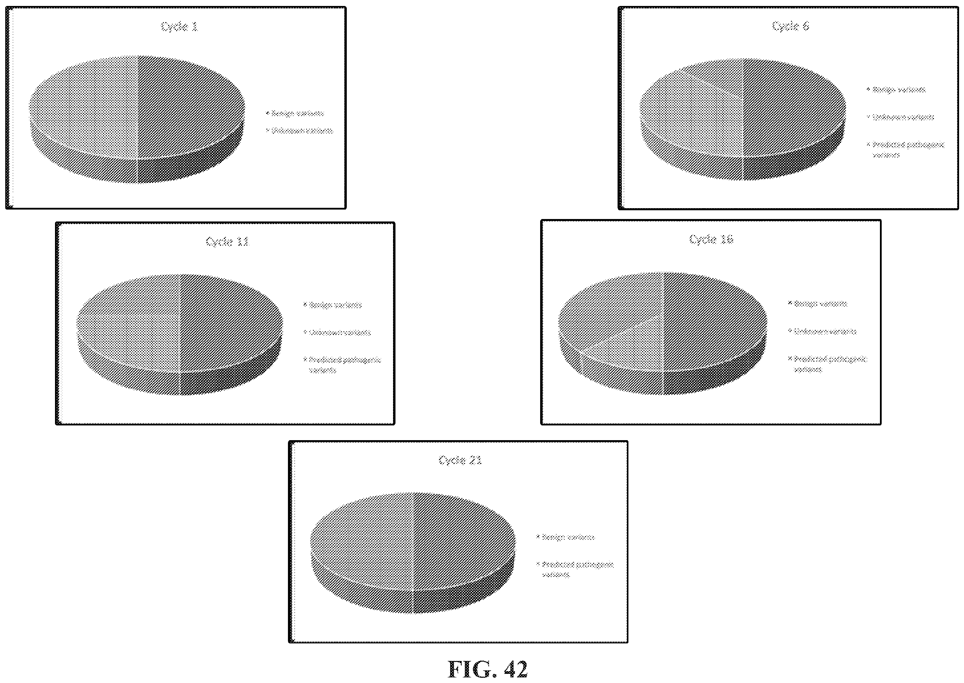

To reduce the sampling bias. we trained an ensemble of eight models that use the same set of benign traming variants and eight different sets of pathogenic variants. Initially, we randomly sampled unknown variants to represent pathogenic variants. Next, iteratively, the ensemble of models are used to score a set of unknown variants that were not involved in the prior training cycle. The top scored pathogenic variants are then obtained to replace 5% of random unknown variants in the prior cycle. Note that we retained 25% more top scored pathogenic variants than needed, so that we can sample eight different sets of scored pathogenic variants to replace unknown variants which increases randomness for the eight models. Then the new pathogenic training set is formed and a new cycle of training isexecuted . This process is repeated till initial randomly-sampled unknown variants are all replaced by highly confident pathogenic variants predicted by the ensemble models. **FIG. 42** is an illustration of the iterative balanced sampling process.

## Balancing the Bening and Unknown Training Sets

The sampling scheme of unknown variants that match benign variants is useful in reducing the bias of our model training. When the unknown variants are sampled randomly, deep learning models often extract biased information and present trivial solutions. For example, if an amino acid substitution K->M occurs more frequently in unknown variants than benign variants, the deep learning models tend to always classify K->M substitutions as pathogenic. Thus, it is important to balance the distribution of amino acid substitutions between the two training sets.

Higher mutable classes like CpG transitions have a huge representation bias in the benign common variants. The orthologous variants from other primates also follow the human mutation rates, implying enrichment of the highly mutable classes in the overall benign training set. If the sampling procedure of unknown variants is not well controlled and balanced, deep learning models tend to be more likely to classify CpG transitions as benign, compared to a less represented class such as transversion or non CpG transitions.

To prevent deep learning models from converging to a trivial, non-biological solution, we consider balancing the trinucleotide contexts of benign and unknown variants. The trinucleotide is formed by the base before the variant, the reference base of the variant, and the base after the variant. And the reference base of the variant can be changed into the other three nucleotides. In total, there are 64x3 trinucleotide contexts.

## Iterative Balanced Sampling

### Cycle 1

We sampled the unknown variants to match the exact number of benign variants for each trinucleotide context. In other words, at the first cycle, we mirrored the benign and pathogenic training sets in terms of trinucleotide contexts of variants. The intuition behind such a sampling methodology is that there are equal representation of variants with identical mutation rates between the benign and unknown sets. This prevents the model from converging to a trivial solution based on mutation rates.

### Cycle 2 to Cycle 20

For cycle 2, we applied the trained model from cycle 1 to score a set of unknown variants that are not involved in cycle 1, and replaced 5% of the unknown variants with the top predicted pathogenic variants. This set is purely generated by the model and we applied no balancing for trinucleotide contexts in this set. The rest of 95% of the unknown variants required for training are sampled to be 95% of the counts of cach trinucleotide context in benign variants.

The intuition is that since cycle 1 uses completely matched training sets, the top predicted pathogenic variants are generated without any mutation rate bias. Hence, There is no need to account for any bias in this set. The rest of 95% of the data is still controlled for the trinucleotide context mutation rate to prevent the model from converging to a trivial solution.

For each cycle, the percentage of the replaced unknown variants increases by 5%. For cycle 3, we replaced 5% of the unknown variants with the top predicted pathogenic variants from the model of cycle 3. Accumulatively, the fraction of pathogenic variants increases to 10% and that of the trinucleotide-context-mirrored unknown variants is reduced to 90%. The sampling process is similar for the rest cycles.

### Cycle 21

For cycle 21, the last cycle, the entire pathogenic training set comprise purely of the top pathogenic variants predicted from the deep learning models. Since we have explicitly controlled for the mutation rate bias at every cycle, the pathogenic variants are reliable to use as training data and not affected by the mutation rate bias. Thus, the last cycle of training produces the final deep learning model for pathogenicity prediction.

## Matching the Labeled Benign and Unlabeled Training Sets

Balanced sampling of unlabeled variants is crucial for removing biases that are unrelated to the deleteriousness of the variant. In absence of proper control of confounding effects, deep learning can easily pick up on inadvertently introduced biases to discriminate between the classes. Human common vanants tend to be enriched with variants from highly mutable classes, such as those on CpG islands. Likewise, primate polymorphisms also follow human mutational rates, implying enrichment of highly mutable variants in the overall benign traitung set. If the sampling procedure of unlabeled variants is not well controlled and balanced, deep learning networks tend to rely on mutational rate bias to classify variants, thus they are more likely to classify CpG transitions as benign, compared to less represented classes such as transversion or non-CpG transition. We sampled exactly the same number of unlabeled variants as labeled benign variants in each of the 96 trinucleotide contexts (discussed previously).

When matching unlabeled variants for the primate variants in the labeled benign dataset, we disallowed unlabeled variants from being selected from regions of the human genome where that primate species was not aligned in the multiple sequence alignment, since it was not possible to call a variant in that primate species at that position.

Within each of the 96 trinucleotide contexts, we corrected for sequencing coverage for primate variants. Because of the large number of humans sequenced, common variants in the human population are observed frequently enough that they are well-ascertained even in areas with low sequencing coverage. The same is not true for primate variants, since only a small number of individuals were sequenced. We split the genome into 10 bins based on sequencing coverage in ExAC/gnomAD exomes. For each bin, we measured the fraction of primate variants in the labeled benign dataset versus the unlabeled dataset. We calculated the probability that a primate variant is a member of the labeled benign dataset, based only on sequencing coverage, using linear regression **(FIG. 24).** When selecting unlabeled variants to match the primate variants in the labeled benign dataset, we weighted the probability of sampling a variant based on the sequencing coverage at that position using the regression coefficients.

## Generation of Benign and Unknown Variants

## Common Variants in Human Population

Recent studies demonstrated that common variants in human populations are generally benign. gnomAD provides 90,958 nonsynonymous SNPs with minor allele frequency (MAF) >= 0.1 % within canonical coding regions, according to one implementation, Variants that passed filters are retained. Indels are excluded. Variants that occur in the stan or stop codons are removed, as well as protein-truncating variants. Scrutinizing subpopulations, the total number of missense variants with MAF >= 0.1% within each subpopulation increases to 245,360, according to one implementation. These variants forms part of the training set of benign variants.

## Common Polymorphism in Great Apes

As coding regions are known to be highly conservative, it is straightforward to assume if a polymorphism is segregating in a great ape population at high frequency, it may also have a mild impact on human fitness. Polymorphism data of bonobo, chimp, gorilla, and orangutan from great ape genome projects and other studies were merged with SNPs of rhesus and marmoset from dbSNP.

## Generation of Unknown Variants

All the possible variants are generated from each base position of canonical coding regions by substituting the nucleotide at the position to the other three nucleotides. New codons are formed leading to potential changes of amino acids at the positions. Synonymous changes are filtered.

Variants observed in gnomAD dataser are removed. Variants that occur in the start or stop codons are removed, as well as variants that form stop codons. For SNPs with multiple gene annotations, the canonical gene annotation is selected to represent the SNP's annotation. In total, 68,258.623 unknown variants are generated, according to one implementation.

## Additional Filtering of Variants

In some regions of human genome, it is known to be difficult to align reads. Inclusion of those regions cause confounding effects on the training and testing datasets. For example, regions under high selective pressure tend to have limited numbers of polymorphisms. Whereas, regions that are hard to sequence also have fewer polymorphisms. To avoid such confounding inputs to our models, we removed variants from genes that were not sequenced by gnomAD.

Generally benign variants are discovered in the well-sequenced regions which tend to be conservative across multiple species. While unknown variants are randomly sampled across genomes, which include some poorly-covered regions. This causes ascertainment bias between the benign and unknown sets. To reduce the bias, we filtered out variants with read depths < 10 in gnomAD. We also filtered out all variants which have more than 10% missing data in the flanking sequence alignments across all the mammal species.

### Data for Validation and Testing

For validating and testing the pathogenicity models, we randomly sampled from the big pool of benign variants two sets of 10,000 benign variants for validation and testing, respectively, according to one implementation. The rest of benign variants are used for training the deep learning models. These variants are specifically sampled from orthologous primate variants to ensure fair comparison between methods as some methods are trained on human common variants. We also randomly sampled two sets of 10,000 unknown variants for validation and testing, separately, according to one implementation. We ensure that the number of unknown variants within each of the 192 trinucleotide contexts match that of benign variants for validation and testing sets, respectively.

We evaluated the performance of multiple methods in clinical settings using *de novo* variants of affected children with autism or developmental disability disorder (DDD) and their unaffected siblings. Totally, according to one implementation, there are 3821 missense *de novo* variants from the cases with DDD and 2736 missense *de novo* variants from the cases with autism. There are 1231 missense *de novo* variants for unaffected siblings, according to one implementation.

### Deep Learning Network Architecture

The pathogenicity prediction network receives five direct inputs and two indirect inputs via the secondary structure and solvent accessibility networks. The five direct inputs are the 51-length amino acid sequences $\times$ 20-depth (encoding the 20 different amino acids), and comprise the reference human amino acid sequence without the variant (1a), the alternative human amino acid sequence with the variant substituted in (1b), the PFM from the multiple sequence alignment of primate species (1c), the PFM from the multiple sequence alignment of marmnalian species (1d), and the PFM from the multiple sequence alignment of more distant vertebrate species (1e). The secondary structure and solvent accessibility networks each receive as input a PFM from the multiple sequence alignment (1f) and (1g), and provide their outputs as inputs into the main pathogenicity prediction network. The secondary structure and solvent accessibility networks were pre-trained on known protein crystal structures for the Protein DataBank, and allow backpropagation during the pathogenicity model training,

The five direct input channels are passed through an upsampling convolution layer of 40 kernels with linear activations. The human reference amino acid sequence (1a) is merged with the PFMs from primate, mammal, and vertebrate multiple sequence alignments (Merge 1a). Similarly, the human attemative amino acid sequence (1b), is merged with the PFMs from primate, mammal, and vertebrate multiple sequence alignments (Merge 1b). This creates two parallel tracks, one for the reference sequence, and one with the alternate sequence with the variant substituted in.

The merged feature map of both reference channel and the alternative channel (Merge 1a and Merge 1b) are passed through a series of six residual blocks (Layers 2a to 7a, Merge 2a and Layers 2b to 7b, Merge 2b). The output of the residual blocks (Merge 2a and Merge 2b) are concatenated together to form a feature map of size (51,80) (Merge 3a, Merge 3b) which fully mixes the data from the reference and alternative channels. Next, the data has two paths for passing through the network in parallel, either through a series of six residual blocks containing two convolutional layers each, as defined in section 2.1 (Merge 3 to 9, Layers 9 to 46 excluding layer 21,34). or via skip connections, which connect the output of every two residual blocks after passing through a 1D convolution (Layer 21, Layer 37, Layer 47). Finally, the merged activations (Merge 10) are fed to another residual block (layers 48 to 53, Merge 11). The activations from Merge 11 are given to a 1D convolution with filter size 1 and sigmoid activation (Layer 54), then passed through a global max pooling layer that picks a single value representing the network's prediction for variant pathogenicity. A schematic illustration of the model is shown in **FIG. 3** and Supplementary Table 16 **(FIGs. 4A, 4B,** and **4C).**

### Model Overview

We developed semi-supervised deep convolutional neural network (CNN) models to predict pathogenicity of variants. The input features to the models include protein sequences and conservation profiles flanking variants, and depletion of missense variants in specific gene regions. We also predicted the changes caused by variants to secondary structure and solvent accessibility by deep learning models and integrated that into our pathogenicity prediction model. For training the model, we generated benign variants from common variants of human subpopulations and orthologous variants from primates. However, we are still lacking reliable sources for pathogenic variants. We initially trained the model with benign and unknown variants, and then used a semi-supervised iterative balanced sampling (IBS) algorithm to gradually replace unknown variants with a set of pathogenic variants predicted with high confidence. Finally, we demonstrated that our model outperformed the existing methods in distinguishing de *novo* variants causing developmental disability disorder in humans from benign ones.

## Adoption of Residual Block

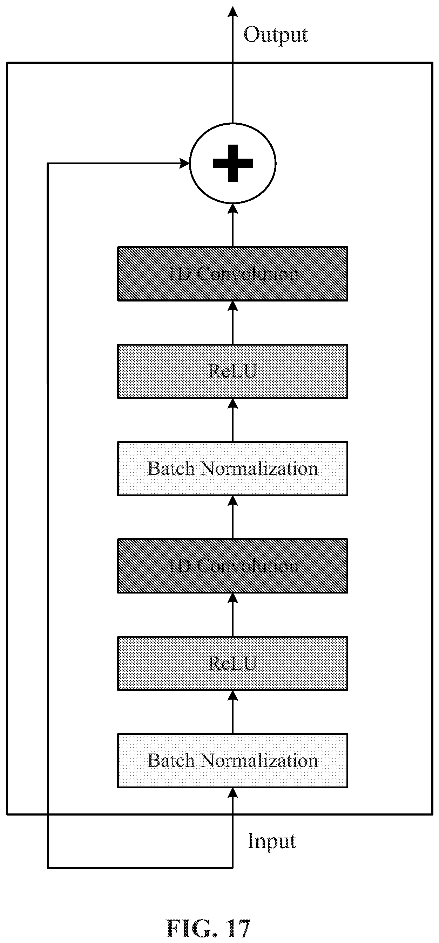

**FIG. 17** illustrates a residual block. Both our deep learning model of pathogenicity prediction and deep learning models for predicting secondary structure and solvent accessibility adopt the definition of residual blocks that was first illustrated in the. Structure of a residual block is shown in figure below. The input layer is first batch normalized, followed by the nonlinear activation "ReLU". The activation is then passed through a ID convolution layer. This intermediate output from the 1D convolution layer is again batch normalized and ReLU activated, followed by another 1D convolution layer. At the end of the second 1D convolution, we merge its output with the original input. In such an architecture, the input is preserved in its original state and the residual connections are kept free of non-linear activations of the model.

Atrous/dilated convolutions allow for large receptive fields with few trainable parameters. An atrous/dilated convolution is a convolution where the kernel is applied over an area larger than its length by skipping input values with a certain step, also called atrous convolution rate or dilation factor. Atrous/dilated convolutions add spacing between the elements of a convolution filter/kernel so that neighboring input entries (e.g., nucleotides, amino acids) at larger intervals are considered when a convolution openinon is performed. This enables incorporation of long-range contextual dependencies in the input. The atrous convolutions conserve pamal convolution calculations for reuse as adjacent nucleotides are processed.

## Novelty of Our Model

Our method differs from the existing methods for predicting pathogenicity of variants in three ways. First, our method adopts a novel architecture of semi-supervised deep convolutional neural networks. Second, reliable benign variants are obtained from human common variants from gnomAD and primate variants, while the highly con fident pathogenic training set is generated through iterative balanced sampling and training, to avoid circular training and testing of models using the identical human curated variant databases. Third, deep learning models for secondary structure and solvent accessibility are integrated into the architecture of our pathogenicity model. The information obtained from the structure and solvent models are not limited to label prediction for specific amino acid residues. Rather, the readout layer is removed from the structure and solvent models, and the pre-trained models are merged with the pathogenicity model. While training the pathogenicity model, the structure and solvent pretrained layers also backpropagate to minimize the error. This helps the pre-trained structure and solvent model to focus on the pathogenicity prediction problem.

## Training Secondary Structure and Solvent Accessibility Models

## Data Preparation

We trained deep convolutional neural networks to predict the 3-state secondary structure and the 3-state solvent accessibility of the proteins. Protein annotations from PDB are used for training the models. Sequences with more than 25% similarity with their sequence profile are removed, according to one implementation. In total, 6,293 protein sequences are used for training, 392 for validation, and 499 for testing, according to one implementation.

The position-specific scoring matrix (PSSM) conservation profiles for the proteins are generated by running PSI-BLAST with E-value threshold 0.001 and 3 iterations to search UniRef90. Any unknown amino acid is set as blank, as well as its secondary structure. We also run PSI.B LAST with similar parameter settings for all human genes to collect their PSSM conservation profiles. These matrices are used for integrating the structure model to pathogenicity prediction. The amino acids of protein sequences are then converted into one-hot encoding vectors. And the protein sequences and PSSM matrices are reshaped to a matrix of Lx20, where L is the length of a protein. The three predicted labels for secondary structure include helix (H), beta sheet (B) and coils (C). The three labels for solvent accessibility include buried (B), intermediate (I) and exposed (E). One label corresponds to one amino acid residue. The labels are coded as one-hot encoding vectors of dimension=3.

## Model Arhitecture and Training

We trained two end-to-end deep convolutional neural network models to predict the 3-state secondary structure and 3-state solvent accessibility of proteins, respectively. The two models have similar configurations, including two input channels, one for protein sequences and the other for protein conservation profiles. Each input channel has the dimension L x 20, where L denotes the length of a protein.

Each of the input channel is passed through a ID convolution layer (layer 1a and 1b) with 40 kernels and linear activations. This layer is used to upsample the input dimensions from 20 to 40. Note all the other layers throughout the model use 40 kernels. The two layer (1a and 1b) activations are merged together by summing the values across each of the 40 dimensions (i.e., merge mode = 'sum'). The output of the merge node is passed through a single layer of 1D convolution (layer 2) followed by linear activation.

The activations from layer 2 is passed through a series of 9 residual blocks (layers 3 to 11) as defined above. The activations of layer 3 is red to layer 4 and layer 4's activation is fed to layer 5 and so on. There are also skip connections that directly sum the output of every 3rd residual block (layers *5,* 8 and 11). The merged activations is then fed to two ID convolutions (layers 12 and 13) with ReLU activations. The activations from layer 13 is given to the softmax readout layer. The softmax computes the probabilities of the three-class outputs for the given input

For the best secondary structure model, the ID convolutions have an atrous rate of 1. For the solvent accessibility model, the last 3 residual blocks (layers 9, 10 and 11) have an atrous rate of 2 to increase the coverage of the kernels. The secondary structure of a protein is strongly dependent on the interactions of amino acids in close proximiry. Thus models with higher kernel coverage improves the performance slightly. On the other hand, solvent accessibility is affected by the long-range interactions between umino acids. Therefore, for the model with high coverage of kernels using atrous convolutions, its accuracy is more than 2% higher than that of the short-coverage models.

The table below provides details about activations and parameters for each layer of the 3-state secondary structure prediction model, according to one implementation.

| Layer | Type | Number of Kernels, Window size | Shape | Atrous rate | Activation |
|---|---|---|---|---|---|
| Input Sequence (layer 1a) | Convolution 1D | 40,1 | (L,40) | 1 | Linear |
| Input PSSM (layer 1b) | Convolution 1D | 40,1 | (L,40) | 1 | Linear |
| Merging Sequence + PSSM | Merge (mode = Concatenate) | - | (L,80) | - | - |
| Layer 2 | Convolution 1D | 40,5 | (L,40) | 1 | Linear |
| Layer 3 | Convolution 1D | 40,5 | (L,40) | 1 | ReLU |
| Layer 4 | Convolution 1D | 40,5 | (L,40) | 1 | ReLU |
| Layer 5 | Convolution 1D | 40,5 | (L,40) | 1 | ReLU |
| Layer 6 | Convolution 1D | 40,5 | (L,40) | 1 | ReLU |
| Layer 7 | Convolution 1D | 40,5 | (L,40) | 1 | ReLU |
| Layer 8 | Convolution 1D | 40,5 | (L,40) | 1 | ReLU |
| Layer 9 | Convolution 1D | 40,5 | (L,40) | 1 | ReLU |
| Layer 10 | Convolution 1D | 40,5 | (L,40) | 1 | ReLU |
| Layer 11 | Convolution 1D | 40,5 | (L,40) | 1 | ReLU |
| Merge activations | Merge - layer 5, 8 and 11.mode='sum' | - | (L,40) | - | - |
| Layer 12 | Convolution 1D | 40,5 | (L,40) | 1 | ReLU |
| Layer 13 | Convolution 1D | 40,5 | (L,40) | 1 | ReLU |
| Output layer | Convolution 1D | 1,3 | (L,3) | - | Softmax |

The details of the solvent accessibility model is shown in the table below, according to one implementation.

| Layer | Type | Number of Kernels, Window size | Shape | Atrous rate | Activation |
|---|---|---|---|---|---|
| Input Sequence (layer 1a) | Convolution 1D | 40,1 | (L,40) | 1 | Linear |
| Input PSSM (layer 1b) | Convolution 1D | 40,1 | (L,40) | 1 | Linear |
| Merging Sequence + PSSM | Merge (mode = Concatenate) | - | (L,80) | - | - |
| Layer 2 | Convolution 1D | 40,5 | (L,40) | 1 | Linear |
| Layer 3 | Convolution 1D | 40,5 | (L,40) | 1 | ReLU |
| Layer 4 | Convolution 1D | 40,5 | (L,40) | 1 | ReLU |
| Layer 5 | Convolution 1D | 40,5 | (L,40) | 1 | ReLU |
| Layer 6 | Convolution 1D | 40,5 | (L,40) | 1 | ReLU |
| Layer 7 | Convolution 1D | 40,5 | (L,40) | 1 | ReLU |
| Layer 8 | Convolution 1D | 40,5 | (L,40) | 1 | ReLU |
| Layer 9 | Convolution 1D | 40,5 | (L,40) | 2 | ReLU |
| Layer 10 | Convolution 1D | 40,5 | (L,40) | 2 | ReLU |
| Layer 11 | Convolution 1D | 40,5 | (L,40) | 2 | ReLU |
| Merge activations | Merge - layer 5, 8 and 11,mode='sum' | - | (L,40) | - | - |
| Layer 12 | Convolution 1D | 40,5 | (L,40) | 1 | ReLU |
| Layer 13 | Convolution 1D | 40,5 | (L,40) | 1 | ReLU |
| Output layer | Convolution 1D | 1,3 | (L,3) | - | Softmax |

The secondary structure class of a specific amino acid residue is determined by the largest predicted softmax probabilities. The model is trained with accumuland categorical cross entropy loss function for the whole protein sequence using ADAM optimizer for optimizing the backpropagation.

The best testing accuracy for the 3-state secondary structure prediction model is 80.32%, similar to the state-of-the-art accuracy predicted by DeepCNF model on a similar training dataset.

The best testing accuracy for the 3-state solvent accessibility prediction model is 64.83%, similar to the current best accuracy predicted by RaptorX on a similar training dataset.

We integrated the pretrained 3-state secondary structure and solvent accessibility prediction models into our pathogenicity prediction model as explained below,

**Training Models to Predict Pathogenicity of Variants**

## Input Features for Pathogenicity Prediction Model

As discussed above, for the pathogenicity prediction problem, there is a benign variant training set and an unknown variant training set for training the pathogenicity model. For each variant, we prepared the following input features to feed into our model.

The first input feature of each variant is its flanking amino acid sequence, i.e., 25 amino acids on each side of the variant obtained from the reference sequence of hg19, to provide the deep learning models of the sequence context of the variant. In total, this flanking reference sequence has 51 amino acids in length.

The second feature is the alternative amino acid that caused the variant. Instead of providing the reference-alternative amino acid pair directly, we provide the alternative flanking sequence to the model. The alternative flanking sequence is the same as the reference flanking sequence in the first feature, except that the middle position of the sequence contains the alternative amino acid, instead of the reference amino acid.

Both the sequences are then converted to one-hot encoded vectors of length 51 x 20, where each amino acid is represented by a vector of 20 zeros or ones.

Then three position weight matrices (PWM) are generated from multiple sequence aligraments (MSA) of 99 vertebrates for the variant, including one for 12 primates, one for 4.7 mammals excluding primates, and one for 40 vertebrates excluding primates and mammals. Each PWM has the dimension of L x 20, where L is the length of flanking sequences around the variant (in our case, L represents 51 amino acids). It comprises counts of amino acids seen in each category of species.

We also generate the PSSM matrices for the variant-flanking sequences of 51 amino acids from psi blast. This is used for the integration of 3-state secondary structure and solvent accessibility prediction models for pathogenicity prediction.

We train the pathogenicity model with the reference sequence (input), alternate sequence (input2), PWM matrices for primates (input3), mammals (input4), vertebrates (input5), and information from 3-state secondary structure and solvent accessibility models.

## Deep Learning Model Training

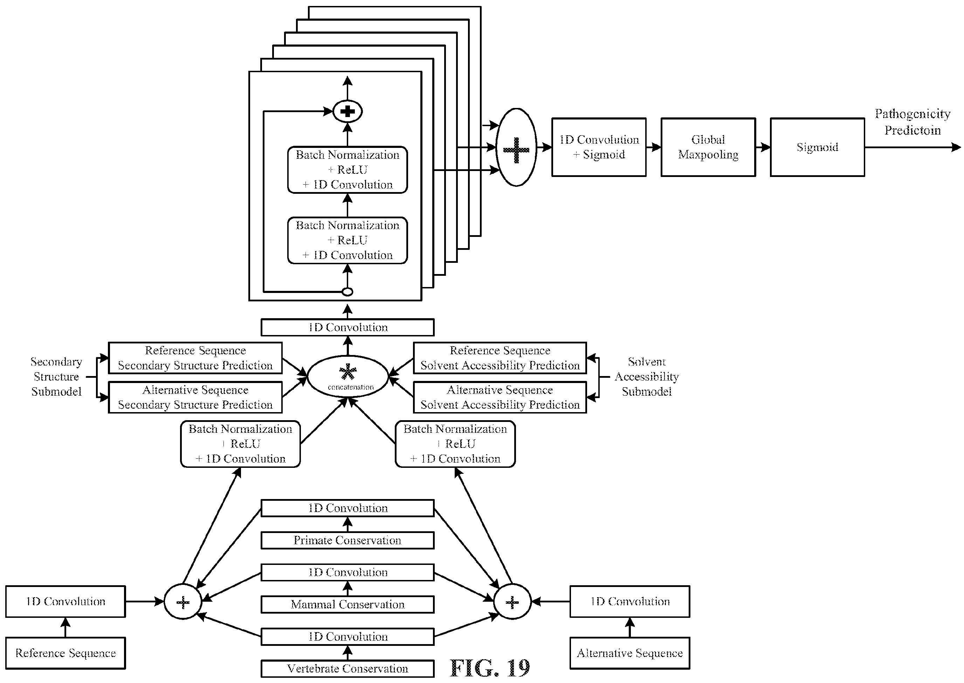

FIG. 19 is a block diagram that provides an overview of the deep learning models workflow. The pathogenicity training models comprises five direct inputs and four indirect inputs. The five direct input features include reference sequence (1a), alternative sequence (1b), primate conservation (1c), mammal conservation (1d). and vertebrate conservation (1e). The indirect inputs include reference-sequence-based secondary structure (If), attemative-sequence-based secondary structure (1g), reference-sequence-based solvent accessibility (1h), and alternative-sequence-based solvent accessibility (1i).

For indirect inputs 1f and 1g, we load the pretrained layers of secondary structure prediction model, excluding the softmax layer. For input 1f. the pretrained layers are based on the human reference sequence for the variants along with the PSSM generated by PSI-BLAST for the variant. Likewise, for input Lg, the pretrained layers of the secondary structure prediction models are based on the human alternative sequence as the input along with the PSSM matrix. Inputs 1h and 1i correspond to the similar preirained channels containing the solvent accessibility information for reference and alternative sequences of the variant, respectively.

The five direct input channels are passed through an apsampling convolution layer of 40 kernels with linear activations. The layers 1a, 1c, 1d and 1c are merged with values summed across the 40 feature dimensions to produce layer 2a . In other words, the feature map of the reference sequence is merged with the three types of conservation feature maps. Similarly, 1b,1c,1d and 1e are merged with values summed across the 40 feature dimensions to generate layer 2b, i.e., the features of the alternative sequence is merged with the three types of conservation features.

The layers 2a and 2b are batch normalized with the activation of ReLU and each passed through a ID convolution layer of filter size 40 (3a and 3b). The outputs of layers 3a and 3b are merged with 1f, 1g, 1h and 1i with the feature maps concatenated to each other. In other words, the feature maps of reference sequence with conservation profile, and alternative sequence with the conservation profile are merged with the secondary structure feature maps of the reference and alternative sequence and the solvent accessibility feature maps of the reference and alternative sequence (layer 4).

The outputs of layer 4 are passed through six residual blocks (layers 5,6,7.8,9,10). The last three residual blocks have an atrous rate of 2 for the 1D convolutions, to give higher coverage for the kernels. The output of layer 10 is passed through a 1D convolution of filter size 1 and activation sigmoid (layer 11) . The output of layer 11 is passed through a global maxpool that picks a single value for the variant. This value represents the pathogenicity of the varuint. The details of one implementation of the pathogenicity prediction model are shown in the table below.

| Layer | Type | Number of Kernels, Window size | Shape | Atrous rate | Activation |
|---|---|---|---|---|---|
| Reference sequence ( 1a ) | Convolution 1D | 40,1 | (51,40) | 1 | Linear |
| Alternative sequence ( 1b ) | Convolution 1D | 40,1 | (51,40) | 1 | Linear |
| Primate conservation ( 1c ) | Convolution 1D | 40,1 | (51,40) | 1 | Linear |
| Mammal Conservation ( 1d ) | Convolution 1D | 40,1 | (51,40) | 1 | Linear |
| Vertebrate Conservation ( 1e ) | Convolution 1D | 40,1 | (51, 40) | 1 | Linear |
| Reference-sequence-based Secondary structure | Input layer | - | (51,40) | - | - |

| (1f) | | | | | |
|---|---|---|---|---|---|
| Alternative-sequence-based secondary structure (1g) | Input layer | - | (51,40) | - | - |
| Reference-sequence-based solvent accessibility (1h) | Input layer | - | (51,40) | - | - |
| Alternative-sequence-based solvent accessibility (1i) | Input layer | - | (51,40) | - | - |
| Reference sequence merge (2a) | Merge (mode =Sum) (1a,1c,1d,1e) | - | (51,40) | - | - |
| Alternative sequence merge (2b) | Merge (mode =Sum) (1b,1c,1d,1e) | - | (51,40) | - | - |
| 3a | Convolution 1D (2a) | 40,5 | (51,40) | 1 | ReLU |
| 3b | Convolution 1D (2b) | 40,5 | (51,40) | 1 | ReLU |
| 4 | Merge (mode = concatenate) (3a,3b,1f,1g,1h,1i) | - | (51,240) | - | - |
| 5 | Convolution 1D | 40,5 | (51,40) | 1 | ReLU |
| 6 | Convolution 1D | 40,5 | (51,40) | 1 | ReLU |
| 7 | Convolution 1D | 40,5 | (51,40) | 1 | ReLU |
| 8 | Convolution 1D | 40,5 | (51,40) | 1 | ReLU |
| 9 | Convolution 1D | 40,5 | (51,40) | 2 | ReLU |
| 10 | Convolution 1D | 40,5 | (51,40) | 2 | ReLU |

| | | | | | |
|---|---|---|---|---|---|
| 11 | Convolution 1D | 40,1 | (51,1) | 2 | Sigmoid |
| 12 | Global max pooling | - | 1 | -- | - |
| Output layer | Activation layer | - | 1 | -- | Sigmoid |

<u>Ensembles</u>

In one implementation. for each cycle of our method, we ran eight different models that train on the same benign data set and eight different unknown data sets and averaged the prediction of evaluation data sets across the eight models. Sampling bias can be reduced and well controlled when multiple randomly-sampled sets of unknown variants are presented to the model.

Furthermore, adoption of ensemble-of-ensembles approach can improve the performance of our model on our evaluation dataset. CADD uses an ensemble of 10 models and gets the average score across all the 10 models to score a variant. Here we attempted to use a similar ensemble upproach. We benchmarked the results using one ensemble and then increased the number of ensembles to assess the performance gain. Note that each ensemble has eight models that train on the same benign data set und eight different unknown data sets. For different ensembles, the seed values of the random number generator are distinct so that the random variant sets are drawn differently from cach other.

The detailed results according to one implementation are shown in the table below.

| Number of Ensembles | -log(pvalue) on DDD dataset ( mean of mean of ensembles ) | -log(pvalue) on DDD dataset ( median of mean of ensembles) |
|---|---|---|
| 1 | 3.4e-27 | 3.4e-27 |
| 5 | 2.74e-29 | 3.8e-29 |
| 10 | 2.98e-29 | 2.55e-29 |
| 15 | 4.06e-29 | 3.88e-29 |
| 20 | 3.116e-29 | 3.05e-29 |
| 25 | 3.77e-29 | 3.31e-29 |
| 30 | 3.81e-29 | 3.34e-29 |

Compared with one ensemble, 5 ensembles and 10 ensembles produced more significant p-values when evaluated using DDD datasets. But increasing the number of ense mbles fails to improve the performance further, indicating a saturation for ensembles. The ensembles reduce sampling bias with a large variety of unknown variants. However, we also required matching the 192 trinucleotide contexts between benign and pathogenic classes, which limits our sampling space substantially, leading to the quick saturation. We conclude that ensemble-of-ensembles approach improves the model performance significantly and further enriches our understanding of models.

**Early Stopping for Training Pathogenicity Model**

Since there lacks reliable annotated pathogenic variant samples, it is challenging to define stopping criteria for model training. To avoid using pathogenic variants in model evaluation, in one implementation, we used the 10,000 benign validation variants from orthologous primates and 10,000 trinucleotide-context-matched unknown variants. After training each epoch of the model, we evaluated the benign validation variants and the unknown validation variants. We used Wilcoxon rank-sum test to assess the difference of the probability distributions of both of the validation variant sets.

The p-value of the test becomes more significant with improvements in the model's ability to disunguish benign variants from a set of unknown variants. We stop the training if no improvement is observed in the model's ability to distinguish between the two distributions during any five consecutive epochs of model training.

Earlier, we had set aside two separate sets of 10.000 withheld primate variants from training, which we termed the validation set and the test set. We used the validation set of 10,000 withheld primate variants and 10,000 unlabeled variants that were matched for trinucleotide context for evaluating early sropping during model training. After each training epoch, we evaluated the ability of the deep neural network to discriminate between variants in the labeled benign validation set and the unlabeled matched controls, measuring the difference in the distributions of the predicted scores using the Wilcoxon rank-sum test. We stopped the training once no further improvement was observed after five consecutive training epochs to prevent overfitting.

### Benchmarking of Classifler Performance

We assessed the classification accuracy of two versions of the deep learning network, one trained with common human variants only, and one trained with the full benign labeled dataset including both common human variants and primate variants, in addition to the following classifiers: SIFT, PolyPhen-2 , CADD, REVEL, M-CAP, LRT, MutationTaster, MutationAssessor, FATHMM, PROVEAN, VEST3, MetaSVM, MetaLR, MutPred, DANN, FATHMM-MKL_coding, Eigen, GenoCanyon, and GERP++ 13,32-48. To obtain the scores for each of the other classifiers, we downloaded the scores for all missense variants from dbNSFP 49 (https://sites.google.com/site/jpopgen/dbNSFP), and benchmarked the methods on the 1 0,000 withheld primate variant test set, and on *de novo* variants in DDD cases versus controls. We selected SIFT, PolyPhen-2, and CADD for inclusion in the main paper because they are among the most widely used methods, and REVEL, because across the different modes of evaluation, it stood out as being one of the best of the 20 existing classifiers we evaluated. The performance for all classifiers we evaluated is provided in **FIG. 28A.**

For evaluating the impact of available training data size on the performance of the deep learning network, we trained deep learning networks at each data point in **FIG. 6,** by randomly sampling from the labeled benign training set of 385,236 primate and common human variants. To reduce random noise in the performance of the classifiers, we performed this training procedure five times, each time using a random instantiation of initial parameter weights, and showed the median performance on both the 10,000 withheld primate variants and the DDD case vs controls dataset in **FIG. 6.** By chance, the performance of the median classifier with the full dataset of 385,236 labeled benign variants was slightly better than the one we used for the rest of the paper on the DDD dataset ($P < 10^{-29}$ instead of $P < 10^{-28}$ by Wilcoxon rank-sum test). To show that variants from each individual primate species contributes to classification accuracy whereas variants from each individual mammal species lower classification accuracy, we trained deep learning networks using a training dataset comprising 83,546 human variants plus a constant number of randomly selected variants for each species, according to one implementation. According to one implementation, the constant number of variants we added to the training set (23,380) is the tonal number of variants available in the species with the lowest number of missense variants, i.e, bonobo. To reduce noise, we again repeated the training procedure five times, and reported the median performance of the classifier.

### Model Evaluation

In one implementation, we trained 21 cycles of deep learning models following the iterative balanced sampling procedure. We performed two types of evaluations to assess the performance of our classifiers. We also compared our models with Polyphen2, SIFT, and CADD on the two metrics and assessed the potential of application of our models for clinical annotation.

### Method 1: Benian Test Set Accuracy

In one implementation, we evaluated the 10,000 benign variants and unknown variants by computing their predicted probabilities using an ensemble of eight different trained models. We also obtained their predicted probabilities scored by the other existing methods mentioned above.

We then obtained the median of predicted probabilities across the unknown test variants for each of the methods used in the evaluation. Using the median score, we found the number of benign variants that scored above or below the median depending on the annotation of benign and pathogenic variants used by each of the methods. SIFT, CADD and our method label pathogenic variants as 1 and benign variants as 0. Thus, we counted the number of benign variants that scored below the median. Polyphen uses the opposite annotation and we counted the number of benign variants above the median. The ratio of the number of benign variants scored above/below the median divided by the total number of benign variants represents the prediction accuracy of benign variants.

Benign accuracy = Total number of benign variants above (below*) the median + Total number of benign variants

Our reasoning behind this evaluation method relies on the analysis of selective pressure of variants in gnomAD. For the singletons in gnomAD, the ratio of missense variants to synonymous variants is - 2.26:1. While for the common variants (MAF>0.1%) in gnomAD, the missense-to-synonymous ratio is ~ 1.06 : 1. This indicates that from a set of random unknown variants, approximately 50% are expected to be purged by natural selection and the remaining 50% tend to be mild and likely become common in the popolation.

| Method | Accuracy of withheld benign set |
|---|---|
| Polyphen | 0.74 |
| SIFT | 0.69 |
| CADD | 0.77 |
| Our Model | 0.85 |

As shown in the table above, our method outperforms the second best method CADD by more than 8%. This shown an significant unprovement in our model's ability to classify the benign variants. While such a demonstration proves the ability of our model, the following Method 2 shown the usefulness of our model on clinical datasets for clinical interpretation.

## Method 2 : Clinical Dataset Evaluation

In one implementation, we evaluated these pathogenicity prediction methods on clinical datasets, including developmental disability disorder (DDD) case-control dataset. DDD dataset comprise 3.821 *de novo* missense variants from affected children and 1,231 *de novo* missense variants from their unaffected siblings. Our hypothesis is that the *de novo* variants from affected children tend to be more deleterious than the *de novo* variants from their unaffected siblings.

As clinical test datasets do not clearly label pathogenic variants, we used the separation between the two sets of *de novo,* variants (from affected and unaffected) to gauge the performance of those methods. We applied Wilcoxon rank-sum test to evaluate how well the separation of these two sets of *de novo* variants is.

| Method | -log10(p-value) on DDD dataset |
|---|---|
| Polyphen | 15.02 |
| SIFT | 13.52 |
| CADD | 13.83 |
| DL | 28.35 |

According to the table above, our semi-supervised deep learning models performs significantly better in distinguishing the affected set of *de novo* variants from the unaffected set. This shows that our model is more appropriate for clinical interpretation than the existing methods. This also validates that the general approach of extracting features from genome sequences and conservation profiles is superior to the manually-crafted features based on human curated datasets.

## Benign Prediction Accuracy on a Withheld Test Set of 10,000 Primate Variants

We used the 10,000 withheld primate variants in the test dataset to benchmark the deep learning network as well as the other 20 classifiers. Because the different classifiers had widely varying score distributions, we used 10,000 randomly selected unlabeled variants that were matched to the test set by tri-nucleotide context to identify the 50th-percentile threshold for each classifier. To ensure fair comparison between the methods, we benchmarked each classifier on the fraction of variants in the 10,000 withheld primate variant test set that were classified as benign at the 50th-percentile threshold for that classifier.

Our reasoning behind using the 50th-percentile to identify benign variants is based on the selective pressure observed for missense variants in the ExAC/gnomAD dataset. For variants occuring at singleton allele frequency, the missense:synonymous ratio is ~ 2.2:1, whereas for common variants (> 0.1% allele frequency), the missense:synonymous ratio is ~ 1.06 : 1. This indicates that approximately 50% of missense variants are expected to be purged by natural selection at common allele frequencies, and the remaining 50% are mild enough to have the potential to become common in the population via genetic drift.

For each of the classifiers, the fraction of withheld primate test variants predicted as benign using the 50th-percentile threshold is shown (**FIG. 28A** and Supplementary Table 17 (**FIG. 34**)).

## Analysis of *de novo* Variants from DDD Study

We benchmarked the classification methods on their ability to discriminate between *de novo* missense variants in the DDD affected individuals, versus *de novo* missense variants in unaffected sibling controls. For each classifier, we reported the p-value from the Wilcoxon rank-sum test of the difference between the prediction scores for the two distributions (**FIGs. 28B** and **28C** and Supplementary Table 17 (**FIG. 34**)).

Given that our two metrics for analyzing the performance of the model are derived from different sources and methodologies, we tested if the performance of the classifiers on the two different metrics was correlated. Indeed, we found that these two metrics were correlated, with a spearman $\rho = 0.57$ ($P < 0.01$) between benign classification accuracy on the withheld primate test set, and the Wilcoxon rank-sum p-value for *de novo* missense variants in DDD cases vs controls. This shows that there is good agreement between the withheld primate test set accuracy and the DDD case vs. control p-value for benchmarking the classifiers (**FIG. 30A**).

Furthermore, we tested if the deep learning network could assist in discovery of genes associated with disease. We tested enrichment of *de novo* mutations in genes by comparing the observed number of *de novo* mutations to the number expected under a null mutation model.

We examined the performance of the deep learning network comparing results from all missense *de novo* mutations versus results from missense mutations with a score > 0.803. Testing all missense *de novo*s used the default missense rate, whereas testing filtered missense *de novo*s used missense mutation rates calculated from sites with scores > 0.803. Each gene required four tests, one testing protein truncating enrichment, and one testing enrichment of protein-altering *de novo* mutations, both tested for just the DDD cohort, and for a larger meta-analysis of neurodevelopmental trio sequencing cohorts. The enrichment of protein-altering *de novo* mutations was combined by Fisher's method with a test of the clustering of missense *de novo* mutations within the coding sequence (Supplementary Table 20 and 21). The p-value for each gene was taken from the minimum of the four tests, and genome-wide significance was determined as $P < 6.757$ x $10^{-7}$ ($\alpha$=0.05, 18,500 genes with four tests).

## Computing Receiver Operator Curve Characteristics and Classification Accuracy within 605 DDD Disease-Associated Genes

To test if the deep learning network was indeed discriminating between pathogenic and benign variants within the same gene, rather than favoring pathogenicity in genes with a *de novo* dominant mode of inheritance, we identified a set of 605 genes that were associated with neurodevelopmental disease with p-value < 0.05 in the DDD cohort (calculated using *de novo* protein-truncating variation alone) (Supplementary Table 18). We report the Wilcoxon rank-sum p-value for all the classifiers in their ability to separate the probability distributions of variants in the 605 genes in DDD and control dataset (**FIG. 28C** and Supplementary Table 19 (**FIG. 35**)).

Within this set of 605 genes, we observe an enrichment ratio for *de novo* missense variants that is three times what is expected by mutational rate alone. This indicates that *de novo* missense variants in DDD affected patients comprise approximately 67% pathogenic variants, and 33% background variants, while the *de novo* missense variants in healthy controls are comprised largely of background variants, except for instances of incomplete peactrance.

To calculate the maximum possible AUC for a classifier that perfectly discriminates between pathogenic and benign variants, we took into account that only 67% of the *de novo* missense variants in affected individuals within the 605 genes were pathogenic, and the rest were background. To construct a receiver operator characteristics curve, we treated classification of *de novo* DDD variants as pathogenic as true positive calls, and treated classification of *de novo* variants in healthy controls as pathogenic as being false positive calls. Hence, a perfect classifier would classify 67% of *de novo* variants in the DDD patients as true positives, 33% of *de novo* variants in the DDD patients as false negatives, and 100% of *de novo* variants in controls as true negatives. Visualization of the receiver operator curve would show only a single point with 67% True Positive rate and 0% False Positive rate, connected to the (0%,0%) and (100%,100%) corners of the plot by straight lines, yielding a maximum AUC of 0.837 for a classifier with perfect discrimination of benign and pathogenic mutations **(FIG. 30B** and Supplementary Table 19 **(FIG. 35)).**

We calculated the classification accuracy of the deep learning network for separating pathogenic and benign variants at a binary threshold by estimating the expected fraction of pathogenic variants within 605 genes in the combined DDD and healthy control datasets. Since the DDD dataset contained 379 *de novo* variants with an excess of 249 *de novo* missense variants over expectation, and the control dataset contained 65 *de novo* variants, we expected 249 pathogenic variants out of 444 total variants **(FIG. 22A).** We selected the threshold for each classifier that separated the 444 *de novo* missense variants into benign or pathogenic categories according to this expected proportion, and used this as a binary cutoff to evaluate the accuracy of each classifier. For our deep learning model, this threshold was attained at a cutoff of $\geq$ 0.803, with a True Positive Rate of 65%, and a False Positive Rate of 14%. To calculate the classification accuracy adjusted for the presence of-33% background variants in the DDD individuals, we assumed that the 33% of *de novo* DDD variants which were background would be classified at the same False Positive Rate as we observed in the healthy controls. This corresponds to 14% $\times$ 0.33 = 4.6% of the true positive classification events in the DDD dataset actually being False Positives from background variants. We estimate the adjusted True Positive Rate for the deep learning network to be (65% - 4.6%) / 67% = 90%. We report the average of the True Positive Rate and the True Negative Rate, which is 88% for the deep learning network **(FIG. 30C** and Supplementary Table 19 **(FIG. 35)).** This estimate is likely to underestimate the true accuracy of the classifier, due to the high prevalence of incomplete penetrance in neurodevelopmental disorders.

## ClinVar Classification Accuracy

Most of the existing classifiers are trained on ClinVar; even classifiers that do not directly train on ClinVar may be affected by using prediction scores from classifiers that are trained on ClinVar. In addition, common human vanants are highly enriched for benign ClinVar consequence, because allele frequency is part of the criteria for assigning benign consequence to a variant.

We tried to minimize the circularity in the ClinVar dataset to make it suitable for analysis by using only ClinVar variants that were added in 2017, as the other classification methods were published in previous years. Even among the 2017 ClinVar variants, we excluded any variant present at common allele frequencies (> 0.1%) in ExAC, or present in HGMD, LSDB. or Uniprot. After filtering all such variants, and excluding variants of uncertain significance and those with conflicting annotations, we were left with 177 variants with benign annotation and 969 variants with pathogenic annotation in ClinVar.

We scored all ClinVar variants using both the deep learning network and existing methods. We selected the threshold for each classifier that separated the Clin Var variants into benign or pathogenic categories according to the observed proportion of benign and pathogenic variants within this dataset, and used this as a binary cutoff to evaluate the accuracy of each classifier. We report the average of the True Positive Rate and the True Negative Rate for each classifier **(FIGs. 31A** and **318).** The performance of classifiers on the ClinVar dataset were not significantly correlated with the performance of the classifiers on either classification accuracy on the 10,000 withheld primare variants or the Wilcoxon rank-sum p-value for the DDD cases vs controls dataset **(FIGs. 31A** and **31B**).

We hypothesize that existing classifiers accurately model the behavior of human experts, but that human heuristics may not be fully optimal for discriminating between pathogenic and benign mutations in empirical data. One such example is Grantham score, which provides a distance metric for characterizing the similarity or dissimilarity of amino acid substitutions. We computed the mean Grantham score for the pathogenic and benign variants within the complete ClinVar dataset (~42,000 variants), and compared this with mean Grantham score for *de novo* variants in DDD effected and unaffected individuals within the 605 genes. To correct for presence of ~33% background variants in the DDD affected individuals, we increased the difference in the Grantham score between DDD cases vs controls by 50%, which was still smaller than the difference between pathogenic and benign variants in ClinVar. One possibility is that human experts place too much weight on measures that are easy to measure, such as amino acid substitution distance, while underweighting factors such as protein structure, that are more difficult for a human expert to quantify.

## Interpreting the Deep Learning Models

Understanding the means by which machine learning algorithms solve problems is often difficult. We visualized the initial layers of the deep leaning network to understand the features that it had learned to extract in order to predict variant pathogenicity. We calculated the correlation coefficients for different amino acids within the first three layers (the first convolutional layer following two upsampling layers) of the pretrained 3-state secondary structure prediction models, and showed that the weights of the convolutional layers learn features very similar to the BLOSUM62 matrix or Grantham distance.

To compute the correlation coefficients between the different amino acids, we started with weights of the first convolutional layer preceded by three upsampling layers (layers 1a ,1b and 1c) in the secondary structure model. We performed matrix multiplication between the three layers, resulted in a matrix with dimensions (20,5,40), where 20 is the number of amino acids, 5 is the window size of the convolutional layer, and 40 is the number of kernels. We reshaped the matrix to have the dimension (20,200) by flattening the last two dimensions, obtaining a matrix in which the weights operating on each of the 20 amino acids were represented as a 200-length vector. We calculated the correlation matrix between the 20 amino acids. Since each dimension represents each amino acid, by calculating the correlation coefficient matrix we are calculating the correlation between the amino acids, and how similar they appear to the deep learning network, based on what it has leaned from the training data. The visualization of the correlation coefficient matrix is shown in **FIG. 27** (amino acids sorted by BLOSUM62 matrix order), and shows two prominent clusters, comprising the hydrophobic amino acids (Methionine, Isoleucine, Leucine. Valine, Phenylalanine, Tyrosine, Tryptophan), and the hydrophilic amino acids (Asparagine, Aspartic Acid, Glutamic Acid, Glutamine, Arginine, and Lysine). The outputs of these initial layers become the inputs for later layers, enabling the deep learning network to construct increasingly complex hierarchical representations of the data.

To illustrate the window of amino acid sequence used by the neural network in its predictions, we perturbed each position in and around 5000 randomly selected variants to observe its effects on the predicted PrimateAI score for the variant (**FIG. 25B).** We systematically zeroed out the inputs at each nearby amino acid position (-25 to +25) around the variant, and measured the change in the neural network's predicted pathogenicity of the variant, and plotted the average absolute value of the change across the 5000 variants. Amino acids near the variant have the greatest effect, in a roughly symmetric distribution, gradually tailing off as distance from the variant increases. Importantly, the model makes its predictions based not only on the amino acid at the position of the variant, but by using information from a wider window, as would be needed to recognize protein motifs. Consistent with the relatively compact size of protein subdomains, we empirically observed that extending the size of the window to more than 51 amino acids did not further improve accuracy.

To evaluate the sensitivity of the deep learning classifier to alignment, we examined the effects of the depth of the alignment on the accuracy of variant classification as follows. We split the data into five bins based on the number of species in the alignment, and evaluated the accuracy of the network in each bin **(FIG. 57).** We found that the accuracy of the network at separating a set of withheld benign mutations from randomly selected mutations that were matched for trinucleotide context (as in **FIG. 21D,** but performed separately for each bin) is strongest at the top three bins and noticeably weaker in the bottom two bins. The 99 vertebrate multi-species alignment comprises 11 non-human primates, 50 mammals, and 38 vertebrates, with the bottom two bins representing proteins that have sparse alignment infomnation from other non-private mammals. The deep learning network is robust and accurate when alignment information extends throughout primates and mammals, with conservation information from more distant vertebrates being less important.

## Definition of Canonical Coding Regions

To define canonical coding regions, multiple alignments of 99 vertebrate genomes with human for coding DNA sequence (CDS) regions (knownCanonical.exonNuc.fa.gz) were downloaded from UCSC genome browser. For human, the coordinates of exons are under Build hg19. Exons are merged in form gene. Genes on autosomes and chrX are retained. Nonhomologous genes were removed, where the list of homologous genes are downloaded from NCBI ftp://ftp.nchi.nih.gov/pub/HomoloGene/current/homologene.data. For SNPs with multiple gene annotations, the longest transcript is selected to represent the SNP's annotation.

## Human, Ape, and Mammal Polymorphism Data

We downloaded human exome polymorphism data from a recent large-scale study, the genome Aggregation Database (gnomAD), which collected the whole-exome sequencing data of 123,136 individuals from 8 subpopulations across the world. We then extracted variants that pass the filters and fall within the canonical coding regions.

The great ape genome sequencing project provides whole genome sequencing data of 24 chimpanzees, 13 bonobos, 27 gorillas and 10 orangutans (including 5 Sumatran Orangutan and 5 Bornean Orangutan). The study on chimpanzee and bonobos provides WGS of an additional 25 apes. As all the sequencing data were mapped to hg19, we downloaded the VCF files from these studies and directly extracted variants within the canonical coding regions.

To compare with other apes and mammals, we also downloaded SNPs of a few other species from dbSNP, including rhesus, marmoset, pig, cow, goat, mouse and chicken. We discarded other species, such as dog, cat or sheep, as dbSNP provides lunit numbers of variants for those species. We initially lifted over SNPs of each species to hgl9. It turns out about 20% of variants are mapped to pseudogene regions. We then obtained the exon coordinates of each species from multiple alignment file of 100 vertebrates of canonical coding regions and extracted variants within those exons. Then those extracted SNPs were lifted over to hg19. If variants are on a different genome build of the species from the alignment, we first lifted variants to the genome build of the alignment.

As cow SNP data comes from various studies, we downloaded from dbSNP all the large batches of cow variants (16 batches with VCF files > 100MB), and evaluated the quality of different batches of cow SNPs by computing missense-to-synonymous ratios for each batch. The median of missense-to-synonymous ratios is 0.781 and the median absolute deviation (MAD) is 0.160 (mean is 0.879 and SD is 0.496). Two batches with outlier ratios (snpBatch_1000_BULL_GENOMES_1059190.gz with ratio of 1.391 and snpBatch_COFACTOR_GENOMICS_1059634.gz with ratio of 2.568) were excluded from further analysis.

## Evaluation of Properties of Polymorphisms in Apes and Mammals

To demonstrate the usability of great apes SNPs, we devised the enrichment score that measures the ratio of the number of singletons and common SNPs (allele frequency (AF) > 0.1%). Synonymous variants are known to be benign and generally evolve neutrally without any selection pressure. Deleterious missense variants are gradually purged by natural selection, thus its allele frequency distribution tends to be in excess of rare variants compared with synonymous variants.

We focused on those gnomAD SNPs that overlap with SNPs observed in primates, mammals and poultry. We counted the number of synonymous and missense variants per species. For missense variants, we further classified into two types, those that share identical amino acid changes in another species, termed as "missense identical", and those that have different amino acid changes in another species, termed as "missense different". The enrichment scores were then computed per species as the ratio of the numbers of singletons vs common variants.

In addition, we performed Chi-squared ($\chi2$) test of homogeneity on 2x2 contingency table to compare enrichment scores between synonymous and missense identical variants for each species. All the primates demonstrate no significant difference in enrichment scores between synonymous and missense identical variants, while cow, mouse and chicken show significant difference.

The result revealed that those SNPs that share identical amino acid changes in great apes tend to have very similar enrichment scores as synonymous SNPs, implying they tend to have mild effect on human healthy. While those that have different amino acid changes or that are absent in great apes have enrichment scores that significantly deviate from that of synonymous SNPs. Missense polymorphisms from non-primate species also have different allele frequency distribution from synonymous variants. The conclusion is that SNPs that share identical amino acid changes in great apes can be added to the training set of benign variants.

Our assumption is that most variants are independently derived, not generated by identity-by-descent (IBD). Thus, we performed enrichment analysis of rare variants in IBD SNPs to evaluate different behavior of their enrichment scores. IBD SNPs are defined as human SNPs that appear in both human and two or more great ape species, including chimpanzee, bonobo, gorilla. S. orangutan, and B. orangutan. Then the enrichment scores, defined as the number of singletons divided by the number of common variants (AF > 0.1%), are calculated separately for missense variants and synonymous variants, which are considered neutral and serve as the baseline for comparison.

## Fixed Substitutions Among Mammal Species

## Enrichment Analysis of Fixed Substitutions

We also studied the rare variant enrichment analysis of inter-species substitutions. We downloaded the phylogenetic tree of 100 vertebrate species from UCSC genome browser (http://hgdownload.soe.ucsc.edu/golden Path/hg19/multiz100way/hg19.100way.commonNames.nh ). Then we calculated pairwise phylogenetic distance and selected closely-related species pairs (distance < 0.3). To obtain primate species pairs, we downloaded alignments (hg38) of 19 mammalian (16 primate) genomes with human for CDS regions from the UCSC genome browser. Four primate pairs were added to 13 vertebrate pairs. The following table shown genetic distance of multiple pairs of closely-related species, according to one implementation.

| Species1 | Species2 | Distance |
|---|---|---|
| Chimp | Bonobo | 0.00799195 |
| Rhesus | Crab-eating macaque | 0.00576807 |
| Marmoset | Squirrel monkey | 0.0680206 |
| Proboscis monkey | Golden snub-nosed monkey | 0.01339514 |
| Baboon | Green-monkey | 0.045652 |
| Horse | White-rhinoceros | 0.084397 |
| Sheep | Domestic-goat | 0.1 |
| Mouse | Rat | 0.176098 |
| Chinchilla | Brush-tailed-rat | 0.16 |
| Cape-golden-mole | Tenrec | 0.265936 |
| Chinese-hamster | Golden-hamster | 0.08 |
| David-myotis-bat | Microbat | 0.08254 |
| Dolphin | Killer-whale | 0.074368 |
| Saker-falcon | Peregrine-falcon | 0.2 |
| Chicken | Turkey | 0.126972 |
| Green-sea-turtle | Painted-turtle | 0.2 |
| Chinese-softshell-turtle | Spiny-softshell-turtle | 0.2 |

We took the multiple alignments of 19 mammalian or 99 vertebrate genomes with human within canonical coding regions and obtained nucleotide substitutions between each selected pair of vertebrates. These substitutions were mapped to hunum exome SNPs from gmmiAD, requiring identical codon changes between species pair and human variants. We classified variants into three types, synonymous variants, missense variants that share identical amino acid changes in another species, and missense variants that have different amino acid changes in another species. The enrichment scores were computed for each class per species pair.

## Comparison of Intraspecies and Interspecies Polymorphisms

Six species were selected to perform comparison of intraspecies and interspecies polymorphisms, including chimpanzee, rhesus, marmoset, goat, mouse and chicken, as both intraspecies and interspecies variants are available for these species. Comparison of enrichment scores of intraspecies and interspecies variants is similar to comparison of adds ratios of two 2x2 contingency tables. Woolf test is usually applied to evaluate homogeneity of odds ratios between contingency tables. Therefore, we utilized Woolf test to evaluate the difference of enrichment scores between intraspecies and interspecies polymorphisms.

## Per-Gene Enrichment Analysis

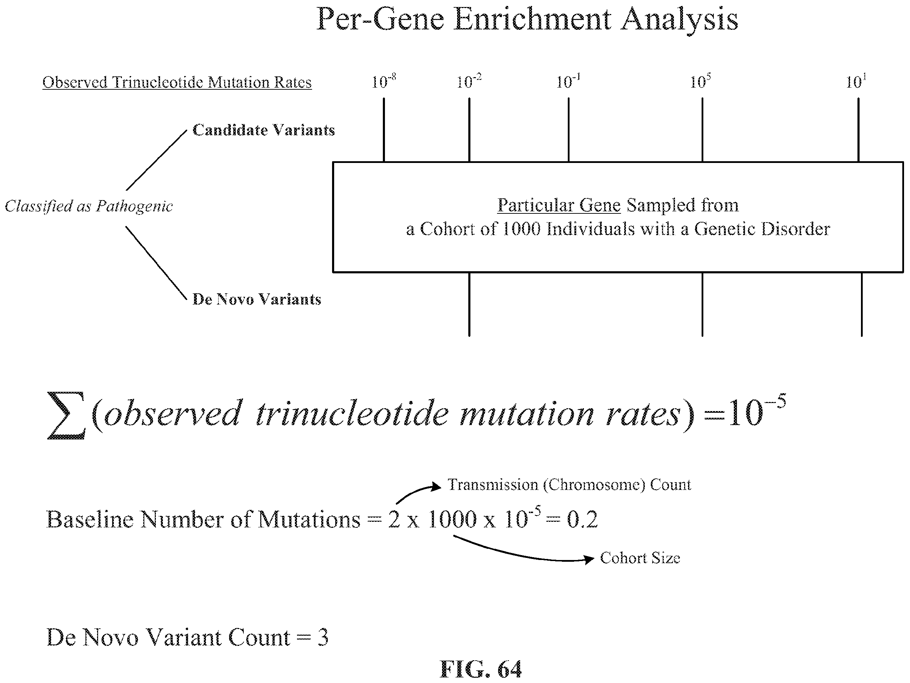

FIG. 64 shows one implementation of per-gene enrichment analysis. In one implementation, the deep convolutional neural network-based variant pathogenicity classifier is further configured to implement a per-gene enrichment analysis which confirms pathogenicity of variants that have been determined to be pathogenic. For a particular gene sampled from a cohort of individuals with a genetic disorder, the per-gene enrichment analysis includes applying the deep convolutional neural network-based variant pathogenicity classifier to identify candidate variants in the particular gene that are pathogenic, determining a baseline number of mutations for the particular gene based on summing observed trinucleotide mutation rates of the candidate variants and multiplying the sum with a transmission count and a size of the cohort, applying the deep convolutional neural nenwork-based variant pathogenicity classifier to identify de novo missense variants in the particular gene that are pathogenic, and comparing the baseline number of mutations with a count of the de novo missense variants. Based on an output of the comparison, the per-gene enrichment analysis confinns that the particular gene is associated with the genetic disorder and that the de novo missense variants are pathogenic. In some implementations, the genetic disorder is autism spectrum disorder (abbreviated ASD). In other implementations, the genetic disorder is developmental delay disorder (abbreviated DDD).

In the example shown in **FIG. 64,** five candidate variants in a particular gene have been classified as pathogenic by the deep convolutional neural network-based variant pathogenicity classifier. These five candidate variants have respective observed trinucleotide mutation rates of $10^{-8}$ $10^{-2}$, $10^{-1}$, $10^5$, and $10^1$, The baseline number of mutations for the particular gene is determined to be $10^{-5}$ based on summing the respective observed trinucleotide mutanon rates of the five candidate variants and multiplying the sum with a transmission/chromaosome count (2) and a size of the cohort (1000). This is then compared with the *de novo* variant count (3).

In some implementations, the deep convolutional neural network-based variant pathogenicity classifier is further configured to perform the comparison using a statistical test that produces a p-value as the output.

In other implementations, the deep convolutional neural network-based variant pathogenicity classifier is further configured to compare the baseline number of mutations with the count of the de novo missense variants and, based on the output of the comparison, confirm that the particular gene is not associated with the genetic disorder and that the de novo missense variants are benign.

## Genome-Wide Enrichment Analysis

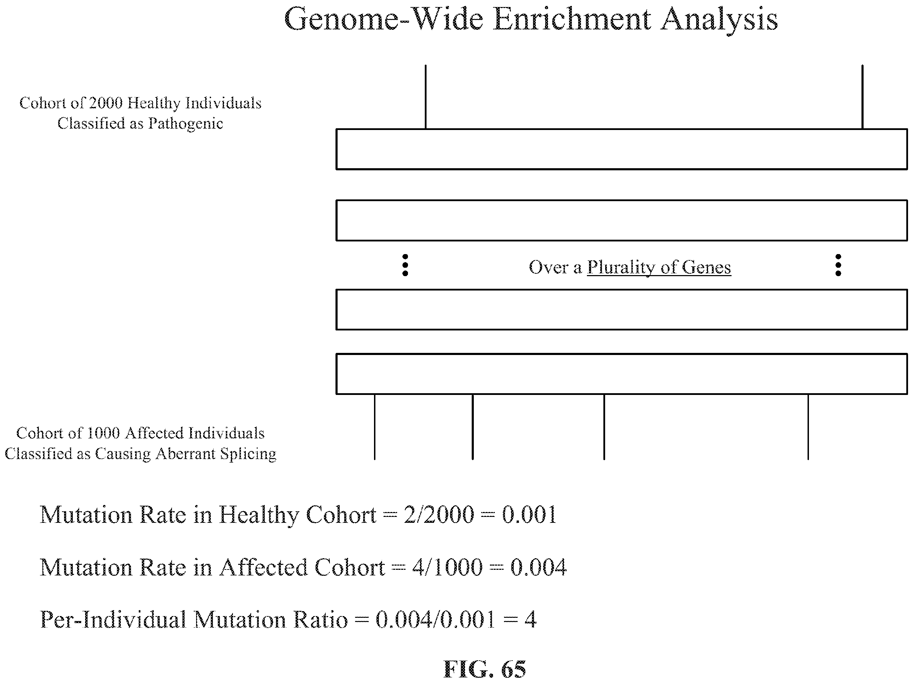

**FIG. 65** shows one implementation of genome-wide enrichment analysis. In another implementation, the deep convolutional neural network-based variant pathogenicity classifier is further configured to implement a genome-wide enrichment analysis which confirms pathogenicity of variants that have been determined to be pathogenic. The genome-wide enrichment analysis includes applying the deep convolutional neural network-based variant pathogenicity classifier to identify a first set of de novo missense variants that are pathogenic in a plurality of genes sampled from a cohort of healthy individuals, applying the deep convolutional neural network-based variant pathogenicity classifier to identify a second set of de novo missense variants that are pathogenic in the plurality of genes sampled from a cohort of individuals with a genetic disorder, and comparing respective counts of the first and second sets, and based on an output of the comparison confirmi ng that the second set of de novo missense variants are enriched in the cohort of individuals with the genetic disorder and therefore pathogenic. In some implementations, the genetic disorder is autism spectrum disorder (abbreviated ASD). In other implementations, the genetic disorder is developmental delay disorder (abbreviated DDD).

In some implementations, the deep convolutional neural network-based variant pathogenicity classifier is further configured to perform the comparison using a statistical test that produces a p-value as the output. In one implementation, the comparison is further parametrized by respective cohort sizes.

In some implementations, the deep convolutional neural network-based variant pathogenicity classifier is further configured to compare the respective counts of the first and second sets, and based on the output of the comparison confirming that the second set of de novo missense variants are not enriched in the cohort of individuals with the genetic disorder and therefore benign.

In the example shown in **FIG. 65.** mutation rate in the healthy cohort (0.001) and mutation rate in affected cohort (0.004) is illustrared, along with per-individual mutation ration (4).

## Particular Implementations

We describe systems, methods, and articles of manufacture for constructing a variant pathogenicity classifier. One or more features of an implementation can be combined with the base implementation. Implementations that are not mutually exclusive are taught to be combinable. One or more features of an implementation can be combined with other implementations. This disclosure periodically reminds the user of these options. Omission from some implementations of recitations that repeat these options should not be taken as limiting the combinations taught in the preceding sections - these recitations are hereby incorporated forward by reference into each of the following implementations.

A system implementation of the technology disclosed includes one or more processors coupled to the memory. The memory is loaded with computer instructions to train a splice site detector that identifies splice sites in genomic sequences (e.g., nucleotide sequences).

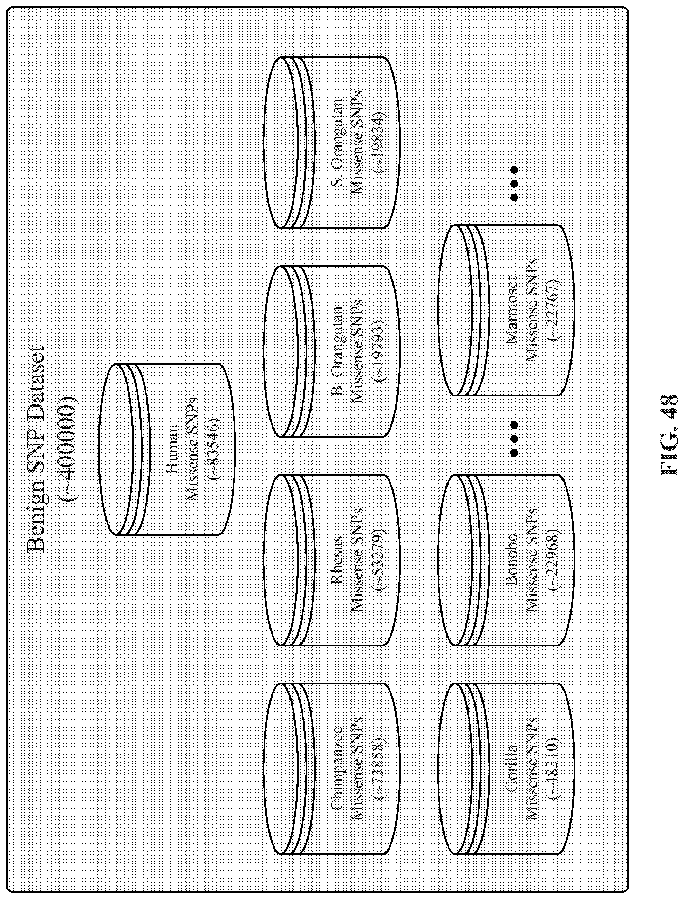

As shown in **FIGs. 48** and **19,** the system trains a convolutional neural network-based variant pathogenicity classifier, which runs on numerous processors coupled to memory. The system uses benign mining examples and pathogenic training examples of protein sequence pairs generated from benign variants and pathogenic variants. The benign variants include common human missense variants and non-human primate missense variants occurring on almmative non-human primate codon sequences that share matching reference codon sequences with humans. The phrase "protein sequence pairs" refers to a reference protein sequence and an attemative protein sequence, wherein the reference protein sequence comprises reference amino acids formed by reference triplet nucleotide bases (reference codons) and the alternative protein sequence comprises alternative amino acids formed by alternative triplet nucleotide bases (alternative codons) such that the alternative protein sequence is produce as a result of a variant occurring in the reference tnplet nucleotide bases (reference codons) forming the reference amino acids of the reference protein sequence. The variant can be a SNP, an insertion or a deletion.

This system implementation and other systems disclosed optionally include one or more of the following features. System can also include features described in connection with methods disclosed. In the interest of conciseness, alternative combinations of system features are not individually enumerated. Features applicable to systems, methods, and articles of manufacture are nor repeated for each statutory class set of base features. The reader will understand how features identified in this section can readily be combined with base features in other statutory classes.

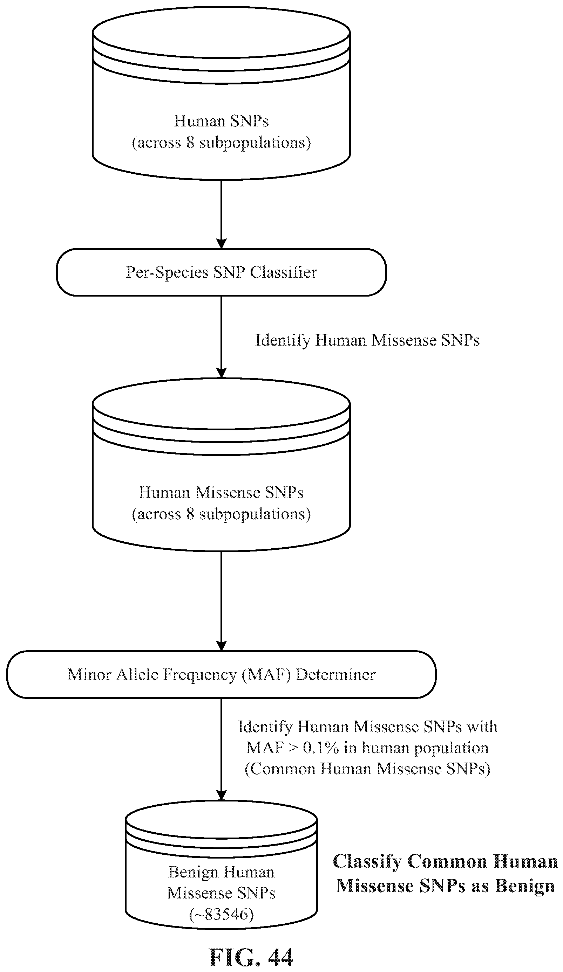

As shown in **FIG. 44,** the common human missense variants have a minor allele frequency (abbreviated MAF) greater than 0.1% across a human population variant dataset sampled from at least 100000 humans.

As shown in **FIG. 44,** the sampled humans belong to different human subpopulations and the common human missense variants have a MAF greater than 0.1% within respective human subpopulation variant datasets.

The human subpopulations include African/African American (abbreviated AFR), American (abbreviated AMR), Ashkenazi Jewish (abbreviated ASJ), East Asian (abbreviated EAS), Finnish (abbreviated FIN), Non-Finnish European (abbreviated NFE), South Asian (abbreviated SAS), and Others (abbreviated OTH).

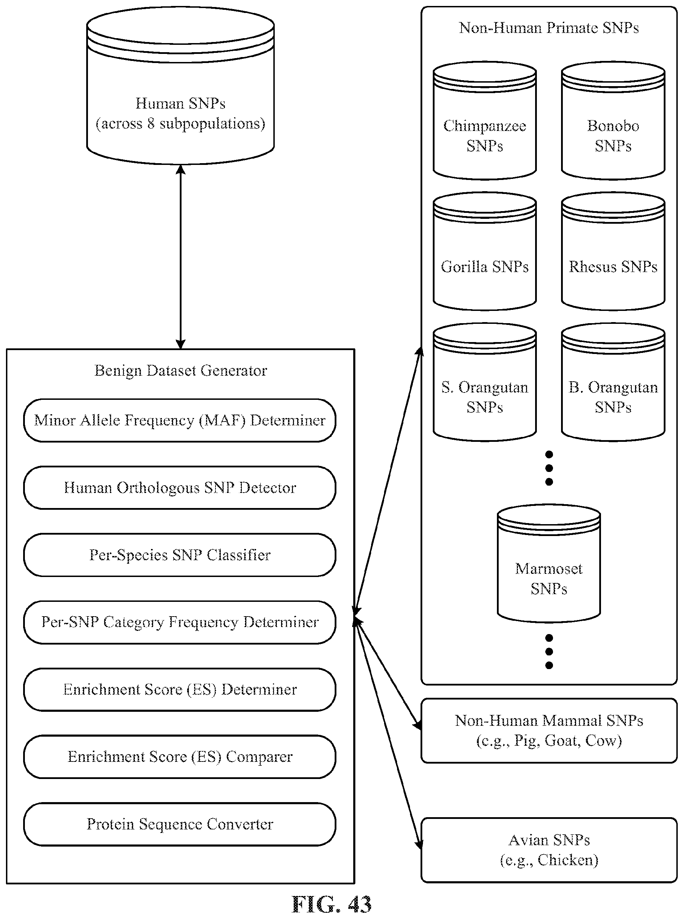

As shown in **FIGs. 43** and **44.** the non-human primate missense variants include missense variants from a plurality of non-human primate species, including Chimpanzee, Bonobo, Gorilla. B. Orangutan. S. Orangutan. Rhesus, and Marmoset.

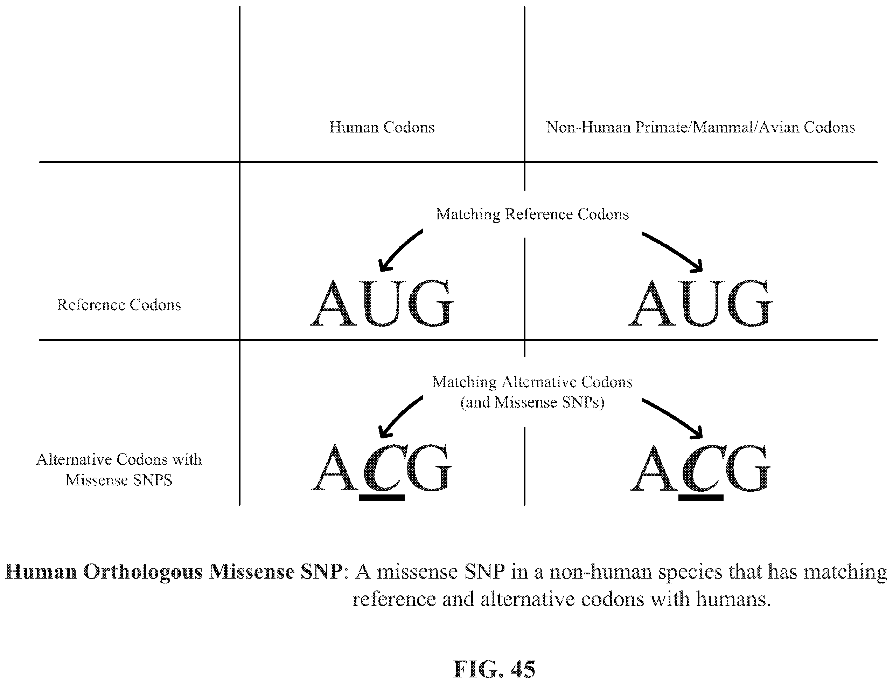

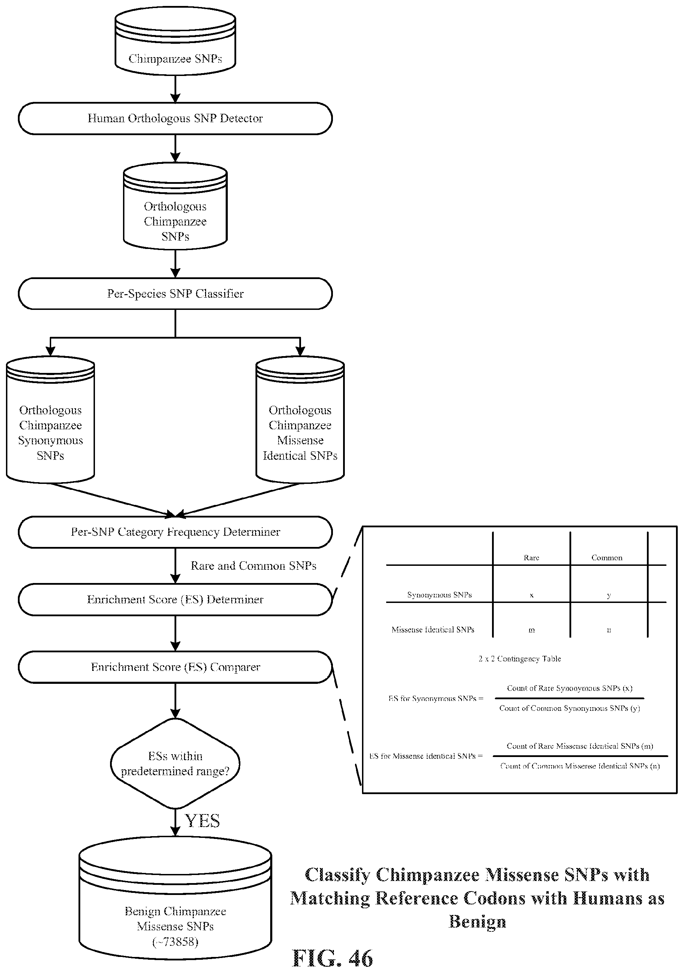

As shown in **FIGs. 45** and **46,** based on an enrichment analysis, the system accepts a particular non-human primate species for inclusion of missense variants of the particular non-human primate species among the benign variants. The enrichment analysis includes, for the particular non-human primate species, comparing a first enrichment score of synonymous variants of the particular non-human primate species to a second enrichment score of missense identical variants of the particular non-human primate species,

**FIG. 45** shows one implementation of human orthologous missense SNPs. A missense SNP in a non-human species that has matching reference and alternative codons with humans. As shown in **FIG. 45,** the missense identical variants are missense variants that share matching reference and alternative codon sequences with humans.

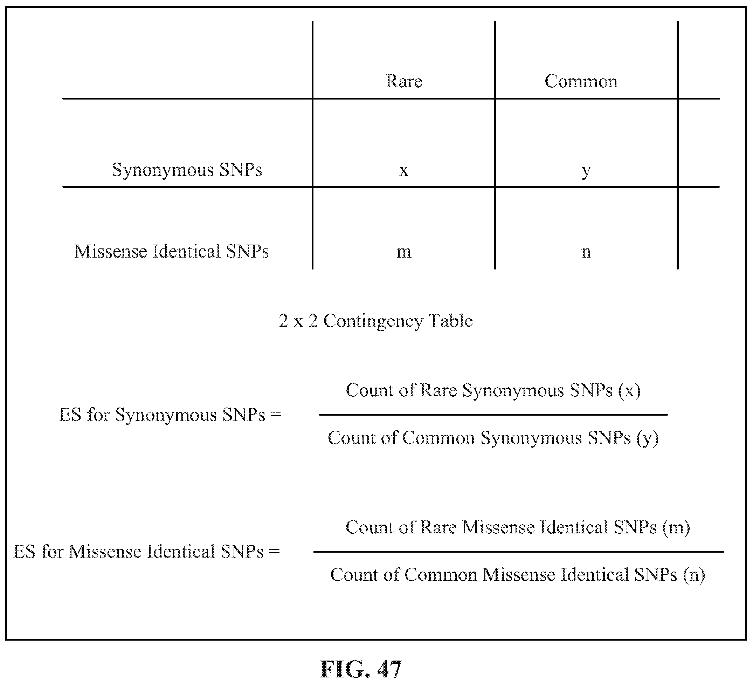

As shown in **FIGs. 46** and **47,** the first enrichment score is produced by determining a ratio of rare synonymous variants with a MAF less than 0.1% over common synonymous variants with a MAF greater than 0.1%. The second enrichment score is produced by determining a ratio of rare missense identical variants with a MAF less than 0.1% over common missense identical variants with a MAF greater than 0.1%. Rare variants include singleton variants.

As shown in **FIGs. 46** and **47,** a difference between the first enrichment score and the second enrichment score is within a predetermined range, further including accepting the particular non-human primate species for inclusion of missense variants of the particular non-human primate among the benign variants. The difference being in the predetermined range indicates that the missense identical variants are under a same degree of natural selection as the synonymous variants and therefore benign as the synonynmus variants,

As shown in **FIG. 48,** the system repeatedly applies the enrichment analysis to accept a plurality of non-human primate species for inclusion of missense variants of the non-human primate species among the benign variants. The system further includes, a chi-squared test of homogeneity to compare a first enrichment score of synonymous variants and a second enrichment score of missense identical variants for each of the non-human primate species.

As shown in **FIG. 48,** a count of the non-human primate missense variants is at least 100000. the count of the non-human primate missense variants is 385236. A count of the common human missense variants is at least 50000. The count of the common human missense variants is 83546.

Other implementations may include a non-transitory computer readable storage medium storing instructions executable by a processor to perform actions of the system described above. Yet another implementation may include a method performing actions of the system described above.

Another system implementation of the technology disclosed includes constructing a single nucleotide polymorphism (abbreviated SNP) pathogenicity classifier. The system trains a convolutional neural network-based SNP pathogenicity classifier, which runs on numerous processors coupled to memory, using benign training examples and pathogenic training examples of amino acid sequences expressed by benign SNPs and pathogenic SNPs. The benign training examples include first and second sets of nucleotide sequences, expressed as amino acid sequence pairs, each amino acid sequence including a central amino acid flanked by upstream and downstream amino acids. Each amino acid sequence pair includes a reference sequence of amino acids expressed by a reference mucleotide sequence and an alternative sequence of amino acids expressed by an alternative nucleotide sequence containing a SNP.

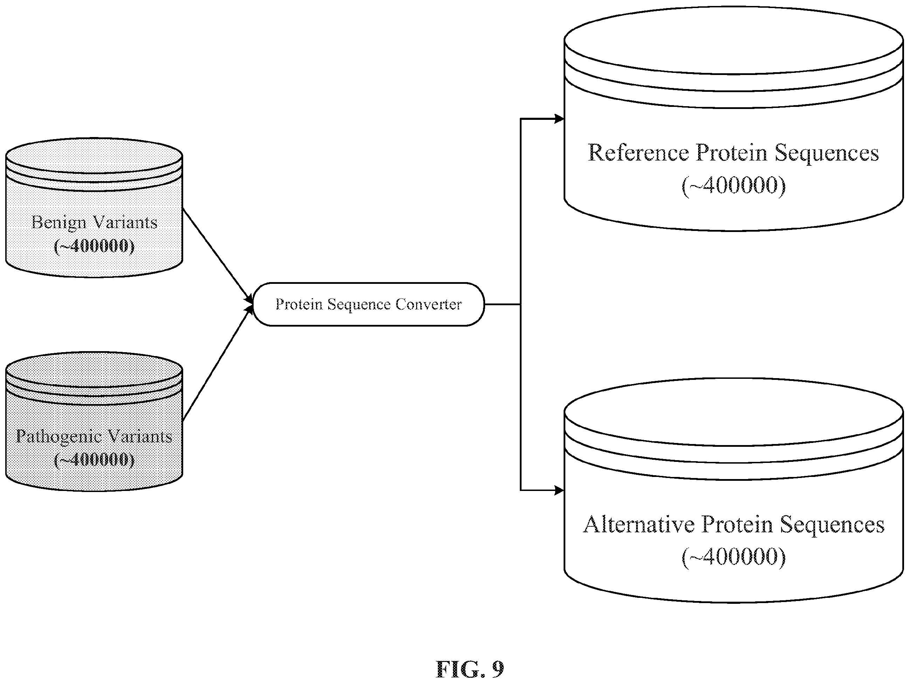

As shown in **FIG. 9.** the first set comprises human nucleotide sequence pairs, with each pair including a human alternative nuelcotide sequence containing a SNP and having a minor allele frequency (abbreviated MAF) deemed to be common within a human population. The second set comprises a non-human primate reference nucleotide sequence paired with a non-human primate alternative nucleotide sequence. The non-human primate reference nucleotide sequence has an orthologous human nucleotide reference sequence. The non-human primate alternative nucleotide sequence contains a SNP.

Each of the features discussed in this particular implementation section for the first system implementation apply equally to this system implementation. As indicated above, all the system features are not repeated here and should be considered repeated by reference.

Other implementations may include a non-transitory computer readable storage medium storing instructions executable by a processor to perform actions of the system described above. Yet another implementation may include a method performing actions of the system described above.

As shown in **FIGs. 48** and **19,** a first method implementation of the technology disclosed includes constructing a variant pathogenicity classifier, the method including. The method further includes, training a convolutional neural network-based variant pathogenicity classifier, which runs on numerous processors coupled to memory, using benign training examples and pathogenic training examples of protein sequence pairs generated from benign variants and pathogenic variants. The benign variants include common human missense variants and non-human primate missense variants occurring on allernative non-human primate codon sequences that share matching reference codon sequences with humans.

Each of the features discussed in this particular implementation section for the first system implementation apply equally to this method implementation. As indicated above, all the system features are not repeated here and should be considered repeated by reference.

Other implementations may include a non-transitory computer readable storage medium storing instructions executable by a processor to perform the method described above. Yet another implementation may include a system including memory and one or more processors operable to execute instructions, stored in the memory, to perform the method described above.

As shown in **FIGs. 48** and **19,** a second method implementation of the technology disclosed includes constructing a single nucleonde polymorphism (abbreviated SNP) pathogenicity classifier. The method further includes raining a convolutional neural network-based SNP pathogenicity classifier, which runs on numerous processors coupled to memory, using benign training examples and pathogenic training examples of amino acid sequences expressed by benign SNPs and pathogenic SNPs. The benign training examples include first and second sets of nucleotide sequences, expressed as amino acid sequence pairs, each amino acid sequence including a central amino acid flanked by upstream and downstream amino acids, and each amino acid sequence pair including a reference sequence of amino acids expressed by a reference nucleotide sequence and an alternative sequence of amino acids expressed by an alternative nucleotide sequence containing a SNP. The first set comprises human nucleotide sequence pairs, with each pair including a human alternative nucleotide sequence containing a SNP and having a minor allele frequency (abbreviated MAF) deemed to be common within a human population. The second set comprises a non-human primate reference nucleotide sequence paired with a non-human primate alternative nucleotide sequence. The non-human primate reference nucleotide sequence has an orthologous human nucleotide reference sequence and the non-human primate alternative nucleotide sequence contains a SNP.

Each of the features discussed in this particular implementation section for the second system implementation apply equally to this method implementation. As indicated above, all the system features are not repeated here and should be considered repeated by reference.

Other implementations may include a non-transitory computer readable storage medium storing instructions executable by a processor to perform the method described above. Yet another implementation may include a system including memory and one or more processors operable to execute instructions, stored in the memory, to perform the method described above.

We describe systems, methods, and articles of manufacture for using a deep convolutional neural network-based a variant pathogenicity classifier with secondary structure and solvent accessibility classifiers. One or more features of an implementation can be combined with the base implementation. Implementations that are not mutually exclusive are taught to be combinable. One or more features of an implementation can be combined with other implementations. This disclosure periodically reminds the user of these options. Omission from some implementations of recitations that repeat these options should not be taken as limiting the combinations taught in the preceding sections - these recitations are hereby incorporated forward by reference into each of the following implementations.

A system implementation of the technology disclosed includes one or more processors coupled to the memory. The memory is loaded with computer instructions to run a deep convolutional neural network-based variant pathogenicity classifier with secondary structure and solvent accessibility classifiers.

The system comprises a first secondary structure subnetwork, running on numerous processors coupled to memory, trained to predict a three-state secondary structure at amino acid locations within a protein sequence. The system further includes a second solvent accessibility subnetwork, running on numerous processors coupled to memory, trained to predict a three-state solvent accessibility at amino acid locations within a protein sequence.

The three-state secondary structure refers to one of a plurality of DNA secondary structure states alpha helix (H), beta sheet (B), and coils (C).

The three-state solvent accessibility refers to one of a plurality of protein solvent accessibility states buried (B), intermediate (I), and exposed (E).

A positional frequency matrix (abbreviated PFM) generator, running on at least one of the numerous processors, applied to three sequence groups of primates, mammals, and vertebrates excluding primates and mammals to generate a primate PFM, a mammal PFM, and a vertebrate PFM.

In other words, this includes applying the PFM generator to the primates sequence data to generate a primate PFM, applying the PFM generator to the mammals sequence data to generate a mammal PFM, and applying the PFM generator to the vertebrates sequence data not including the primates and mammals sequence data to generate a vertebrate PFM.

An input processor that accepts a variant amino acid sequence with a target variant amino acid flanked upstream and downstream by at least 25 amino acids in each direction, wherein a single nucleotide variant produces the target variant amino acid. A supplemental data allocator, running on at least one of the numerous processors, that allocates a reference amino acid sequence with a target reference amino acid flanked upstream and downstream by at least 25 amino acids in each direction, aligned with the variant amino acid sequence. Following this, it allocates reference state classifications produced by the first and second subnetworks for the reference amino acid sequence. After this the supplemental data allocator allocates variant state classifications produced by the first and second subnetworks for the variant amino acid sequence. Finally, it allocates primate, mammal, and vertebrate PFMs aligned with the reference amino acid sequence.

In the context of this application, the phrase "aligned with" refers to position-wise determining primate, mammal, and vertebrate PFMs for each amino position in the reference amino acid sequence or the alternative amino acid sequence and encoding and storing the results of the determining on the position-wise or ordinal position-basis in the same order as the amino acid positions occur in the reference amino acid sequence or the alternative amino acid sequence.

The system also includes a deep convolutional neural network, rmming on the numerous processors, trained to classify the variant amino acid sequence as benign or pathogenic based on processing the variant amino acid sequence, the allocated reference amino acid sequence, the allocated reference and variant state classifications, and the allocated PFMs. The system includes an output processor that reports at least a pathogenicity score for the variant amino acid sequence.

This system implementation and other systems disclosed optionally include one or more of the following features. System can also include features described in connection with methods disclosed. In the interest of conciseness, alternative combinations of system features are not individually enumerated. Features applicable to systems, methods, and articles of manufacture are not repeated for each statutory class set of base features. The reader will understand how fearures identified in this section can readily be combined with base features in other statutory classes.

The system comprising the deep convolutional neural network-based variant pathogenicity classifier, further configured to classify the single nucleotide variant as benign or pathogenic based on the pathogenicity score.

The system comprises the deep convolutional neural network-based variant pathogenicity classifier in which the deep convolutional neural network accepts, in parallel, as input at least the variant amino acid sequence, the allocated reference amino acid sequence, the allocated variant secondary structure state classification, the allocated reference secondary structure state classification, the allocated variant solvent accessibility state classification, the allocated reference solvent accessibility state classification, the allocated primate PFM, the allocated mammal PFM, and the allocated vertebrate PFM.

The system is configured to use the batch normalization layers, the ReLU non-linearity layers, and the dimensionality altering layers to pre-process the variant amino acid sequence, the allocated reference amino acid sequence, the allocated primate PFM, the allocated mammal PFM, and the allocated vertebrate PFM. The system is further configured to sum the pre-proecssed characterizations and concatenate the sums with the allocated variant secondary structure state classification, the allocated reference secondary structure state classification, the allocated variant solvent accessibility state classification, and the allocated reference solvent accessibility state classification to produce a concatenated input. The system processes the concatenated input through a dimensionality altering layer and accept the processed concatenated input to initiate residual blocks of the deep convolutional neural network.

The deep convolutional neural network comprises groups of residual blocks arranged in a sequence from lowest to highest. The deep convolutional neural network is parameterized by a number of residual blocks, a number of skip connections, and a number of residual connections without non-linear activations. The deep convolutional neural network comprises dimensionality altering layers that reshape spatial and feature dimensions of a preceding input.

The system is further configured to train to classify, as pathogenic, a single nucleotide variant that produces a target variant amino acid from a target reference amino acid that is conserved in aligned reference amino acid sequences across primates, mammals, and vertebrates.

The conservation represents functional significance of the target reference amino acid and is determined from the PFWs. The system is further configured to train to classify, as pathogenic, a single nucleotide variant that causes different secondary structures between a variant amino acid sequence and a reference variant amino acid sequence.

The system is further configured to train to classify, as pathogenic, a single nucleotide variant that causes different solvent accessibilities between a variant amino acid sequence and a reference variant amino acid sequence.

A PFM represents conservation of amino acids in a human protein sequence across aligned protein sequences of other species by determining, on a location-by-location basis, frequency of occurrence an amino acid in the human protein sequence across the aligned protein sequences of the other species.

The three states of secondary structure are helix, sheet, and coil, the first secondary structure subnetwork is trained to accept an input protein sequence and primate, mannual, and vertebrate PFMs aligned with amino acid locations within the input protein sequence, and predict the three-state secondary structure at each of the amino acid locations. The three states of solvent accessibility are exposed, buried, and intermediate.

The second solvent accessibility subnetwork is trained to accept an input protein sequence and primate, mammal, and vertebrate PFMs aligned with amino acid locations within the input protein sequence, and predict the three-state solvent accessibility at each of the amino acid locations. The input protein sequence is a reference protein sequence. The input protein sequence is an alternative protein sequence. The first secondary structure subnetwork comprises groups of residual blocks arranged in a sequence from lowest to highest. The first secondary structure subnetwork is parameterized by a number of residual blocks, a number of skip connections, and a number of residual connections without non-linear activations.

The first secondary structure subnetwork comprises dimensionality altering layers that reshape spatial and feature dimensions of a preceding input. The second solvent accessibility subnetwork comprises groups of residual blocks arranged in a sequence from lowest to highest. The second solvent accessibility subnetwork is parameterized by a number of residual blocks, a number of skip connections, and a number of residual connections without non-linear activations. The second solvent accessibility subnetwork comprises dimensionality altering layers that reshape spatial and feature dimensions of a preceding input.

Each residual block comprises at least one batch normalization layer, at least one rectified linear unit (abbreviated ReLU) layer, at least one dimensionality altering layer, and at least one residual connection. Each residual block comprises two batch normalization layers, two ReLU non-linearity layers, two dimensionality altering layers, and one residual connection.

The deep convolutional neural network, the first secondary structure subnetwork, and the second solvent accessibility subnetwork each comprise an ultimate classification layer. The ultimate classification layer is a sigmoid-based layer. The ultimate classification layer is a sofimax-based layer.

The system is further configured to ablate ultimate classification layers of the first secondary structure subnetwork and the second solvent accessibility subnetwork for collaboration with the deep convolutional neural network.

The system is further configured to, during training of the deep convolutional neural network, further train the first secondary structure subnetwork and the second solvent accessibility subnetwork on pathogenicity classification, including hack propagating errors to the subnetworks and updating subnetwork weights.

The second solvent accessibility subnetwork comprises at least on atrous convolution layer. The system is further configured to classify developmental delay disorder (abbreviated DDD)-causing variants as pathogenic. The variant amino acid sequence and the reference amino acid sequence share flanking amino acids. The system is further configured to use one-hot encoding to encode inputs to the deep convolutional neural network.

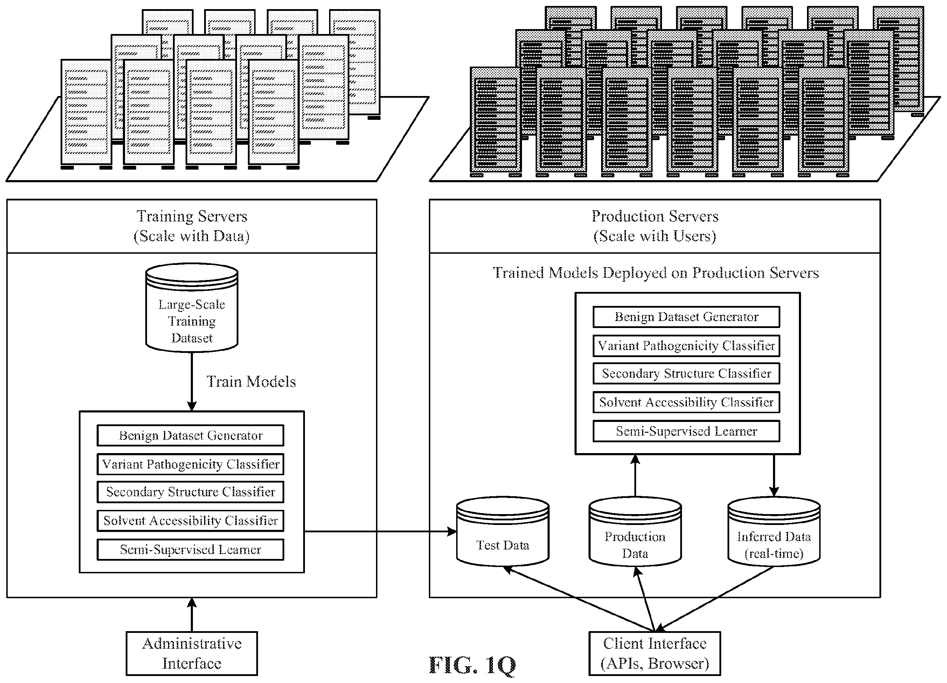

**FIG. 1Q** shows an example computing environment in which the technology disclosed can be operated. The deep convolutional neural network, the first secondary structure subnetwork, and the second solvent accessibility subnetwork are trained on one or more training servers. The trained deep convolutional neural network, the first trained secondary structure subnetwork, and the trained second solvent accessibility subnetwork are deployed on one or more production servers that receive input sequences from requesting clients. The production servers process the input sequences through at least one of the deep convolutional neural network, the first secondary structure subnetwork, and the second solvent accessibility subnetwork to produce outputs that are transmitted to the clients.

Other implementations may include a non-transitory computer readable storage medium storing instructions executable by a processor to perform actions of the system described above. Yet another implementation may include a method performing actions of the system described above.

Another system implementation of the technology disclosed includes a deep convolutional neural network-based variant pathogenicity classifier, running on numerous processors coupled to memory. The system includes, a positional frequency matrix (abbreviated PFM) generator, running on at least one of the numerous processors, applied to two sequence groups of primates and mammals to generate a primate PFM and a mammal PFM. The system also includes an input processor that accepts a variant amino acid sequence with a target variant amino acid flanked upstream and downstream by at least 25 amino acids in each direction, wherein a single nucleotide variant produces the target variant amino acid. The system also includes a supplemental data allocator, running on at least one of the numerous processors, that allocates a reference amino acid sequence with a target reference amino acid flanked upstream and downstream by at least 25 amino acids in each direction, aligned with the variant amino acid sequence. It also allocates primate and mammal PFMs aligned with the reference amino acid sequence. The system further includes a deep convolutional neural network, running on the numerous processors, trained to classify the variant amino acid sequence as benign or pathogenic based on processing the variant amino acid sequence, the allocated reference amino acid sequence, and the allocated PFMs. Finally, the system includes, an output processor that reports at least a pathogenicity score for the variant amino acid sequence.

This system implementation and other systems disclosed optionally include one or more of the following features. System can also include features described in connection with methods disclosed. In the interest of conciseness, alternative combinations of system features are not individually enumerated. Features applicable to systems, methods, and articles of manufacture are not repeated for each statutory class set of base features. The reader will understand how features identified in this section can readily be combined with base features in other statutory classes.

The system is further configured to classify the single nucleotide variant as benign or pathogenic based on the pathogenicity score. The deep convolutional neural network, in parallel, accepts and processes the variant amino acid sequence, the allocated reference amino acid sequence, the allocated primate PFM, and the allocated mammal PFM. The system is further configured to train to classify, as pathogenic, a single nucleotide variant that produces a target variant amino acid from a target reference amino acid that is conserved in reference amino acid sequences across primates and mammals. The conservation represents functional significance of the target reference amino acid and is determined from the PFWs.

Each of the features discussed in this particular implementation section for the first system implementation apply equally to this system implementation. As indicated above, all the system features are not repeated here and should be considered repeated by reference.

Other implementations may include a non-transitory computer readable storage medium storing instructions executable by a processor to perform actions of the system described above. Yet another implementation may include a method performing actions of the system described above.

A first method implementation of the technology disclosed includes running a first secondary structure subnetwork on numerous processors coupled to memory, trained to predict a three-state secondary structure at amino acid locations within a protein sequence. Running a second solvent accessibility subnetwork on numerous processors coupled to memory, trained to predict a three-state solvent accessibility at amino acid locations within a protein sequence. Running on at least one of the numerous processors, a positional frequency matrix (abbreviated PFM) generator, applied to three sequence groups of primates, mammals, and vertebrates excluding primates and mammals to generate a primate PFM, a mammal PFM. and a vertebrate PFM. Accepting a variant amino acid sequence an input processor with a target variant amino acid flanked upstream and downstream by at least 25 amino acids in each direction. A single nucleotide variant produces the target variant amino acid. Running on at least one of the numerous processors, a supplemental data allocator that allocates a reference amino acid sequence with a target reference amino acid flanked upstream and downstream by at least 25 amino acids in each direction, aligned with the variant amino acid sequence. It also allocates reference state classifications produced by the first and second subnetworks for the reference amino acid sequence. It further allocates variant state classifications produced by the first and second subnetworks for the variant amino acid sequence. It allocates primate, mammal, and vertebrate PFMs aligned with the reference emino acid sequence. Running on the numerous processors, a deep convolutional neural network trained to classify the variant amino acid sequence as benign or pathogenic based on processing the variant amino acid sequence, the allocated reference amino acid sequence, the allocated reference and variant state classifications, and the allocated PFMs. Reporting at least a pathogenicity score for the variant amino acid sequence through an output processor.

Each of the features discussed in this particular implementation section for the first system implementation apply equally to this method implementation. As indicated above, all the system features are not repeated here and should be considered repeated by reference.

Other implementations may include a non-transitory computer readable storage medium storing instructions executable by a processor to perform the method described above. Yet another implementation may include a system including memory and one or more processors operable to execute instructions, stored in the memory, to perform the method described above.

A second method implementation of the technology disclosed includes running on numerous processors coupled to memory, a deep convolutional neural network-based variant pathogenicity classifier. Running a positional frequency matrix (abbreviated PFM) generator on at least one of the numerous processors, applied to two sequence groups of primates and mammals to generate a primate PFM and a mammal PFM. Accepting in an input processor, a variant amino acid sequence with a target variant amino acid flanked upstream and downstream by at least 25 amino acids in each direction. A single nucleotide variant produces the target variant amino acid. Running a supplemental data allocator on at least one of the numerous processors, that allocates a reference amino acid sequence with a target reference amino acid flanked upstream and downstream by at least 25 amino acids in each direction, aligned with the variant amino acid sequence and allocates primate and mammal PFMs aligned with the reference amino acid sequence. Running a deep convolutional neural network on the numerous processors, trained to classify the variant amino acid sequence as benign or pathogenic based on processing the variant amino acid sequence. the allocated reference amino acid sequence, and the allocated PFMs. Reporting at least a pathogenicity score for the variant amino acid sequence in an output processor.

Each of the features discussed in this particular implementation section for the second system implementation apply equally to this method implementation. As indicated above, all the system features are not repeated here and should be considered repeated by reference.

Other implementations may include a non-transitory computer readable storage medium storing instructions executable by a processor to perform the method described above. Yet another implementation may include a system including memory and one or more processors operable to execute instructions, stored in the memory, to perform the method described above.

Yet another system implementation of the technology disclosed includes a system that generates large-scale pathogenic training data for training a single nucleotide polymorphism (abbreviated SNP) pathogenicity classifier.

As shown in **FIG. 19,** the system trains the SNP pathogenicity classifier, which runs on numerous processors coupled to memory, using a training set of benign SNPs and a training set of elite predicted pathogenic SNPs that are culled from a synthetic set of combinatorically generated SNPs. In the context of this application, elite predicted pathogenic SNPs are those SNPs are produced/selected at the end of each cycle based on their average or maxinmm pathogenicity scores, as outputted by the ensemble. The term "elite" is borrowed from the genetic algorithm vocabulary and is intended to have a meaning typically given in genetic algorithm publications.

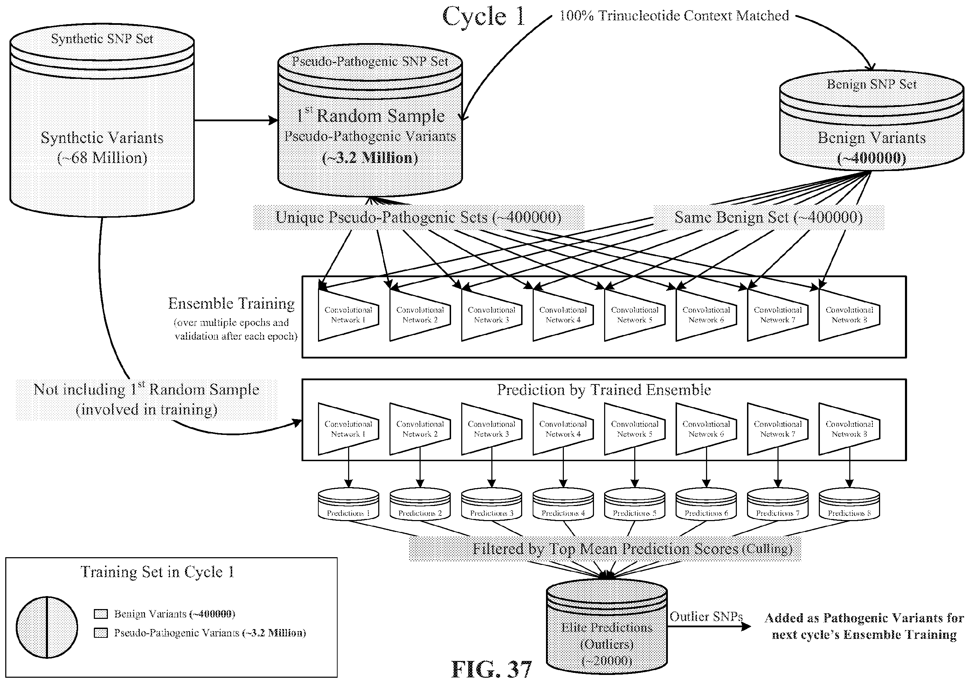

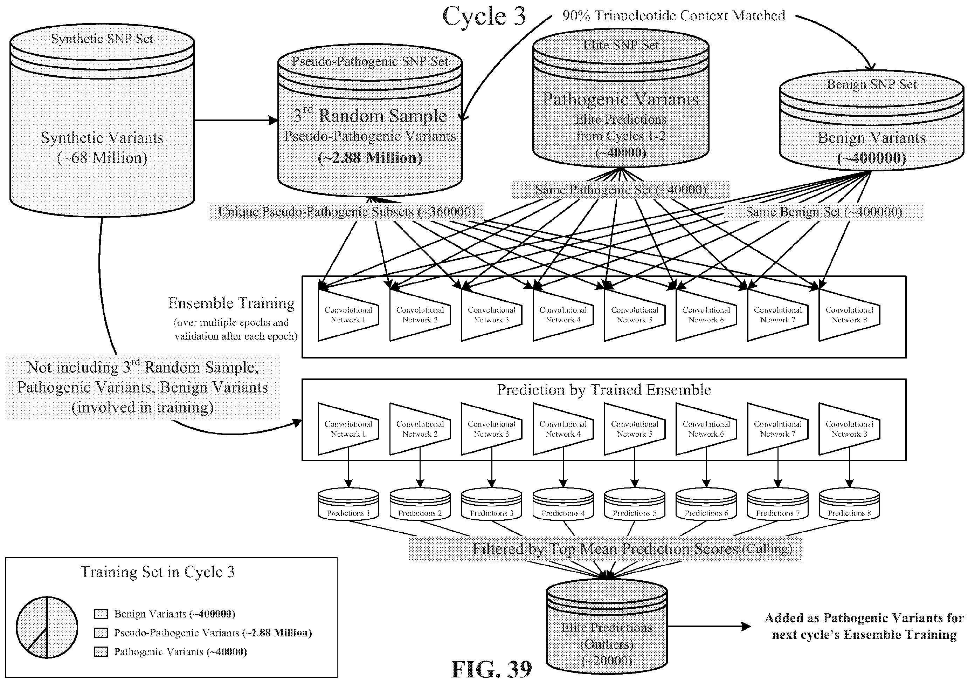

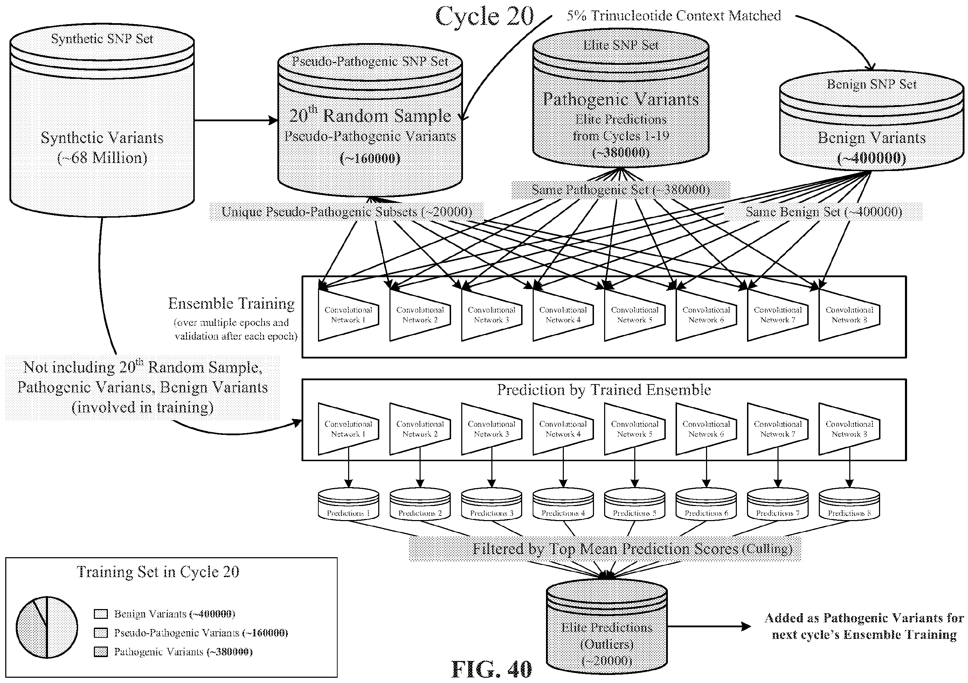

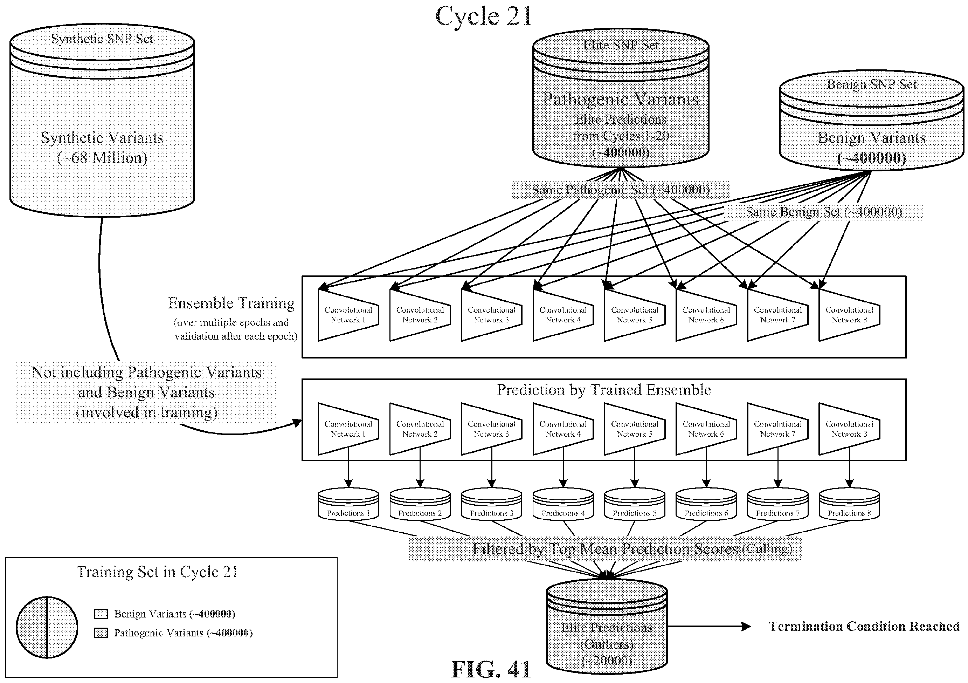

As shown in **FIGs. 37, 38, 39, 40, 41,** and **42,** the system builds the clite set iteratively in cycles, beginning with no predicted SNPs and accumulating a full set of predicted SNPs by calling outlier SNPs from the synthetic set. The synthetic set comprises pseudo-pathogenic SNPs that are combinatorically generated SNPs not present in the benign set and decrease in set membership as the outlier SNPs are iteratively culled from the synthetic set for inclusion in the elite set. In the context of this application, the term "culling" means filtering, replacing, updating, or selecting a previous population with a new population. The term "culling" is borrowed from the genetic algorithm vocabulary and is intended to have a meaning typically given in genetic algorithm publications.

As shown in **FIGs. 37, 38, 39, 40, 41,** and **42,** the system trains and applies an ensemble of SNP pathogenicity classifiers to cull the outlier SNPs from the synthetic set, iteratively in cycles. This includes training the ensemble using a common training set of benign SNPs, a common training set of elite predicted pathogenic SNPs, and separate training sets of pseudo-pathogenic SNPs sampled from the synthetic set without replacement. This also includes applying the trained ensemble to cull the outlier SNPs from the synthetic set and accumulate culled outlier SNPs in the common elite set by applying the trained ensemble to score at least some SNPs from the synthetic set that were not used for traning the ensemble in a current cycle and using the scores to select, from the scored SNPs, current cycle outlier SNPs to accumulate in the common elite set.

In the context of this application, "pseudo-pathogenic SNPs" are those SNPs that are labelled as pathogenic for training purposes and sampled from the synthetically-generated variants without replacement during the training.

Also, the training set of elite predicted pathogenic SNPs is built iteratively over multiple cycles.

As shown in **FIGs. 37, 38, 39, 40, 41,** and **42,** the system then stores in memory, classifier parameters derived by the training, a common elite set completed over the cycles and within a predetermined spread of the common benign set, and the common benign set for training the SNP pathogenicity classifier.

As shown in **FIGs. 37, 38, 39, 40, 41,** and **42,** the elite predicted pathogenic SNPs are top 5% of the SNPs predicted by the ensemble. In some implementanons, they are fixed number of top-scored SNPs, such as 20000.

The SNP pathogenicity classifier and the ensemble of SNP pathogenicity classifiers are each deep convolutional neural networks (abbreviated DCNN). The ensemble includes 4 to 16 DCNNs. As shown in **FIGs. 37, 38, 39, 40. 41,** and **42,** the ensemble includes 8 DCNNs.

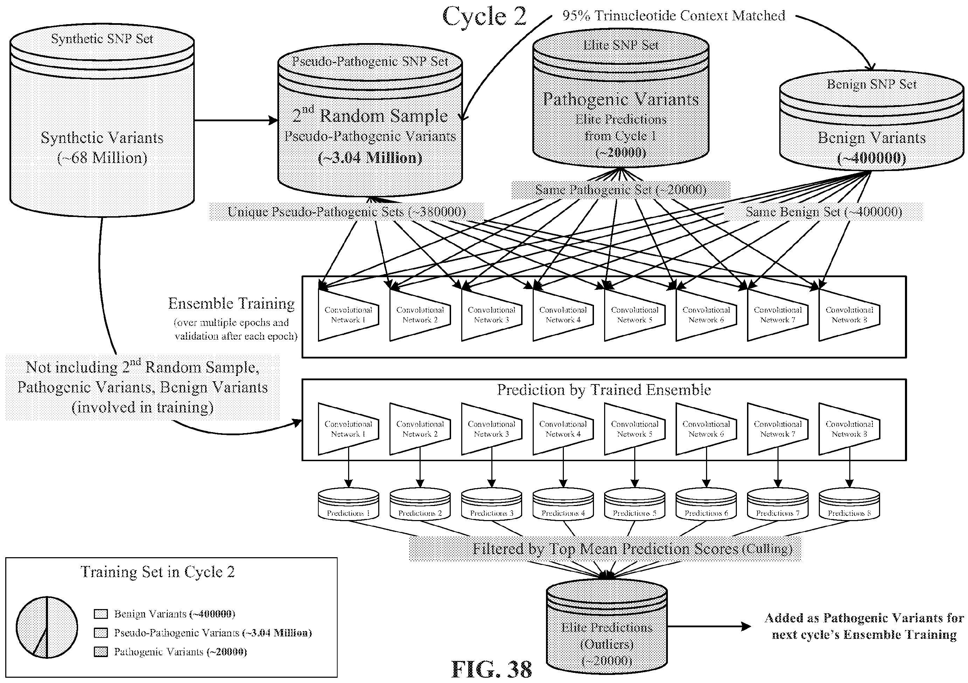

As shown in **FIGs. 37, 38. 39, 40, 41,** and **42,** the system trains the ensemble of DCCNs in epochs during the cycles, concluding the training for a particular cycle when predictions on a validation sample form discrete probability distribution clusters of benign and pathogenic predictions.

As shown in **FIGs. 37, 38, 39, 40. 41,** and **42,** the system uses the scores to select the current cycle outlier SNPs by summing scores from the ensemble of DCCNs.

As shown in **FIGs. 37, 38, 39, 40, 41,** and **42,** the system uses the scores to select the current cycle outlier SNPs by taking a maximum mean value for each of the SNPs scored by the ensemble of DCNNs.

As shown in **FIGs. 37, 38, 39, 40, 41,** and **42,** the sampling without replacement during a current cycle results in disjoint separate training sets of pseudo-pathogenic SNPs during the current cycle.

The system continues the cycles until a termination condition is reached. The termination condition can be a predetermined number of cycles. As shown in **FIGs. 37, 38, 39, 40, 41,** and **42,** the predetermined number of cycles is 21.

As shown in **FIGs. 37, 38, 39, 40, 41,** and **42,** the termination condition is when an elite predicted pathogenic set size is within a predetermined spread of a benign set size.

The classifier parameters can be at least convolution filter **weights** and learning rate.

The system can select one of the SNP pathogenicity classifiers in the ensemble as the SNP pathogenicity classifier. The selected SNP pathogenicity classifier can be the one that outpredicted other SNP pathogenicity classifiers in the ensemble on a validation sample evaluated in an ultimate cycle.

As shown in **FIGs. 37, 38, 39, 40, 41,** and **42, the** common elite set completed over the cycles can have at least 400000 elite predicted pathogenic SNPs.

As shown in **FIGs. 37, 38, 39, 40, 41,** and **42,** the system, in each cycle, can match trinucleotide context between the benign SNPs and the sampled pseudo-pathogenic SNPs to prevent mutation rate bias in the elite predicted pathogenic SNPs.

As shown in **FIGs. 37, 38, 39, 40, 41,** and **42,** the sampling of psendo-pathogenic SNPs from the synthetic set can decrease by 5% in each successive cycle.

As shown **in FIGs. 37, 38, 39, 40, 41,** and **42,** the system can filter the synthetic SNPs scored in the current cycle by pseudo-pathogenic SNPs sampled in the current cycle for training, the elite predicted pathogenic SNPs, and the benign SNPs used in the current cycle for training.

Each of the features discussed in this particular implementation section for the first system implementation apply equally to this system implementation. As indicated above, all the system features are not repeated here and should be considered repeated by reference.

Other implementations may include a non-transitory computer readable storage medium storing instructions executable by a processor to perform actions of the system described above. Yet another implementation may include a system including memory and one or more processors operable to execute instructions, stored in the memory, to perform actions of the system described above.

Yet another implementation of the technology disclosed includes a convolutional neural network (abbreviated CNN)-based semi-supervised learner, as shown in **FIG. 36.**

As shown in **FIG. 36,** the semi-supervised learner can include an ensemble of CNNs, running on numerous processors coupled to memory, that is iteratively trained on a benign training set and a pathogenic training set.

As shown in **FIG. 36**, the semi-supervised learner can include a set augmenter, running on at least one of the processors, that progressively augments a set size of the pathogenic training set based on the trained ensemble's evaluation of a synthetic set;

In each iteration, the evaluation produces an elite predicted pathogenic set that is added to the pathogenic training set by the set augmenter.

The semi-supervised learner can include a builder that uses at least one of the CNNs, an augmented pathogenic training set, and the benign training set to construct and train a single nucleotide polymorphism (abbreviated SNP) pathogenicity classifier.

Each of the features discussed in this particular implementation section for the first system implementation apply equally to this system implementation. As indicated above, all the system features are not repeated here and should be considered repeated by reference.

Other implementations may include a non-transitory computer readable storage medium storing instructions executable by a processor to perform actions of the system described above. Yet another implementation may include a system including memory and one or more processors operable to execute instructions, stored in the memory, to perform actions of the system described above.

The preceding description is presented to enable the making and use of the technology disclosed. Various modifications to the disclosed implementations will be apparent, and the general principles defined herein may be applied to other implementations and applications without departing from the spirit and scope of the technology disclosed. Thus, the technology disclosed is not intended to be limited to the implementations shown, but is to be accorded the widest scope consistent with the principles and features disclosed herein. The scope of the technology disclosed is defined by the appended claims.

## Computer System

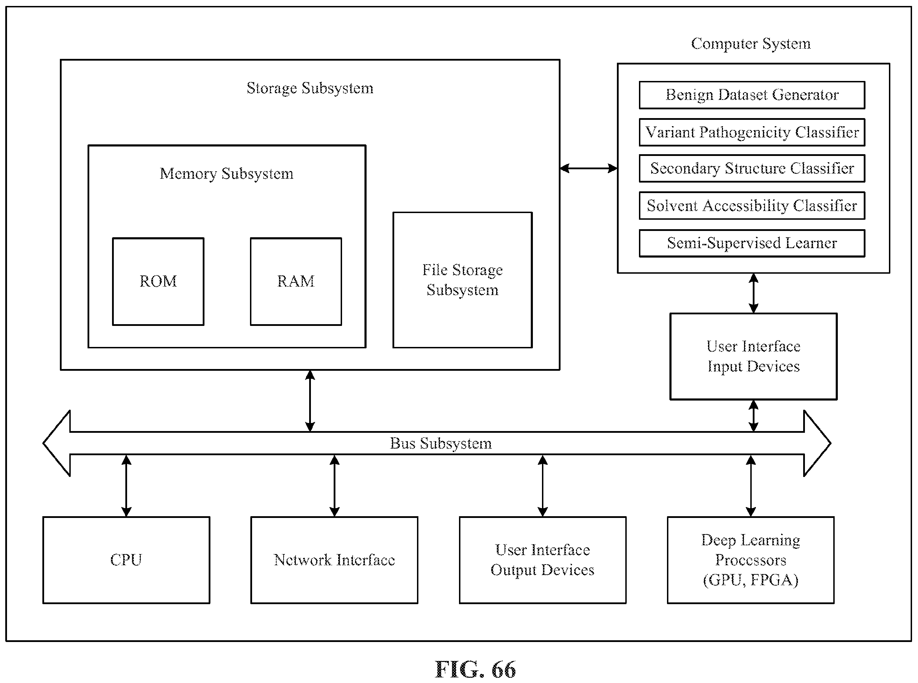

**FIG. 66** is a simplified block diagram of a computer system that can be used to implement the technology disclosed. Computer system typically iuctues at least one processor that communicates with a number of peripheral devices via bus subsystem. These peripheral devices can include a storage subsystem including, for example, memory devices and a file storage subsystem, user interface input devices, user interface output devices, and a network interface subsystem. The input and output devices allow user interaction with computer system. Network interface subsystem provides an interface to outside networks, including an interface to corresponding interface devices in other computer systems.

In one implementation, the neural networks such as benign dataset generator, variant pathogenicity classifier, secondary structure classifier, solvent accessibility classifier, and semi-supervised learner are communicably linked to the storage subsystem and user interface input devices.

User interface input devices can include a keyboard; pointing devices such as a mouse, trackball, touchpad, or graphics tablet; a scanner; a touch screen incorporated into the display: audio input devices such as voice recognition systems and microphones; and other types of input devices. In general, use of the term "input device" is intended to include all possible types of devices and ways to input information into computer system.

User interface output devices can include a display subsystem, a printer, a fax machine, or non-visual displays such as audio output devices. The display subsystem can include a cathode ray tube (CRT), a flat-panel device such as a liquid crystal display (LCD), a projection device, or some other mechanism for creating a visible image. The display subsystem can also provide a non-visual display such as audio output devices. In general, use of the term "output device" is intended to include all possible types of devices and ways to output information from computer system to the user or to another machine or computer system.

Storage subsystem stores programming and data constructs that provide the functionality of some or all of the modules and methods described herein. These software modules are generally executed by processor alone or in combination with other processors.

Memory used in the storage subsystem can include a number of memories including a main random access memory (RAM) for storage of instructions and data during program execution and a read only memory (ROM) in which fixed instructions are stored. A file storage subsystem can provide persistent storage for program and data files, and can include a hard disk drive, a floppy disk drive along with associated removable media, a CD-ROM drive, an optical drive, or removable media cartridges. The modules implementing the functionality of certain implementations can be stored by file storage subsystem in the storage subsystem, or in other machines accessible by the processor.

Bus subsystem provides a mechanism for letting the various components and subsystems of computer system communicate with each other as intended. Although bus subsystem is shown schematically as a single bus, alternative implementations of the bus subsystem can use multiple busses.

Computer system itself can be of varying types including a personal computer, a portable computer, a workstation, a computer terminal, a network computer, a television, a mainframe, a server farm, a widely-distributed set of loosely networked computers, or any other data processing system or user device. Due to the ever-changing nature of computers and networks, the description of computer system depicted in **FIG. 66** is intended only as a specific example for purposes of illustrating the technology disclosed. Many other configurations of computer system are possible having more or less components than the computer system depicted in **FIG. 66.**

The deep learning processors can be GPUs or FPGAs and can be hosted by a deep learning cloud platforms such as Google Cloud Platform, Xilinx, and Cirrascale. Examples of deep learning processors include Google's Tensor Processing Unit (TPU), rackmount solutions like GX4 Rackmount Series, GX8 Rackmount Series, NVIDIA DGX-1, Microsoft' Stratix V FPGA, Graphcore's Intelligent Processor Unit (IPU), Qualcomm's Zeroth platform with Snapdragon processors, NVIDIA's Volta, NVIDIA's DRIVE PX, NVIDIA's JETSON TX1/TX2 MODULE, Intel's Nirvana, Movidius VPU, Fujitsu DPI, ARM's DynamicIQ, IBM TrueNorth, and others.

Various embodiments of the present invention are described in the following numbered clauses.

1. A method of constructing a variant pathogenicity classifier, the method including:

training a convolutional neural network-based variant pathogenicity classifier, which runs on numerous processors coupled to memory, using benign training examples and pathogenic training examples of protein sequence pairs generated from benign variants and pathogenic variants; and

wherein the benign variants include common human missense variants and non-human primate missense variants occurring on alternative non-human primate codon sequences that share matching reference codon sequences with humans.

2. The method of clause 1, wherein the common human missense variants have a minor allele frequency (abbreviated MAF) greater than 0.1 % across a **human** population variant dataset sampled from at least 100000 humans.

3. The method of any of clauses 1-2, wherein the sampled humans belong to different human subpopulations and the common human missense variants have a MAF greater than 0.1% within respective human subpopulation variant datasets.

4. The method of any of clauses 1-3, wherein the human subpopulations include African/African American (abbreviated AFR), American (abbreviated AMR), Ashkenazi Jewish (abbreviated ASJ), East Asian (abbreviated EAS), Finnish (abbreviated FIN), Non-Finnish European (abbreviated NFE), South Asian (abbreviated SAS), and Others (abbreviated OTH).

5. The method of any of clauses 1-4, wherein the non-human primate missense variants include missense variants from a plurality of non-human primate species, including Chimpanzee, Bonobo, Gorilla, B. Orangutan, S. Orangutan, Rhesus, and Marmoset.

6. The method of any of clauses 1-5, further including, based on an enrichment analysis, accepting a particular non-human primate species for inclusion of missense variants of the particular non-human primate species among the benign variants, wherein the enrichment analysis includes, for the particular non-human primate species, comparing a first enrichment score of synonymous variants of the particular non-human primate species to a second enrichment score of missense identical variants of the particular non-human primate species.

7. The method of any of clauses 1-6, wherein the missense identical variants are missense variants that share matching reference and alternative codon sequences with humans.

8. The method of any of clauses 1-7, wherein the first enrichment score is produced by determining a ratio of rare synonymous variants with a MAF less than 0.1% over common synonymous variants with a MAF greater than 0.1%.

9. The method of any of clauses 1-8, wherein the second enrichment score is produced by determining a ratio of rare missense identical variants with a MAF less than 0.1% over common missense identical variants with a MAF greater than 0.1 %.

10. The method of any of clauses 1-9, wherein rare variants include singleton variants.

11. The method of any of clauses 1-10, wherein a difference between the first enrichment score and the second enrichment score is within a predetermined range, further including accepting the particular non-human primate species for inclusion of missense variants of the particular non-human primate among the benign variants.

12. The method of any of clauses 1-11, wherein the difference being in the predetermined range indicates that the missense identical variants are under a same degree of natural selection as the synonymous variants and therefore benign as the synonymous variants.

13. The method of any of clauses 1-12, further including repeatedly applying the enrichment analysis to accept a plurality of non-human primate species for inclusion of missense variants of the non-human primate species among the benign variants.

14. The method of any of clauses 1-13, further including using a chi-squared test of homogeneity to compare a first enrichment score of synonymous variants and a second enrichment score of missense identical variants for each of the non-human primate species.

15. The method of any of clauses 1-14, wherein a count of the non-human primate missense variants is at least 100000.

16. The method of any of clauses 1-15, wherein the count of the non-human primate missense variants is 385236.

17. The method of any of clauses 1-16, wherein a count of the common human missense variants is at least 50000.

18. The method of any of clauses 1-17, wherein the count of the common human missense variants is 83546.

19. A method of constructing a single nucleotide polymorphism (abbreviated SNP) pathogenicity classifier, the method including:

    training a convolutional neural network-based SNP pathogenicity classifier, which runs on numerous processors coupled to memory, using benign training examples and pathogenic training examples of amino acid sequences expressed by benign SNPs and pathogenic SNPs;

wherein the benign training examples include first and second sets of nucleotide sequences, expressed as amino acid sequence pairs, each amino acid sequence including a central amino acid flanked by upstream and downstream amino acids, and each amino acid sequence pair including

a reference sequence of amino acids expressed by a reference nucleotide sequence and

an alternative sequence of amino acids expressed by an alternative nucleotide sequence containing a SNP;

the first set comprises human nucleotide sequence pairs, with each pair including a human alternative nucleotide sequence containing a SNP and having a minor allele frequency (abbreviated MAF) deemed to be common within a human population; and

the second set comprises a non-human primate reference nucleotide sequence paired with a non-human primate alternative nucleotide sequence, wherein

the non-human primate reference nucleotide sequence has an orthologous human nucleotide reference sequence and

the non-human primate alternative nucleotide sequence contains a SNP.

20. A non-transitory computer readable storage medium impressed with computer program instructions to construct a variant pathogenicity classifier, when executed on a processor, implement a method comprising:

training a convolutional neural network-based variant pathogenicity classifier, which runs on numerous processors coupled to memory, using benign training examples and pathogenic training examples of protein sequence pairs generated from benign variants and pathogenic variants; and

wherein the benign variants include common human missense variants and non-human primate missense variants occurring on alternative non-human primate codon sequences that share matching reference codon sequences with humans.

21. A system including one or more processors coupled to memory, the memory loaded with computer instructions to construct a variant pathogenicity classifier, the instructions, when executed on the processors, implement actions comprising:

training a convolutional neural network-based variant pathogenicity classifier, which runs on numerous processors coupled to memory, using benign training examples and pathogenic training examples of protein sequence pairs generated from benign variants and pathogenic variants; and

wherein the benign variants include common human missense variants and non-human primate missense variants occurring on alternative non-human primate codon sequences that share matching reference codon sequences with humans.

22. A non-transitory computer readable storage medium impressed with computer program instructions to construct a single nucleotide polymorphism (abbreviated SNP) pathogenicity classifier, when executed on a processor, implement a method comprising:

training a convolutional neural network-based SNP pathogenicity classifier, which runs on numerous processors coupled to memory, using benign training examples and pathogenic training examples of amino acid sequences expressed by benign SNPs and pathogenic SNPs;

wherein the benign training examples include first and second sets of nucleotide sequences, expressed as amino acid sequence pairs, each amino acid sequence including a central amino acid flanked by upstream and downstream amino acids, and each amino acid sequence pair including

a reference sequence of amino acids expressed by a reference nucleotide sequence and

an alternative sequence of amino acids expressed by an alternative nucleotide sequence containing a SNP;

the first set comprises human nucleotide sequence pairs, with each pair including a human alternative nucleotide sequence containing a SNP and having a minor allele frequency (abbreviated MAF) deemed to be common within a human population; and

the second set comprises a non-human primate reference nucleotide sequence paired with a non-human primate alternative nucleotide sequence, wherein

the non-human primate reference nucleotide sequence has an orthologous human nucleotide reference sequence and

the non-human primate alternative nucleotide sequence contains a SNP.

23. A system including one or more processors coupled to memory, the memory loaded with computer instructions to construct a single nucleotide polymorphism (abbreviated SNP) pathogenicity classifier, the instructions, when executed on the processors, implement actions comprising:

training a convolutional neural network-based SNP pathogenicity classifier, which runs on numerous processors coupled to memory, using benign training examples and pathogenic training examples of amino acid sequences expressed by benign SNPs and pathogenic SNPs;

wherein the benign training examples include first and second sets of nucleotide sequences, expressed as amino acid sequence pairs, each amino acid sequence including a central amino acid flanked by upstream and downstream amino acids, and each amino acid sequence pair including

a reference sequence of amino acids expressed by a reference nucleotide sequence and

an alternative sequence of amino acids expressed by an alternative nucleotide sequence containing a SNP;

the first set comprises human nucleotide sequence pairs, with each pair including a human alternative nucleotide sequence containing a SNP and having a minor allele frequency (abbreviated MAF) deemed to be common within a human population; and

the second set comprises a non-human primate reference nucleotide sequence paired with a non-human primate alternative nucleotide sequence, wherein

the non-human primate reference nucleotide sequence has an orthologous human nucleotide reference sequence and

the non-human primate alternative nucleotide sequence contains a SNP.

## APPENDIX

The following is a bibliography of potentially relevant references listed in a paper authored by the inventors. The subject matter of the paper is covered in the US Provisionals to which this Application claims priority to/benefit of. These references can be made available by the Counsel upon request or may be accessible via Global Dossier. The paper is the first listed reference.

1. Laksshman Sundaram, Hong Gao, Samskruthi Reddy Padigepati, Jeremy F. McRae, Yanjun Li, Jack A. Kosmicki, Nondas Fritzilas, Jörg Hakenberg, Anindita Dutta, John Shon, Jinbo Xu, Serafim Batzloglou, Xiaolin Li & Kyle Kai-How Farh. Predicting the clinical impact of human mutation with deep neural networks. Nature Genetics volume 50, pages1161-1170 (2018). Accessible at https://www.nature.com/articles/s41588-018-0167-z.
2. MacArthur, D. G. et al. Guidelines for investigating causality of sequence variants in human disease. Nature 508, 469-476, doi:10.1038/nature13127 (2014).
3. Rehm, H. L., J. S. Berg, L. D. Brooks, C. D. Bustamante, J. P. Evans, M. J. Landrum, D. H. Ledbetter, D. R. Maglott, C. L. Martin, R. L. Nussbaum, S. E. Plon, E. M. Ramos, S. T. Sherry, M. S. Watson. ClinGenthe Clinical Genome Resource. N. Engl. J. Med. 372, 2235-2242 (2015).
4. Bamshad, M. J., S. B. Ng, A. W. Bigham, H. K. Tabor, M. J. Emond, D. A. Nickerson, J. Shendure. Exome sequencing as a tool for Mendelian disease gene discovery. Nat. Rev. Genet. 12, 745-755 (2011).

5. Rehm, H. L. Evolving health care through personal genomics. Nature Reviews Genetics 18, 259-267 (2017).

6. Richards, S. et al. Standards and guidelines for the interpretation of sequence variants: a joint consensus recommendation of the American College of Medical Genetics and Genomics and the Association for Molecular Pathology. Genet Med 17, 405-424, doi:10.1038/gim.2015.30 (2015).

7. Lek, M. et al. Analysis of protein-coding genetic variation in 60,706 humans. Nature 536, 285-291, doi:10.1038/nature19057 (2016).

8. Mallick, S. et al. The Simons Genome Diversity Project: 300 genomes from 142 diverse populations. Nature 538, 201-206, doi:10.1038/nature18964 (2016).

9. Genomes Project Consortium et al. A global reference for human genetic variation. Nature 526, 68-74, doi:10.1038/nature15393 (2015).

10. Liu, X., X. Jian, E. Boerwinkle. dbNSFP: A lightweight database of human nonsynonymous SNPs and their functional predictions. Human Mutation 32, 894-899 (2011).

11. Chimpanzee Sequencing Analysis Consortium. Initial sequence of the chimpanzee genome and comparison with the human genome. Nature 437, 69-87, doi:10.1038/nature04072 (2005).

12. Takahata, N. Allelic genealogy and human evolution. Mol Biol Evol 10, 2-22 (1993).

13. Asthana, S., Schmidt, S. & Sunyaev, S. A limited role for balancing selection. Trends Genet 21, 30-32, doi:10.1016/j.tig.2004.11.001 (2005).

14. Leffler, E. M., Z. Gao, S. Pfeifer, L. Ségurel, A. Auton, O. Venn, R. Bowden, R. Bontrop, J.D. Wall, G. Sella, P. Donnelly. Multiple instances of ancient balancing selection shared between humans and chimpanzees. Science 339, 1578-1582 (2013).

15. Samocha, K. E. et al. A framework for the interpretation of de novo mutation in human disease. Nat Genet 46, 944-950, doi:10.1038/ng.3050 (2014).

16. Ohta, T. Slightly deleterious mutant substitutions in evolution. Nature 246, 96-98 (1973).

17. Reich, D. E. & Lander, E. S. On the allelic spectrum of human disease. Trends Genet 17, 502-510 (2001).

18. Whiffin, N., E. Minikel, R. Walsh, A. H. O'Donnell-Luria, K. Karczewski, A. Y. Ing, P. J. Barton, B. Funke, S. A. Cook, D. MacArthur, J. S. Ware. Using high-resolution variant frequencies to empower clinical genome interpretation. Genetics in Medicine 19, 1151-1158 (2017).

19. Prado-Martinez, J. et al. Great ape genome diversity and population history. Nature 499, 471-475 (2013).

20. Klein, J., Satta, Y., O'HUigin, C. & Takahata, N. The molecular descent of the major histocompatibility complex. Annu Rev Immunol 11, 269-295, doi:10.1146/annurev.iy.11.040193.001413 (1993).

21. Kimura, M. The neutral theory of molecular evolution. (Cambridge University Press, 1983).

22. de Manuel, M. et al. Chimpanzee genomic diversity reveals ancient admixture with bonobos. Science 354, 477-481, doi:10.1126/science.aag2602 (2016).

23. Locke, D. P. et al. Comparative and demographic analysis of orang-utan genomes. Nature 469, 529-533 (2011).

24. Rhesus Macaque Genome Sequencing Analysis Consortium et al. Evolutionary and biomedical insights from the rhesus macaque genome. Science 316, 222-234, doi:10.1126/science.1139247 (2007).

25. Worley, K. C., W. C. Warren, J. Rogers, D. Locke, D. M. Muzny, E. R. Mardis, G. M. Weinstock, S. D. Tardif, K. M. Aagaard, N. Archidiacono, N. A. Rayan. The common marmoset genome provides insight into primate biology and evolution. Nature Genetics 46, 850-857 (2014).

26. Sherry, S. T. et al. dbSNP: the NCBI database of genetic variation. Nucleic Acids Res 29, 308-311 (2001).

27. Schrago, C. G. & Russo, C. A. Timing the origin of New World monkeys. Mol Biol Evol 20, 1620-1625, doi:10.1093/molbev/msg172 (2003).

28. Landrum, M. J. et al. ClinVar: public archive of interpretations of clinically relevant variants. Nucleic Acids Res 44, D862-868, doi:10.1093/nar/gkv1222 (2016).

29. Brandon, E. P., Idzerda, R. L. & McKnight, G. S. Targeting the mouse genome: a compendium of knockouts (Part II). Curr Biol 5, 758-765 (1995).

30. Lieschke, J. G., P. D. Currie. Animal models of human disease: zebrafish swim into view. Nature Reviews Genetics 8, 353-367 (2007).

31. Sittig, L. J., P. Carbonetto, K. A. Engel, K. S. Krauss, C. M. Barrios-Camacho, A. A. Palmer. Genetic background limits generalizability of genotype-phenotype relationships. Neuron 91, 1253-1259 (2016).

32. Bazykin, G. A. et al. Extensive parallelism in protein evolution. Biol Direct 2, 20, doi:10.1186/1745-6150-2-20 (2007).

33. Ng, P. C. & Henikoff, S. Predicting deleterious amino acid substitutions. Genome Res 11, 863-874, doi:10.1101/gr.176601 (2001).

34. Adzhubei, I. A. et al. A method and server for predicting damaging missense mutations. Nat Methods 7, 248-249, doi:10.1038/nmeth0410-248 (2010).

35. Chun, S., J. C. Fay. Identification of deleterious mutations within three human genomes. Genome research 19, 1553-1561 (2009).

36. Schwarz, J. M., C. Rödelsperger, M. Schuelke, D. Seelow. MutationTaster evaluates disease-causing potential of sequence alterations. Nat. Methods 7, 575-576 (2010).

37. Reva, B., Antipin, Y. & Sander, C. Predicting the functional impact of protein mutations: application to cancer genomics. Nucleic Acids Res 39, e118, doi:10.1093/nar/gkr407 (2011).

38. Dong, C. et al. Comparison and integration of deleteriousness prediction methods for nonsynonymous SNVs in whole exome sequencing studies. Hum Mol Genet 24, 2125-2137, doi:10.1093/hmg/ddu733 (2015).

39. Carter, H., Douville, C., Stenson, P. D., Cooper, D. N. & Karchin, R. Identifying Mendelian disease genes with the variant effect scoring tool. BMC Genomics 14 Suppl 3, S3, doi:10.1186/1471-2164-14-S3-S3 (2013).

40. Choi, Y., Sims, G. E., Murphy, S., Miller, J. R. & Chan, A. P. Predicting the functional effect of amino acid substitutions and indels. PLoS One 7, e46688, doi:10.1371/journal.pone.0046688 (2012).

41. Gulko, B., Hubisz, M. J., Gronau, I. & Siepel, A. A method for calculating probabilities of fitness consequences for point mutations across the human genome. Nat Genet 47, 276-283, doi:10.1038/ng.3196 (2015).

42. Shihab, H. A. et al. An integrative approach to predicting the functional effects of non-coding and coding sequence variation. Bioinformatics 31, 1536-1543, doi:10.1093/bioinformatics/btv009 (2015).

43. Quang, D., Chen, Y. & Xie, X. DANN: a deep learning approach for annotating the pathogenicity of genetic variants. Bioinformatics 31, 761-763, doi:10.1093/bioinformatics/btu703 (2015).

44. Bell, C. J., D. L. Dinwiddie, N. A. Miller, S. L. Hateley, E. E. Ganusova, J. Midge, R. J. Langley, L. Zhang, C. L. Lee, R. D. Schilkey, J. E. Woodward, H. E. Peckham, G. P. Schroth, R. W. Kim, S. F. Kingsmore. Comprehensive carrier testing for severe childhood recessive diseases by next generation sequencing. Sci. Transl. Med. 3, 65ra64 (2011).

45. Kircher, M., D. M. Witten, P. Jain, B. J. O'Roak, G. M. Cooper, J. Shendure. A general framework for estimating the relative pathogenicity of human genetic variants. Nat. Genet. 46, 310-315 (2014).

46. Smedley, D. et al. A Whole-Genome Analysis Framework for Effective Identification of Pathogenic Regulatory Variants in Mendelian Disease. Am J Hum Genet 99, 595-606, doi:10.1016/j.ajhg.2016.07.005 (2016).

47. Ioannidis, N. M. et al REVEL: an ensemble method for predicting the pathogenicity of rare missense variants. Am J Hum Genet 99, 877-885, doi:10.1016/j.ajhg.2016.08.016 (2016).

48. Jagadeesh, K. A., A. M. Wenger, M. J. Berger, H. Guturu, P. D. Stenson, D. N. Cooper, J. A. Bernstein, G. Bejerano. M-CAP eliminates a majority of variants of uncertain significance in clinical exomes at high sensitivity. Nature genetics 48, 1581-1586 (2016).

49. Grimm, D. G. The evaluation of tools used to predict the impact of missense variants is hindered by two types of circularity. Human mutation 36, 513-523 (2015).

50. He, K., X. Zhang, S. Ren, J. Sun. in Proceedings of the IEEE conference on computer vision and pattern recognition. 770-778.

51. Heffernan, R. et al. Improving prediction of secondary structure, local backbone angles, and solvent accessible surface area of proteins by iterative deep learning. Sci Rep 5, 11476, doi:10.1038/srep11476 (2015).

52. Wang, S., J. Peng, J. Ma, J. Xu. Protein secondary structure prediction using deep convolutional neural fields. Scientific reports 6, 18962-18962 (2016).

53. Harpak, A., A. Bhaskar, J. K. Pritchard. Mutation Rate Variation is a Primary Determinant of the Distribution of Allele Frequencies in Humans. PLoS Genetics 12 (2016).

54. Payandeh, J., Scheuer, T., Zheng, N. & Catterall, W. A. The crystal structure of a voltage-gated sodium channel. Nature 475, 353-358 (2011).

55. Shen, H. et al. Structure of a eukaryotic voltage-gated sodium channel at near-atomic resolution. Science 355, eaa14326, doi:10.1126/science.aa14326 (2017).

56. Nakamura, K. et al. Clinical spectrum of SCN2A mutations expanding to Ohtabara syndrome. Neurology 81, 992-998, doi:10.1212/WNL.0b013e3182a43e57 (2013).

57. Henikoff, S. & Henikoff, J. G. Amino acid substitution matrices from protein blocks. Proc Natl Acad Sci U SA 89, 10915-10919 (1992).

58. Li, W. H., C. I. Wu, C. C. Luo. Nonrandomness of point mutation as reflected in nucleotide substitutions in pseudogenes and its evolutionary implications. Journal of Molecular Evolution 21, 58-71 (1984).

59. Grantham, R. Amino acid difference formula to help explain protein evolution. Science 185, 862-864 (1974).

60. LeCun, Y., L. Bottou, Y. Bengio, P. Haffner. in Proceedings of the IEEE 2278-2324.

61. Vissers, L. E., Gilissen, C. & Veltman, J. A. Genetic studies in intellectual disability and related disorders. Nat Rev Genet 17, 9-18, doi:10.1038/nrg3999 (2016).

62. Neale, B. M. et al Patterns and rates of exonic de novo mutations in autism spectrum disorders. Nature 485, 242-245, doi:10.1038/nature11011 (2012).

63. Sanders, S. J. et al. De novo mutations revealed by whole-exome sequencing are strongly associated with autism. Nature 485, 237-241, doi:10.1038/nature10945 (2012).

64. De Rubeis, S. et al. Synaptic, transcriptional and chromatin genes disrupted in autism. Nature 515, 209-215, doi:10.1038/nature13772 (2014).

65. Deciphering Developmental Disorders Study. Large-scale discovery of novel genetic causes of developmental disorders. Nature 519, 223-228, doi:10.1038/nature14135 (2015).

66. Deciphering Developmental Disorders Study. Prevalence and architecture of de novo mutations in developmental disorders. Nature 542, 433-438, doi:10.1038/nature21062 (2017).

67. Iossifov, I. et al. The contribution of de novo coding mutations to autism spectrum disorder. Nature 515, 216-221, doi:10.1038/nature13908 (2014).

68. Zhu, X., Need, A. C., Petrovski, S. & Goldstein, D. B. One gene, many neuropsychiatric disorders: lessons from Mendelian diseases. Nat Neurosci 17, 773-781, doi:10.1038/nn.3713 (2014).

69. Leffler, E. M., K. Bullaughey, D. R. Matute, W. K. Meyer, L. Ségurel, A. Venkat, P. Andolfatto, M. Przeworski. Revisiting an old riddle: what determines genetic diversity levels within species? PLoS biology 10, e1001388 (2012).

70. Estrada, A. et al. Impending extinction crisis of the world's primates: Why primates matter. Science advances 3, e1600946 (2017).

71. Kent, W. J., C. W. Sugnet, T. S. Furey, K. M. Roskin, T. H. Pringle, A.M. Zahler, D. Haussler. The human genome browser at UCSC. Genome Res. 12, 996-1006 (2002).

72. Tyner, C. et al. The UCSC Genome Browser database: 2017 update. Nucleic Acids Res 45, D626-D634, doi:10.1093/nar/gkw1134 (2017).

73. Kabsch, W. & Sander, C. Dictionary of protein secondary structure: pattern recognition of hydrogen-bonded and geometrical features. Biopolymers 22, 2577-2637, doi:10.1002/bip.360221211 (1983).

74. Joosten, R. P. et al. A series of PDB related databases for everyday needs. Nucleic Acids Res 39, D411-419, doi:10.1093/nar/gkq1105 (2011).

75. He, K., Zhang, X., Ren, S. & Sun, J. in European Conference on Computer Vision. 630-645 (Springer).

76. Ionita-Laza, I., McCallum, K., Xu, B. & Buxbaum, J. D. A spectral approach integrating functional genomic annotations for coding and noncoding variants. Nat Genet 48, 214-220, doi:10.1038/ng.3477 (2016).

77. Li, B. et aL Automated inference of molecular mechanisms of disease from amino acid substitutions. Bioinformatics 25, 2744-2750, doi:10.1093/bioinformatics/btp528 (2009).

78. Lu, Q. et al. A statistical framework to predict functional non-coding regions in the human genome through integrated analysis of annotation data. Sci Rep 5, 10576, doi:10.1038/srep10576 (2015).

79. Shihab, H. A. et al. Predicting the functional, molecular, and phenotypic consequences of amino acid substitutions using hidden Markov models. Hum Mutat 34, 57-65, doi:10.1002/humu.22225 (2013).

80. Davydov, E. V. et al. Identifying a high fraction of the human genome to be under selective constraint using GERP++. PLoS Comput Biol 6, e1001025, doi:10.1371/journal.pcbi.1001025 (2010).

81. Liu, X., Wu, C., Li, C. & Boerwinkle, E. dbNSFP v3.0: A One-Stop Database of Functional Predictions and Annotations for Human Nonsynonymous and Splice-Site SNVs. Hum Mutat 37, 235-241, doi:10.1002/humu.22932 (2016).

82. Jain, S., White, M. & Radivojac, P. in Proceedings of the Thirty-First AAAI Conference on Artificial Intelligence. 2066-2072.

83. de Ligt, J. et al. Diagnostic exome sequencing in persons with severe intellectual disability. N Engl J Med 367, 1921-1929, doi:10.1056/NEJMoa1206524 (2012).

84. Iossifov, I. et al. De novo gene disruptions in children on the autistic spectrum. Neuron 74, 285-299, doi:10.1016/j.neuron.2012.04.009 (2012).

85. O'Roak, B. J. et al. Sporadic autism exomes reveal a highly interconnected protein network of de novo mutations. Nature 485, 246-250, doi:10.1038/nature10989 (2012).

86. Rauch, A. et al. Range of genetic mutations associated with severe non-syndromic sporadic intellectual disability: an exome sequencing study. Lancet 380, 1674-1682, doi:10.1016/S0140-6736(12)61480-9 (2012).

87. Epi, K. C. et al. De novo mutations in epileptic encephalopathies. Nature 501, 217-221, doi:10.1038/nature12439 (2013).

88. Euro, E.-R. E. S. C., Epilepsy Phenome/Genome, P. & Epi, K. C. De novo mutations in synaptic transmission genes including DNM1 cause epileptic encephalopathies. Am J Hum Genet 95, 360-370, doi:10.1016/j.ajhg.2014.08.013 (2014).

89. Gilissen, C. et al. Genome sequencing identifies major causes of severe intellectual disability. Nature 511, 344-347, doi:10.1038/nature13394 (2014).

90. Lelieveld, S. H. et al. Meta-analysis of 2,104 trios provides support for 10 new genes for intellectual disability. Nat Neurosci 19, 1194-1196, doi:10.1038/nn.4352 (2016).

91. Famiglietti, M. L. et al. Genetic variations and diseases in UniProtKB/Swiss-Prot: the ins and outs of expert manual curation. Hum Mutat 35, 927-935, doi:10.1002/humu.22594 (2014).

92. Horaitis, O., Talbot, C. C., Jr., Phommarinh, M., Phillips, K. M. & Cotton, R. G. A database of locus-specific databases. Nat Genet 39, 425, doi:10.1038/ng0407-425 (2007).

93. Stenson, P. D. et al. The Human Gene Mutation Database: building a comprehensive mutation repository for clinical and molecular genetics, diagnostic testing and personalized genomic medicine. Hum Genet 133, 1-9, doi:10.1007/s00439-013-1358-4 (2014).

**Claims**

1. A computer-implemented method comprising:

   inputting, into a neural network-based variant pathogenicity classifier trained to classify a variant amino acid sequence as benign or pathogenic:

      a reference amino acid sequence for a protein;
      a variant amino acid sequence for the protein comprising a variant amino acid; and
      a conservation profile representing conservation of amino acids in a reference protein sequence across aligned protein sequences of other species; and

   generating, as an output of the neural network-based variant pathogenicity classifier, a pathogenicity score indicating a likelihood that the variant amino acid is benign or pathogenic.

2. The computer-implemented method according to claim 1:

   (a) wherein the conservation profile is determined, on a location-by-location basis, by the frequency of occurrence of an amino acid in the reference protein sequence across the aligned protein sequences of other species; and/or
   (b) wherein the step of inputting into the neural network-based variant pathogenicity classifier includes inputting reference and variant state classifications.

3. The computer-implemented method of claim 1 or claim 2, wherein the conservation profile comprises a position-specific frequency matrix generated from a multiple sequence alignment of the other species.

4. The computer-implemented method of claim 3, wherein the position-specific frequency matrix is a position-specific score matrix.

5. The computer-implemented method of any one of claims 1-4, wherein the conservation profile comprises one or more of:

      a primate conservation profile generated from amino acids of primate species aligned with the reference amino acid sequence;
      a mammal conservation profile generated from amino acids from mammal species aligned with the reference amino acid sequence; or
      a vertebrate conservation profile generated from amino acids from vertebrate species aligned with the reference amino acid sequence.

6. The computer-implemented method of any one of claims 1-5, further comprising inputting, into the neural network-based variant pathogenicity classifier, a three-state secondary structure prediction for each amino acid in the reference amino acid sequence.

7. The computer-implemented method of claim 6, further comprising generating, utilizing a secondary structure neural network, the three-state secondary structure prediction.

8. The computer-implemented method of any one of claims 1-7, further comprising inputting, into the neural network-based variant pathogenicity classifier, a three-state solvent accessibility prediction for each amino acid in the reference amino acid sequence.

9. The computer-implemented method of claim 8, further comprising generating, utilizing a solvent accessibility neural network, the three-state solvent accessibility prediction.

10. The computer-implemented method of any one of claims 1-9, wherein the variant amino acid of the variant amino acid sequence is caused by a nucleotide substitution of a nucleotide within the reference amino acid sequence.

11. The computer-implemented method of any one of claims 1-10, wherein the neural network-based variant pathogenicity classifier is trained utilizing benign training data comprising variants from primates matched to common human variants according to a trinucleotide context, optionally wherein the benign training data comprises primate variants that are identical-by-state (IBS) with human missense variants or human synonymous variants.

12. A non-transitory computer readable storage medium impressed with computer program instructions that, when executed on a processor, cause a system to perform a computer-implemented method according to any one of claims 1 to 11.

13. A system including one or more processors coupled to memory, the memory loaded with computer instructions that, when executed on the one or more processors, implement actions comprising a computer-implemented method according to any one of claims 1 to 11.

14. A computer-implemented method of training a neural network-based variant pathogenicity classifier to classify a variant amino acid sequence as benign or pathogenic, the method comprising:

training the neural network-based variant pathogenicity classifier, which runs on numerous processors coupled to memory, using as input benign training examples of protein sequence pairs and pathogenic training examples of protein sequence pairs, wherein the benign training examples and the pathogenic training examples, respectively, include benign variants and pathogenic variants, paired with reference sequences,

wherein the benign training examples include a first set of benign variants from humans paired with human reference sequences and a second set of benign variants from non-human primates paired with non-human primate reference sequences,

wherein the benign variants from the humans include common human missense variants, and

wherein the benign variants from the non-human primates include non-human primate missense variants occurring on alternative non-human primate codon sequences that match non-human primate reference codon sequences with human reference codon sequences; and

whereby the neural network-based variant pathogenicity classifier after training, is configured to receive a reference amino acid sequence for a protein and a variant amino acid sequence for the protein comprising a variant amino acid, and to generate a classification score output that indicates whether the variant is benign or pathogenic.

15. The computer-implemented method according to claim 14, wherein the neural network-based variant pathogenicity classifier after training, is configured to further receive a conservation profile representing conservation of amino acids in a reference protein sequence across aligned protein sequences of related other species.

# Feed-Forward Neural Network

**FIG. 1A**

EP 4 597 451 A2

## Convolutional Neural Network

**FIG. 1B**

# Training Convolutional Neural Network

**FIG. 1C**

EP 4 597 451 A2

# Sub-Sampling Layers of Convolutional Neural Networks

| 44 | 51 | 66 | 61 |
|----|----|----|----|
| 34 | 10 | 21 | 50 |
| 21 | 9  | 4  | 35 |
| 75 | 44 | 1  | 3  |

Feature Map

| 35 | 50 |
|----|----|
| 38 | 11 |

Average Pooling

| 51 | 66 |
|----|----|
| 75 | 35 |

Max Pooling

**FIG. 1D**

EP 4 597 451 A2

# Non-Linear Layers of Convolutional Neural Networks

| 100 | -43 | 76 | -21 |
|-----|-----|-----|-----|
| -90 | -10 | 40 | 21 |
| -19 | 44 | 1 | -35 |
| 221 | 64 | 41 | 23 |

| 100 | 0 | 76 | 0 |
|-----|-----|-----|-----|
| 0 | 0 | 40 | 21 |
| 0 | 44 | 1 | 0 |
| 221 | 64 | 41 | 23 |

0,0

ReLU Activation Function

**FIG. 1E**

EP 4 597 451 A2

# Convolution Layers of Convolutional Neural Networks

Input

2048 Dimensions

| Convolution 1 16 Kernels of size 3 × 3 | | Convolution 1 16 Kernels of size 3 × 3 |

16 Feature maps

Convolution 1

ReLU 1

Pool 1 (AVG Pooling)
16 Kernels of size 3 × 3

Pool 1

Convolution 2
16 Kernels of size 3 × 3

ReLU 2

Pool 2 (AVG Pooling)
32 Kernels of size 2 × 2

Feature Vector
512 Dimensions

## FIG. 1F

# Residual Connections used in Convolutional Neural Networks

**FIG. 1G**

Residual Block and Skip-Connections used in
Convolutional Neural Networks

FIG. 1H

EP 4 597 451 A2

# Batch Normalization Forward Pass with Convolutional Neural Networks

$$\mu_{\mathcal{B}} = \frac{1}{n} \sum_{i=1}^{n} x_i^{(\ell-1)}$$

$$\sigma_{\mathcal{B}}^2 = \frac{1}{n} \sum_{i=1}^{n} \left( x_i^{(\ell-1)} - \mu_{\mathcal{B}} \right)^2$$

$$\hat{x}^{(\ell-1)} = \frac{x^{(\ell-1)} - \mu_{\mathcal{B}}}{\sqrt{\sigma_{\mathcal{B}}^2 + \epsilon}}$$

$$x^{(\ell)} = \gamma^{(\ell)} \hat{x}^{(\ell-1)} + \beta^{(\ell)}$$

**FIG. 1I**

EP 4 597 451 A2

# Batch Normalization – Inference with Convolutional Neural Networks

$$\hat{x}^{(\ell-1)} = \frac{x^{(\ell-1)} - \mu_{\mathcal{D}}}{\sqrt{\sigma_{\mathcal{D}}^2 + \epsilon}}$$

$$x_i^{(\ell)} = \gamma^{(\ell)} \hat{x}_i^{(\ell-1)} + \beta^{(\ell)}$$

**FIG. 1J**

# Batch Normalization Backward Pass with Convolutional Neural Networks

$$\nabla_{\gamma^{(\ell)}} \mathcal{L} = \sum_{i=1}^{n} \left( \nabla_{x^{(\ell+1)}} \mathcal{L} \right)_i \cdot \hat{x}_i^{(\ell)}$$

$$\nabla_{\beta^{(\ell)}} \mathcal{L} = \sum_{i=1}^{n} \left( \nabla_{x^{(\ell+1)}} \mathcal{L} \right)_i$$

**FIG. 1K**

# Batch Normalization in Convolution Layers

```
conv_model.add(layers.Conv2D(32, 3, activation='relu'))  <———  After a Conv layer
conv_model.add(layers.BatchNormalization())

dense_model.add(layers.Dense(32, activation='relu'))  <———  After a Dense layer
dense_model.add(layers.BatchNormalization())
```

**FIG. 1L**

EP 4 597 451 A2

# 1D Convolution used in Convolutional Neural Networks

**FIG. 1M**

# Global Average Pooling (GAP) in Convolutional Neural Networks

**FIG. 1N**

# Dilated Convolutions

input $x_{t-3}$ $x_{t-2}$ $x_{t-1}$ $x_t$

layer 1

output $x_{t+1}$

FIG. 1O

EP 4 597 451 A2

# Stacked Dilated Convolutions

**FIG. 1P**

Training Servers
(Scale with Data)

Large-Scale
Training
Dataset

Train Models

Benign Dataset Generator

Variant Pathogenicity Classifier

Secondary Structure Classifier

Solvent Accessibility Classifier

Semi-Supervised Learner

Administrative
Interface

Production Servers
(Scale with Users)

Trained Models Deployed on Production Servers

Benign Dataset Generator

Variant Pathogenicity Classifier

Secondary Structure Classifier

Solvent Accessibility Classifier

Semi-Supervised Learner

Test Data

Production
Data

Inferred Data
(real-time)

Client Interface
(APIs, Browser)

**FIG. 1Q**

EP 4 597 451 A2

FIG. 2

PAH – phenylalanine hydroxylase
NH₂ – amino-terminus
COOH – carboxyl-terminus

EP 4 597 451 A2

105

**FIG. 3**

| Layer | Input to the Layer | Type | Number of Kernel & Window size | Shape | Activation |
|---|---|---|---|---|---|
| Layer 1a | Reference Sequence | Convolution 1D | 40,1 | (51,40) | Linear |
| Layer 1b | Alternative Sequence | Convolution 1D | 40,1 | (51,40) | Linear |
| Layer 1c | Primate Conservation | Convolution 1D | 40,1 | (51,40) | Linear |
| Layer 1d | Mammal Conservation | Convolution 1D | 40,1 | (51,40) | Linear |
| Layer 1e | Vertebrate Conservation | Convolution 1D | 40,1 | (51,40) | Linear |
| Pre-trained Secondary Structure Prediction Model (Trainable Network) | Primate + Mammal + Vertebrate Conservation | - | - | (51,40) | - |
| Pre-trained Solvent Accessibility Model (Trainable Network) | Primate + Mammal + Vertebrate Conservation | - | - | (51,40) | - |
| Layer 1f | Pre-trained Structure Prediction Model output | Convolution 1D | 40,1 | (51,40) | Linear |
| Layer 1g | Pre-trained Solvent Accessibility Model output | Convolution 1D | 40,1 | (51,40) | Linear |
| Merge 1a | Layer 1a, Layer 1c, Layer 1d, Layer 1e, Layer 1f, Layer 1g | Add | - | (51,40) | - |
| Merge 1b | Layer 1b, Layer 1c, Layer 1d, Layer 1e, Layer 1f, Layer 1g | Add | - | (51,40) | - |
| Layer 2a | Merge 1a | Batch Normalization | - | (51,40) | - |
| Layer 3a | Layer 2a | Activation | - | (51,40) | Relu |
| Layer 4a | Layer 3a | Convolution 1D | 40,5 | (51,40) | Linear |
| Layer 5a | Layer 4a | Batch Normalization | - | (51,40) | - |
| Layer 6a | Layer 5a | Activation | - | (51,40) | Relu |
| Layer 7a | Layer 6a | Convolution 1D | 40,5 | (51,40) | Linear |
| Layer 2b | Merge 1b | Batch Normalization | - | (51,40) | - |
| Layer 3b | Layer 2b | Activation | - | (51,40) | Relu |
| Layer 4b | Layer 3b | Convolution 1D | 40,5 | (51,40) | Linear |

# FIG. 4A

| Layer 5b | Layer 4b | Batch Normalization | | (51,40) | |
|---|---|---|---|---|---|
| Layer 6b | Layer 5b | Activation | - | (51,40) | Relu |
| Layer 7b | Layer 6b | Convolution 1D | 40,5 | (51,40) | Linear |
| Merge 2a | Layer 7a, Layer 7b | Concatenate | - | (51,80) | - |
| Merge 2b | Layer 7a, Layer 7b | Concatenate | | (51,80) | |
| Layer 8a | Merge 2a | Convolution 1D | 40,5 | (51,40) | Linear |
| Layer 8b | Merge 2b | Convolution 1D | 40,5 | (51,40) | Linear |
| Layer 9 | Layer 8a | Batch Normalization | - | (51,40) | - |
| Layer 10 | Layer 9 | Activation | - | (51,40) | Relu |
| Layer 11 | Layer 10 | Convolution 1D | 40,5 | (51,40) | Linear |
| Layer 12 | Layer 11 | Batch Normalization | | (51,40) | |
| Layer 13 | Layer 12 | Activation | - | (51,40) | Relu |
| Layer 14 | Layer 13 | Convolution 1D | 40,5 | (51,40) | Linear |
| Merge3 | Layer 8a, Layer 14 | Add | - | (51,40) | - |
| Layer 15 | Merge3 | Batch Normalization | | (51,40) | |
| Layer 16 | Layer 15 | Activation | - | (51,40) | Relu |
| Layer 17 | Layer 16 | Convolution 1D | 40,5 | (51,40) | Linear |
| Layer 18 | Layer 17 | Batch Normalization | - | (51,40) | - |
| Layer 19 | Layer 18 | Activation | - | (51,40) | Relu |
| Layer 20 | Layer 19 | Convolution 1D | 40,5 | (51,40) | Linear |
| Merge4 | Merge3, Layer 20 | Add | | (51,40) | |
| Layer 21 | Merge4 | Convolution 1D | 40,1 | (51,40) | Linear |
| Layer 22 | Merge4 | Batch Normalization | | (51,40) | |
| Layer 23 | Layer 22 | Activation | - | (51,40) | Relu |
| Layer 24 | Layer 23 | Convolution 1D | 40,5 | (51,40) | Linear |
| Layer 25 | Layer 24 | Batch Normalization | - | (51,40) | - |
| Layer 26 | Layer 25 | Activation | - | (51,40) | Relu |
| Layer 27 | Layer 26 | Convolution 1D | 40,5 | (51,40) | Linear |
| Merge5 | Merge4, Layer 27 | Add | | (51,40) | |
| Layer 28 | Merge 5 | Batch Normalization | - | (51,40) | - |
| Layer 29 | Layer 28 | Activation | - | (51,40) | Relu |
| Layer 30 | Layer 29 | Convolution 1D | 40,5 | (51,40) | Linear |
| Layer 31 | Layer 30 | Batch Normalization | - | (51,40) | - |
| Layer 32 | Layer 31 | Activation | - | (51,40) | Relu |

**FIG. 4B**

| | | | | | |
|---|---|---|---|---|---|
| Layer 33 | Layer 32 | Convolution 1D | 40,5 | (51,40) | Linear |
| Merge 6 | Merge 5, Layer 33 | Add | | (51,40) | |
| Layer 34 | Merge6 | Convolution 1D | 40,1 | (51,40) | Linear |
| Layer 35 | Merge6 | Batch Normalization | | (51,40) | |
| Layer 36 | Layer 35 | Activation | - | (51,40) | Relu |
| Layer 37 | Layer 36 | Convolution 1D | 40,5 | (51,40) | Linear |
| Layer 38 | Layer 37 | Batch Normalization | - | (51,40) | - |
| Layer 39 | Layer 38 | Activation | | (51,40) | Relu |
| Layer 40 | Layer 39 | Convolution 1D | 40,5 | (51,40) | Linear |
| Merge 7 | Merge6, Layer 40 | Add | | (51,40) | |
| Layer 41 | Merge7 | Batch Normalization | - | (51,40) | - |
| Layer 42 | Layer 41 | Activation | - | (51,40) | Relu |
| Layer 43 | Layer 42 | Convolution 1D | 40,5 | (51,40) | Linear |
| Layer 44 | Layer 43 | Batch Normalization | | (51,40) | |
| Layer 45 | Layer 44 | Activation | ' | (51,40) | Relu |
| Layer 46 | Layer 45 | Convolution 1D | 40,5 | (51,40) | Linear |
| Merge8 | Merge 7, Layer 46 | Add | - | (51,40) | - |
| Layer 47 | Merge 8 | Convolution 1D | 40,1 | (51,40) | Linear |
| Merge9 | Layer 8b, Layer 21, Layer 34, Layer 47 | Add | - | (51,40) | - |
| Layer 48 | Merge 10 | Batch Normalization | | (51,40) | |
| Layer 49 | Layer 48 | Activation | - | (51,40) | Relu |
| Layer 50 | Layer 49 | Convolution 1D | 40,5 | (51,40) | Linear |
| Layer 51 | Layer 50 | Batch Normalization | - | (51,40) | - |
| Layer 52 | Layer 51 | Activation | | (51,40) | Relu |
| Layer 53 | Layer 52 | Convolution 1D | 40,1 | (51,40) | Linear |
| Merge 10 | Merge 9, Layer 53 | Add | | (51,40) | |
| Layer 54 | Merge 10 | Convolution 1D | 1,1 | (51,1) | Sigmoid |
| Output Layer | Layer 54 | GlobalMaxPooling1D | | | 1 |

**FIG. 4C**

FIG. 5

FIG. 6

| Layer | Input to the Layer | Type | Number of Kernel & Kernel Window size | Shape | Activation |
|---|---|---|---|---|---|
| Layer 1a | Input PSTXT | Convolution 1D | 40,1 | (51,40) | Linear |
| Layer 2a | Layer 1a | Convolution 1D | 40,1 | (51,40) | Linear |
| Layer 1b | Input PSTXT | Convolution 1D | 40,1 | (51,40) | Linear |
| Layer 2b | Layer 1b | Convolution 1D | 40,1 | (51,40) | Linear |
| Layer 3 | Layer 2a | Batch Normalization | - | (51,40) | - |
| Layer 4 | Layer 3 | Activation | - | (51,40) | Relu |
| Layer 5 | Layer 4 | Convolution 1D | 40,5 | (51,40) | Linear |
| Layer 6 | Layer 5 | Batch Normalization | - | (51,40) | - |
| Layer 7 | Layer 6 | Activation | - | (51,40) | Relu |
| Layer 9 | Layer 8 | Convolution 1D | 40,5 | (51,40) | Linear |
| Merge1 | Layer 2a, Layer 9 | Add | - | (51,40) | - |
| Layer 10 | Merge1 | Batch Normalization | - | (51,40) | - |
| Layer 11 | Layer 10 | Activation | - | (51,40) | Relu |
| Layer 12 | Layer 11 | Convolution 1D | 40,5 | (51,40) | Linear |
| Layer 13 | Layer 12 | Batch Normalization | - | (51,40) | - |
| Layer 14 | Layer 13 | Activation | - | (51,40) | Relu |
| Layer 15 | Layer 14 | Convolution 1D | 40,5 | (51,40) | Linear |
| Merge2 | Merge1, Layer15 | Add | - | (51,40) | - |
| Layer 16 | Merge2 | Convolution 1D | 40,1 | (51,40) | Linear |
| Layer 17 | Merge2 | Batch Normalization | - | (51,40) | - |
| Layer 18 | Layer 17 | Activation | - | (51,40) | Relu |
| Layer 19 | Layer 18 | Convolution 1D | 40,5 | (51,40) | Linear |
| Layer 20 | Layer 19 | Batch Normalization | - | (51,40) | - |
| Layer 21 | Layer 20 | Activation | - | (51,40) | Relu |
| Layer 22 | Layer 21 | Convolution 1D | 40,5 | (51,40) | Linear |
| Merge3 | Merge2, Layer 22 | Add | - | (51,40) | - |

**FIG. 7A**

| | | | | | |
|---|---|---|---|---|---|
| Layer 23 | Merge 3 | Batch Normalization | - | (51,40) | - |
| Layer 24 | Layer 23 | Activation | - | (51,40) | Relu |
| Layer 25 | Layer 24 | Convolution 1D | 40,3 | (51,40) | Linear |
| Layer 26 | Layer 25 | Batch Normalization | - | (51,40) | - |
| Layer 27 | Layer 26 | Activation | - | (51,40) | Relu |
| Layer 28 | Layer 27 | Convolution 1D | 40,3 | (51,40) | Linear |
| Merge4 | Merge3, Layer 28 | Add | - | (51,40) | - |
| Layer 29 | Merge4 | Convolution 1D | 40,1 | (51,40) | Linear |
| Layer 30 | Merge4 | Batch Normalization | - | (51,40) | - |
| Layer 31 | Layer 30 | Activation | - | (51,40) | Relu |
| Layer 32 | Layer 31 | Convolution 1D | 40,5 | (51,40) | Linear |
| Layer 33 | Layer 32 | Batch Normalization | - | (51,40) | - |
| Layer 34 | Layer 33 | Activation | - | (51,40) | Relu |
| Layer 35 | Layer 34 | Convolution 1D | 40,3 | (51,40) | Linear |
| Merge5 | Merge4, Layer 35 | Add | - | (51,40) | - |
| Layer 36 | Merge5 | Batch Normalization | - | (51,40) | - |
| Layer 37 | Layer 36 | Activation | - | (51,40) | Relu |
| Layer 38 | Layer 37 | Convolution 1D | 40,3 | (51,40) | Linear |
| Layer 39 | Layer 38 | Batch Normalization | - | (51,40) | - |
| Layer 40 | Layer 39 | Activation | - | (51,40) | Relu |
| Layer 41 | Layer 40 | Convolution 1D | 40,5 | (51,40) | Linear |
| Merge6 | Merge5, Layer 41 | Add | - | (51,40) | - |
| Layer 42 | Merge 6 | Convolution 1D | 40,1 | (51,40) | Linear |
| Merge7 | Layer 29, Layer 10, Layer 38, Layer 42 | Add | - | (51,40) | - |
| Layer 43 | Merge 7 | Batch Normalization | - | (51,40) | - |
| Layer 44 | Layer 43 | Activation | - | (51,40) | Relu |
| Layer 45 | Merge 44 | Convolution 1D | 40,1 | (51,40) | Linear |
| Layer 46 | Layer 45 | Batch Normalization | - | (51,40) | - |
| Layer 47 | Layer 46 | Activation | - | (51,40) | Relu |
| Layer 48 | Layer 45 | Convolution 1D | 40,1 | (51,40) | Linear |
| Merge 8 | Merge 7, Layer 48 | Add | - | (51,40) | - |
| Output layer | Merge 8 | Convolution 1D | 3,1 | (51,3) | Softmax |

**FIG. 7B**

113

| Layer | Input to the Layer | Type | Number of Kernel & Kernel Window size | Shape | Activation |
|---|---|---|---|---|---|
| Layer 1a | Input PSSM | Convolution 1D | 40,1 | (51,40) | Linear |
| Layer 2a | Layer 1a | Convolution 1D | 40,1 | (51,40) | Linear |
| Layer 1b | Input PSSM | Convolution 1D | 40,1 | (51,40) | Linear |
| Layer 2b | Layer 1b | Convolution 1D | 40,1 | (51,40) | Linear |
| Layer 3 | Layer 2a | Batch Normalization | | (51,40) | |
| Layer 4 | Layer 3 | Activation | - | (51,40) | Relu |
| Layer 5 | Layer 4 | Convolution 1D | 40,5 | (51,40) | Linear |
| Layer 6 | Layer 5 | Batch Normalization | - | (51,40) | - |
| Layer 7 | Layer 7 | Activation | | (51,40) | Relu |
| Layer 9 | Layer 8 | Convolution 1D | 40,5 | (51,40) | Linear |
| Merge1 | Layer 2b, Layer 9 | Add | | (51,40) | |
| Layer 10 | Merge1 | Batch Normalization | - | (51,40) | - |
| Layer 11 | Layer 10 | Activation | | (51,40) | Relu |
| Layer 12 | Layer 11 | Convolution 1D | 40,5 | (51,40) | Linear |
| Layer 13 | Layer 12 | Batch Normalization | | (51,40) | |
| Layer 14 | Layer 13 | Activation | - | (51,40) | Relu |
| Layer 15 | Layer 14 | Convolution 1D | 40,5 | (51,40) | Linear |
| Merge2 | Merge1, Layer15 | Add | - | (51,40) | - |
| Layer 16 | Merge2 | Convolution 1D | 40,1 | (51,40) | Linear |
| Layer 17 | Merge2 | Batch Normalization | - | (51,40) | - |
| Layer 18 | Layer 17 | Activation | | (51,40) | Relu |
| Layer 19 | Layer 18 | Convolution 1D | 40,5 | (51,40) | Linear |
| Layer 20 | Layer 19 | Batch Normalization | | (51,40) | |
| Layer 21 | Layer 20 | Activation | - | (51,40) | Relu |
| Layer 22 | Layer 21 | Convolution 1D | 40,5 | (51,40) | Linear |
| Merge3 | Merge2, Layer 22 | Add | | (51,40) | |

## FIG. 8A

| Layer 23 | Merge 3 | Batch Normalization | - | (51,40) | - |
|---|---|---|---|---|---|
| Layer 24 | Layer 23 | Activation | - | (51,40) | Relu |
| Layer 25 | Layer 24 | Convolution 1D | 40,5 | (51,40) | Linear |
| Layer 26 | Layer 25 | Batch Normalization | - | (51,40) | - |
| Layer 27 | Layer 26 | Activation | - | (51,40) | Relu |
| Layer 28 | Layer 27 | Convolution 1D | 40,5 | (51,40) | Linear |
| Merge4 | Merge3, Layer 28 | Add | - | (51,40) | - |
| Layer 29 | Merge4 | Convolution 1D | 40,1 | (51,40) | Linear |
| Layer 30 | Merge4 | Batch Normalization | - | (51,40) | - |
| Layer 31 | Layer 30 | Activation | - | (51,40) | Relu |
| Layer 32 | Layer 31 | Convolution 1D | 40,5 | (51,40) | Linear |
| Layer 33 | Layer 32 | Batch Normalization | - | (51,40) | - |
| Layer 34 | Layer 33 | Activation | - | (51,40) | Relu |
| Layer 35 | Layer 34 | Convolution 1D | 40,5 | (51,40) | Linear |
| Merge5 | Merge4, Layer 35 | Add | - | (51,40) | - |
| Layer 36 | merge5 | Batch Normalization | - | (51,40) | - |
| Layer 37 | Layer 36 | Activation | - | (51,40) | Relu |
| Layer 38 | Layer 37 | Convolution 1D | 40,5 | (51,40) | Linear |
| Layer 39 | Layer 38 | Batch Normalization | - | (51,40) | - |
| Layer 40 | Layer 39 | Activation | - | (51,40) | Relu |
| Layer 41 | Layer 40 | Convolution 1D | 40,5 | (51,40) | Linear |
| Merge6 | Merge5, Layer 41 | Add | - | (51,40) | - |
| Layer 42 | Merge 6 | Convolution 1D | 40,1 | (51,40) | Linear |
| Merge7 | Layer 29, Layer 42, Layer 36, Layer 42 | Add | - | (51,40) | - |
| Layer 43 | Merge 7 | Batch Normalization | - | (51,40) | - |
| Layer 44 | Layer 43 | Activation | - | (51,40) | Relu |
| Layer 45 | Merge 44 | Convolution 1D | 40,1 | (51,40) | Linear |
| Layer 46 | Layer 45 | Batch Normalization | - | (51,40) | - |
| Layer 47 | Layer 46 | Activation | - | (51,40) | Relu |
| Layer 48 | Layer 47 | Convolution 1D | 40,1 | (51,40) | Linear |
| Merge 8 | Merge 7, Layer 48 | Add | - | (51,40) | - |
| Output layer | Merge 8 | Convolution 1D | 3,1 | (51,3) | Softmax |

**FIG. 8B**

FIG. 9

FIG. 10

FIG. 11

FIG. 12

FIG. 13

FIG. 14

**FIG. 15**

**FIG. 16**

Output

Input

**FIG. 17**

FIG. 18

FIG. 19

FIG. 20

**FIGs. 21D, 21E, 21F**

EP 4 597 451 A2

FIGs. 22A, 22B, 22C

FIGs. 22D, 22E

FIGs. 23A, 23B

**FIGs. 23C, 23D**

FIG. 24

COL1A2 gene chr7:94,038,883~94,039,564 (300 ~ 350 a.a.)

FIG. 25A

IDS gene  chrX:148,585,752~148,584,937 (59 ~ 109 a.a.)

**FIG. 25B**

MYC gene chr8:128,752,873-128,753,184 (345 ~ 450 a.a.)

FIG. 25C

FIG. 26

EP 4 597 451 A2

FIG. 27

FIG. 28A

FIG. 28B

FIG. 28C

FIG. 29A

FIG. 29B

FIGs. 30A, 30B

FIG. 30C

FIG. 31A

EP 4 597 451 A2

**FIG. 31B**

|  | Training Accuracy | Validation Accuracy | Testing Accuracy |
|---|---|---|---|
| 3-State Secondary Structure | 80.32 % | 79.17 % | 79.86 % |
| 3-State Solvent Accessibility | 64.83 % | 60.53 % | 60.31 % |

**FIG. 32**

EP 4 597 451 A2

| Models | Replicates | Withheld Primate Variant Accuracy | DDD Case vs Control -log(pvalue) | DDD 605 disease genes -log(pvalue) |
|---|---|---|---|---|
| Use DL predicted secondary structure labels | 1 | 0.914 | 28.60 | 15.61 |
| | 2 | 0.919 | 32.32 | 15.94 |
| | 3 | 0.915 | 29.48 | 16.38 |
| | 4 | 0.917 | 30.35 | 15.62 |
| | 5 | 0.916 | 29.11 | 16.22 |
| | Median | 0.916 | 29.48 | 15.94 |
| Replace DL predicted secondary structure labels with DSSP secondary structure labels of human proteins when available | 1 | 0.916 | 29.48 | 15.94 |
| | 2 | 0.913 | 31.72 | 16.24 |
| | 3 | 0.915 | 31.09 | 15.69 |
| | 4 | 0.915 | 30.49 | 16.09 |
| | 5 | 0.915 | 30.57 | 14.79 |
| | Median | 0.915 | 30.79 | 16.09 |

FIG. 33

EP 4 597 451 A2

|  | Withheld Primate Variant Accuracy | DDD Case vs Control -log(pvalue) |
|---|---|---|
| SIFT | 0.7490 | 14.1233 |
| PolyPhen-2 | 0.7618 | 13.7044 |
| CADD | 0.7939 | 14.8167 |
| M-CAP | 0.7667 | 20.6078 |
| LRT | 0.7598 | 6.2960 |
| MutationTaster | 0.5332 | 2.2811 |
| MutationAssessor | 0.6879 | 11.5202 |
| FATHMM | 0.5981 | 8.1690 |
| PROVEAN | 0.6995 | 14.0747 |
| VEST3 | 0.7978 | 19.7068 |
| MetaSVM | 0.7215 | 15.9829 |
| MetaLR | 0.6991 | 15.5842 |
| REVEL | 0.7686 | 21.5892 |
| MutPred | 0.6240 | 7.3044 |
| DANN | 0.7666 | 9.8976 |
| MKL_coding | 0.7657 | 6.2469 |
| Eigen | 0.8003 | 13.5364 |
| GenoCanyon | 0.8058 | 7.2196 |
| integrated_fitCons | 0.5331 | 0.4954 |
| GERP | 0.7430 | 2.8285 |
| PrimateAI Human | 0.8411 | 23.4536 |
| PrimateAI Human + Primates | 0.9156 | 28.8346 |

FIG. 34

| | -log(pvalue) | AUC | Threshold | Classification Accuracy |
|---|---|---|---|---|
| SIFT | 5.6793 | 0.6903 | 0.001 | 0.7949 |
| PolyPhen-2 | 8.5522 | 0.7430 | 0.956 | 0.8197 |
| CADD | 6.4772 | 0.7087 | 5.040 | 0.7643 |
| M-CAP | 7.7143 | 0.7283 | 0.081 | 0.7348 |
| LRT | 6.0285 | 0.6970 | 1.00E-06 | 0.7604 |
| MutationTaster | 2.1366 | 0.6117 | 1.000 | 0.6564 |
| MutationAssessor | 6.6779 | 0.7115 | 2.240 | 0.7096 |
| FATHMM | 2.5979 | 0.6194 | -1.260 | 0.6401 |
| PROVEAN | 7.3447 | 0.7188 | -4.310 | 0.7833 |
| VEST3 | 7.0357 | 0.7173 | 0.679 | 0.7953 |
| MetaSVM | 6.2556 | 0.7041 | -0.3371 | 0.7406 |
| MetaLR | 5.8707 | 0.6971 | 0.349 | 0.7407 |
| REVEL | 8.4115 | 0.7392 | 0.456 | 0.8056 |
| MutPred | 5.3370 | 0.7036 | 0.526 | 0.6949 |
| DANN | 3.1588 | 0.6397 | 0.996 | 0.7046 |
| MKL_coding | 1.1649 | 0.5773 | 0.965 | 0.5840 |
| Eigen | 7.1659 | 0.7320 | 0.577 | 0.7844 |
| GenoCanyon | 4.6763 | 0.6740 | 0.999 | 0.6875 |
| integrated_fitCons | 0.8315 | 0.5554 | 0.706 | 0.5736 |
| GERP | 1.0330 | 0.5718 | 5.060 | 0.6330 |
| PrimateAI Human + Primates | 15.3064 | 0.8154 | 0.802 | 0.8772 |

**FIG. 35**

FIG. 36

FIG. 37

FIG. 38

FIG. 39

FIG. 40

**Cycle 20** — 5% Trinucleotide Context Matched

Synthetic SNP Set — Synthetic Variants (~68 Million)

Pseudo-Pathogenic SNP Set — 20th Random Sample — Pseudo-Pathogenic Variants (~160000)

Elite SNP Set — Pathogenic Variants — Elite Predictions from Cycles 1-19 (~380000)

Benign SNP Set — Benign Variants (~400000)

Unique Pseudo-Pathogenic Subsets (~20000)

Same Pathogenic Set (~380000)

Same Benign Set (~400000)

Ensemble Training (over multiple epochs and validation after each epoch)

Convolutional Network 1, Convolutional Network 2, Convolutional Network 3, Convolutional Network 4, Convolutional Network 5, Convolutional Network 6, Convolutional Network 7, Convolutional Network 8

Not including 20th Random Sample, Pathogenic Variants, Benign Variants (involved in training)

Prediction by Trained Ensemble

Convolutional Network 1, Convolutional Network 2, Convolutional Network 3, Convolutional Network 4, Convolutional Network 5, Convolutional Network 6, Convolutional Network 7, Convolutional Network 8

Predictions 1, Predictions 2, Predictions 3, Predictions 4, Predictions 5, Predictions 6, Predictions 7, Predictions 8

Filtered by Top Mean Prediction Scores (Culling)

Elite Predictions (Outliers) (~20000)

Added as Pathogenic Variants for next cycle's Ensemble Training

Training Set in Cycle 20
- Benign Variants (~400000)
- Pseudo-Pathogenic Variants (~160000)
- Pathogenic Variants (~380000)

EP 4 597 451 A2

FIG. 41

FIG. 42

**FIG. 43**

Human SNPs
(across 8 subpopulations)

Per-Species SNP Classifier

Identify Human Missense SNPs

Human Missense SNPs
(across 8 subpopulations)

Minor Allele Frequency (MAF) Determiner

Identify Human Missense SNPs with
MAF > 0.1% in human population
(Common Human Missense SNPs)

Benign Human
Missense SNPs
(~83546)

**Classify Common Human
Missense SNPs as Benign**

FIG. 44

|  | Human Codons | Non-Human Primate/Mammal/Avian Codons |
|---|---|---|
| | Matching Reference Codons | |
| Reference Codons | AUG | AUG |
| Alternative Codons with Missense SNPS | Matching Alternative Codons (and Missense SNPs) ACG | ACG |

**Human Orthologous Missense SNP**: A missense SNP in a non-human species that has matching reference and alternative codons with humans.

FIG. 45

Chimpanzee SNPs

↓

Human Orthologous SNP Detector

↓

Orthologous
Chimpanzee
SNPs

↓

Per-Species SNP Classifier

Orthologous
Chimpanzee
Synonymous
SNPs

Orthologous
Chimpanzee
Missense
Identical SNPs

↓

Per-SNP Category Frequency Determiner

Rare and Common SNPs

↓

Enrichment Score (ES) Determiner

↓

Enrichment Score (ES) Comparer

↓

ESs within
predetermined range?

YES

↓

Benign Chimpanzee
Missense SNPs
(~73858)

|  | Rare | Common |
|---|---|---|
| Synonymous SNPs | x | y |
| Missense Identical SNPs | m | n |

2 x 2 Contingency Table

$$\text{ES for Synonymous SNPs} = \frac{\text{Count of Rare Synonymous SNPs (x)}}{\text{Count of Common Synonymous SNPs (y)}}$$

$$\text{ES for Missense Identical SNPs} = \frac{\text{Count of Rare Missense Identical SNPs (m)}}{\text{Count of Common Missense Identical SNPs (n)}}$$

**Classify Chimpanzee Missense SNPs with
Matching Reference Codons with Humans as
Benign**

**FIG. 46**

|                         | Rare | Common |
|-------------------------|------|--------|
| Synonymous SNPs         | x    | y      |
| Missense Identical SNPs | m    | n      |

2 x 2 Contingency Table

$$\text{ES for Synonymous SNPs} = \frac{\text{Count of Rare Synonymous SNPs (x)}}{\text{Count of Common Synonymous SNPs (y)}}$$

$$\text{ES for Missense Identical SNPs} = \frac{\text{Count of Rare Missense Identical SNPs (m)}}{\text{Count of Common Missense Identical SNPs (n)}}$$

**FIG. 47**

Benign SNP Dataset (~400000)
Human Missense SNPs (~83546)
Chimpanzee Missense SNPs (~73858)
Rhesus Missense SNPs (~53279)
B. Orangutan Missense SNPs (~19793)
S. Orangutan Missense SNPs (~19834)
Gorilla Missense SNPs (~48310)
Bonobo Missense SNPs (~22968)
Marmoset Missense SNPs (~22767)

FIG. 48

FIG. 49A

**FIG. 49B**

**FIG. 49C**

**FIG. 49D**

**FIG. 49E**

EP 4 597 451 A2

FIGs. 50A, 50B

FIGs. 50C, 50D

**FIG. 51**

EP 4 597 451 A2

FIGs. 52A, 52B

FIGs. 52C, 52D

FIG. 53

FIG. 54

FIG. 55

Fraction of gnomAD common missense SNPs discovered by human cohorts of different sizes

FIG. 56

**FIG. 57**

| Species (and number of individuals) | Source | Number of missense variants matching human codons | Number of unique missense variants contributed |
|---|---|---|---|
| Human common variants (MAF > 0.1%, 123136 individuals) | gnomAD / ExAC database | 83,546 | 83,546 |
| Chimpanzee (24) | Prado-Martinez et al., Nature (2013) | 76,293 | 73,858 |
| Bonobo (13) | Prado-Martinez et al., Nature (2013) | 25,277 | 22,968 |
| Gorilla (27) | Prado-Martinez et al., Nature (2013) | 52,052 | 48,310 |
| Orangutan, Bornean subspecies (5) | Prado-Martinez et al., Nature (2013) | 21,621 | 19,793 |
| Orangutan, Sumatran subspecies (5) | Prado-Martinez et al., Nature (2013) | 27,567 | 19,834 |
| Chimpanzee (35) | de Manuel et al., Science (2016) | 75,580 | 30,327 |
| Orangutan (individual data not available) | dbSNP | 18,627 | 10,554 |
| Rhesus (16, individual data not available) | dbSNP | 62,393 | 53,279 |
| Marmoset (9, individual data not available) | dbSNP | 25,048 | 22,767 |
| Total | | | 385,236 |

**FIG. 58**

| Human population allele frequency in 123,136 exomes | Expected number of synonymous variants | Expected number of missense variants | Missense: synonymous ratio |
| --- | --- | --- | --- |
| singleton | 986,141.22 | 2,429,287.85 | 2.46 |
| singletons~0.01% | 1,077,630.37 | 2,416,751.61 | 2.24 |
| 0.01%~0.1% | 166,849.26 | 364,292.75 | 2.18 |
| >0.1% | 90,221.42 | 202,918.38 | 2.24 |

**FIG. 59**

EP 4 597 451 A2

| | Total number of ClinVar variants of interest |
|---|---|
| Original ClinVar file | 666,403 |
| Retaining only hg19 records | 324,698 |
| Extracting only single nucleotide variants | 264,623 |
| Removing non-coding variants | 186,769 |
| Ignoring all synonymous changes and missense changes creating or disrupting a stop codon | 122,884 |
| Excluding variants of unknown significance and variants with conflicting annotations | 42,438 |

**FIG. 60**

| Species | Number of variants with benign annotations in ClinVar | Number of variants with pathogenic annotations in ClinVar |
| --- | --- | --- |
| Chimpanzee | 218 | 21 |
| Bonobo | 85 | 7 |
| Gorilla | 167 | 23 |
| Orangutan | 160 | 12 |
| Rhesus | 87 | 11 |
| Marmoset | 25 | 7 |
| Mouse | 30 | 4 |
| Pig | 74 | 30 |
| Cow | 77 | 39 |
| Goat | 60 | 16 |
| Chicken | 9 | 8 |
| Zebrafish | 2 | 6 |

FIG. 61

EP 4 597 451 A2

**Table 1 | Additional genes achieving genome-wide significance in intellectual disability when considering only missense de novo mutations (DNMs) with PrimateAI scores ≥0.803**

| HGNC symbol | Protein-truncating variants | Missense | | P value | | Phenotypic abnormalities observed in multiple individuals |
|---|---|---|---|---|---|---|
| | | PrimateAI score ≥ 0.803 | All missense | PrimateAI score ≥ 0.803 | All missense | |
| ACTL6B | 0 | 3 | 3 | $1.5 \times 10^{-7}$ | $2.4 \times 10^{-6}$ | Microcephaly |
| EBF3 | 3 | 3 | 3 | $5.2 \times 10^{-8}$ | $5.4 \times 10^{-6}$ | Growth delay, eye abnormality, strabismus, ataxia |
| EFTUD2 | 2 | 4 | 4 | $1.5 \times 10^{-7}$ | $1.5 \times 10^{-5}$ | Microcephaly, low-set ears, microtia, choanal atresia |
| HECW2 | 1 | 8 | 8 | $2.8 \times 10^{-10}$ | $6.7 \times 10^{-7}$ | Seizures, myopathy, abnormal calvarium |
| KDM6A | 2 | 3 | 3 | $2.3 \times 10^{-7}$ | $9.8 \times 10^{-6}$ | Eyelid, dental abnormalities, hypotonia |
| KIF5C | 0 | 3 | 3 | $3.0 \times 10^{-7}$ | $2.8 \times 10^{-6}$ | Cerebral hypoplasia |
| MAP2K1 | 0 | 5 | 5 | $3.1 \times 10^{-8}$ | $2.7 \times 10^{-6}$ | Hypertelorism, low-set ears, polyhydramnois |
| PPP1CB | 0 | 6 | 6 | $1.5 \times 10^{-8}$ | $1.6 \times 10^{-6}$ | Abnormality of the forehead, short stature |
| PRKD1 | 0 | 6 | 6 | $8.6 \times 10^{-8}$ | $1.7 \times 10^{-5}$ | Skin, digital, and cardiac abnormalities; sparse hair |
| SOX11 | 1 | 3 | 3 | $3.1 \times 10^{-7}$ | $2.4 \times 10^{-6}$ | Hypermetropia, nail hypoplasia |
| TBR1 | 4 | 4 | 4 | $1.3 \times 10^{-10}$ | $4.2 \times 10^{-7}$ | Autistic behavior |
| TLK2 | 3 | 5 | 5 | $4.7 \times 10^{-8}$ | $6.3 \times 10^{-7}$ | Nose, eyelid abnormalities, slanted palpebral fissure |
| TRIP12 | 6 | 2 | 4 | $1.4 \times 10^{-7}$ | $5.4 \times 10^{-7}$ | Joint laxity |
| U2AF2 | 0 | 4 | 4 | $2.6 \times 10^{-7}$ | $1.2 \times 10^{-5}$ | Seizures; eye, palatal, philtrum abnormalities |

Counts of protein truncating and missense DNMs are provided. P values for gene enrichment are shown when the statistical test was run only with missense mutations with PrimateAI score ≥ 0.803, and when it was repeated for all missense mutations.

FIG. 62

EP 4 597 451 A2

**Table 2 | Comparison of the difference in Grantham score, protein surface-exposure, and amino acid sequence conservation between human expert annotated variants in ClinVar and de novo variants in DDD cases versus controls**

| | Grantham score | Protein surface-exposure | Sequence conservation |
|---|---|---|---|
| ClinVar pathogenic variants | 91.1 | 0.53 | 0.87 |
| ClinVar benign variants | 67.4 | 0.41 | 0.54 |
| Difference in human expert annotations | +23.7 | +0.12 | **+0.33** |
| De novo variants in DDD patients | 84.9 | 0.51 | 0.90 |
| De novo variants in healthy controls | 72.7 | 0.29 | 0.73 |
| Difference in affected versus unaffected individuals | +12.2 | +0.22 | +0.17 |

Mean scores are shown for missense mutations with non-conflicting annotations in the ClinVar database, and for de novo variants present in DDD cases versus controls within 605 disease-associated genes. Protein surface-exposure reflects the fraction of amino acids predicted as exposed residues by the solvent accessibility neural network, and sequence conservation shows the fraction of amino acids with sequence identity in the 100-vertebrate alignment. Numbers in bold highlight differences in the heuristics favored by human experts compared to empirical data.

## FIG. 63

# Per-Gene Enrichment Analysis

Observed Trinucleotide Mutation Rates    $10^{-8}$    $10^{-2}$    $10^{-1}$    $10^{5}$    $10^{1}$

**Candidate Variants**

*Classified as Pathogenic*

**De Novo Variants**

Particular Gene Sampled from
a Cohort of 1000 Individuals with a Genetic Disorder

$$\sum (observed\ trinucleotide\ mutation\ rates) = 10^{-5}$$

Transmission (Chromosome) Count

Baseline Number of Mutations = 2 x 1000 x $10^{-5}$ = 0.2

Cohort Size

De Novo Variant Count = 3

**FIG. 64**

EP 4 597 451 A2

# Genome-Wide Enrichment Analysis

Cohort of 2000 Healthy Individuals
Classified as Pathogenic

Over a <u>Plurality of Genes</u>

Cohort of 1000 Affected Individuals
Classified as Causing Aberrant Splicing

Mutation Rate in Healthy Cohort = 2/2000 = 0.001

Mutation Rate in Affected Cohort = 4/1000 = 0.004

Per-Individual Mutation Ratio = 0.004/0.001 = 4

**FIG. 65**

EP 4 597 451 A2

FIG. 66

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62573144, Hong Gao, Kai-How Farh, Laksshman Sundaram and Jeremy Francis McRae
- US 62573149, Kai-How Farh, Laksshman Sundaram, Samskruthi Reddy Padigepati and Jeremy Francis McRae
- US 62573153, Hong Gao, Kai-How Farh, Laksshman Sundaram and Jeremy Francis McRae
- US 62582898, Hong Gao, Kai-How Farh and Laksshman Sundaram
- US 20181 W, Laksshman Sundaram, Kai-How Farh, Hong Gao, Samskruthi Reddy Padigepati and Jeremy Francis McRae
- WO 07010252 A
- GB 20071003798 W
- US 20090088327 A
- US 20160085910
- US 20130296175
- WO 04018497 A
- US 7057026 B
- WO 9106678 A
- WO 07123744 A

- US 7329492 B
- US 7211414 B
- US 7315019 B
- US 7405281 B
- US 20080108082
- US 5641658 A
- US 20020055100
- US 7115400 B
- US 20040096853
- US 20040002090
- US 20070128624
- US 20080009420
- US 20070099208 A1
- US 20070166705 A1
- US 20080280773 A1
- US 018255
- WO 004018497 A
- US 20074016675 A1
- US 2013030867 W
- WO 2014142831 A

**Non-patent literature cited in the description**

- **A. V. D. OORD** ; **S. DIELEMAN** ; **H. ZEN** ; **K. SIMONYAN** ; **0. VINYALS** ; **A. GRAVES** ; **N. KALCHBRENNER** ; **A. SENIOR** ; **K. KAVUKCUOGLU**. WAVENET: A GENERATIVE MODEL FOR RAW AUDIO. *arXiv:1609.03499*, 2016
- **S. 0. ARIK** ; **M. CHRZANOWSKI** ; **A. COATES** ; **G. DIAMOS** ; **A. GIBIANSKY** ; **Y. KANG.** ; **X. LI** ; **J. MILLER** ; **A. NG** ; **J. RAIMAN**. DEEP VOICE: REAL-TIME NEURAL TEXT-TO-SPEECH. *arXiv:1702.07825*, 2017
- **F. YU** ; **V. KOLTUN**. MULTI-SCALE CONTEXT AGGREGATION BY DILATED CONVOLUTIONS. *arXiv:1511.07122*, 2016
- **K. HE** ; **X. ZHANG** ; **S. REN** ; **J. SUN**. DEEP RESIDUAL LEARNING FOR IMAGE RECOGNITION. *arXiv:1512.03385*, 2015
- **R.K. SRIVASTAVA** ; **K. GREFF** ; **J. SCHMIDHUBER**. HIGHWAY NETWORKS. *arXiv: 1505.00387*, 2015
- **G. HUANG** ; **Z. LIU** ; **L. VAN DER MAATEN** ; **K. Q. WEINBERGER**. DENSELY CONNECTED CONVOLUTIONAL NETWORKS. *arXiv:1608.06993*, 2017

- **C. SZEGEDY** ; **W. LIU** ; **Y. JIN.** ; **P. SERMANET** ; **S. REED** ; **D. ANGUELOV** ; **D. ERHAN** ; **V. VANHOUCKE** ; **A. RABINOVICH**. GOING DEEPER WITH CONVOLUTIONS. *arXiv: 1409.4842*, 2014
- **S. IOFFE** ; **C. SZEGEDY**. BATCH NORMALIZATION: ACCELERATING DEEP NETWORK TRAINING BY REDUCING INTERNAL COVARIATE SHIFT. *arXiv: 1502.03167*, 2015
- **J. M. WOLTERINK** ; **T. LEINER** ; **M. A. VIERGEVER** ; **I. ISGUM**. DILATED CONVOLUTIONAL NEURAL NETWORKS FOR CARDIOVASCULAR MR SEGMENTATION IN CONGENITAL HEART DISEASE. *arXiv:1704.03669*, 2017
- **L. C. PIQUERAS**. AUTOREGRESSIVE MODEL BASED ON A DEEP CONVOLUTIONAL NEURAL NETWORK FOR AUDIO GENERATION. *Tampere University of Technology*, 2016
- **J. WU**. Introduction to Convolutional Neural Networks. *Nanjing University*, 2017
- CONVOLUTIONAL NETWORKS. **I.J. GOODFELLOW** ; **D. WARDE-FARLEY** ; **M. MIRZA** ; **A. COURVILLE** ; **Y. BENGIO**. Deep Learning. MIT Press, 2016

- **J. GU** ; **Z. WANG** ; **J. KUEN** ; **L. MA** ; **A. SHAHROUDY** ; **B. SHUAI** ; **T. LIU** ; **X. WANG** ; **G. WANG**. RECENT ADVANCES IN CONVOLUTIONAL NEURAL NETWORKS. *arXiv:1512,07108*, 2017
- **T. CHING et al.** *Opportunities And Obstacles for Deep Learning In Biology And Medicine*, 2017, www. biorxiv.org:142760
- **ANGERMUELLER C** ; **PÄRNAMAA T** ; **PARTS L** ; **STEGLE O.** Deep Learning For Computational Biology.. *Mol Syst Biol.*, 2016, vol. 12, 878
- **PARK Y** ; **KELLIS M.** Deep Learning For Regulatory Genomics. *Nat. Biotechnol.*, 2015, vol. 33, 825-826
- **MIN, S** ; **LEE, B.** ; **YOON, S.** Deep Learning In Bioinformatics.. *Brief. Bioinfonn. bbw068*, 2016
- **LEUNG MK** ; **DELONG A.** ; **ALIPANAHI B et al.** *Machine Learning In Genomic Medicine: A Review of Computational Problems and Data Sets*, 2016
- **LIBBRECHT MW** ; **NOBLE WS.** Machine Learning Applications In Genetics and Genomics. *Nature Reviews Genetics*, 2015, vol. 16 (6), 321-32
- **A. V. D. OORD** ; **S. DIELEMAN** ; **H. ZEN** ; **K. SIMONYAN** ; **O. VINYALS** ; **A. GRAVES** ; **N. KALCHBRENNER** ; **A. SENIOR** ; **K. KAVUKEUO-GLU**. WAVENET: A GENERATIVE MODEL FOR RAW AUDIO. *arXiv:1609.03499*, 2016
- **BENTLEY et al.** *Nature*, 2008, vol. 456, 53-39
- **LIZARDI et al.** *Nat. Genet.*, 1998, vol. 19, 225-232
- **DUNN, TAMSEN** ; **BERRY, GWENN** ; **EMIG-AGIUS. DOROTHEA** ; **JIANG, YU** ; **IYER, ANITA** ; **UDAR, NITIN** ; **STRÖMBERG, MICHAEL.** *Pisces: An Accurate and Versatile Single Sample Somatic and Germline Variant Caller*, 2017, 595-595
- **T SAUNDERS. CHRISTOPHER** ; **WONG, WENDY** ; **SWAMY, SAJANI** ; **BECQ, JENNIFER** ; **J MURRAY, LISA** ; **CHEETHAM, KEIRA**. Strelka: Accurate somatic small-variant calling from sequenced tumor-normal sample pairs.. *Bioinformatics*, 2012, vol. 28, 1811-7
- **KIM, S** ; **SCHEFFLER. K** ; **HALPERN, A.L** ; **BEKRITSKY, M.A.** ; **NOH, E.** ; **KÄLLBERG, M.** ; **CHEN, X** ; **BEYTER, D** ; **KRUSCHE, P** ; **SAUNDERS, C.T.** *Strelka2: Fast and accurate variant calling for clinical sequencing applications*, 2017
- **STROMBERG, MICHAEL** ; **ROY, RAJAT** ; **LAJU-GIE, JULIEN** ; **JIANG, YU** ; **LI, HAOCHEN** ; **MARGULIES, ELLIOTT.** *Nirvana: Clinical Grade Variant Annotator.*, 2017, 596-596
- **LAKSSHMAN SUNDARAM** ; **HONG GAO** ; **SAM-SKRUTHI REDDY PADIGEPATI** ; **JEREMY F. MCRAE** ; **YANJUN LI** ; **JACK A. KOSMICKI** ; **NONDAS FRITZILAS** ; **JÖRG HAKENBERG** ; **ANINDITA DUTTA** ; **JOHN SHON**. Predicting the clinical impact of human mutation with deep neural networks. *Nature Genetics*, 2018, vol. 50, 1161-1170, https://www.nature.com/articles/s41588-018-0167-z
- **MACARTHUR, D. G. et al.** Guidelines for investigating causality of sequence variants in human disease.. *Nature*, 2014, vol. 508, 469-476
- **REHM, H. L** ; **J. S. BERG** ; **L. D. BROOKS** ; **C. D. BUSTAMANTE** ; **J. P. EVANS** ; **M. J. LANDRUM** ; **D. H. LEDBETTER** ; **D. R. MAGLOTT** ; **C. L. MARTIN** ; **R. L. NUSSBAUM.** ClinGenthe Clinical Genome Resource.. *N. Engl. J. Med.*, 2015, vol. 372, 2235-2242
- **BAMSHAD, M. J** ; **S. B. NG** ; **A. W. BIGHAM** ; **H. K. TABOR** ; **M. J. EMOND** ; **D. A. NICKERSON** ; **J. SHENDURE.** Exome sequencing as a tool for Mendelian disease gene discovery.. *Nat. Rev. Genet.*, 2011, vol. 12, 745-755
- **REHM, H. L.** Evolving health care through personal genomics. *Nature Reviews Genetics*, 2017, vol. 18, 259-267
- **RICHARDS, S. et al.** Standards and guidelines for the interpretation of sequence variants: a joint consensus recommendation of the American College of Medical Genetics and Genomics and the Association for Molecular Pathology.. *Genet Med*, 2015, vol. 17, 405-424
- **LEK, M. et al.** Analysis of protein-coding genetic variation in 60,706 humans.. *Nature*, 2016, vol. 536, 285-291
- **MALLICK, S. et al.** The Simons Genome Diversity Project: 300 genomes from 142 diverse populations.. *Nature*, 2016, vol. 538, 201-206
- **GENOMES PROJECT CONSORTIUM et al.** A global reference for human genetic variation.. *Nature*, 2015, vol. 526, 68-74
- **LIU, X.** ; **X. JIAN** ; **E. BOERWINKLE.** dbNSFP: A lightweight database of human nonsynonymous SNPs and their functional predictions.. *Human Mutation*, 2011, vol. 32, 894-899
- Chimpanzee Sequencing Analysis Consortium. Initial sequence of the chimpanzee genome and comparison with the human genome.. *Nature*, 2005, vol. 437, 69-87
- **TAKAHATA, N.** Allelic genealogy and human evolution.. *Mol Biol Evol*, 1993, vol. 10, 2-22
- **ASTHANA, S.** ; **SCHMIDT, S.** ; **SUNYAEV, S.** A limited role for balancing selection. *Trends Genet*, 2005, vol. 21, 30-32

- **LEFFLER, E. M** ; **Z. GAO** ; **S. PFEIFER** ; **L. SÉGUREL** ; **A. AUTON** ; **O. VENN** ; **R. BOWDEN** ; **R. BONTROP** ; **J.D. WALL** ; **G. SELLA**. Multiple instances of ancient balancing selection shared between humans and chimpanzees. *Science*, 2013, vol. 339, 1578-1582
- **SAMOCHA, K. E. et al.** A framework for the interpretation of de novo mutation in human disease.. *Nat Genet*, 2014, vol. 46, 944-950
- **OHTA, T.** Slightly deleterious mutant substitutions in evolution.. *Nature*, 1973, vol. 246, 96-98
- **REICH, D. E.** ; **LANDER, E. S.** On the allelic spectrum of human disease.. *Trends Genet*, 2001, vol. 17, 502-510
- **WHIFFIN, N.** ; **E. MINIKEL** ; **R. WALSH** ; **A. H. O'DONNELL-LURIA** ; **K. KARCZEWSKI** ; **A. Y. ING** ; **P. J. BARTON** ; **B. FUNKE** ; **S. A. COOK** ; **D. MACARTHUR**. Using high-resolution variant frequencies to empower clinical genome interpretation.. *Genetics in Medicine*, 2017, vol. 19, 1151-1158
- **PRADO-MARTINEZ, J. et al.** Great ape genome diversity and population history.. *Nature*, 2013, vol. 499, 471-475
- **KLEIN, J.** ; **SATTA, Y** ; **O'HUIGIN, C.** ; **TAKAHATA, N.** The molecular descent of the major histocompatibility complex.. *Annu Rev Immunol*, 1993, vol. 11, 269-295
- **KIMURA, M.** The neutral theory of molecular evolution.. Cambridge University Press, 1983
- **DE MANUEL, M. et al.** Chimpanzee genomic diversity reveals ancient admixture with bonobos.. *Science*, 2016, vol. 354, 477-481
- **LOCKE, D. P. et al.** Comparative and demographic analysis of orang-utan genomes.. *Nature*, 2011, vol. 469, 529-533
- **RHESUS MACAQUE et al.** Evolutionary and biomedical insights from the rhesus macaque genome. *Science*, 2007, vol. 316, 222-234
- **WORLEY, K. C.** ; **W. C. WARREN** ; **J. ROGERS** ; **D. LOCKE** ; **D. M. MUZNY** ; **E. R. MARDIS** ; **G. M. WEINSTOCK** ; **S. D. TARDIF** ; **K. M. AAGAARD** ; **N. ARCHIDIACONO**. The common marmoset genome provides insight into primate biology and evolution. *Nature Genetics*, 2014, vol. 46, 850-857
- **SHERRY, S. T. et al.** dbSNP: the NCBI database of genetic variation.. *Nucleic Acids Res*, 2001, vol. 29, 308-311
- **SCHRAGO, C. G.** ; **RUSSO, C. A.** Timing the origin of New World monkeys.. *Mol Biol Evol*, 2003, vol. 20, 1620-1625
- **LANDRUM, M. J. et al.** ClinVar: public archive of interpretations of clinically relevant variants.. *Nucleic Acids Res*, 2016, vol. 44, D862-868
- **BRANDON, E. P** ; **IDZERDA, R. L.** ; **MCKNIGHT, G. S.** Targeting the mouse genome: a compendium of knockouts (Part II).. *Curr Biol*, 1995, vol. 5, 758-765

- **LIESCHKE, J. G** ; **P. D. CURRIE**. Animal models of human disease: zebrafish swim into view.. *Nature Reviews Genetics*, 2007, vol. 8, 353-367
- **SITTIG, L. J.** ; **P. CARBONETTO** ; **K. A. ENGEL** ; **K. S. KRAUSS** ; **C. M. BARRIOS-CAMACHO** ; **A. A. PALMER**. Genetic background limits generalizability of genotype-phenotype relationships.. *Neuron*, 2016, vol. 91, 1253-1259
- **BAZYKIN, G. A. et al.** Extensive parallelism in protein evolution.. *Biol Direct*, 2007, vol. 2, 20
- **NG, P. C.** ; **HENIKOFF, S.** Predicting deleterious amino acid substitutions.. *Genome Res*, 2001, vol. 11, 863-874
- **ADZHUBEI, I. A. et al.** A method and server for predicting damaging missense mutations.. *Nat Methods*, 2010, vol. 7, 248-249
- **CHUN, S.** ; **J. C. FAY.** Identification of deleterious mutations within three human genomes.. *Genome research*, 2009, vol. 19, 1553-1561
- **SCHWARZ, J. M** ; **C. RÖDELSPERGER** ; **M. SCHUELKE** ; **D. SEELOW**. MutationTaster evaluates disease-causing potential of sequence alterations.. *Nat. Methods*, 2010, vol. 7, 575-576
- **REVA, B.** ; **ANTIPIN, Y.** ; **SANDER, C.** Predicting the functional impact of protein mutations: application to cancer genomics.. *Nucleic Acids Res*, 2011, vol. 39, e118
- **DONG, C. et al.** Comparison and integration of deleteriousness prediction methods for nonsynonymous SNVs in whole exome sequencing studies.. *Hum Mol Genet*, 2015, vol. 24, 2125-2137
- **CARTER, H.** ; **DOUVILLE, C.** ; **STENSON, P. D** ; **COOPER, D. N.** ; **KARCHIN, R.** Identifying Mendelian disease genes with the variant effect scoring tool.. *BMC Genomics*, 2013, vol. 14 (3), S3
- **CHOI, Y.** ; **SIMS, G. E** ; **MURPHY, S.** ; **MILLER, J. R.** ; **CHAN, A. P.** Predicting the functional effect of amino acid substitutions and indels.. *PLoS One*, 2012, vol. 7, e46688
- **GULKO, B.** ; **HUBISZ, M. J.** ; **GRONAU, I.** ; **SIEPEL, A.** A method for calculating probabilities of fitness consequences for point mutations across the human genome. *Nat Genet*, 2015, vol. 47, 276-283
- **SHIHAB, H. A. et al.** An integrative approach to predicting the functional effects of non-coding and coding sequence variation.. *Bioinformatics*, 2015, vol. 31, 1536-1543
- **QUANG, D** ; **CHEN, Y.** ; **XIE, X.** DANN: a deep learning approach for annotating the pathogenicity of genetic variants.. *Bioinformatics*, 2015, vol. 31, 761-763
- **BELL, C. J.** ; **D. L. DINWIDDIE** ; **N. A. MILLER** ; **S. L. HATELEY** ; **E. E. GANUSOVA** ; **J. MIDGE** ; **R. J. LANGLEY** ; **L. ZHANG** ; **C. L. LEE** ; **R. D. SCHILKEY**. Comprehensive carrier testing for severe childhood recessive diseases by next generation sequencing.. *Sci. Transl. Med.*, 2011, vol. 3, 65-64

- **KIRCHER, M** ; **D. M. WITTEN** ; **P. JAIN** ; **B. J. O'ROAK** ; **G. M. COOPER** ; **J. SHENDURE.** A general framework for estimating the relative pathogenicity of human genetic variants. *Nat. Genet.*, 2014, vol. 46, 310-315
- **SMEDLEY, D. et al.** A Whole-Genome Analysis Framework for Effective Identification of Pathogenic Regulatory Variants in Mendelian Disease.. *Am J Hum Genet*, 2016, vol. 99, 595-606
- **IOANNIDIS, N. M. et al.** REVEL: an ensemble method for predicting the pathogenicity of rare missense variants.. *Am J Hum Genet*, 2016, vol. 99, 877-885
- **JAGADEESH, K. A** ; **A. M. WENGER** ; **M. J. BERGER** ; **H. GUTURU** ; **P. D. STENSON** ; **D. N. COOPER** ; **J. A. BERNSTEIN** ; **G. BEJERANO**. M-CAP eliminates a majority of variants of uncertain significance in clinical exomes at high sensitivity.. *Nature genetics*, 2016, vol. 48, 1581-1586
- **GRIMM, D. G.** The evaluation of tools used to predict the impact of missense variants is hindered by two types of circularity.. *Human mutation*, 2015, vol. 36, 513-523
- **HE, K** ; **X. ZHANG** ; **S. REN** ; **J. SUN**. *Proceedings of the IEEE conference on computer vision and pattern recognition.*, 770-778
- **HEFFERNAN, R. et al.** Improving prediction of secondary structure, local backbone angles, and solvent accessible surface area of proteins by iterative deep learning.. *Sci Rep*, 2015, vol. 5, 11476
- **WANG, S** ; **J. PENG** ; **J. MA** ; **J. XU.** Protein secondary structure prediction using deep convolutional neural fields.. *Scientific reports*, 2016, vol. 6, 18962-18962
- **HARPAK, A** ; **A. BHASKAR** ; **J. K. PRITCHARD.** Mutation Rate Variation is a Primary Determinant of the Distribution of Allele Frequencies in Humans.. *PLoS Genetics*, 2016, vol. 12
- **PAYANDEH, J.** ; **SCHEUER, T** ; **ZHENG, N** ; **CATTERALL, W. A.** The crystal structure of a voltage-gated sodium channel.. *Nature*, 2011, vol. 475, 353-358
- **SHEN, H. et al.** Structure of a eukaryotic voltage-gated sodium channel at near-atomic resolution.. *Science*, 2017, vol. 355, eaa14326
- **NAKAMURA, K. et al.** Clinical spectrum of SCN2A mutations expanding to Ohtabara syndrome.. *Neurology*, 2013, vol. 81, 992-998
- **HENIKOFF, S.** ; **HENIKOFF, J. G.** Amino acid substitution matrices from protein blocks.. *Proc Natl Acad Sci U SA*, 1992, vol. 89, 10915-10919
- **LI, W. H** ; **C. I. WU** ; **C. C. LUO.** Nonrandomness of point mutation as reflected in nucleotide substitutions in pseudogenes and its evolutionary implications. *Journal of Molecular Evolution*, 1984, vol. 21, 58-71
- **GRANTHAM, R.** Amino acid difference formula to help explain protein evolution.. *Science*, 1974, vol. 185, 862-864
- **LECUN, Y** ; **L. BOTTOU** ; **Y. BENGIO** ; **P. HAFFNER**. *Proceedings of the IEEE*, 2278-2324
- **VISSERS, L. E.** ; **GILISSEN, C.** ; **VELTMAN, J. A.** Genetic studies in intellectual disability and related disorders.. *Nat Rev Genet*, 2016, vol. 17, 9-18
- **NEALE, B. M. et al.** Patterns and rates of exonic de novo mutations in autism spectrum disorders.. *Nature*, 2012, vol. 485, 242-245
- **SANDERS, S. J. et al.** De novo mutations revealed by whole-exome sequencing are strongly associated with autism.. *Nature*, 2012, vol. 485, 237-241
- **DE RUBEIS, S. et al.** Synaptic, transcriptional and chromatin genes disrupted in autism.. *Nature*, 2014, vol. 515, 209-215
- Deciphering Developmental Disorders Study. Large-scale discovery of novel genetic causes of developmental disorders.. *Nature*, 2015, vol. 519, 223-228
- Deciphering Developmental Disorders Study. Prevalence and architecture of de novo mutations in developmental disorders.. *Nature*, 2017, vol. 542, 433-438
- **IOSSIFOV, I. et al.** The contribution of de novo coding mutations to autism spectrum disorder.. *Nature*, 2014, vol. 515, 216-221
- **ZHU, X** ; **NEED, A. C** ; **PETROVSKI, S.** ; **GOLDSTEIN, D. B.** One gene, many neuropsychiatric disorders: lessons from Mendelian diseases.. *Nat Neurosci*, 2014, vol. 17, 773-781
- **LEFFLER, E. M** ; **K. BULLAUGHEY** ; **D. R. MATUTE** ; **W. K. MEYER** ; **L. SÉGUREL** ; **A. VENKAT** ; **P. ANDOLFATTO** ; **M. PRZEWORSKI.** Revisiting an old riddle: what determines genetic diversity levels within species. *PLoS biology*, 2012, vol. 10, e1001388
- **ESTRADA, A. et al.** Impending extinction crisis of the world's primates: Why primates matter.. *Science advances*, 2017, vol. 3, e1600946
- **KENT, W. J.** ; **C. W. SUGNET** ; **T. S. FUREY** ; **K. M. ROSKIN** ; **T. H. PRINGLE** ; **A.M. ZAHLER** ; **D. HAUSSLER.** The human genome browser at UCSC.. *Genome Res.*, 2002, vol. 12, 996-1006
- **TYNER, C. et al.** The UCSC Genome Browser database: 2017 update.. *Nucleic Acids Res*, 2017, vol. 45, D626-D634
- **KABSCH, W.** ; **SANDER, C.** Dictionary of protein secondary structure: pattern recognition of hydrogen-bonded and geometrical features.. *Biopolymers*, 1983, vol. 22, 2577-2637
- **JOOSTEN, R. P. et al.** A series of PDB related databases for everyday needs.. *Nucleic Acids Res*, 2011, vol. 39, D411-419
- **HE, K** ; **ZHANG, X.** ; **REN, S.** ; **SUN, J.** European Conference on Computer Vision.. Springer, 630-645

- **IONITA-LAZA, I ; MCCALLUM, K ; XU, B. ; BUXBAUM, J. D.** A spectral approach integrating functional genomic annotations for coding and noncoding variants.. *Nat Genet*, 2016, vol. 48, 214-220
- **LI, B. et al.** Automated inference of molecular mechanisms of disease from amino acid substitutions. *Bioinformatics*, 2009, vol. 25, 2744-2750
- **LU, Q. et al.** A statistical framework to predict functional non-coding regions in the human genome through integrated analysis of annotation data.. *Sci Rep*, 2015, vol. 5, 10576
- **SHIHAB, H. A. et al.** Predicting the functional, molecular, and phenotypic consequences of amino acid substitutions using hidden Markov models.. *Hum Mutat*, 2013, vol. 34, 57-65
- **DAVYDOV, E. V. et al.** Identifying a high fraction of the human genome to be under selective constraint using GERP++.. *PLoS Comput Biol*, 2010, vol. 6, e1001025
- **LIU, X ; WU, C. ; LI, C ; BOERWINKLE, E.** dbNSFP v3.0: A One-Stop Database of Functional Predictions and Annotations for Human Nonsynonymous and Splice-Site SNVs.. *Hum Mutat*, 2016, vol. 37, 235-241
- **JAIN, S ; WHITE, M. ; RADIVOJAC, P.** *Proceedings of the Thirty-First AAAI Conference on Artificial Intelligence.*, 2066-2072
- **DE LIGT, J. et al.** Diagnostic exome sequencing in persons with severe intellectual disability. *N Engl J Med*, 2012, vol. 367, 1921-1929
- **IOSSIFOV, I. et al.** De novo gene disruptions in children on the autistic spectrum.. *Neuron*, 2012, vol. 74, 285-299
- **O'ROAK, B. J. et al.** Sporadic autism exomes reveal a highly interconnected protein network of de novo mutations.. *Nature*, 2012, vol. 485, 246-250
- **RAUCH, A. et al.** Range of genetic mutations associated with severe non-syndromic sporadic intellectual disability: an exome sequencing study.. *Lancet*, 2012, vol. 380, 1674-1682
- **EPI, K. C. et al.** De novo mutations in epileptic encephalopathies.. *Nature*, 2013, vol. 501, 217-221
- **EURO, E.-R. E. S. C.** Epilepsy Phenome/Genome, P. & Epi, K. C. De novo mutations in synaptic transmission genes including DNM1 cause epileptic encephalopathies.. *Am J Hum Genet*, 2014, vol. 95, 360-370
- **GILISSEN, C. et al.** Genome sequencing identifies major causes of severe intellectual disability.. *Nature*, 2014, vol. 511, 344-347
- **LELIEVELD, S. H. et al.** Meta-analysis of 2,104 trios provides support for 10 new genes for intellectual disability.. *Nat Neurosci*, 2016, vol. 19, 1194-1196
- **FAMIGLIETTI, M. L. et al.** Genetic variations and diseases in UniProtKB/Swiss-Prot: the ins and outs of expert manual curation.. *Hum Mutat*, 2014, vol. 35, 927-935
- **HORAITIS, O. ; TALBOT, C. C., JR ; PHOMMARINH, M ; PHILLIPS, K. M. ; COTTON, R. G.** A database of locus-specific databases. *Nat Genet*, 2007, vol. 39, 425
- **STENSON, P. D. et al.** The Human Gene Mutation Database: building a comprehensive mutation repository for clinical and molecular genetics, diagnostic testing and personalized genomic medicine.. *Hum Genet*, 2014, vol. 133, 1-9